# EUROPEAN PATENT SPECIFICATION

(11) **EP 3 401 314 B1**
(45) Date of publication and mention of the grant of the patent: **08.11.2023**
(21) Application number: 17206812.4
(22) Date of filing: 14.03.2014
(51) Int. Cl.: C07D 401/06, C07D 417/06, A61K 31/496, A61P 35/00

(54) **COVALENT INHIBITORS OF KRAS G12C**
KOVALENTE INHIBITOREN VON KRAS-G12C
INHIBITEURS COVALENTS DE KRAS G12C

(30) Priority: 15.03.2013 US 201361852123 P; 10.10.2013 US 201361889480 P; 13.03.2014 JO 201498
(43) Date of publication of application: 14.11.2018
(62) Divisional of application: 14720375.6
(73) Proprietor: Araxes Pharma LLC, San Diego, CA 92121 (US)
(72) Inventor: REN, Pingda, San Diego, CA California 92130 (US); LIU, Yi, San Diego, CA California 92130 (US); LI, Liansheng, San Diego, CA California 92130 (US); FENG, Jun, San Diego, CA California 92130 (US); WU, Tao, Carlsbad, CA California 92009 (US)
(74) Representative: Grünecker Patent- und Rechtsanwälte PartG mbB

(56) References cited:
- EP-A1- 1 348 434
- EP-A1- 2 133 334
- EP-B1- 0 655 442
- WO-A1-02/04420
- WO-A1-97/30992
- WO-A1-02/080928
- WO-A1-2004/033427
- WO-A1-2006/097261
- WO-A2-98/35951
- WO-A2-2007/047146
- US-A- 4 656 181
- US-A1- 2004 106 634
- US-A1- 2005 119 266
- SUNDBERG R J ET AL: "THE O-STYRYLNITRENE ROUTE TO 2-SUBSTITUTED INDOLES. PYROLYSIS OF O-AZIDOSTYRENES", JOURNAL OF ORGANIC CHEMISTRY, WILLIAMS & WILKINS, US, vol. 37, no. 5, 1 January 1972 (1972-01-01), pages 719-724, XP002474920, ISSN: 0022-3263, DOI: 10.1021/JO00970A010

## Description

### BACKGROUND

### Technical Field

Ras represents a group of closely related monomeric globular protein of 189 amino acids (21 kDa molecular mass) which is associated with the plasma membrane and which binds either GDP or GTP. Ras acts as a molecular switch. When Ras contains bound GDP it is in the resting or off position and is "inactive". In response to exposure of the cell to certain growth promoting stimuli, Ras is induced to exchange its bound GDP for a GTP. With GTP bound, Ras is "switched on" and is able to interact with and activate other proteins (its "downstream targets"). The Ras protein itself has a very low intrinsic ability to hydrolyze GTP back to GDP, thus turning itself into the off state. Switching Ras off requires extrinsic proteins termed GTPase-activating proteins (GAPs) that interact with Ras and greatly accelerate the conversion of GTP to GDP. Any mutation in Ras which affects its ability to interact with GAP or to convert GTP back to GDP will result in a prolonged activation of the protein and consequently a prolonged signal to the cell telling it to continue to grow and divide. Because these signals result in cell growth and division, overactive Ras signaling may ultimately lead to cancer.

Structurally, Ras proteins contain a G domain which is responsible for the enzymatic activity of Ras - the guanine nucleotide binding and the hydrolysis (GTPase reaction). It also contains a C-terminal extension, known as the CAAX box, which may be post-translationally modified and is responsible for targeting the protein to the membrane. The G domain is approximately 21-25 kDa in size and it contains a phosphate binding loop (P-loop). The P-loop represents the pocket where the nucleotides are bound in the protein, and this is the rigid part of the domain with conserved amino acid residues which are essential for nucleotide binding and hydrolysis (Glycine 12, Threonine 26 and Lysine 16). The G domain also contains the so called Switch I (residues 30-40) and Switch II (residues 60-76) regions, both of which are the dynamic parts of the protein which are often represented as the "springloaded" mechanism because of their ability to switch between the resting and loaded state. The key interaction is the hydrogen bonds formed by Threonine-35 and glycine-60 with the γ-phosphate of GTP which maintain Switch 1 and Switch 2 regions respectively in their active conformation. After hydrolysis of GTP and release of phosphate, these two relax into the inactive GDP conformation.

The most notable members of the Ras subfamily are HRAS, KRAS and NRAS, mainly for being implicated in many types of cancer. However, there are many other members including DIRAS1; DIRAS2; DIRAS3; ERAS; GEM; MRAS; NKIRAS1; NKIRAS2; NRAS; RALA; RALB; RAP1A; RAP1B; RAP2A; RAP2B; RAP2C; RASD1; RASD2; RASL10A; RASL10B; RASL11A; RASL11B; RASL12; REM1; REM2; RERG; RERGL; RRAD; RRAS; RRAS2.

Mutations in any one of the three main isoforms of RAS (H-Ras, N-Ras, or K-Ras) genes are among the most common events in human tumorigenesis. About 30% of all human tumors are found to carry some mutation in Ras genes. Remarkably, K-Ras mutations are detected in 25-30% of tumors. By comparison, the rates of oncogenic mutation occurring in the N-Ras and H-Ras family members are much lower (8% and 3% respectively). The most common K-Ras mutations are found at residue G12 and G13 in the P-loop and at residue Q61.

G12C is a frequent mutation of K-Ras gene (glycine-12 to cysteine). This mutation had been found in about 13% of cancer occurrences, about 43% of lung cancer occurrences, and in almost 100% of MYH-associates polyposis (familial colon cancer syndrome). However targeting this gene with small molecules is a challenge. Accordingly, there is a need in the art for small molecules for targeting Ras (e.g., K-Ras, H-Ras and/or N-Ras) and use of the same for treatment of various diseases, such as cancer. The present invention provides these and other related advantages.
EP 0655442 relates to new piperazine derivatives and a pharmaceutically acceptable salt thereof.
US 4,656,181 relates to new 1,4-dihydropyridines, to processes for their preparation and to medicaments containing the same, such medicaments being preferably used for their action on the blood vessels, and in particular as agents against coronary diseases or as anti-atheromatic agents.
US 2004/106634 relates to anilinopyrimidines and derivatives thereof which have utility over a wide range of indications, including activity as Jun N-terminal kinase inhibitors.
WO 98/35951 discloses aminopiperazine derivatives and pharmaceutically acceptable salts thereof which are useful as a medicament.
WO 2006/097261 describes chiral cis-imidazolines which are small molecule inhibitors of the MDM2-p53 interaction.
EP1348434 discloses pyridyl amides, which have advantageous pharmacological properties and inhibit the VEGF production and/or significantly reduce the vessel density.
US 2005/119266 relates to pyrrolidine and piperidine derivatives that are inhibitors of trypsin-like serine protease enzymes, especially factor Xa, pharmaceutical compositions containing the same, and methods of using the same as anticoagulant agents for treatment of thromboembolic disorders.
WO 2004/033424 discloses compounds, or pharmaceutically acceptable salts thereof having human 11-p-hydroxysteroid dehydrogenase type 1 enzyme (1 (3HSD1) inhibitory activity and accordingly have value in the treatment of disease states including metabolic syndrome and are useful in methods of treatment of a warm-blooded animal, such as man. The invention also relates to processes for the manufacture of said compounds, to pharmaceutical compositions containing them and to their use in the manufacture of medicaments to inhibit 11 (3HSDlin a warm-blooded animal, such as man.
WO 02/04420 describes piperidine compounds fro use as CCR-3 inhibitor.
WO 02/080928 relates to N-substituted nonarylheterocyclo amidyl compounds that are effective as NMDA NR2B antagonists useful for relieving pain.
EP 2133334 describes inhibitors of histone deacetylases (HDACs), to a process for their preparation, pharmaceutical compositions comprising them, and to their use as therapeutic agents, in particular for the treatment of cancer.
WO 2007/047146 relates to compounds, processes for their syntheses, compositions and methods for the treatment of hepatitis C virus (HCV) infection.
WO 97/30992 discloses compounds that inhibit farnesyl-protein transferase and ras protein farnesylation, thereby making them useful as anti-cancer agents.
Sunderberg et al., Journal of Organic Chemistry 1972, vol. 37, no. 5, pages 719 - 724, describe the syntheis of 2-substituted indoles.

### BRIEF SUMMARY

The present invention provides compounds which are capable of modulating G12C mutant K-Ras, H-Ras and/or N-Ras proteins. In some instances, the compound acts as an electrophile capable of forming a covalent bond with the cysteine residue at position 12 of a K-Ras, H-Ras or N-Ras G12C mutant protein.

In some aspects of the invention, the compounds described herein are included in pharmaceutical compositions. In some embodiments, the pharmaceutical composition comprises a pharmaceutically acceptable carrier.

The present invention provides a compound having one of the following structures (VIa'), (VIb'), (VIc'), (VId'), (VIe'), (VIf') or (VIg'): 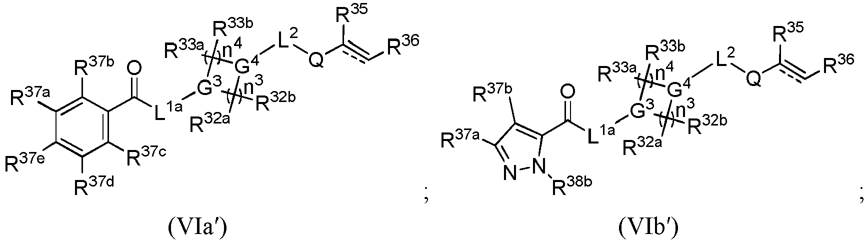 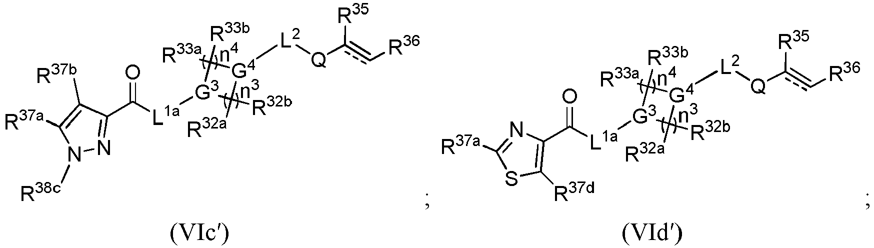 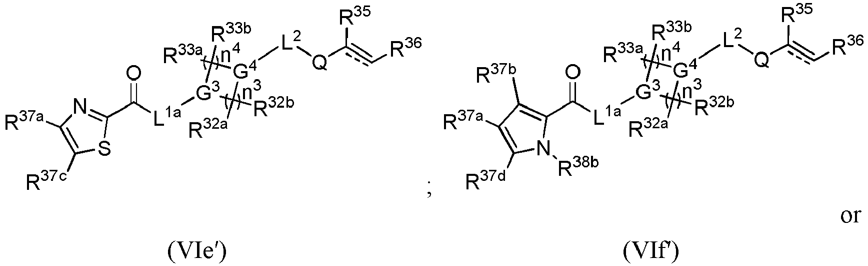 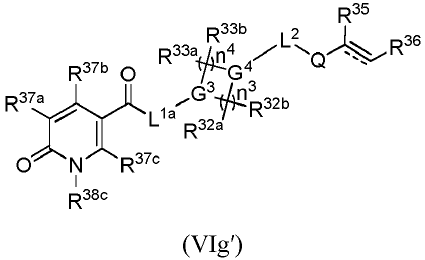 or a pharmaceutically acceptable salt, tautomer, stereoisomer or prodrug thereof, wherein:
i) G³ is N and G⁴ is CR; or
ii) G³ is CR and G⁴ is N; or
iii) G³ is N and G⁴ is N,

wherein R is H, cyano, halo or C₁-C₆alkyl;
L^{1a} is a bond, -NH-, alkylene or heteroalkylene;
L² is a bond or alkylene;
R^{32a} and R^{32b} are, at each occurrence, independently H, -OH, -NH₂, -CO₂H, cyano, cyanoalkyl, C₁-C₆alkyl, C₃-C₈cycloalkyl, hydroxylalkyl, aminoalkyl, carboxylalkyl or aminocarbonyl; or R^{32a} and R^{32b} join to form a carbocyclic or heterocyclic ring; or R^{32a} is H, -OH, -NH₂, -CO₂H, cyano, C₁-C₆alkyl, C₃-C₈cycloalkyl, hydroxylalkyl, aminoalkyl, carboxylalkyl or aminocarbonyl and R^{32b} joins with R^{33b} to form a carbocyclic or heterocyclic ring;
R^{33a} and R^{33b} are, at each occurrence, independently H, -OH, -NH₂, -CO₂H, cyano, cyanoalkyl, C₁-C₆alkyl, C₃-C₈cycloalkyl, hydroxylalkyl, aminoalkyl, carboxylalkyl or aminocarbonyl; or R^{33a} and R^{33b} join to form a carbocyclic or heterocyclic ring; or R^{33a} is H, -OH, -NH₂, -CO₂H, cyano, C₁-C₆alkyl, C₃-C₈cycloalkyl, hydroxylalkyl, aminoalkyl, carboxylalkyl or aminocarbonyl and R^{33b} joins with R^{32b} to form a carbocyclic or heterocyclic ring;
R^{37a}, R^{37b}, R^{37c}, R^{37d} and R^{37e} are each independently H, halo, oxo, hydroxyl, cyano, aminocarbonyl, formyl, C₁-C₆alkyl, C₁-C₆alkylsulfonyl, C₁-C₆haloalkyl, C₃-C₈cycloalkyl, C₁-C₆alkoxy, C₁-C₆hydroxylalkyl, C₁-C₆alkoxyalkyl, C₁-C₆aminoalkyl, heterocyclyl or aryl, wherein: i) R^{37a} or R^{37e} is aryl, or ii) R^{37a} or R^{37e}, or both, are chloro, bromo or iodo;
R^{38a}, R^{38b} and R^{38c} are each independently H, C₁-C₆alkyl or aryl;
n³ and n⁴ are each independently 1, 2 or 3;
Q is -C(=O)-, -NR³⁴C(=O)-, -S(=O)₂- or - NR³⁴S(=O)₂-;
R³⁴ is H, C₁-C₆alkyl or hydroxylalkyl;
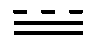 is a carbon-carbon double bond or a carbon-carbon triple bond; and
R³⁵ and R³⁶ are each independently H, cyano, C₁-C₆alkyl, aminoalkyl, alkylaminoalkyl, or hydroxylalkyl, wherein at least one of R³⁵ or R³⁶ is H
wherein each occurrence of alkyl, alkylene, heteroalkylene, cyanoalkyl, cycloalkyl, hydroxylalkyl, aminoalkyl, carboxylalkyl, aminocarbonyl, alkylsulfonyl, carbocyclic ring, heterocyclic ring, haloalkyl, alkoxy, alkoxyalkyl, heterocyclyl, aryl or alkylaminoalkyl is optionally substituted unless otherwise specified.

### BRIEF DESCRIPTION OF THE SEVERAL VIEWS OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

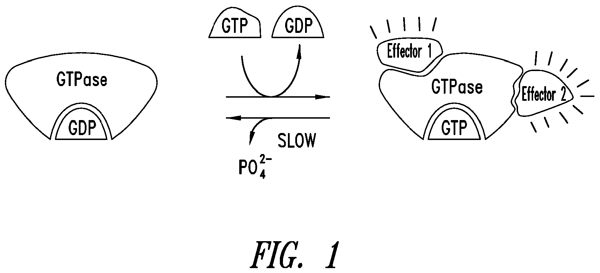

Fig. 1 Shows the enzymatic activity of Ras.

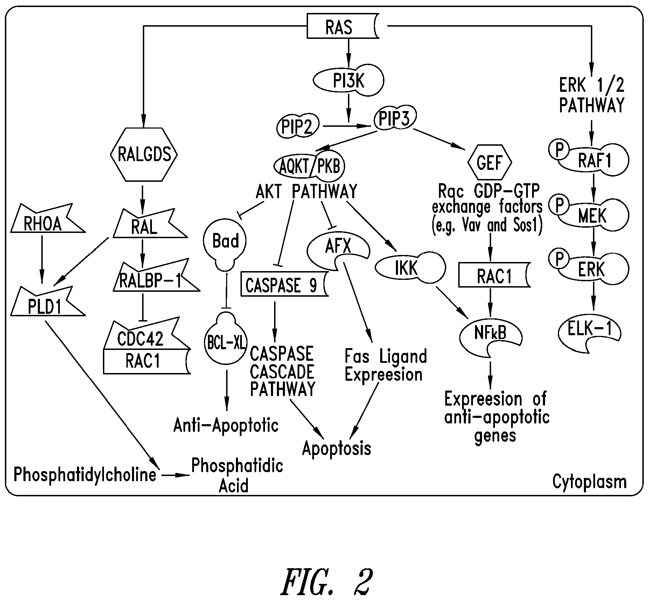

Fig. 2 shows a signal transduction pathway for Ras.

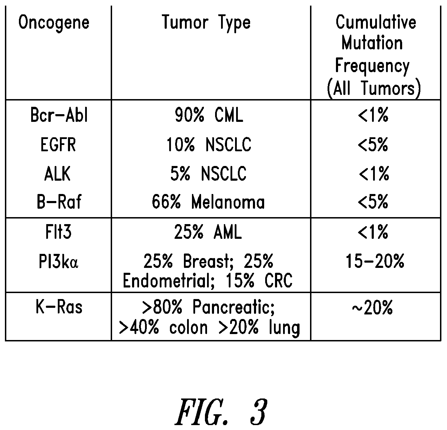

Fig. 3 Shows some common oncogenes, their respective tumor type and cumulative mutation frequencies (all tumors).

### DETAILED DESCRIPTION

Unless defined otherwise, all technical and scientific terms used herein have the same meaning as is commonly understood by one of skill in the art to which this invention belongs.

As used in the specification and claims, the singular form "a", "an" and "the" include plural references unless the context clearly dictates otherwise.

"Amino" refers to the -NH₂ radical.

"Carboxy" or "carboxyl" refers to the -CO₂H radical.

"Cyano" refers to the -CN radical.

"Hydroxy" or "hydroxyl" refers to the -OH radical.

"Imino" refers to the =NH substituent.

"Nitro" refers to the -NO₂ radical.

"Oxo" refers to the =O substituent.

"Thioxo" refers to the =S substituent.

"Alkyl" refers to a straight or branched hydrocarbon chain radical consisting solely of carbon and hydrogen atoms, which is saturated or unsaturated (*i*.*e*., contains one or more double and/or triple bonds), having from one to twelve carbon atoms (C₁-C₁₂ alkyl), preferably one to eight carbon atoms (Ci-Cs alkyl) or one to six carbon atoms (C₁-C₆ alkyl), and which is attached to the rest of the molecule by a single bond, *e*.*g*., methyl, ethyl, *n*-propyl, 1-methylethyl (*iso*-propyl), *n*-butyl, *n*-pentyl, 1,1-dimethylethyl (*t*-butyl), 3-methylhexyl, 2-methylhexyl, ethenyl, prop-1-enyl, but-1-enyl, pent-1-enyl, penta-1,4-dienyl, ethynyl, propynyl, butynyl, pentynyl, and hexynyl. Alkyl includes alkenyls (one or more carbon-carbon double bonds) and alkynyls (one or more carbon-carbon triple bonds). Unless stated otherwise specifically in the specification, an alkyl group is optionally substituted.

"Alkylene" or "alkylene chain" refers to a straight or branched divalent hydrocarbon chain linking the rest of the molecule to a radical group, consisting solely of carbon and hydrogen, which is saturated or unsaturated *(*i.e., contains one or more double and/or triple bonds), and having from one to twelve carbon atoms, *e.g.,* methylene, ethylene, propylene, *n*-butylene, ethenylene, propenylene, *n*-butenylene, propynylene, and *n*-butynylene. The alkylene chain is attached to the rest of the molecule through a single or double bond and to the radical group through a single or double bond. The points of attachment of the alkylene chain to the rest of the molecule and to the radical group can be through one carbon or any two carbons within the chain. Unless stated otherwise specifically in the specification, an alkylene chain is optionally substituted.

"Alkenylene" is an alkylene, as defined above, which comprises one or more carbon-carbon double bonds. Unless stated otherwise specifically in the specification, an alkenylene is optionally substituted

"Alkylenecarbonyl" refers to a radical of the formula -C(=O)Rₐ-, where Rₐ is an alkylene chain as defined above. Unless stated otherwise specifically in the specification, an alkylenecarbonyl is optionally substituted.

"Alkenylenecarbonyl" refers to an alkylenecarbonyl, as defined above, which comprises one or more carbon-carbon double bonds. Unless stated otherwise specifically in the specification, an alkenylenecarbonyl is optionally substituted.

"Alkoxy" refers to a radical of the formula -ORₐ where Rₐ is an alkyl radical as defined above containing one to twelve carbon atoms. Unless stated otherwise specifically in the specification, an alkoxy group is optionally substituted.

"Alkylamino" refers to a radical of the formula -NHRₐ or -NRₐRₐ where each Rₐ is, independently, an alkyl radical as defined above containing one to twelve carbon atoms. Unless stated otherwise specifically in the specification, an alkylamino group is optionally substituted.

"Aminoalkyl" refers to an alkyl group comprising at least one amino substituent. The amino substituent can be on a tertiary, secondary or primary carbon. Unless stated otherwise specifically in the specification, an aminoalkyl group is optionally substituted.

"Alkylaminoalkyl" refers to an alkyl group comprising at least one alkylamino substituent. The alkylamino substituent can be on a tertiary, secondary or primary carbon. Unless stated otherwise specifically in the specification, an alkylaminoalkyl group is optionally substituted.

"Aminocarbonyl" refers to a radical of the formula -C(=O)NRₐR_{b}, where Rₐ and R_{b} are each independently H or alkyl. Unless stated otherwise specifically in the specification, an alkoxy group is optionally substituted.

"Aryl" refers to a hydrocarbon ring system radical comprising hydrogen, 6 to 18 carbon atoms and at least one aromatic ring. For purposes of this invention, the aryl radical is a monocyclic, bicyclic, tricyclic or tetracyclic ring system, which may include fused or bridged ring systems. Aryl radicals include aryl radicals derived from aceanthrylene, acenaphthylene, acephenanthrylene, anthracene, azulene, benzene, chrysene, fluoranthene, fluorene, as-indacene, s-indacene, indane, indene, naphthalene, phenalene, phenanthrene, pleiadene, pyrene, and triphenylene. Unless stated otherwise specifically in the specification, the term "aryl" or the prefix "ar-" (such as in "aralkyl") is meant to include aryl radicals that are optionally substituted.

"Arylene" refers to a divalent aryl group which links the rest of the molecule (*e*.*g.,* compound of structure V-VI) to a radical group and/or to the rest of the molecule. Unless stated specifically otherwise, an arylene is optionally substituted.

"Aralkyl" refers to a radical of the formula -R_{b}-R_{c} where R_{b} is an alkylene chain as defined above and R_{c} is one or more aryl radicals as defined above, for example, benzyl, and diphenylmethyl. Unless stated otherwise specifically in the specification, an aralkyl group is optionally substituted.

"Carboxyalkyl" refers to a radical of the formula -R_{b}-R_{c} where R_{b} is an alkylene chain as defined above and R_{c} is a carboxy group as defined above. Unless stated otherwise specifically in the specification, carboxyalkyl group is optionally substituted.

"Cyanoalkyl" refers to a radical of the formula -R_{b}-R_{c} where R_{b} is an alkylene chain as defined above and R_{c} is a cyano group as defined above. Unless stated otherwise specifically in the specification, a cyanoalkyl group is optionally substituted.

"Cycloalkyl" or "carbocyclic ring" refers to a stable non-aromatic monocyclic or polycyclic hydrocarbon radical consisting solely of carbon and hydrogen atoms, which may include fused or bridged ring systems, having from three to fifteen carbon atoms, preferably having from three to ten carbon atoms, and which is saturated or unsaturated and attached to the rest of the molecule by a single bond. Monocyclic radicals include, for example, cyclopropyl, cyclobutyl, cyclopentyl, cyclohexyl, cycloheptyl, and cyclooctyl. Polycyclic radicals include, for example, adamantyl, norbornyl, decalinyl, and 7,7-dimethyl-bicyclo[2.2.1]heptanyl. A "cycloalkenyl" is a cycloalkyl comprising one or more carbob-carbon double bonds within the ring. Unless otherwise stated specifically in the specification, a cycloalkyl (or cycloalkenyl) group is optionally substituted.

The term "bicycloalkyl" refers to a structure consisting of two cycloalkyl moieties, unsubstituted or substituted, that have two or more atoms in common. If the cycloalkyl moieties have exactly two atoms in common they are said to be "fused". Examples include bicyclo[3.1.0]hexyl, and perhydronaphthyl. If the cycloalkyl moieties have more than two atoms in common they are said to be "bridged". Examples include bicyclo[3.2.1]heptyl ("norbornyl"), and bicyclo[2.2.2]octyl.

As used herein, the term "heteroatom" or "ring heteroatom" is meant to include oxygen (O), nitrogen (N), sulfur (S), and phosphorus (P).

The term "heteroalkyl," by itself or in combination with another term, means, unless otherwise stated, a straight or branched chain, or cyclic hydrocarbon radical, or combinations thereof, comprising of at least one carbon atoms and at least one heteroatom selected from the group comprising of O, N, P, Si and S, and wherein the nitrogen, phosphorus, and sulfur atoms may optionally be oxidized and the nitrogen heteroatom may optionally be quaternized. The heteroatom(s) O, N, P and S and Si may be placed at any interior position of the heteroalkyl group or at the position at which alkyl group is attached to the remainder of the molecule. The alkyl portion of the moiety is unsubstituted or substituted.

"Heteroalkylene" refers to an alkylene group comprising at least one heteroatom (e.g., N, O or S). In some embodiments, the heteroatom is within the alkylene chain (*i.e.,* the heteroalkylene comprises at least one carbon-heteroatom-carbon bond. In other embodiments, the heteroatom is at a terminus of the alkylene and thus serves to join the alkylene to the remainder of the molecule (e.g., M1-H-A-M2, where M1 and M2 are portions of the a molecule, H is a heteroatom and A is an alkylene). Unless stated otherwise specifically in the specification, a heteroalkylene is optionally substituted.

"Heteroalkylenecarbonyl" refers to a radical of the formula -C(=O)Rₐ-, where Rₐ is a heteroalkylene chain as defined above. Unless stated otherwise specifically in the specification, a heteroalkylenecarbonyl is optionally substituted.

The term "heterobicycloalkyl" refers to a bicycloalkyl structure, which is unsubstituted or substituted, in which at least one carbon atom is replaced with a heteroatom independently selected from oxygen, nitrogen, and sulfur.

The term "spiroalkyl" refers to a structure, which is unsubstituted or substituted, which comprises at least two cycloalkyl units joined at single carbon. In various embodiments the spiroalkyl rings can be 1-18 carbons.

The term "heterospiroalkyl" refers to a spiroalkyl structure, which is unsubstituted or substituted, in which at least one carbon atom is replaced with a heteroatom independently selected from oxygen, nitrogen, and sulfur.

"Cycloalkylalkyl" refers to a radical of the formula -R_{b}R_{d} where R_{b} is an alkylene chain as defined above and R_{d} is a cycloalkyl radical as defined above. Unless stated otherwise specifically in the specification, a cycloalkylalkyl group is optionally substituted.

"Fused" refers to any ring structure described herein which is fused to an existing ring structure in the compounds of the invention. When the fused ring is a heterocyclyl ring or a heteroaryl ring, any carbon atom on the existing ring structure which becomes part of the fused heterocyclyl ring or the fused heteroaryl ring is replaced with a nitrogen atom.

"Halo" or "halogen" refers to bromo, chloro, fluoro or iodo.

"Haloalkyl" refers to an alkyl radical, as defined above, that is substituted by one or more halo radicals, as defined above, *e.g.,* trifluoromethyl, difluoromethyl, trichloromethyl, 2,2,2-trifluoroethyl, 1,2-difluoroethyl, 3-bromo-2-fluoropropyl, and 1,2-dibromoethyl. Unless stated otherwise specifically in the specification, a haloalkyl group is optionally substituted.

"Heterocyclyl" or "heterocyclic ring" refers to a stable 3- to 18-membered non-aromatic ring radical which consists of two to twelve carbon atoms and from one to six heteroatoms selected from the group consisting of nitrogen, oxygen and sulfur. Unless stated otherwise specifically in the specification, the heterocyclyl radical is a monocyclic, bicyclic, tricyclic or tetracyclic ring system, which may include fused or bridged ring systems; and the nitrogen, carbon or sulfur atoms in the heterocyclyl radical is optionally oxidized; the nitrogen atom is optionally quaternized; and the heterocyclyl radical is partially or fully saturated. Examples of such heterocyclyl radicals include dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. Unless stated otherwise specifically in the specification. Unless stated otherwise specifically in the specification, a heterocyclyl group is optionally substituted.

"Heterocycloalkylene" refers to a divalent saturated heterocyclyl group which links the rest of the molecule (*e.g.,* compound of structure VI) to a radical group and/or to the rest of the molecule. Unless stated specifically otherwise, a heterocycloalkylene is optionally substituted.

"Heterocycloalkylenecarbonyl" refers to a radical of the formula -RₐC(=O)-, wherein Rₐ is a heterocycloalkylene as defined above. Unless stated specifically otherwise, a heterocycloalkylenecarbonyl is optionally substituted.

"*N*-heterocyclyl" refers to a heterocyclyl radical as defined above containing at least one nitrogen and where the point of attachment of the heterocyclyl radical to the rest of the molecule is through a nitrogen atom in the heterocyclyl radical. Unless stated otherwise specifically in the specification, a *N-*heterocyclyl group is optionally substituted.

"Heterocyclylalkyl" or "heterocycloalkyl" refers to a radical of the formula -R_{b}Rₑ where R_{b} is an alkylene chain as defined above and Rₑ is a heterocyclyl radical as defined above, and if the heterocyclyl is a nitrogen-containing heterocyclyl, the heterocyclyl is optionally attached to the alkyl radical at the nitrogen atom. Unless stated otherwise specifically in the specification, a heterocyclylalkyl group is optionally substituted.

"Heteroaryl" refers to a 5- to 14-membered ring system radical comprising hydrogen atoms, one to thirteen carbon atoms, one to six heteroatoms selected from the group consisting of nitrogen, oxygen and sulfur, and at least one aromatic ring. For purposes of this invention, the heteroaryl radical may be a monocyclic, bicyclic, tricyclic or tetracyclic ring system, which may include fused or bridged ring systems; and the nitrogen, carbon or sulfur atoms in the heteroaryl radical may be optionally oxidized; the nitrogen atom may be optionally quaternized. Examples include azepinyl, acridinyl, benzimidazolyl, benzothiazolyl, benzindolyl, benzodioxolyl, benzofuranyl, benzooxazolyl, benzothiazolyl, benzothiadiazolyl, benzo[*b*][1,4]dioxepinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzotriazolyl, benzo[4,6]imidazo[1,2-a]pyridinyl, carbazolyl, cinnolinyl, dibenzofuranyl, dibenzothiophenyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyridinyl, oxadiazolyl, 2-oxoazepinyl, oxazolyl, oxiranyl, 1-oxidopyridinyl, 1-oxidopyrimidinyl, 1-oxidopyrazinyl, 1-oxidopyridazinyl, 1-phenyl-1*H*-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, quinazolinyl, quinoxalinyl, quinolinyl, quinuclidinyl, isoquinolinyl, tetrahydroquinolinyl, thiazolyl, thiadiazolyl, triazolyl, tetrazolyl, triazinyl, and thiophenyl (i.e. thienyl). Unless stated otherwise specifically in the specification, a heteroaryl group is optionally substituted.

"Heteroarylene" refers to a divalent saturated heteroaryl group which links the rest of the molecule (*e.g.,* compound of structure VI) to a radical group and/or to the rest of the molecule. Unless stated specifically otherwise, a heteroarylene is optionally substituted.

"Heteroarylenecarbonyl" refers to a radical of the formula -RₐC(=O)-, wherein Rₐ is a heteroarylene as defined above. Unless stated specifically otherwise, a heteroarylenecarbonyl is optionally substituted.

"*N*-heteroaryl" refers to a heteroaryl radical as defined above containing at least one nitrogen and where the point of attachment of the heteroaryl radical to the rest of the molecule is through a nitrogen atom in the heteroaryl radical. Unless stated otherwise specifically in the specification, an *N*-heteroaryl group is optionally substituted.

"Heteroarylalkyl" refers to a radical of the formula -R_{b}R_{f} where R_{b} is an alkylene chain as defined above and R_{f} is a heteroaryl radical as defined above. Unless stated otherwise specifically in the specification, a heteroarylalkyl group is optionally substituted.

"Hydroxylalkyl" refers to an alkyl group comprising at least one hydroxyl substituent. The -OH substituent may be on a primary, secondary or tertiary carbon. Unless stated otherwise specifically in the specification, a hydroxylalkyl group is optionally substituted.

"Thioalkyl" refers to a radical of the formula -SRₐ where Rₐ is an alkyl radical as defined above containing one to twelve carbon atoms. Unless stated otherwise specifically in the specification, a thioalkyl group is optionally substituted.

The term "substituted" used herein means any of the above groups (*e*.*g*.., alkyl, alkylene, alkenylene, alkenylenecarbonyl, alkoxy, alkylamino, aminoalkyl, alkylaminoalkyl, thioalkyl, aryl, arylene, aralkyl, carboxyalkyl, cyanoalkyl, cycloalkyl, cycloalkylalkyl, heteroalkyl, heteroalkylene, heteroalkylenecarbonyl, heterobicycloalkyl, spiroalkyl, heterospiroalkyl, haloalkyl, heterocyclyl, heterocycloalkylene, heterocycloalkylenecarbonyl, *N-*heterocyclyl, heterocyclylalkyl, heteroaryl, heteroarylene, heteroarylenecarbonyl, *N*-heteroaryl, hydroxylalkyl, thioalkyl and/or heteroarylalkyl) wherein at least one hydrogen atom is replaced by a bond to a non-hydrogen atoms such as: a halogen atom such as F, Cl, Br, and I; an oxygen atom in groups such as hydroxyl groups, alkoxy groups, and ester groups; a sulfur atom in groups such as thiol groups, thioalkyl groups, sulfone groups, sulfonyl groups, and sulfoxide groups; a nitrogen atom in groups such as amines, amides, alkylamines, dialkylamines, arylamines, alkylarylamines, diarylamines, N-oxides, imides, and enamines; a silicon atom in groups such as trialkylsilyl groups, dialkylarylsilyl groups, alkyldiarylsilyl groups, and triarylsilyl groups; and other heteroatoms in various other groups. "Substituted" also means any of the above groups in which one or more hydrogen atoms are replaced by a higher-order bond (e.g., a double- or triple-bond) to a heteroatom such as oxygen in oxo, carbonyl, carboxyl, and ester groups; and nitrogen in groups such as imines, oximes, hydrazones, and nitriles. For example, "substituted" includes any of the above groups in which one or more hydrogen atoms are replaced with -NR_{g}Rₕ, -NR_{g}C(=O)Rₕ, -NR_{g}C(=O)NR_{g}Rₕ, -NR_{g}C(=O)ORₕ, -NR_{g}SO₂Rₕ, -OC(=O)NR_{g}Rₕ, -OR_{g}, -SR_{g}, -SOR_{g}, -SO₂R_{g}, -OSO₂R_{g}, -SO₂OR_{g}, =NSO₂R_{g}, and -SO₂NR_{g}Rₕ. "Substituted also means any of the above groups in which one or more hydrogen atoms are replaced with -C(=O)R_{g}, -C(=O)OR_{g}, -C(=O)NR_{g}Rₕ, -CH₂SO₂R_{g}, -CH₂SO₂NR_{g}Rₕ. In the foregoing, R_{g} and Rₕ are the same or different and independently hydrogen, alkyl, alkoxy, alkylamino, thioalkyl, aryl, aralkyl, cycloalkyl, cycloalkylalkyl, haloalkyl, heterocyclyl, *N*-heterocyclyl, heterocyclylalkyl, heteroaryl, *N-*heteroaryl and/or heteroarylalkyl. "Substituted" further means any of the above groups in which one or more hydrogen atoms are replaced by a bond to an amino, cyano, hydroxyl, imino, nitro, oxo, thioxo, halo, alkyl, alkoxy, alkylamino, thioalkyl, aryl, aralkyl, cycloalkyl, cycloalkylalkyl, haloalkyl, heterocyclyl, *N*-heterocyclyl, heterocyclylalkyl, heteroaryl, *N-*heteroaryl and/or heteroarylalkyl group. In addition, each of the foregoing substituents is optionally substituted with one or more of the above substituents.

"Electrophile" or "electrophilic moiety" is any moiety capable of reacting with a nucleophile (e.g., a moiety having a lone pair of electrons, a negative charge, a partial negative charge and/or an excess of electrons, for example a -SH group). Electrohiles typically are electron poor or comprise atoms which are electron poor. In certain embodiments an electrohile contains a positive charge or partial positive charge, has a resonance structure which contains a positive charge or partial positive charge or is a moiety in which delocalization or polarization of electrons results in one or more atom which contains a positive charge or partial positive charge. In some embodiments, the electrophiles comprise conjugated double bonds, for example an α,β-unsaturated carbonyl or α,β-unsaturated thiocarbonyl compound.

The term "effective amount" or "therapeutically effective amount" refers to that amount of a compound described herein that is sufficient to effect the intended application including disease treatment, as defined below. The therapeutically effective amount may vary depending upon the intended treatment application (in vivo), or the subject and disease condition being treated, e.g., the weight and age of the subject, the severity of the disease condition, and the manner of administration, which can readily be determined by one of ordinary skill in the art. The term also applies to a dose that will induce a particular response in target cells, e.g. reduction of platelet adhesion and/or cell migration. The specific dose will vary depending on the particular compounds chosen, the dosing regimen to be followed, whether it is administered in combination with other compounds, timing of administration, the tissue to which it is administered, and the physical delivery system in which it is carried.

As used herein, "treatment" or "treating" refer to an approach for obtaining beneficial or desired results with respect to a disease, disorder or medical condition including a therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the subject, notwithstanding that the subject may still be afflicted with the underlying disorder. In certain embodiments, for prophylactic benefit, the compositions are administered to a subject at risk of developing a particular disease, or to a subject reporting one or more of the physiological symptoms of a disease, even though a diagnosis of this disease may not have been made.

A "therapeutic effect," as that term is used herein, encompasses a therapeutic benefit and/or a prophylactic benefit as described above. A prophylactic effect includes delaying or eliminating the appearance of a disease or condition, delaying or eliminating the onset of symptoms of a disease or condition, slowing, halting, or reversing the progression of a disease or condition, or any combination thereof.

The term "co-administration," "administered in combination with," and their grammatical equivalents, as used herein, encompass administration of two or more agents to an animal, including humans, so that both agents and/or their metabolites are present in the subject at the same time. Co-administration includes simultaneous administration in separate compositions, administration at different times in separate compositions, or administration in a composition in which both agents are present.

"Pharmaceutically acceptable salt" includes both acid and base addition salts.

"Pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid, and phosphoric acid, and organic acids such as acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, *p-*toluenesulfonic acid, trifluoroacetic acid, and undecylenic acid.

"Pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include the sodium, potassium, lithium, ammonium, calcium, magnesium, iron, zinc, copper, manganese, and aluminum salts. Preferred inorganic salts are the ammonium, sodium, potassium, calcium, and magnesium salts. Salts derived from organic bases include salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, *N*-ethylpiperidine, and polyamine resins. Particularly preferred organic bases are isopropylamine, diethylamine, ethanolamine, trimethylamine, dicyclohexylamine, choline and caffeine.

The terms "antagonist" and "inhibitor" are used interchangeably, and they refer to a compound having the ability to inhibit a biological function of a target protein, whether by inhibiting the activity or expression of the protein, such as K-Ras, H-Ras or N-Ras G12C. Accordingly, the terms "antagonist" and "inhibitors" are defined in the context of the biological role of the target protein. While preferred antagonists herein specifically interact with (e.g. bind to) the target, compounds that inhibit a biological activity of the target protein by interacting with other members of the signal transduction pathway of which the target protein is a member are also specifically included within this definition. A preferred biological activity inhibited by an antagonist is associated with the development, growth, or spread of a tumor.

The term "agonist" as used herein refers to a compound having the ability to initiate or enhance a biological function of a target protein, whether by inhibiting the activity or expression of the target protein. Accordingly, the term "agonist" is defined in the context of the biological role of the target polypeptide. While preferred agonists herein specifically interact with (e.g. bind to) the target, compounds that initiate or enhance a biological activity of the target polypeptide by interacting with other members of the signal transduction pathway of which the target polypeptide is a member are also specifically included within this definition.

As used herein, "agent" or "biologically active agent" refers to a biological, pharmaceutical, or chemical compound or other moiety. Non-limiting examples include a simple or complex organic or inorganic molecule, a peptide, a protein, an oligonucleotide, an antibody, an antibody derivative, antibody fragment, a vitamin derivative, a carbohydrate, a toxin, or a chemotherapeutic compound. Various compounds can be synthesized, for example, small molecules and oligomers (e.g., oligopeptides and oligonucleotides), and synthetic organic compounds based on various core structures. In addition, various natural sources can provide compounds for screening, such as plant or animal extracts.

"Signal transduction" is a process during which stimulatory or inhibitory signals are transmitted into and within a cell to elicit an intracellular response. A modulator of a signal transduction pathway refers to a compound which modulates the activity of one or more cellular proteins mapped to the same specific signal transduction pathway. A modulator may augment (agonist) or suppress (antagonist) the activity of a signaling molecule.

An "anti-cancer agent", "anti-tumor agent" or "chemotherapeutic agent" refers to any agent useful in the treatment of a neoplastic condition. One class of anti-cancer agents comprises chemotherapeutic agents. "Chemotherapy" means the administration of one or more chemotherapeutic drugs and/or other agents to a cancer patient by various methods, including intravenous, oral, intramuscular, intraperitoneal, intravesical, subcutaneous, transdermal, buccal, or inhalation or in the form of a suppository.

The term "cell proliferation" refers to a phenomenon by which the cell number has changed as a result of division. This term also encompasses cell growth by which the cell morphology has changed (e.g., increased in size) consistent with a proliferative signal.

The term "selective inhibition" or "selectively inhibit" refers to a biologically active agent refers to the agent's ability to preferentially reduce the target signaling activity as compared to off-target signaling activity, via direct or indirect interaction with the target.

"Subject" refers to an animal, such as a mammal, for example a human. The methods described herein can be useful in both human therapeutics and veterinary applications. In some embodiments, the subject is a mammal, and in some embodiments, the subject is human.

"Mammal" includes humans and both domestic animals such as laboratory animals and household pets (*e*.*g*., cats, dogs, swine, cattle, sheep, goats, horses, rabbits), and non-domestic animals such as wildlife.

"Radiation therapy" means exposing a subject, using routine methods and compositions known to the practitioner, to radiation emitters such as alpha-particle emitting radionuclides (e.g., actinium and thorium radionuclides), low linear energy transfer (LET) radiation emitters (i.e. beta emitters), conversion electron emitters (e.g*.* strontium-89 and samarium-153-EDTMP, or high-energy radiation, including without limitation x-rays, gamma rays, and neutrons.

An "anti-cancer agent", "anti-tumor agent" or "chemotherapeutic agent" refers to any agent useful in the treatment of a neoplastic condition. One class of anti-cancer agents comprises chemotherapeutic agents. "Chemotherapy" means the administration of one or more chemotherapeutic drugs and/or other agents to a cancer patient by various methods, including intravenous, oral, intramuscular, intraperitoneal, intravesical, subcutaneous, transdermal, buccal, or inhalation or in the form of a suppository.

"Prodrug" is meant to indicate a compound that may be converted under physiological conditions or by solvolysis to a biologically active compound described herein. Thus, the term "prodrug" refers to a precursor of a biologically active compound that is pharmaceutically acceptable. In some aspects, a prodrug is inactive when administered to a subject, but is converted in vivo to an active compound, for example, by hydrolysis. The prodrug compound often offers advantages of solubility, tissue compatibility or delayed release in a mammalian organism (see, e.g., Bundgard, H., Design of Prodrugs (1985), pp. 7-9, 21-24 (Elsevier, Amsterdam). A discussion of prodrugs is provided in Higuchi, T., et al., "Pro-drugs as Novel Delivery Systems," A.C.S. Symposium Series, Vol. 14, and in Bioreversible Carriers in Drug Design, ed. Edward B. Roche, American Pharmaceutical Association and Pergamon Press, 1987. The term "prodrug" is also meant to include any covalently bonded carriers, which release the active compound in vivo when such prodrug is administered to a mammalian subject. Prodrugs of an active compound, as described herein, are typically prepared by modifying functional groups present in the active compound in such a way that the modifications are cleaved, either in routine manipulation or in vivo, to the parent active compound. Prodrugs include compounds wherein a hydroxy, amino or mercapto group is bonded to any group that, when the prodrug of the active compound is administered to a mammalian subject, cleaves to form a free hydroxy, free amino or free mercapto group, respectively. Examples of prodrugs include acetate, formate and benzoate derivatives of a hydroxy functional group, or acetamide, formamide and benzamide derivatives of an amine functional group in the active compound.

The term "in vivo" refers to an event that takes place in a subject's body.

The invention disclosed herein is also meant to encompass the *in vivo* metabolic products of the disclosed compounds. Such products may result from, for example, the oxidation, reduction, hydrolysis, amidation, and esterification of the administered compound, primarily due to enzymatic processes. Accordingly, the invention includes compounds produced by a process comprising administering a compound of this invention to a mammal for a period of time sufficient to yield a metabolic product thereof. Such products are typically identified by administering a radiolabelled compound of the invention in a detectable dose to an animal, such as rat, mouse, guinea pig, monkey, or to human, allowing sufficient time for metabolism to occur, and isolating its conversion products from the urine, blood or other biological samples.

"Stable compound" and "stable structure" are meant to indicate a compound that is sufficiently robust to survive isolation to a useful degree of purity from a reaction mixture, and formulation into an efficacious therapeutic agent.

Often crystallizations produce a solvate of the compound of the invention. As used herein, the term "solvate" refers to an aggregate that comprises one or more molecules of a compound of the invention with one or more molecules of solvent. In some embodiments, the solvent wise water, in which case the solvate is a hydrate. Alternatively, in other embodiments, the solvent is an organic solvent. Thus, the compounds of the present invention may exist as a hydrate, including a monohydrate, dihydrate, hemihydrate, sesquihydrate, trihydrate, and tetrahydrate, as well as the corresponding solvated forms. In some aspects, the compound of the invention is a true solvate, while in other cases, the compound of the invention merely retains adventitious water or is a mixture of water plus some adventitious solvent.

"Optional" or "optionally" means that the subsequently described event of circumstances may or may not occur, and that the description includes instances where said event or circumstance occurs and instances in which it does not. For example, "optionally substituted aryl" means that the aryl radical may or may not be substituted and that the description includes both substituted aryl radicals and aryl radicals having no substitution.

A "pharmaceutical composition" refers to a formulation of a compound of the invention and a medium generally accepted in the art for the delivery of the biologically active compound to mammals, *e.g.,* humans. Such a medium includes all pharmaceutically acceptable carriers, diluents or excipients therefor.

"Pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

The compounds of the invention, or their pharmaceutically acceptable salts may contain one or more asymmetric centers and may thus give rise to enantiomers, diastereomers, and other stereoisomeric forms that are defined, in terms of absolute stereochemistry, as (*R*)- or (*S*)- or, as (D)- or (L)- for amino acids. The present invention is meant to include all such possible isomers, as well as their racemic and optically pure forms. Optically active (+) and (-), (*R*)- and (*S*)-, or (D)- and (L)-isomers may be prepared using chiral synthons or chiral reagents, or resolved using conventional techniques, for example, chromatography and fractional crystallization. Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC). When the compounds described herein contain olefinic double bonds or other centres of geometric asymmetry, and unless specified otherwise, it is intended that the compounds include both E and Z geometric isomers. Likewise, all tautomeric forms are also intended to be included.

The present invention includes all manner of rotamers and conformationally restricted states of a compound of the invention.

A "stereoisomer" refers to a compound made up of the same atoms bonded by the same bonds but having different three-dimensional structures, which are not interchangeable. The present invention contemplates various stereoisomers and mixtures thereof and includes "enantiomers", which refers to two stereoisomers whose molecules are nonsuperimposeable mirror images of one another.

A "tautomer" refers to a proton shift from one atom of a molecule to another atom of the same molecule. The present invention includes tautomers of any said compounds.

The chemical naming protocol and structure diagrams used herein are a modified form of the I.U.P.A.C. nomenclature system, using the ACD/Name Version 9.07 software program and/or ChemDraw Ultra Version 11.0.1 software naming program (CambridgeSoft). For complex chemical names employed herein, a substituent group is typically named before the group to which it attaches. For example, cyclopropylethyl comprises an ethyl backbone with a cyclopropyl substituent. Except as described below, all bonds are identified in the chemical structure diagrams herein, except for all bonds on some carbon atoms, which are assumed to be bonded to sufficient hydrogen atoms to complete the valency.

### Compounds

In an aspect, the invention provides compounds which are capable of selectively binding to and/or modulating a G12C mutant K-Ras, H-Ras or N-Ras protein.. In some embodiments, the compounds modulate the G12C mutant K-Ras, H-Ras or N-Ras protein by reaction with an amino acid. In some embodiment the compounds of the invention selectively react with the G12C mutant K-Ras, H-Ras or N-Ras proteins by forming an irreversible covalent bond with the cysteine at the 12 position. By binding to the Cystine 12 the compounds of the invention may lock the switch II of the G12C mutant K-Ras, H-Ras or N-Ras into an inactive stage. This inactive stage may be distinct from those observed for GTP and GDP bound K-Ras, H-Ras or N-Ras. Some compounds of the invention are also able to perturb the switch I conformation. Because effector binding to K-Ras, H-Ras or N-Ras is highly sensitive to the conformation of switch I and II, the irreversible binding of these compounds may disrupt K-Ras, H-Ras or N-Ras downstream signaling.

The compound of the present invention has one of the following structures (VIa'), (VIb'), (VIc'), (VId'), (VIe'), (VIf') or (VIg'): 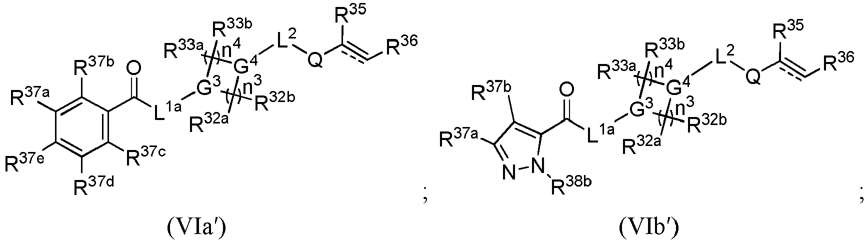 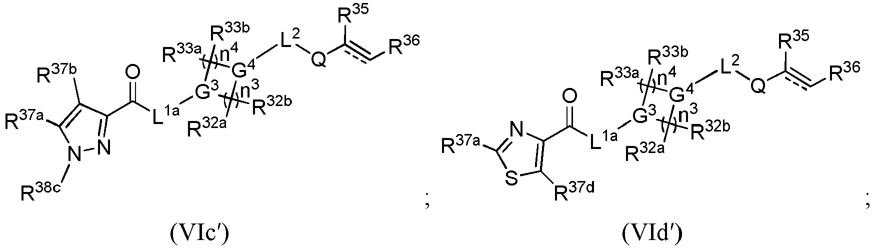 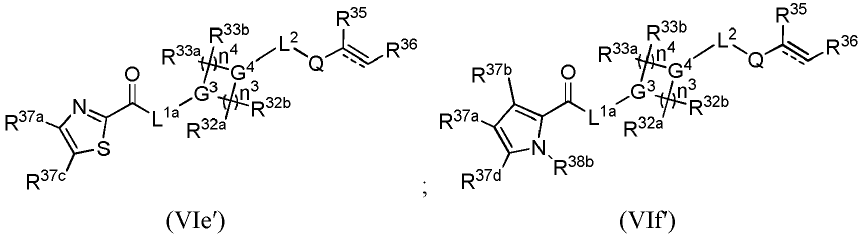 or 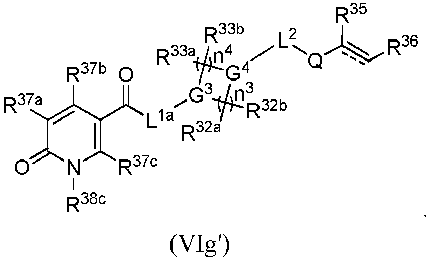 wherein:
or a pharmaceutically acceptable salt, tautomer, stereoisomer or prodrug thereof,
wherein:
   i) G³ is N and G⁴ is CR; or
   ii) G³ is CR and G⁴ is N; or
   iii) G³ is N and G⁴ is N,

   wherein R is H, cyano, halo or C₁-C₆alkyl;
   L^{1a} is a bond, -NH-, alkylene or heteroalkylene;
   L² is a bond or alkylene;
   R^{32a} and R^{32b} are, at each occurrence, independently H, -OH, -NH₂, -CO₂H, cyano, cyanoalkyl, C₁-C₆alkyl, C₃-C₈cycloalkyl, hydroxylalkyl, aminoalkyl, carboxylalkyl or aminocarbonyl; or R^{32a} and R^{32b} join to form a carbocyclic or heterocyclic ring; or R^{32a} is H, -OH, -NH₂, -CO₂H, cyano, C₁-C₆alkyl, C₃-C₈cycloalkyl, hydroxylalkyl, aminoalkyl, carboxylalkyl or aminocarbonyl and R^{32b} joins with R^{33b} to form a carbocyclic or heterocyclic ring;
   R^{33a} and R^{33b} are, at each occurrence, independently H, -OH, -NH₂, -CO₂H, cyano, cyanoalkyl, C₁-C₆alkyl, C₃-C₈cycloalkyl, hydroxylalkyl, aminoalkyl, carboxylalkyl or aminocarbonyl; or R^{33a} and R^{33b} join to form a carbocyclic or heterocyclic ring; or R^{33a} is H, -OH, -NH₂, -CO₂H, cyano, C₁-C₆alkyl, C₃-C₈cycloalkyl, hydroxylalkyl, aminoalkyl, carboxylalkyl or aminocarbonyl and R^{33b} joins with R^{32b} to form a carbocyclic or heterocyclic ring;
   R^{37a}, R^{37b}, R^{37c}, R^{37d} and R^{37e} are each independently H, halo, oxo, hydroxyl, cyano, aminocarbonyl, formyl, C₁-C₆alkyl, C₁-C₆alkylsulfonyl, C₁-C₆haloalkyl, C₃-C₈cycloalkyl, C₁-C₆alkoxy, C₁-C₆hydroxylalkyl, C₁-C₆alkoxyalkyl, C₁-C₆aminoalkyl, heterocyclyl or aryl, wherein: i) R^{37a} or R^{37e} is aryl, or ii) R^{37a} or R^{37e}, or both, are chloro, bromo or iodo;
   R^{38a}, R^{38b} and R^{38c} are each independently H, C₁-C₆alkyl or aryl;
   n³ and n⁴ are each independently 1, 2 or 3;
   Q is -C(=O)-, -NR³⁴C(=O)-, -S(=O)₂- or - NR³⁴S(=O)₂-;
   R³⁴ is H, C₁-C₆alkyl or hydroxylalkyl;
   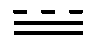 is a carbon-carbon double bond or a carbon-carbon triple bond; and
   R³⁵ and R³⁶ are each independently H, cyano, C₁-C₆alkyl, aminoalkyl, alkylaminoalkyl, or hydroxylalkyl, wherein at least one of R³⁵ or R³⁶ is H
   wherein each occurrence of alkyl, alkylene, heteroalkylene, cyanoalkyl, cycloalkyl, hydroxylalkyl, aminoalkyl, carboxylalkyl, aminocarbonyl, alkylsulfonyl, carbocyclic ring, heterocyclic ring, haloalkyl, alkoxy, alkoxyalkyl, heterocyclyl, aryl or alkylaminoalkyl is optionally substituted unless otherwise specified.

In various embodiments, n³ is 2 and n⁴ is 2. In still other embodiments, n³ is 1 and n⁴ is 1. In some more embodiments, n³ is 2 and n⁴ is 1.

In other of the foregoing embodiments, R^{37a}, R^{37b}, R^{37c}, R^{37d} and R^{37e} are each independently H, -OH, halo, oxo, C₁-C₆alkyl, C₁-C₆alkoxy, heterocyclyl or aryl.

In some embodiments, R^{37a} or R^{37e} is aryl. In some more specific embodiments, R^{37a} is aryl, such as phenyl.

In some different embodiments, the aryl is unsubstituted. In some other embodiments, the aryl is substituted. For example, in some embodiments the aryl is substituted with one or more halo substituents. In some of these embodiments, the halo substituents are selected from fluoro and chloro.

In other of the foregoing embodiments, R^{37a} is halo. For example, in some embodiments halo is chloro, bromo or iodo.

In some embodiments, R^{37a} or R^{37e} has one of the following structures:

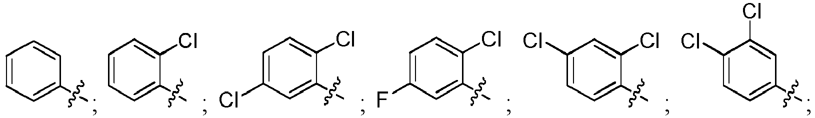

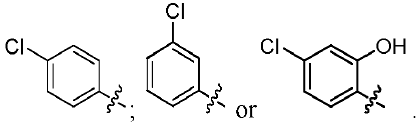

In some other embodiments of the compounds of structure (VI), Q is - C(=O)-.

In other of the foregoing embodiments, at least one of R³⁵ or R³⁶ is H, for example, in some embodiment each of R³⁵ and R³⁶ are H.

In some different embodiments, L^{1a} is a bond.

In some embodiments, L^{1a} is -NH, alkylene, or heteroalkyleneIn some of these embodiments, L^{1a} is substituted alkylene. In various other embodiments, L^{1a} is unsubstituted alkylene. For example, in some embodiments L^{1a} is

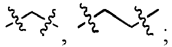

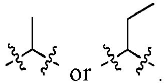

In some different embodiments, L^{1a} is substituted heteroalkylene. In some other embodiments, L^{1a} is unsubstituted heteroalkylene. In some of the foregoing embodiments, L^{1a} is aminoalkylene or thioalkylene, for example aminoalkylene. For example, in some embodiments L^{1a} has one of the following structures:

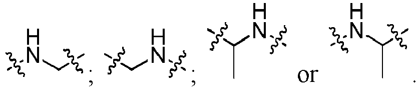

In other embodiments, L^{1a} is

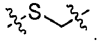

In other of the foregoing embodiments, at least one of R^{32a}, R^{32b}, R^{33a} or R^{33b} is H. For example, in some embodiments each of R^{32a}, R^{32b}, R^{33a} or R^{33b} is H.

In other of the foregoing embodiments, at least one of R^{32a}, R^{32b}, R^{33a} or R^{33b} is hydroxylalkyl.

In still other embodiments, at least one of R^{32a}, R^{32b}, R^{33a} or R^{33b} is cyano.

In some other different embodiments, at least one of R^{32a}, R^{32b}, R^{33a} or R^{33b} is aminocarbonyl; or at least one of R^{32a}, R^{32b}, R^{33a} or R^{33b} is C₁-C₆ alkyl.

In some more specific embodiments, the compound is selected from a compound in Table 6.

The compounds of the present invention are prepared according to procedures well-known or derivable by one of ordinary skill in the art, for example by procedures analogous to those exemplified in Examples 13, 17, 19, 20, 22 and other examples provided below. Each of the compounds in Table 6 was prepared in such a manner and analyzed by mass spectrometry and/or ¹H NMR. The mass spectrum ([M+H⁺] or [M+Na⁺]) and/or NMR spectrum was found to be consistent with the structures in Table VI.

General Reaction Scheme II illustrates an exemplary procedure for preparing compounds of the present invention

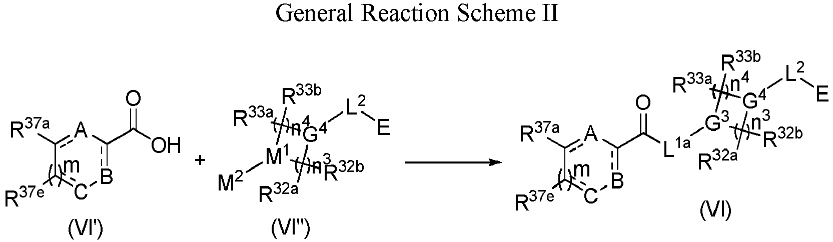

Referring to General Reaction Scheme II, (VI') and (VI") are available from commercial sources and/or are easily prepared according to procedures known in the art. All variables on (VI') and (VI"), with the exception of M¹ and M², are as defined above. In some procedures, M¹ is NH and M² is absent. In other procedures M¹ is N or CH and M² is a precursor to L^{1a} which reacts with an activated acid. For example, in various procedures M² is NH₂, aminoalkyl or other heterosubstituted alkyl. Embodiments where M² comprises a carbanion (or M¹ is a carbanion) are also contemplated such that L¹ is alkylene. Briefly, an appropriately substituted acid (VI') is activated and reacted with an appropriately substituted heterocycle (VI") under appropriate coupling conditions. The L²-E moiety may be present in (VI") as illustrated or may be installed after coupling For example L²-E may be installed before or after coupling via acylation (or thioacylation) using a reagent such as an acid chloride or thionyl chloride.

It should be noted that variations of the above procdure are possible, some of which are exemplified in the examples. For example, in some procedures, the acid moiety is present on the cyclic moiety (VI') and (VI') is appropriately substituted with a nucleophilic moiety to enable coupling to form (VI). Other methods of bond formation, which do not require reaction of an activated acid are also available for preparation of the compounds. It should also ne noted that various substitutions on (VI') and/or (VI") can be present during the coupling step (in protected or unprotected form) or the substituents can be added after (VI') and (VI") are coupled. Methods for inclusion of these substituents are known in the art.

Various options are available to one of ordinary skill in the art to add various substituents and or modify or reorder the above described steps to arrive at different embodiments of compounds of structure VI. The appropriate acid is purchased commercially or made according to well-known procedures.

It will also be appreciated by those skilled in the art that in the processes described herein (e.g., General Reaction Scheme I and II and the below examples) the functional groups of intermediate compounds may need to be protected by suitable protecting groups. Such functional groups include hydroxy, amino, mercapto and carboxylic acid. Suitable protecting groups for hydroxy include trialkylsilyl or diarylalkylsilyl (for example, *t*-butyldimethylsilyl, *t*-butyldiphenylsilyl or trimethylsilyl), tetrahydropyranyl, and benzyl. Suitable protecting groups for amino, amidino and guanidino include *t*-butoxycarbonyl, and benzyloxycarbonyl. Suitable protecting groups for mercapto include -C(O)-R" (where R" is alkyl, aryl or arylalkyl), *p*-methoxybenzyl, and trityl. Suitable protecting groups for carboxylic acid include alkyl, aryl or arylalkyl esters. Protecting groups may be added or removed in accordance with standard techniques, which are known to one skilled in the art and as described herein. The use of protecting groups is described in detail in Green, T.W. and P.G.M. Wutz, Protective Groups in Organic Synthesis (1999), 3rd Ed., Wiley. As one of skill in the art would appreciate, the protecting group may also be a polymer resin such as a Wang resin, Rink resin or a 2-chlorotrityl-chloride resin.

It will also be appreciated by those skilled in the art, although such protected derivatives of compounds of this invention may not possess pharmacological activity as such, they may be administered to a mammal and thereafter metabolized in the body to form compounds of the invention which are pharmacologically active. Such derivatives may therefore be described as "prodrugs". All prodrugs of compounds of this invention are included within the scope of the invention.

It is also understood that one skilled in the art may be able to make these compounds by similar methods or by combining other methods known to one skilled in the art. It is further understood that one skilled in the art would be able to make, in a similar manner as described below, other compounds of of the invention not specifically illustrated below by using the appropriate starting components and modifying the parameters of the synthesis as needed. In general, starting components may be obtained from sources such as Sigma Aldrich, Lancaster Synthesis, Inc., Maybridge, Matrix Scientific, TCI, and Fluorochem USA, etc. or synthesized according to sources known to those skilled in the art (see, for example, Advanced Organic Chemistry: Reactions, Mechanisms, and Structure, 5th edition (Wiley, December 2000)) or prepared as described in this invention.

**Table 5a**

| Reference Compounds | | | | | |
|---|---|---|---|---|---|
| **No.** | **Structure** | **Name** | **No.** | **Structure** | **Name** |
| V-1 | 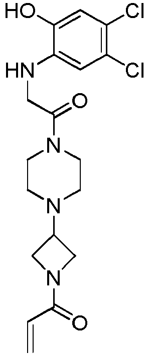 | 1-(3-(4-(2-(4,5-dichloro-2-hydroxyphenylam ino)acetyl)piperaz in-1-yl)azetidin-1-yl)prop-2-en-1-one | V-2 | 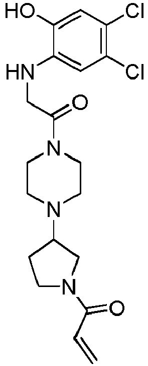 | 1-(3-(4-(2-(4,5-dichloro-2-hydroxyphenylami no)acetyl)piperazi n-1-yl)pyrrolidin-1-yl)prop-2-en-1-one |
| V-3 | 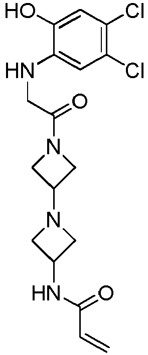 | N-(1'-(2-(4,5-dichloro-2-hydroxyphenylam ino)acetyl)-1,3'-biazetidin-3-yl)acrylamide | V-4 | 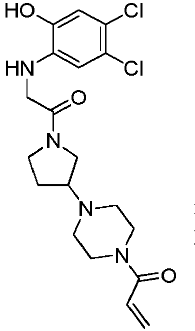 | 1-(4-(1-(2-(4,5-dichloro-2-hydroxyphenylami no)acetyl)pyrrolidi n-3-yl)piperazin-1-yl)prop-2-en-1-one |
| V-5 | 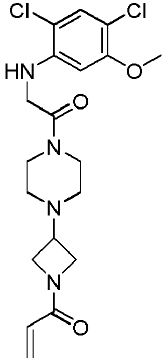 | 1-(3-(4-(2-(2,4-dichloro-5-methoxyphenyla mino)acetyl)piper azin-1-yl)azetidin-1-yl)prop-2-en-1-one | V-6 | 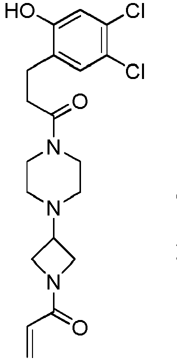 | 1-(3-(4-(3-(4,5-dichloro-2-hydroxyphenyl)pr opanoyl)piperazin-1-yl)azetidin-1-yl)prop-2-en-1-one |
| V-7 | 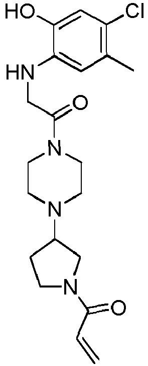 | 1-(3-(4-(2-(4-chloro-2-hydroxy-5-methylphenylami no)acetyl)piperazi n-1-yl)pyrrolidin-1-yl)prop-2-en-1-one | V-8 | 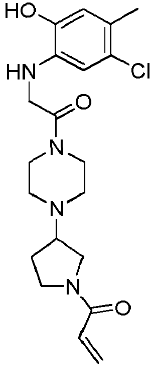 | 1-(3-(4-(2-(5-chloro-2-hydroxy-4-methylphenylamin o)acetyl)piperazin-1-yl)pyrrolidin-1-yl)prop-2-en-1-one |
| V-9 | 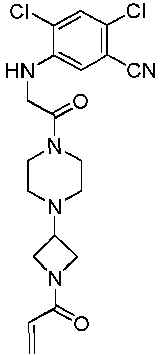 | 5-(2-(4-(1-acryloylazetidin-3-yl)piperazin-1-yl)-2-oxoethylamino)-2,4-dichlorobenzonitr ile | V-10 | 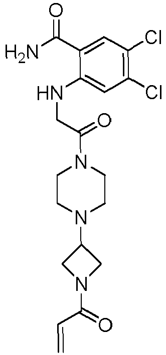 | 2-(2-(4-(1-acryloylazetidin-3-yl)piperazin-1-yl)-2-oxoethylamino)-4,5-dichlorobenzamide |
| V-11 | 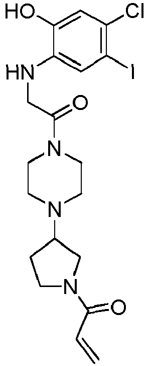 | 1-(3-(4-(2-(4-chloro-2-hydroxy-5-iodophenylamino )acetyl)piperazin-1-yl)pyrrolidin-1-yl)prop-2-en-1-one | V-12 | 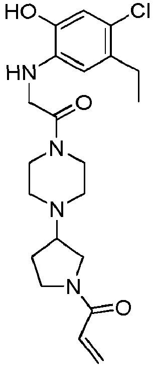 | 1-(3-(4-(2-(4-chloro-5-ethyl-2-hydroxyphenylami no)acetyl)piperazi n-1-yl)pyrrolidin-1-yl)prop-2-en-1-one |

| **No.** | **Structure** | **Name** | **No.** | **Structure** | **Name** |
|---|---|---|---|---|---|
| V-13 | 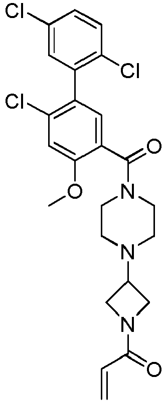 | 1-(3-(4-(2',5',6-trichloro-4-methoxybiphenyl carbonyl)piperazi n-1-yl)azetidin-1-yl)prop-2-en-1-one | V-14 | 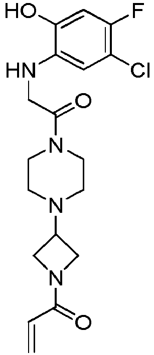 | 1-(3-(4-(2-(5-chloro-4-fluoro-2-hydroxyphenylami no)acetyl)piperazi n-1-yl)azetidin-1-yl)prop-2-en-1-one |
| V-15 | 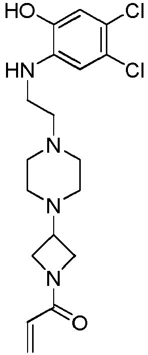 | 1-(3-(4-(2-(4,5-dichloro-2-hydroxyphenylam ino)ethyl)piperazi n-1-yl)azetidin-1-yl)prop-2-en-1-one | V-16 | 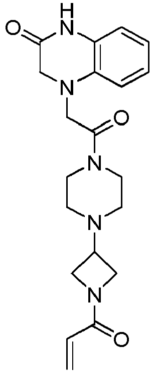 | 4-(2-(4-(1-acryloylazetidin-3-yl)piperazin-1-yl)-2-oxoethyl)-3,4-dihydroquinoxalin -2(1H)-one |
| V-17 | 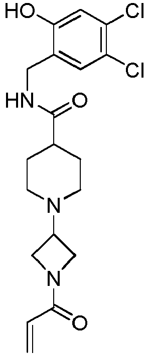 | 1-(1-acryloylazetidin-3-yl)-N-(4,5-dichloro-2-hydroxybenzyl)pi peridine-4-carboxamide | V-18 | 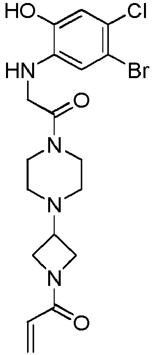 | 1-(3-(4-(2-(5-bromo-4-chloro-2-hydroxyphenylami no)acetyl)piperazi n-1-yl)azetidin-1-yl)prop-2-en-1-one |
| V-19 | 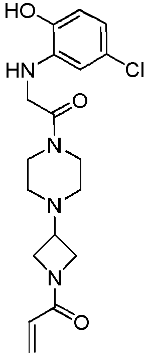 | 1-(3-(4-(2-(5-chloro-2-hydroxyphenylam ino)acetyl)piperaz in-1-yl)azetidin-1-yl)prop-2-en-1-one | V-20 | 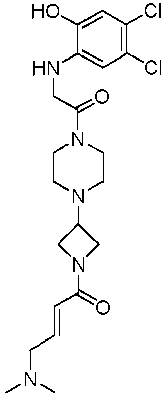 | (E)-1-(3-(4-((4,5-dichloro-2-hydroxyphenyl)gly cyl)piperazin-1-yl)azetidin-1-yl)-4-(dimethylamino)b ut-2-en-1-one |
| V-21 | 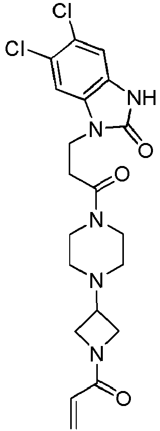 | 1-(3-(4-(1-acryloylazetidin-3-yl)piperazin-1-yl)-3-oxopropyl)-5,6-dichloro-1H-benzo[d] imidazol -2(3H)-one | V-22 | 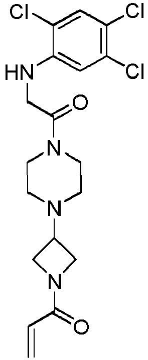 | 1-(3-(4-(2-(2,4,5-trichlorophenylami no)acetyl)piperazi n-1-yl)azetidin-1-yl)prop-2-en-1-one |
| V-23 | 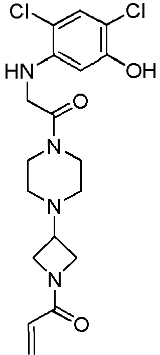 | 1-(3-(4-(2-(2,4-dichloro-5-hydroxyphenylam ino)acetyl)piperaz in-1-yl)azetidin-1-yl)prop-2-en-1-one | V-24 | 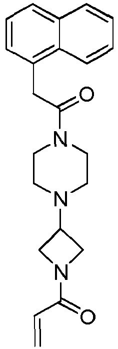 | 1-(3-(4-(2-(naphthalen-1-yl)acetyl)piperazin -1-yl)azetidin-1-yl)prop-2-en-1-one |
| V-25 | 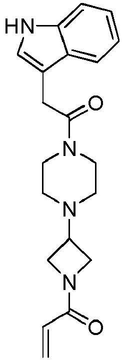 | 1-(3-(4-(2-(1H-indol-3-yl)acetyl)piperazi n-1-yl)azetidin-1-yl)prop-2-en-1-one | V-26 | 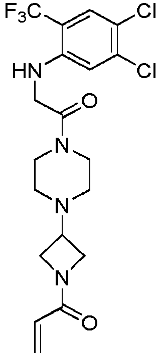 | 1-(3-(4-(2-(4,5-dichloro-2-(trifluoromethyl)p henylamino)acetyl )piperazin-1-yl)azetidin-1-yl)prop-2-en-1-one |
| V-27 | 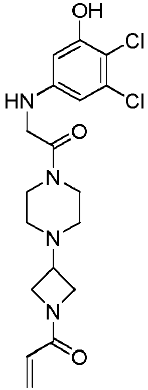 | 1-(3-(4-(2-(3,4-dichloro-5-hydroxyphenylam ino)acetyl)piperaz in-1-yl)azetidin-1-yl)prop-2-en-1-one | V-28 | 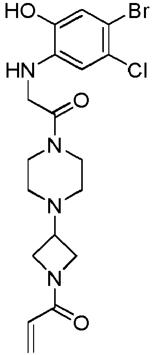 | 1-(3-(4-(2-(4-bromo-5-chloro-2-hydroxyphenylami no)acetyl)piperazi n-1-yl)azetidin-1-yl)prop-2-en-1-one |
| V-29 | 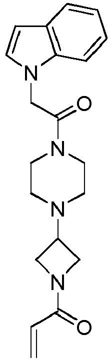 | 1-(3-(4-(2-(1H-indol-1-yl)acetyl)piperazi n-1-yl)azetidin-1-yl)prop-2-en-1-one | V-30 | 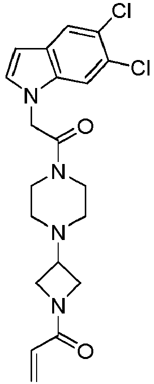 | 1-(3-(4-(2-(5,6-dichloro-1H-indol-1-yl)acetyl)piperazin -1-yl)azetidin-1-yl)prop-2-en-1-one |
| V-31 | 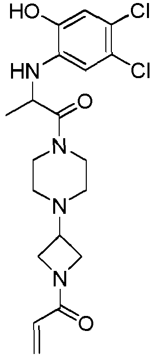 | 1-(3-(4-(2-(4,5-dichloro-2-hydroxyphenylam ino)propanoyl)pip erazin-1-yl)azetidin-1-yl)prop-2-en-1-one | V-32 | 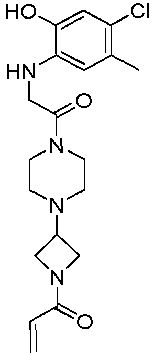 | 1-(3-(4-(2-(4-chloro-2-hydroxy-5-methylphenylamin o)acetyl)piperazin-1-yl)azetidin-1-yl)prop-2-en-1-one |
| V-33 | 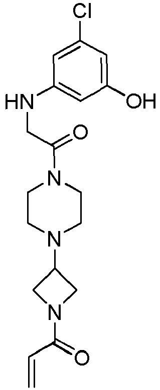 | 1-(3-(4-(2-(3-chloro-5-hydroxyphenylam ino)acetyl)piperaz in-1-yl)azetidin-1-yl)prop-2-en-1-one | V-34 | 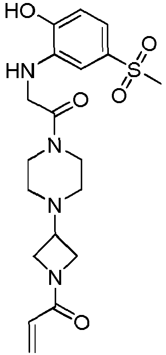 | 1-(3-(4-(2-(2-hydroxy-5-(methylsulfonyl)p henylamino)acetyl )piperazin-1-yl)azetidin-1-yl)prop-2-en-1-one |
| V-35 | 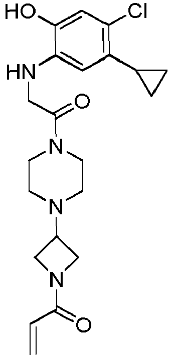 | 1-(3-(4-(2-(4-chloro-5-cyclopropyl-2-hydroxyphenylam ino)acetyl)piperaz in-1-yl)azetidin-1-yl)prop-2-en-1-one | V-36 | 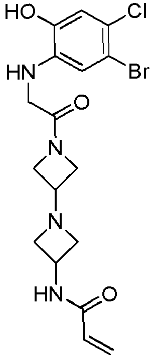 | N-(1'-(2-(5-bromo-4-chloro-2-hydroxyphenylami no)acetyl)-1,3'-biazetidin-3-yl)acrylamide |
| V-37 | 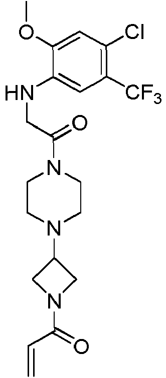 | 1-(3-(4-(2-(4-chloro-2-methoxy-5-(trifluoromethyl)p henylamino)acety l)piperazin-1-yl)azetidin-1-yl)prop-2-en-1-one | V-38 | 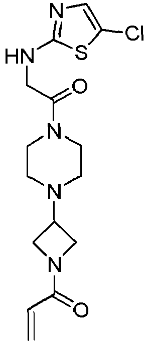 | 1-(3-(4-(2-(5-chlorothiazol-2-ylamino)acetyl)pip erazin-1-yl)azetidin-1-yl)prop-2-en-1-one |
| V-39 | 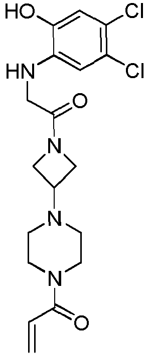 | 1-(4-(1-(2-(4,5-dichloro-2-hydroxyphenylam ino)acetyl)azetidi n-3-yl)piperazin-1-yl)prop-2-en-1-one | V-40 | 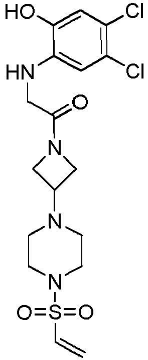 | 2-(4,5-dichloro-2-hydroxyphenylami no)-1-(3-(4-(vinylsulfonyl)pip erazin-1-yl)azetidin-1-yl)ethanone |
| V-41 | 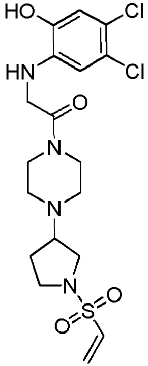 | 2-(4,5-dichloro-2-hydroxyphenylam ino)-1-(4-(1-(vinylsulfonyl)py rrolidin-3-yl)piperazin-1-yl)ethanone | V-42 | 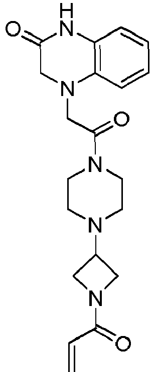 | 4-(2-(4-(1-acryloylazetidin-3 - yl)piperazin-1-yl)-2-oxoethyl)-3,4-dihydroquinoxalin -2(1H)-one |
| V-43 | 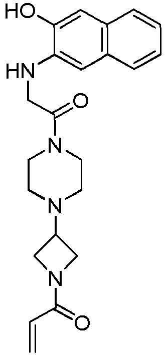 | 1-(3-(4-(2-(3-hydroxynaphthale n-2-ylamino)acetyl)pi perazin-1-yl)azetidin-1-yl)prop-2-en-1-one | V-44 | 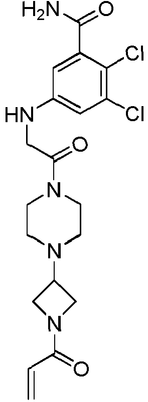 | 5-(2-(4-(1-acryloylazetidin-3-yl)piperazin-1-yl)-2-oxoethylamino)-2,3-dichlorobenzamide |
| V-45 | 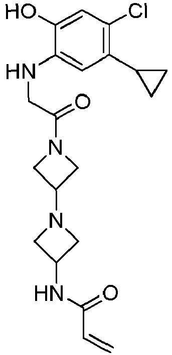 | N-(1'-(2-(4-chloro-5-cyclopropyl-2-hydroxyphenylam ino)acetyl)-1,3'-biazetidin-3-yl)acrylamide | V-46 | 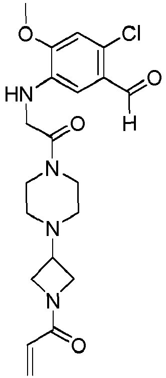 | 5-(2-(4-(1-acryloylazetidin-3-yl)piperazin-1-yl)-2-oxoethylamino)-2-chloro-4-methoxybenzaldeh yde |
| V-47 | 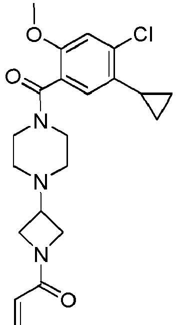 | 1-(3-(4-(4-chloro-5-cyclopropyl-2-methoxybenzoyl) piperazin-1-yl)azetidin-1-yl)prop-2-en-1-one | V-48 | 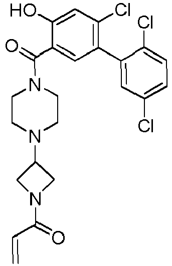 | 1-(3-(4-(2',5',6-trichloro-4-hydroxybiphenylc arbonyl)piperazin-1-yl)azetidin-1-yl)prop-2-en-1-one |
| V-49 | 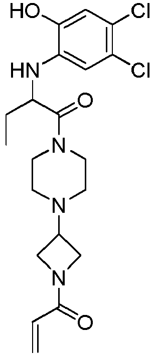 | 1-(4-(1-acryloylazetidin-3-yl)piperazin-1-yl)-2-(4,5-dichloro-2-hydroxyphenylam ino)butan-1-one | V-50 | 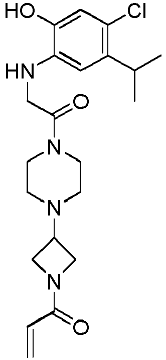 | 1-(3-(4-(2-(4-chloro-2-hydroxy-5-isopropylphenyla mino)acetyl)pipera zin-1-yl)azetidin-1-yl)prop-2-en-1-one |
| V-51 | 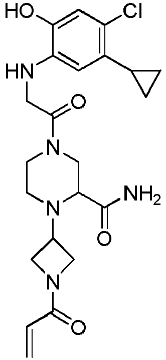 | 1-(1-acryloylazetidin-3-yl)-4-(2-(4-chloro-5-cyclopropyl-2-hydroxyphenylam ino)acetyl)piperaz ine-2-carboxamide | V-52 | 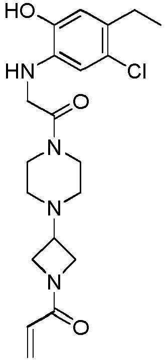 | 1-(3-(4-(2-(5-chloro-4-ethyl-2-hydroxyphenylami no)acetyl)piperazi n-1-yl)azetidin-1-yl)prop-2-en-1-one |
| V-53 | 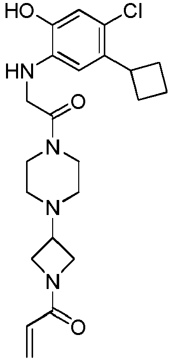 | 1-(3-(4-(2-(4-chloro-5-cyclobutyl-2-hydroxyphenylam ino)acetyl)piperaz in-1-yl)azetidin-1-yl)prop-2-en-1-one | V-54 | 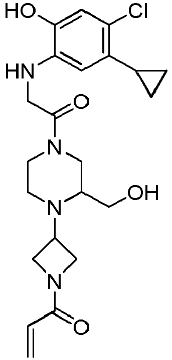 | 1-(3-(4-(2-(4-chloro-5-cyclopropyl-2-hydroxyphenylami no)acetyl)-2-(hydroxymethyl)pi perazin-1-yl)azetidin-1-yl)prop-2-en-1-one |
| V-55 | 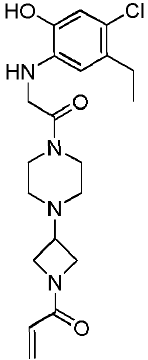 | 1-(3-(4-(2-(4-chloro-5-ethyl-2-hydroxyphenylam ino)acetyl)piperaz in-1-yl)azetidin-1-yl)prop-2-en-1-one | V-56 | 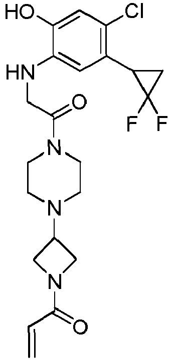 | 1-(3-(4-(2-(4-chloro-5-(2,2-difluorocycloprop yl)-2-hydroxyphenylami no)acetyl)piperazi n-1-yl)azetidin-1-yl)prop-2-en-1-one |
| V-57 | 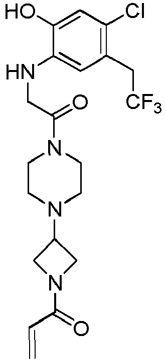 | 1-(3-(4-(2-(4-chloro-2-hydroxy-5-(2,2,2-trifluoroethyl)phe nylamino)acetyl) piperazin-1-yl)azetidin-1-yl)prop-2-en-1-one | V-58 | 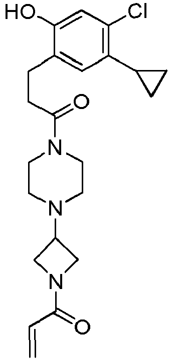 | 1-(3-(4-(3-(4-chloro-5-cyclopropyl-2-hydroxyphenyl)pr opanoyl)piperazin-1-yl)azetidin-1-yl)prop-2-en-1-one |
| V-59 | 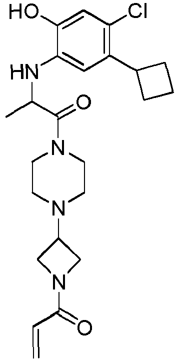 | 1-(3-(4-(2-(4-chloro-5-cyclobutyl-2-hydroxyphenylam ino)propanoyl)pip erazin-1-yl)azetidin-1-yl)prop-2-en-1-one | V-60 | 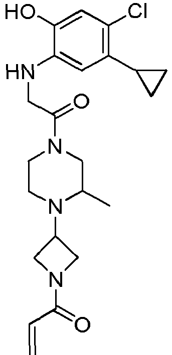 | 1-(3-(4-(2-(4-chloro-5-cyclopropyl-2-hydroxyphenylami no)acetyl)-2-methylpiperazin-1-yl)azetidin-1-yl)prop-2-en-1-one |
| V-61 | 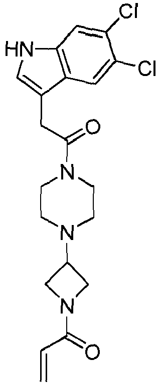 | 1-(3-(4-(2-(5,6-dichloro-1H-indol-3-yl)acetyl)piperazi n-1-yl)azetidin-1-yl)prop-2-en-1-one | V-62 | 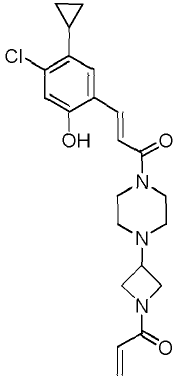 | (E)-1-(4-(1-acryloylazetidin-3 - yl)piperazin-1-yl)-3-(4-chloro-5-cyclopropyl-2-hydroxyphenyl)pr op-2-en-1-one |
| V-63 | 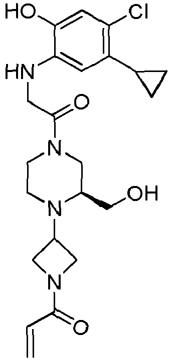 | (S)-1-(3-(4-(2-(4-chloro-5-cyclopropyl-2-hydroxyphenylam ino)acetyl)-2-(hydroxymethyl)p iperazin-1-yl)azetidin-1-yl)prop-2-en-1-one | V-64 | 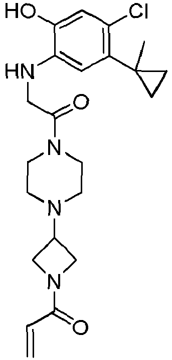 | 1-(3-(4-(2-(4-chloro-2-hydroxy-5-(1-methylcyclopropyl )phenylamino)acet yl)piperazin-1-yl)azetidin-1-yl)prop-2-en-1-one |
| V-65 | 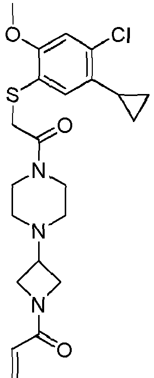 | 1-(3-(4-(2-(4-chloro-5-cyclopropyl-2-methoxyphenylthi o)acetyl)piperazin -1-yl)azetidin-1-yl)prop-2-en-1-one | V-66 | 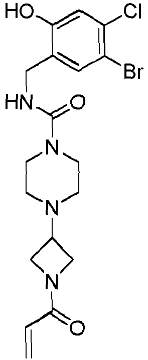 | 4-(1-acryloylazetidin-3 - yl)-N-(5-bromo-4-chloro-2-hydroxybenzyl)pip erazine-1-carboxamide |
| V-67 | 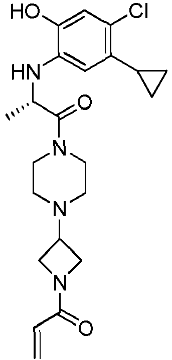 | (S)-1-(3-(4-(2-(4-chloro-5-cyclopropyl-2-hydroxyphenylam ino)propanoyl)pip erazin-1-yl)azetidin-1-yl)prop-2-en-1-one | V-68 | 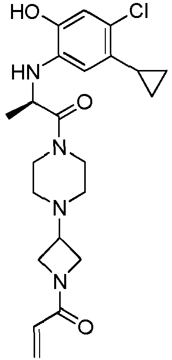 | (R)-1-(3-(4-(2-(4-chloro-5-cyclopropyl-2-hydroxyphenylami no)propanoyl)pipe razin-1-yl)azetidin-1-yl)prop-2-en-1-one |
| V-69 | 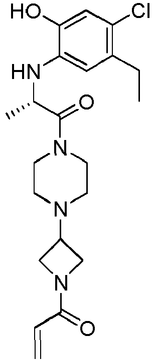 | (S)-1-(3-(4-(2-(4-chloro-5-ethyl-2-hydroxyphenylam ino)propanoyl)pip erazin-1-yl)azetidin-1-yl)prop-2-en-1-one | V-70 | 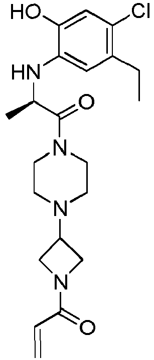 | (R)-1-(3-(4-(2-(4-chloro-5-ethyl-2-hydroxyphenylami no)propanoyl)pipe razin-1-yl)azetidin-1-yl)prop-2-en-1-one |
| V-71 | 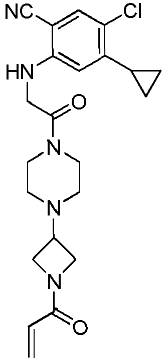 | 2-(2-(4-(1-acryloylazetidin-3-yl)piperazin-1-yl)-2-oxoethylamino)-5-chloro-4-cyclopropylbenzo nitrile | V-72 | 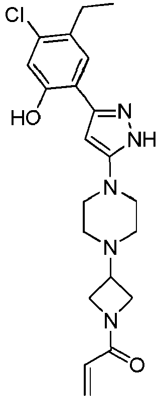 | 1-(3-(4-(3-(4-chloro-5-ethyl-2-hydroxyphenyl)-1H-pyrazol-5-yl)piperazin-1-yl)azetidin-1-yl)prop-2-en-1-one |
| V-73 | 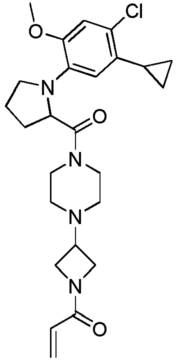 | 1-(3-(4-(1-(4-chloro-5-cyclopropyl-2-methoxyphenyl)p yrrolidine-2-carbonyl)piperazi n-1-yl)azetidin-1-yl)prop-2-en-1-one | V-74 | 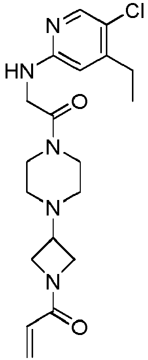 | 1-(3-(4-(2-(5-chloro-4-ethylpyridin-2-ylamino)acetyl)pip erazin-1-yl)azetidin-1-yl)prop-2-en-1-one |
| V-75 | 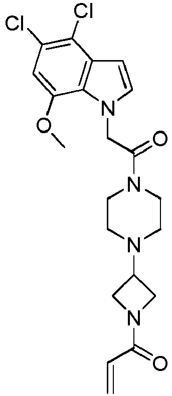 | 1-(3-(4-(2-(4,5-dichloro-7-methoxy-1H-indol-1-yl)acetyl)piperazi n-1-yl)azetidin-1-yl)prop-2-en-1-one | V-76 | 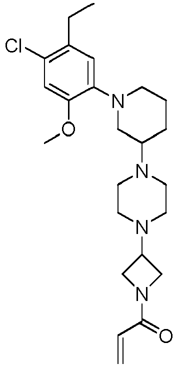 | 1-(3-(4-(1-(4-chloro-5-ethyl-2-methoxyphenyl)pi peridin-3-yl)piperazin-1-yl)azetidin-1-yl)prop-2-en-1-one |

**Table 5b**

| Experimental Mass Spectral Data for Compounds in Table 5 | | | | | | | |
|---|---|---|---|---|---|---|---|
| **No.** | **[M+H⁺]** | **No.** | **[M+H⁺]** | **No.** | **[M+H⁺]** | **No.** | **[M+H⁺]** |
| V-1 | 411.30 | V-2 | 425.25 | V-3 | 399.20 | V-4 | 427.30 |
| V-5 | 449.25* | V-6 | 410.25 | V-7 | 429.35* | V-8 | 407.35 |
| V-9 | 422.25 | V-10 | 462.25* | V-11 | 519.25 | V-12 | 443.30* |
| V-13 | 532.25* | V-14 | 395.30 | V-15 | 399.25 | V-16 | 419.25 |
| V-17 | 434.25* | V-18 | 457.35 | V-19 | 379.30 | V-20 | 470.35 |
| V-21 | 450.35 | V-22 | 433.05 | V-23 | 435.25* | V-24 | 386.25* |
| V-25 | 351.35 | V-26 | 487.30* | V-27 | 413.30 | V-28 | 479.20* |
| V-29 | 353.30 | V-30 | 421.30 | V-31 | 449.25* | V-32 | 393.30 |
| V-33 | 377.30⁺ | V-34 | 423.35 | V-35 | 441.30* | V-36 | 445.20 |
| V-37 | 461.30 | V-38 | 368.30⁺ | V-39 | 411.20 | V-40 | 447.25⁺ |
| V-41 | 463.20 | V-42 | 382.40⁺ | V-43 | 417.35* | V-44 | 440.30 |

| **No.** | **[M+H⁺]** | **No.** | **[M+H⁺]** | **No.** | **[M+H⁺]** | **No.** | **[M+H⁺]** |
|---|---|---|---|---|---|---|---|
| V-45 | 405.35 | V-46 | 421.30 | V-47 | 404.35 | V-48 | 494.30 |
| V-49 | 441.30 | V-50 | 421.35 | V-51 | 462.45 | V-52 | 407.40 |
| V-53 | 433.40 | V-54 | 449.35 | V-55 | 407.30 | V-56 | 455.20 |
| V-57 | 461.40 | V-58 | 418.40 | V-59 | 447.40 | V-60 | 433.45 |
| V-61 | 421.25 | V-62 | 416.35 | V-63 | 449.40 | V-64 | 433.35 |
| V-65 | 451.30 | V-66 | 459.25 | V-67 | 433.20 | V-68 | 433.40 |
| V-69 | 421.35 | V-70 | 421.35 | V-71 | 428.35 | V-72 | 416.35 |
| V-73 | 473.90 | V-74 | 392.30 | V-75 | 451.30 | V-76 | 447.85 |

| | | | | | | | |
|---|---|---|---|---|---|---|---|
| ^{∗} [M+Na]⁺ + [M-H]⁻ | | | | | | | |

**Table 6**

| Representative and Reference Compounds | | | |
|---|---|---|---|
| **No.** | **Structure** | **Name** | **[M+H]⁺** |
| VI-1 | 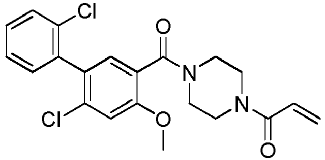 | 1-(4-(2',6-dichloro-4-methoxybiphenylcarbon yl)piperazin-1-yl)prop-2-en-1-one | 441.20* |
| VI-2 | 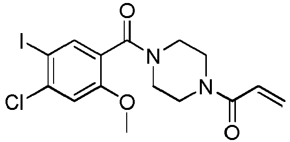 | 1-(4-(4-chloro-5-iodo-2-methoxybenzoyl)piperaz in-1 -yl)prop-2-en-1 -one | 457.05* |
| VI-3 | 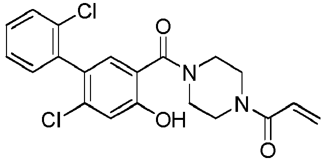 | 1-(4-(2',6-dichloro-4-hydroxybiphenylcarbon yl)piperazin-1-yl)prop-2-en-1-one | 427.15 |
| VI-4 | 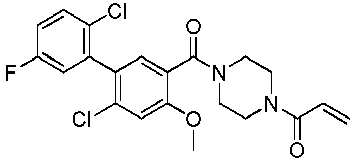 | 1-(4-(2',6-dichloro-5'-fluoro-4-methoxybiphenylcarbon yl)piperazin-1-yl)prop-2-en-1-one | 459.15 |
| VI-5 | 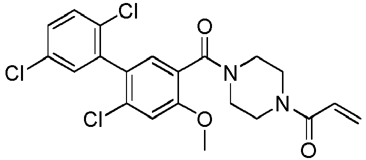 | 1-(4-(2',5',6-trichloro-4-methoxybiphenylcarbon yl)piperazin-1-yl)prop-2-en-1-one | 453.15 |
| VI-6 | 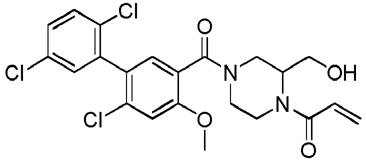 | 1-(2-(hydroxymethyl)-4-(2',5',6-trichloro-4-methoxybiphenylcarbon yl)piperazin-1-yl)prop-2-en-1-one | 483.20^{#} |
| VI-7 | 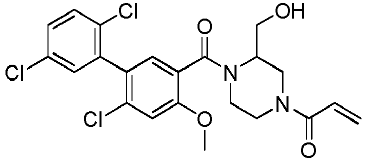 | 1-(3-(hydroxymethyl)-4-(2',5',6-trichloro-4-methoxybiphenylcarbon yl)piperazin-1-yl)prop-2-en-1-one | 485.20 |
| VI-8 | 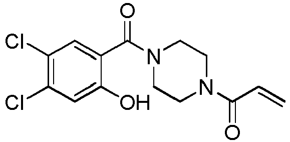 | 1-(4-(4,5-dichloro-2-hydroxybenzoyl)piperaz in-1-yl)prop-2-en-1-one | 327.15 |
| VI-9 | 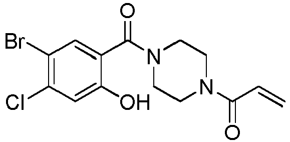 | 1-(4-(5-bromo-4-chloro-2-hydroxybenzoyl)piperaz in-1-yl)prop-2-en-1-one | 388* |
| VI-10 | 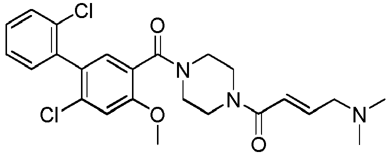 | (E)-1-(4-(2',6-dichloro-4-methoxybiphenylcarbon yl)piperazin-1-yl)-4-(dimethylamino)but-2-en-1-one | 476.23 |
| VI-11 | 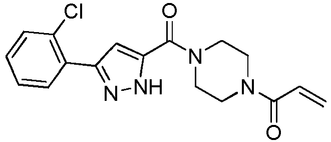 | 1-(4-(3-(2-chlorophenyl)-1H-pyrazole-5-carbonyl)piperazin-1- yl)prop-2-en-1-one | 467.20* |
| VI-12 | 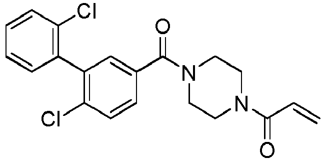 | 1-(4-(2',6-dichlorobiphenylcarbon yl)piperazin-1-yl)prop-2-en-1-one | 389.20 |
| VI-13 | 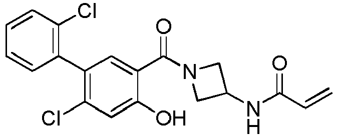 | N-(1-(2',6-dichloro-4-hydroxybiphenylcarbon yl)azetidin-3-yl)acrylamide | 389.30 |
| VI-14 | 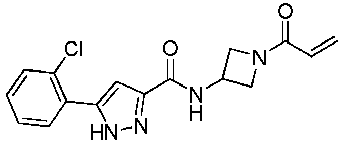 | N-(1-acryloylazetidin-3-yl)-3-(2-chlorophenyl)-1H-pyrazole-5-carboxamide | 331.15 |
| VI-15 | 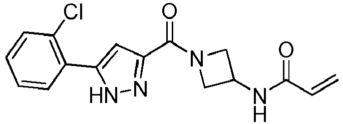 | N-(1-(3-(2-chlorophenyl)- 1H-pyrazole-5-carbonyl)azetidin-3- yl)acrylamide | 331.15 |
| VI-16 | 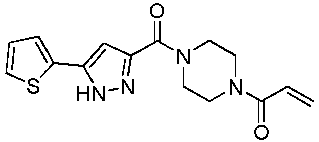 | 1-(4-(5-(thiophen-2-yl)-1H-pyrazole-3-carbonyl)piperazin-1-yl)prop-2-en-1-one | 339.15 |
| VI-17 | 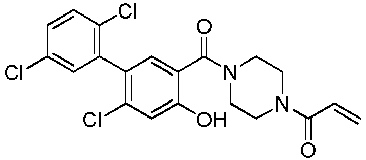 | 1-(4-(2',5',6-trichloro-4-hydroxybiphenylcarbon yl)piperazin-1-yl)prop-2-en-1-one | 438.30⁺ |
| VI-18 | 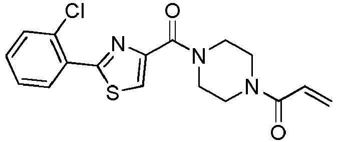 | 1-(4-(2-(2-chlorophenyl)thiazole-4-carbonyl)piperazin-1- yl)prop-2-en-1 -one | 384.15⁺ |
| VI-19 | 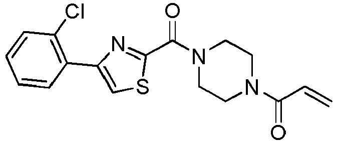 | 1-(4-(4-(2-chlorophenyl)thiazole-2-carbonyl)piperazin-1- yl)prop-2-en-1-one | 384.15⁺ |
| VI-20 | 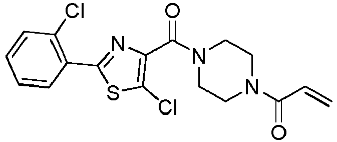 | 1-(4-(5-chloro-2-(2-chlorophenyl)thiazole-4-carbonyl)piperazin-1- yl)prop-2-en-1-one | 418.10* |
| VI-21 | 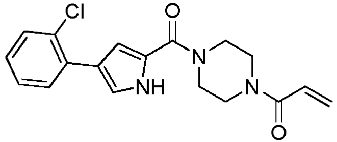 | 1-(4-(4-(2-chlorophenyl)-1H-pyrrole-2-carbonyl)piperazin-1- yl)prop-2-en-1-one | 344.20 |
| VI-22 | 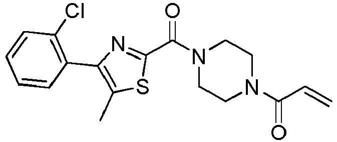 | 1-(4-(4-(2-chlorophenyl)-5-methylthiazole-2-carbonyl)piperazin-1- yl)prop-2-en-1-one | 376.25 |
| VI-23 | 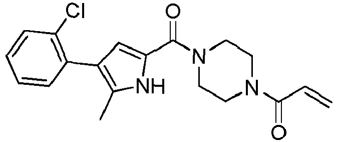 | 1-(4-(4-(2-chlorophenyl)-5 -methyl-1H-pyrrole-2-carbonyl)piperazin-1- yl)prop-2-en-1-one | 356.35⁺ |
| VI-24 | 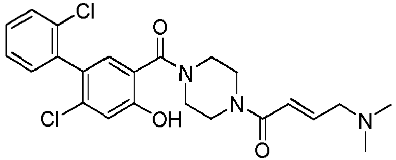 | (E)-1-(4-(2',6-dichloro-4-hydroxybiphenylcarbon yl)piperazin-1-yl)-4-(dimethylamino)but-2-en-1-one | 462.35 |
| VI-25 | 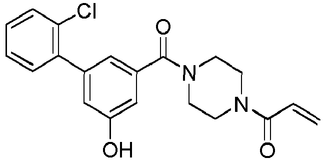 | 1-(4-(2'-chloro-5-hydroxybiphenylcarbon yl)piperazin-1-yl)prop-2-en-1-one | 371.25 |
| VI-26 | 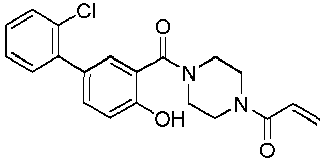 | 1-(4-(2'-chloro-4-hydroxybiphenylcarbon yl)piperazin-1-yl)prop-2-en-1-one | 371.25 |
| VI-27 | 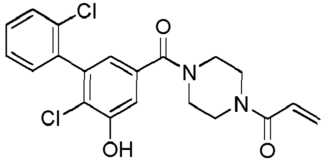 | 1-(4-(2',6-dichloro-5-hydroxybiphenylcarbon yl)piperazin-1-yl)prop-2-en-1-one | 403.35⁺ |
| VI-28 | 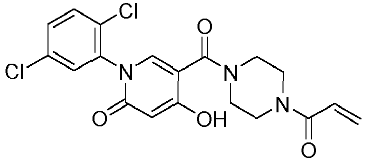 | 5 -(4-acryloylpiperazine-1-carbonyl)-1-(2,5-dichlorophenyl)-4-hydroxypyridin-2(1H)-one | 422.06 |
| VI-29 | 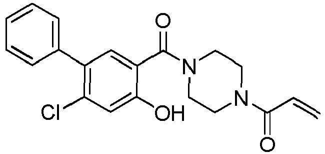 | 1-(4-(6-chloro-4-hydroxybiphenylcarbon yl)piperazin-1-yl)prop-2-en-1-one | 371.25 |
| VI-30 | 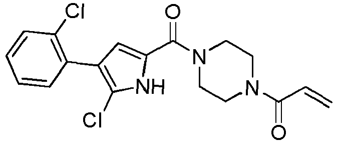 | 1-(4-(5-chloro-4-(2-chlorophenyl)-1H-pyrrole-2-carbonyl)piperazin-1- yl)prop-2-en-1-one | 378.25⁺ |
| VI-31 | 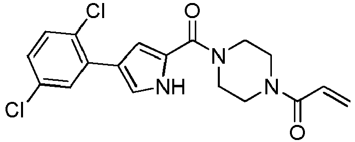 | 1-(4-(4-(2,5-dichlorophenyl)-1H-pyrrole-2-carbonyl)piperazin-1- yl)prop-2-en-1-one | 378.20 |
| VI-32 | 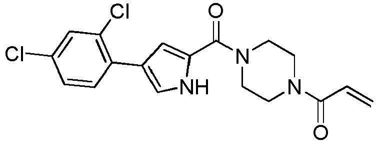 | 1-(4-(4-(2,4-dichlorophenyl)-1H-pyrrole-2-carbonyl)piperazin-1- yl)prop-2-en-1-one | 377.90 |
| VI-33 | 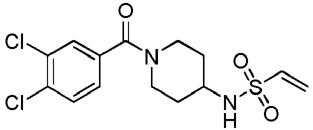 | N-(1-(3,4-dichlorobenzoyl)piperidi n-4-yl)ethenesulfonamide | 363.02 |
| VI-34 | 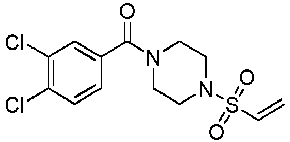 | (3,4-dichlorophenyl)(4-(vinylsulfonyl)piperazin -1-yl)methanone | 349.04 |
| VI-35 | 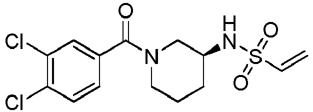 | (S)-N-(1-(3,4-dichlorobenzoyl)piperidi n-3-yl)ethenesulfonamide | 363.06 |
| VI-36 | 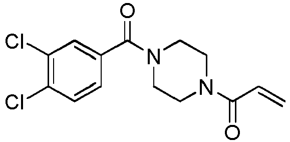 | 1-(4-(3,4-dichlorobenzoyl)piperaz in-1-yl)prop-2-en-1-one | 312.99 |
| VI-37 | 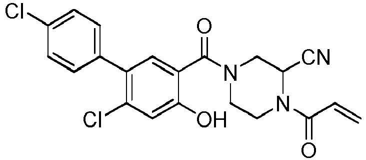 | 1-acryloyl-4-(4',6-dichloro-4-hydroxybiphenylcarbon yl)piperazine-2-carbonitrile | 430.30 |
| VI-38 | 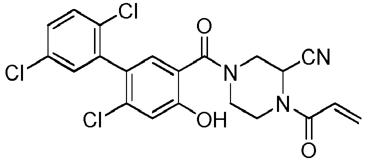 | 1-acryloyl-4-(2',5',6-trichloro-4-hydroxybiphenylcarbon yl)piperazine-2-carbonitrile | 464.30 |
| VI-39 | 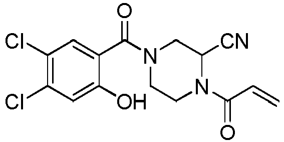 | 1-acryloyl-4-(4,5-dichloro-2-hydroxybenzoyl)piperaz ine-2-carbonitrile | 354.15 |
| VI-40 | 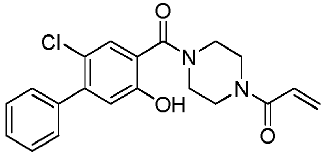 | 1-(4-(2-chloro-5-hydroxybiphenylcarbon yl)piperazin-1-yl)prop-2-en-1-one | 371.25 |
| VI-41 | 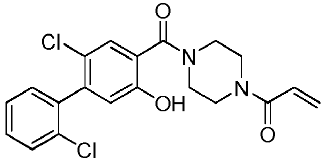 | 1-(4-(2,2'-dichloro-5-hydroxybiphenylcarbon yl)piperazin-1-yl)prop-2-en-1-one | 405.25 |
| VI-42 | 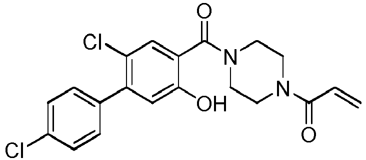 | 1-(4-(2,4'-dichloro-5-hydroxybiphenylcarbon yl)piperazin-1-yl)prop-2-en-1-one | 405.20 |
| VI-43 | 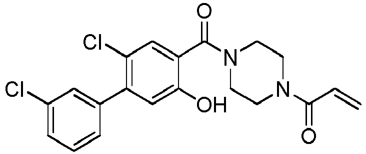 | 1-(4-(2,3'-dichloro-5-hydroxybiphenylcarbon yl)piperazin-1-yl)prop-2-en-1-one | 405.25 |

| | | | |
|---|---|---|---|
| ^{∗} [M+Na]⁺ + [M-H]⁻ # [M] | | | |

### Pharmaceutical Compositions

Other embodiments are directed to pharmaceutical compositions. The pharmaceutical composition comprises any one (or more) of the foregoing compounds and a pharmaceutically acceptable carrier. In some embodiments, the pharmaceutical composition is formulated for oral administration. In other embodiments, the pharmaceutical composition is formulated for injection. In still more embodiments, the pharmaceutical compositions comprise a compound as disclosed herein and an additional therapeutic agent (e.g., anticancer agent). Examples of such therapeutic agents are described herein below.

Suitable routes of administration include oral, intravenous, rectal, aerosol, parenteral, ophthalmic, pulmonary, transmucosal, transdermal, vaginal, otic, nasal, and topical administration. In addition, by way of example only, parenteral delivery includes intramuscular, subcutaneous, intravenous, intramedullary injections, as well as intrathecal, direct intraventricular, intraperitoneal, intralymphatic, and intranasal injections.

In certain embodiments, a compound as described herein is administered in a local rather than systemic manner, for example, via injection of the compound directly into an organ, often in a depot preparation or sustained release formulation. In specific embodiments, long acting formulations are administered by implantation (for example subcutaneously or intramuscularly) or by intramuscular injection. Furthermore, in other embodiments, the drug is delivered in a targeted drug delivery system, for example, in a liposome coated with organ-specific antibody. In such embodiments, the liposomes are targeted to and taken up selectively by the organ. In yet other embodiments, the compound as described herein is provided in the form of a rapid release formulation, in the form of an extended release formulation, or in the form of an intermediate release formulation. In yet other embodiments, the compound described herein is administered topically.

The compounds according to the invention are effective over a wide dosage range. For example, in the treatment of adult humans, dosages from 0.01 to 1000 mg, from 0.5 to 100 mg, from 1 to 50 mg per day, and from 5 to 40 mg per day are examples of dosages that are used in some embodiments. An exemplary dosage is 10 to 30 mg per day. The exact dosage will depend upon the route of administration, the form in which the compound is administered, the subject to be treated, the body weight of the subject to be treated, and the preference and experience of the attending physician.

In some embodiments, a compound of the invention is administered in a single dose. Typically, such administration will be by injection, e.g., intravenous injection, in order to introduce the agent quickly. However, other routes are used as appropriate. In some embodiments, a single dose of a compound of the invention is used for treatment of an acute condition.

In some embodiments, a compound of the invention is administered in multiple doses. In some embodiments, dosing is about once, twice, three times, four times, five times, six times, or more than six times per day. In other embodiments, dosing is about once a month, once every two weeks, once a week, or once every other day. In another embodiment a compound of the invention and another agent are administered together about once per day to about 6 times per day. In another embodiment the administration of a compound of the invention and an agent continues for less than about 7 days. In yet another embodiment the administration continues for more than about 6, 10, 14, 28 days, two months, six months, or one year. In some cases, continuous dosing is achieved and maintained as long as necessary.

Administration of the compounds of the invention may continue as long as necessary. In some embodiments, a compound of the invention is administered for more than 1, 2, 3, 4, 5, 6, 7, 14, or 28 days. In some embodiments, a compound of the invention is administered for less than 28, 14, 7, 6, 5, 4, 3, 2, or 1 day. In some embodiments, a compound of the invention is administered chronically on an ongoing basis, e.g., for the treatment of chronic effects.

In some embodiments, the compounds of the invention are administered in dosages. It is known in the art that due to intersubject variability in compound pharmacokinetics, individualization of dosing regimen is necessary for optimal therapy. Dosing for a compound of the invention may be found by routine experimentation in light of the instant disclosure.

In some embodiments, the compounds described herein are formulated into pharmaceutical compositions. In specific embodiments, pharmaceutical compositions are formulated in a conventional manner using one or more physiologically acceptable carriers comprising excipients and auxiliaries which facilitate processing of the active compounds into preparations which can be used pharmaceutically. Proper formulation is dependent upon the route of administration chosen. Any pharmaceutically acceptable techniques, carriers, and excipients are used as suitable to formulate the pharmaceutical compositions described herein: Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins1999).

In another embodiment, compounds described herein are formulated for oral administration. Compounds described herein are formulated by combining the active compounds with, e.g., pharmaceutically acceptable carriers or excipients. In various embodiments, the compounds described herein are formulated in oral dosage forms that include, by way of example only, tablets, powders, pills, dragees, capsules, liquids, gels, syrups, elixirs, slurries, and suspensions.

In certain embodiments, pharmaceutical preparations for oral use are obtained by mixing one or more solid excipient with one or more of the compounds described herein, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as: for example, maize starch, wheat starch, rice starch, potato starch, gelatin, gum tragacanth, methylcellulose, microcrystalline cellulose, hydroxypropylmethylcellulose, sodium carboxymethylcellulose; or others such as: polyvinylpyrrolidone (PVP or povidone) or calcium phosphate. In specific embodiments, disintegrating agents are optionally added. Disintegrating agents include, by way of example only, cross-linked croscarmellose sodium, polyvinylpyrrolidone, agar, or alginic acid or a salt thereof such as sodium alginate.

In one embodiment, dosage forms, such as dragee cores and tablets, are provided with one or more suitable coating. In specific embodiments, concentrated sugar solutions are used for coating the dosage form. The sugar solutions, optionally contain additional components, such as by way of example only, gum arabic, talc, polyvinylpyrrolidone, carbopol gel, polyethylene glycol, and/or titanium dioxide, lacquer solutions, and suitable organic solvents or solvent mixtures. Dyestuffs and/or pigments are also optionally added to the coatings for identification purposes. Additionally, the dyestuffs and/or pigments are optionally utilized to characterize different combinations of active compound doses.

In certain embodiments, therapeutically effective amounts of at least one of the compounds described herein are formulated into other oral dosage forms. Oral dosage forms include push-fit capsules made of gelatin, as well as soft, sealed capsules made of gelatin and a plasticizer, such as glycerol or sorbitol. In specific embodiments, push-fit capsules contain the active ingredients in admixture with one or more filler. Fillers include, by way of example only, lactose, binders such as starches, and/or lubricants such as talc or magnesium stearate and, optionally, stabilizers. In other embodiments, soft capsules, contain one or more active compound that is dissolved or suspended in a suitable liquid. Suitable liquids include, by way of example only, one or more fatty oil, liquid paraffin, or liquid polyethylene glycol. In addition, stabilizers are optionally added.

In other embodiments, therapeutically effective amounts of at least one of the compounds described herein are formulated for buccal or sublingual administration. Formulations suitable for buccal or sublingual administration include, by way of example only, tablets, lozenges, or gels. In still other embodiments, the compounds described herein are formulated for parental injection, including formulations suitable for bolus injection or continuous infusion. In specific embodiments, formulations for injection are presented in unit dosage form (e.g., in ampoules) or in multi-dose containers. Preservatives are, optionally, added to the injection formulations. In still other embodiments, the pharmaceutical compositions are formulated in a form suitable for parenteral injection as sterile suspensions, solutions or emulsions in oily or aqueous vehicles. Parenteral injection formulations optionally contain formulatory agents such as suspending, stabilizing and/or dispersing agents. In specific embodiments, pharmaceutical formulations for parenteral administration include aqueous solutions of the active compounds in water-soluble form. In additional embodiments, suspensions of the active compounds (e.g., compounds of structure (I)) are prepared as appropriate oily injection suspensions. Suitable lipophilic solvents or vehicles for use in the pharmaceutical compositions described herein include, by way of example only, fatty oils such as sesame oil, or synthetic fatty acid esters, such as ethyl oleate or triglycerides, or liposomes. In certain specific embodiments, aqueous injection suspensions contain substances which increase the viscosity of the suspension, such as sodium carboxymethyl cellulose, sorbitol, or dextran. Optionally, the suspension contains suitable stabilizers or agents which increase the solubility of the compounds to allow for the preparation of highly concentrated solutions. Alternatively, in other embodiments, the active ingredient is in powder form for constitution with a suitable vehicle, e.g., sterile pyrogen-free water, before use.

Methods for the preparation of compositions comprising the compounds described herein include formulating the compounds with one or more inert, pharmaceutically acceptable excipients or carriers to form a solid, semi-solid or liquid. Solid compositions include powders, tablets, dispersible granules, capsules, cachets, and suppositories. Liquid compositions include solutions in which a compound is dissolved, emulsions comprising a compound, or a solution containing liposomes, micelles, or nanoparticles comprising a compound as disclosed herein. Semi-solid compositions include gels, suspensions and creams. The form of the pharmaceutical compositions described herein include liquid solutions or suspensions, solid forms suitable for solution or suspension in a liquid prior to use, or as emulsions. These compositions also optionally contain minor amounts of nontoxic, auxiliary substances, such as wetting or emulsifying agents, pH buffering agents, and so forth.

In certain embodiments, useful aqueous suspensions contain one or more polymers as suspending agents. Useful polymers include water-soluble polymers such as cellulosic polymers, e.g., hydroxypropyl methylcellulose, and water-insoluble polymers such as cross-linked carboxyl-containing polymers. Certain pharmaceutical compositions described herein comprise a mucoadhesive polymer, selected for example from carboxymethylcellulose, carbomer (acrylic acid polymer), poly(methylmethacrylate), polyacrylamide, polycarbophil, acrylic acid/butyl acrylate copolymer, sodium alginate and dextran.

Furthermore, useful pharmaceutical compositions optionally include one or more pH adjusting agents or buffering agents, including acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate and ammonium chloride. Such acids, bases and buffers are included in an amount required to maintain pH of the composition in an acceptable range.

Additionally, useful compositions also, optionally, include one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

Other useful pharmaceutical compositions optionally include one or more preservatives to inhibit microbial activity. Suitable preservatives include mercury-containing substances such as merfen and thiomersal; stabilized chlorine dioxide; and quaternary ammonium compounds such as benzalkonium chloride, cetyltrimethylammonium bromide and cetylpyridinium chloride.

Still other useful compositions include one or more surfactants to enhance physical stability or for other purposes. Suitable nonionic surfactants include polyoxyethylene fatty acid glycerides and vegetable oils, e.g., polyoxyethylene (60) hydrogenated castor oil; and polyoxyethylene alkylethers and alkylphenyl ethers, e.g., octoxynol 10, octoxynol 40.

Still other useful compositions include one or more antioxidants to enhance chemical stability where required. Suitable antioxidants include, by way of example only, ascorbic acid and sodium metabisulfite.

In certain embodiments, aqueous suspension compositions are packaged in single-dose non-reclosable containers. Alternatively, multiple-dose reclosable containers are used, in which case it is typical to include a preservative in the composition.

In alternative embodiments, other delivery systems for hydrophobic pharmaceutical compounds are employed. Liposomes and emulsions are examples of delivery vehicles or carriers useful herein. In certain embodiments, organic solvents such as N-methylpyrrolidone are also employed. In additional embodiments, the compounds described herein are delivered using a sustained-release system, such as semipermeable matrices of solid hydrophobic polymers containing the therapeutic agent. Various sustained-release materials are useful herein. In some embodiments, sustained-release capsules release the compounds for a few weeks up to over 100 days. Depending on the chemical nature and the biological stability of the therapeutic reagent, additional strategies for protein stabilization are employed.

In certain embodiments, the formulations described herein comprise one or more antioxidants, metal chelating agents, thiol containing compounds and/or other general stabilizing agents. Examples of such stabilizing agents, include: (a) about 0.5% to about 2% w/v glycerol, (b) about 0.1% to about 1% w/v methionine, (c) about 0.1% to about 2% w/v monothioglycerol, (d) about 1 mM to about 10 mM EDTA, (e) about 0.01% to about 2% w/v ascorbic acid, (f) 0.003% to about 0.02% w/v polysorbate 80, (g) 0.001% to about 0.05% w/v. polysorbate 20, (h) arginine, (i) heparin, (j) dextran sulfate, (k) cyclodextrins, (1) pentosan polysulfate and other heparinoids, (m) divalent cations such as magnesium and zinc; or (n) combinations thereof.

In some embodiments, the concentration of one or more compounds provided in the pharmaceutical compositions of the present invention is less than 100%, 90%, 80%, 70%, 60%, 50%, 40%, 30%, 20%, 19%, 18%, 17%, 16%, 15%,14%, 13%, 12%, 11%, 10%, 9%, 8%, 7%, 6%, 5%, 4%, 3%, 2%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% w/w, w/v or vivo

In some embodiments, the concentration of one or more compounds of the invention is greater than 90%, 80%, 70%, 60%, 50%, 40%, 30%, 20%, 19.75%, 19.50%, 19.25% 19%, 18.75%, 18.50%, 18.25% 18%, 17.75%, 17.50%, 17.25% 17%, 16.75%, 16.50%, 16.25% 16%, 15.75%, 15.50%, 15.25% 15%, 14.75%, 14.50%, 14.25% 14%, 13.75%, 13.50%, 13.25% 13%, 12.75%, 12.50%, 12.25% 12%, 11.75%, 11.50%, 11.25% 11%, 10.75%, 10.50%, 10.25% 10%, 9.75%, 9.50%, 9.25% 9%, 8.75%, 8.50%, 8.25% 8%, 7.75%, 7.50%, 7.25% 7%, 6.75%, 6.50%, 6.25% 6%, 5.75%, 5.50%, 5.25% 5%, 4.75%, 4.50%, 4.25%, 4%, 3.75%, 3.50%, 3.25%, 3%, 2.75%, 2.50%, 2.25%, 2%, 1.75%, 1.50%, 125% , 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% w/w, w/v, or v/v.

In some embodiments, the concentration of one or more compounds of the invention is in the range from approximately 0.0001% to approximately 50%, approximately 0.001% to approximately 40 %, approximately 0.01% to approximately 30%, approximately 0.02% to approximately 29%, approximately 0.03% to approximately 28%, approximately 0.04% to approximately 27%, approximately 0.05% to approximately 26%, approximately 0.06% to approximately 25%, approximately 0.07% to approximately 24%, approximately 0.08% to approximately 23%, approximately 0.09% to approximately 22%, approximately 0.1% to approximately 21%, approximately 0.2% to approximately 20%, approximately 0.3% to approximately 19%, approximately 0.4% to approximately 18%, approximately 0.5% to approximately 17%, approximately 0.6% to approximately 16%, approximately 0.7% to approximately 15%, approximately 0.8% to approximately 14%, approximately 0.9% to approximately 12%, approximately 1% to approximately 10% w/w, w/v or v/v.

In some embodiments, the concentration of one or more compounds of the invention is in the range from approximately 0.001% to approximately 10%, approximately 0.01% to approximately 5%, approximately 0.02% to approximately 4.5%, approximately 0.03% to approximately 4%, approximately 0.04% to approximately 3.5%, approximately 0.05% to approximately 3%, approximately 0.06% to approximately 2.5%, approximately 0.07% to approximately 2%, approximately 0.08% to approximately 1.5%, approximately 0.09% to approximately 1%, approximately 0.1% to approximately 0.9% w/w, w/v or v/v.

In some embodiments, the amount of one or more compounds of the invention is equal to or less than 10 g, 9.5 g, 9.0 g, 8.5 g, 8.0 g, 7.5 g, 7.0 g, 6.5 g, 6.0 g, 5.5 g, 5.0 g, 4.5 g, 4.0 g, 3.5 g, 3.0 g, 2.5 g, 2.0 g, 1.5 g, 1.0 g, 0.95 g, 0.9 g, 0.85 g, 0.8 g, 0.75 g, 0.7 g, 0.65 g, 0.6 g, 0.55 g, 0.5 g, 0.45 g, 0.4 g, 0.35 g, 0.3 g, 0.25 g, 0.2 g, 0.15 g, 0.1 g, 0.09 g, 0.08 g, 0.07 g, 0.06 g, 0.05 g, 0.04 g, 0.03 g, 0.02 g, 0.01 g, 0.009 g, 0.008 g, 0.007 g, 0.006 g, 0.005 g, 0.004 g, 0.003 g, 0.002 g, 0.001 g, 0.0009 g, 0.0008 g, 0.0007 g, 0.0006 g, 0.0005 g, 0.0004 g, 0.0003 g, 0.0002 g, or 0.0001 g.

In some embodiments, the amount of one or more compounds of the invention is more than 0.0001 g, 0.0002 g, 0.0003 g, 0.0004 g, 0.0005 g, 0.0006 g, 0.0007 g, 0.0008 g, 0.0009 g, 0.001 g, 0.0015 g, 0.002 g, 0.0025 g, 0.003 g, 0.0035 g, 0.004 g, 0.0045 g, 0.005 g, 0.0055 g, 0.006 g, 0.0065 g, 0.007 g, 0.0075 g, 0.008 g, 0.0085 g, 0.009 g, 0.0095 g, 0.01 g, 0.015 g, 0.02 g, 0.025 g, 0.03 g, 0.035 g, 0.04 g, 0.045 g, 0.05 g, 0.055 g, 0.06 g, 0.065 g, 0.07 g, 0.075 g, 0.08 g, 0.085 g, 0.09 g, 0.095 g, 0.1 g, , 0.15 g, 0.2 g, , 0.25 g, 0.3 g, , 0.35 g, 0.4 g, , 0.45 g, 0.5 g, 0.55 g, 0.6 g, , 0.65 g, 0.7 g, 0.75 g, 0.8 g, 0.85 g, 0.9 g, 0.95 g, 1 g, 1.5 g, 2 g, 2.5, 3 g, 3.5, 4 g, 4.5 g, 5 g, 5.5 g, 6 g, 6.5g, 7 g, 7.5g, 8 g, 8.5 g, 9 g, 9.5 g, or 10 g.

In some embodiments, the amount of one or more compounds of the invention is in the range of 0.0001-10 g, 0.0005-9 g, 0.001-8 g, 0.005-7 g, 0.01-6 g, 0.05-5 g, 0.1-4 g, 0.5-4 g, or 1-3 g.

The examples and preparations provided below further illustrate and exemplify the compounds of the present invention and methods of preparing such compounds. In the following examples, and throughout the specification and claims, molecules with a single chiral center, unless otherwise noted, exist as a racemic mixture. Those molecules with two or more chiral centers, unless otherwise noted, exist as a racemic mixture of diastereomers. Single enantiomers/diastereomers may be obtained by methods known to those skilled in the art.

### EXAMPLES

### EXAMPLE 1

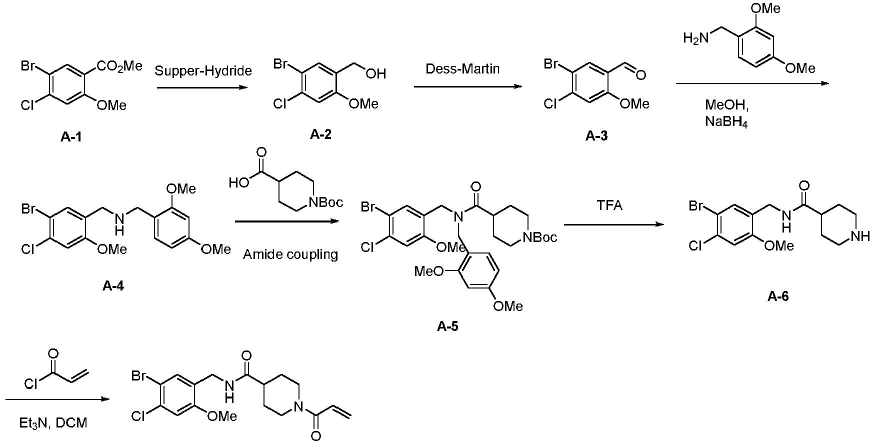

### 5-Bromo-4-chloro-2-methoxyphenyl)methanol

To a solution of methyl 5-bromo-4-chloro-2-methoxybenzoate (1.0 g, 3.6 mmol) in dry THF (10 mL) at 0°C, supper-Hydride (1.0 M, 10 mL) was added and the resulting mixture was stirred at room temperature overnight. The mixture was partitioned between ethyl acetate and water. The organic layer was dried over Na₂SO₄, filtered and concentrated *in vacuo* to afford the desired product. The crude product was used directly in the next step without further purification.

### 5-Bromo-4-chloro-2-methoxybenzaldehyde

To the solution of 5-bromo-4-chloro-2-methoxyphenyl)methanol in dry DCM (5 mL), Dess-Martin reagent (607 mg) was added and the resulting mixture was stirred at room temperature overnight. The mixture was partitioned between DCM and water. The organic layer was dried over Na₂SO₄, filtered and concentrated *in vacuo.* The residue was purified via Isolera One (silica cartridge, 0-60% ethyl acetate/hexanes) to afford the desired product (200 mg, 56% yield). ¹H NMR (300 MHz, CDCl₃) δ: **10.33 (s, 1H),** 8.04 (s, 1H), 7.12 (s, 1H), 3.39 (s, 3H).

### N-(5-bromo-4-chloro-2-methoxybenzyl)-1-(2,4-dimethoxyphenyl)methanamine

The solution of 5-bromo-4-chloro-2-methoxybenzaldehyde (300 mg, 1.2 mmol) and 2,4-dimethoxybenzyl amine (167 mg, 1.2mmol) in MeOH/DCM (4 : 1, 10 mL) was stirred at room temperature for 5 h. To this mixture, NaBH₄ (100 mg, 13 mmol) was added in two portions. The mixture stirred for 1 h and then partitioned between DCM and water. The organic layer was washed with brine, dried over Na₂SO₄, filtered and concentrated in vacuo to afford the desired product. The crude product was used directly in the next step without further purification.

### tert-Butyl 4-((5-bromo-4-chloro-2-methoxybenzyl)(2,4-dimethoxybenzyl)carbamoyl)piperidine-1-carboxylate

The mixture of N-(5-bromo-4-chloro-2-methoxybenzyl)-1-(2,4-dimethoxyphenyl)methanamine (190 mg, 0.47 mmol), 1-(tert-butoxycarbonyl)piperidine-4-carboxylic acid (109 mg, 0.47 mmol), HATU (180 mg, 0.47 mmol) and Et₃N (101 mg, 1 mmol) in DMF (5 mL) was stirred at room temperature for 4 h. The mixture was partitioned between ethyl acetate and water. The organic layer was washed with brine, dried over Na₂SO₄, filtered and concentrated *in vacuo* to afford the desired product. The crude product was used directly in the next step without further purification.

### N-(5-Bromo-4-chloro-2-methoxybenzyl)piperidine-4-carboxamide

The crude tert-Butyl 4-((5-bromo-4-chloro-2-methoxybenzyl)(2,4-dimethoxybenzyl)carbamoyl)piperidine-1-carboxylate was dissolved in 50% TFA in DCM (10 mL) and the resulting mixture was stirred at room temperature for 2 h. The mixture was concentrated in vacuo and the residue was partitioned between DCM and saturate NaHCO₃ aqueous solution. The organic layer was washed with brine, dried over Na₂SO₄, filtered and concentrated *in vacuo* to afford the desired product.

### 1-Acryloyl-N-(5-bromo-4-chloro-2-methoxybenzyl)piperidine-4-carboxamide

To a solution of N-(5-Bromo-4-chloro-2-methoxybenzyl)piperidine-4-carboxamide obtained from above step in DCM (10 mL) at 0°C, Et₃N (0.2 mL, 1.43 mmol) and acryloyl chloride (36.2 mg, 0.4 mmol) were added sequentially and the mixture was stirred at room temperature for 1 h. The mixture was partitioned between

DCM and water. The organic layer was washed with brine, dried over Na₂SO₄, filtered and concentrated in vacuo. The residue was purified via Isolera One (silica cartridge, 0-3% MeOH/DCM) to afford the desired product (13 mg). ¹H NMR **(300 MHz, DMSO-*d6*) δ: 8.27 (t, *J* = 5.6 Hz, 1H), 7.37 (s, 1H), 7.26 (s, 1H), 6.81 (dd, *J* = 10.4, 18.8 Hz, 1H), 6.08 (dd, *J* = 2.4, 16.7 Hz, 1H), 5.66 (dd, *J* = 2.4, 10.4 Hz, 1H), 4.40 (d, *J* = 12.8 Hz, 1H), 4.17 (d, *J* = 5.8 Hz, 2H), 4.07 (d, *J* = 13.1 Hz, 1H), 3.83 (s, 3H), 3.07 (t, *J* = 12.0 Hz, 1 H), 2.68 (t, *J* = 12.7 Hz, 1 H), 2.48-2.50 (m, 1H), 1.75 (d, *J* = 10.8 Hz, 2 H), 1.47-1.51 (m, 2H). ESI-MS *m*/*z*: 417.05 [M+H]⁺.**

### EXAMPLE 2

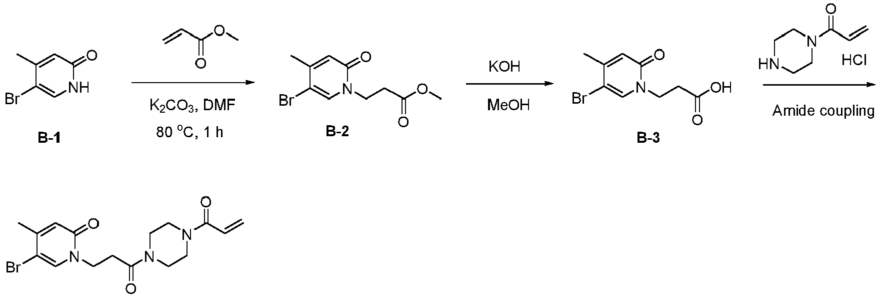

### Methyl 3-(5-bromo-4-methyl-2-oxopyridin-1(2H)-yl)propanoate

To a solution of 5-bromo-4-methylpyridin-2(1H)-one (1.0 g, 5.3 mmol) in DMF (10 mL), K₂CO₃ (1.5 g, 10.6 mmol) was added, followed by addition of methyl acrylate (0.91 g, 10.6 mmol) at 80°C. The mixture was stirred at 80°C for 1 h. The mixture was allowed to cool to room temperature and then partitioned between ethyl acetate and water. The organic layer was washed with brine, dried over Na₂SO₄, filtered and concentrated *in vacuo.* The residue was used directly in the next step without further purification.

### 3-(5-Bromo-4-methyl-2-oxopyridin-1(2H)-yl)propanoic acid

To a solution of the crude product from above step in MeOH (20 mL), aqueous KOH (2M, 5 mL) was added and the resulting mixture was stirred at room temperature overnight. The mixture was acidified with con. HCl and then extracted with ethyl acetate. The organic layer was washed with brine, dried over Na₂SO₄, filtered and concentrated *in vacuo.* The crude product was used directly in the next step without further purification.

### 1-(3-(4-Acryloylpiperazin-1-yl)-3-oxopropyl)-5-bromo-4-methylpyridin-2(1H)-one

A mixture of 3-(5-Bromo-4-methyl-2-oxopyridin-1(2H)-yl)propanoic acid (112 mg, 0.43 mmol), 1-(piperazin-1-yl)prop-2-en-1-one (80 mg, 0.43 mmol), HATU (163 mg, 0.43 mmol) and Et₃N (0.2 mL, 1.43 mmol) in DMF (5 mL) was stirred at room temperature for 2 h. The mixture was partitioned between ethyl acetate and water. The organic layer was washed with brine, dried over Na₂SO₄, filtered and concentrated *in vacuo.* The residue was purified via Isolera One (silica cartridge, 0-6% MeOH/DCM) to afford the desired product (22.8 mg, 14 yield). ¹H NMR **(300 MHz, CDCl₃) δ: 7.73 (s, 1H), 6.55 (dd, *J* = 10.5, 16.7 Hz, 1H), 6.46 (s, 1H), 6.32 (dd, *J* = 1.8, 16.8 Hz, 1H), 5.75 (dd, *J* = 1.8, 10.5 Hz, 1H), 4.20 (t, *J* = 6.0 Hz, 2H), 3.4-3.6 (m, 8H), 2.86 (t, *J* = 6.0 Hz, 2H), 2.22 (s, 3H). ESI-MS *m*/*z*: 382.05 [M+H]⁺.**

### EXAMPLE 3

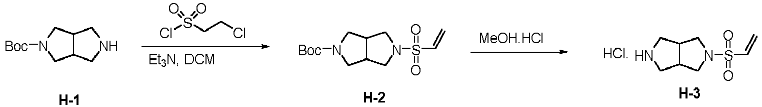

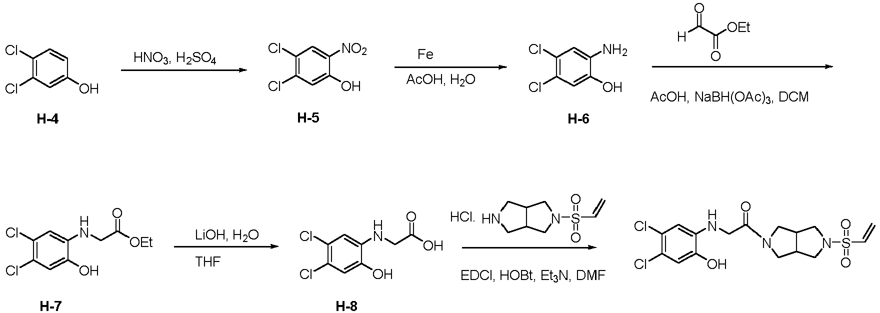

### tert-Butyl 5-(vinylsulfonyl)hexahydropyrrolo [3,4-c] pyrrole-2(1H)-carboxylate

To a stirred mixture of tert-butyl hexahydropyrrolo[3,4-c]pyrrole-2(1H)-carboxylate (1.0 g, 4.71 mmol) in DCM (20 mL) at 0°C, Et₃N (1.43 g, 14.1 mmol) was added. The mixture was stirred at 0°C for 5 min, and then a solution of 2-chloroethanesulfonyl chloride (0.77 g, 4.71 mmol) in DCM (5 mL) was added dropwise. The resulting mixture was stirred at room temperature for 0.5 h. The reaction mixture was poured into water (20 mL) and extracted with DCM (30 mL x 3). The combined organic layer was washed with brine (15 mL x 3), dried over anhydrous Na₂SO₄, filtered and concentrated *in vacuo.* The residue was purified by flash column chromatography on silica gel (10-50% ethyl acetate/petroleum ether) to afford the desired product (0.5 g, 35 % yield) as a solid.

### 2-(Vinylsulfonyl)octahydropyrrolo [3,4-c] pyrrole

A mixture of tert-butyl 5-(vinylsulfonyl)hexahydropyrrolo[3,4-c]pyrrole-2(1H)-carboxylate (150 mg, 0.50 mmol) in HCl/MeOH (20 mL, 2.86 M) was stirred at room temperature for 1 h. The mixture was concentrated *in vacuo* to afford the crude product (110 mg) as a solid which was used directly in next step without further purification.

### 4,5-Dichloro-2-nitrophenol

To a solution of 3,4-dichlorophenol (30 g, 185 mmol) in DCM (300 mL) at -15°C, sulfuric acid (24 g, 278 mmol) was added. To this mixture, nitric acid (19 g, 194 mmol) was added dropwise (over 20 min) and the temperature was controlled between -15 °C to -5 °C. The resulting mixture was stirred at 0°C for 1 h. The mixture was poured into ice and extracted with ethyl acetate. The combined organic layer was washed with water, saturate NaHCO₃ aqueous solution and brine, dried over Na₂SO₄ and then concentrated *in vacuo.* The residue was purified by flash column chromatography on silica gel (petroleum ether/ethyl acetate = 100:1) to afford the desired product (16 g, 42% yield) as a solid.

### 2-Amino-4,5-dichlorophenol

To a stirred solution of 4,5-dichloro-2-nitrophenol (15 g,72 mmol) in acetic acid (150 mL) and water (450 mL), iron powder (16 g, 285 mmol) was added in portions. The resulting mixture was stirred at 50°C for 2 h. The mixture was allowed to cool to room temperature, filtered, and the cake was rinsed with ethyl acetate. The filtrate was extracted with ethyl acetate. The organic layer was washed with water, NaHCO₃ (aq.) and brine, dried over Na₂SO₄ and concentrated *in vacuo* to afford the desired product (10 g, 78%).

### Ethyl 2-((4,5-dichloro-2-hydroxyphenyl)amino)acetate

To a solution of 2-amino-4,5-dichlorophenol (2.0 g, 11.3 mmol) and ethyl-2-oxoacetate (2.26 g, 12.42 mmol) in DCM (50 mL) at room temperature, AcOH (1 mL) was added and the resulting mixture was stirred for 1 h. To this mixture, NaBH(OAc)₃ (7.2 g, 33.9 mmol) was added and then stirred for 16 h. The mixture was concentrated in vacuo and the residue was suspended in DCM. The mixture was filtered through a pad of Celite and the filtrate was washed with brine. The organic layer was dried over anhydrous Na₂SO₄, filtered and concentrated *in vacuo.* The residue was purified by flash column chromatography on silica gel (petroleum ether/ethyl acetate = 10:1) to afford the desired product (2.4 g, 81 % yield) as a solid.

### 2-((4,5-Dichloro-2-hydroxyphenyl)amino)acetic acid

To a solution of ethyl 2-((4,5-dichloro-2-hydroxyphenyl)amino)acetate (2 g, 7.6 mmol) in of 4:1 mixture of tetrahydrofuran and water (30 mL) at room temperature, LiOH.HzO (3.2 g, 76 mmol) were added and the resulting mixture was stirred for 30 min and then acidified with aqueous HCl (1 N) to adjust the pH to 3 - 5. The mixture was extracted with ethyl acetate (40 mL x 3). The combined organic layer was washed with brine (40 mL x 3), dried over anhydrous Na₂SO₄, filtered and concentrated *in vacuo* to afford the crude product (2 g) which was used directly in the next step without further purification.

### 2-((4,5-dichloro-2-hydroxyphenyl)amino)-1-(5-(vinylsulfonyl)hexahydropyrrolo [3,4-c]pyrrol-2(1H)-yl)ethanone

To a stirred solution of 2-((4,5-dichloro-2-hydroxyphenyl)amino)acetic acid (90 mg, 0.385 mmol) in DMF (15 mL) at room temperature, 2-(vinylsulfonyl)octahydropyrrolo[3,4-c]pyrrole (110 mg, 0.46 mmol) was added followed by HOBt (78.05 mg, 0.58 mmol), EDCI.HCl (110.7 mg, 0.46 mmol) and Et₃N (116.7 mg, 1.2 mmol). The reaction mixture was stirred at room temperature for 1 h. The reaction mixture was partitioned between ethyl acetate and brine. The organic layer was dried over anhydrous Na₂SO₄, filtered and concentrated *in vacuo.* The residue was purified by flash column chromatography on silica gel (10-50% ethyl acetate/dichloroethane) to afford the desired product (20 mg, 10 % yield) as a solid. **¹H NMR** (400 MHz, DMSO-*d6*) δ: 10.17 (s, 1H), 6.91 (dd, *J* = 10.0, 16.4 Hz, 1H), 6.79 (s, 1H), 6.62 (s, 1H), 6.17 - 6.10 (m, 2H), 5.25 (t, *J* = 4.0 Hz, 1H), 3.85 - 3.82 (m, 2H), 3.71 - 3.66 (m, 1H), 3.61 - 3.56 (m, 1H), 3.45 - 3.39 (m, 3H), 3.29 - 3.25 (m, 1H), 3.09 - 3.00 (m, 3H), 2.92 - 2.89 (m, 1H). **ESI-MS** *m*/*z:* 420.1 [M+H]⁺.

### EXAMPLE 4

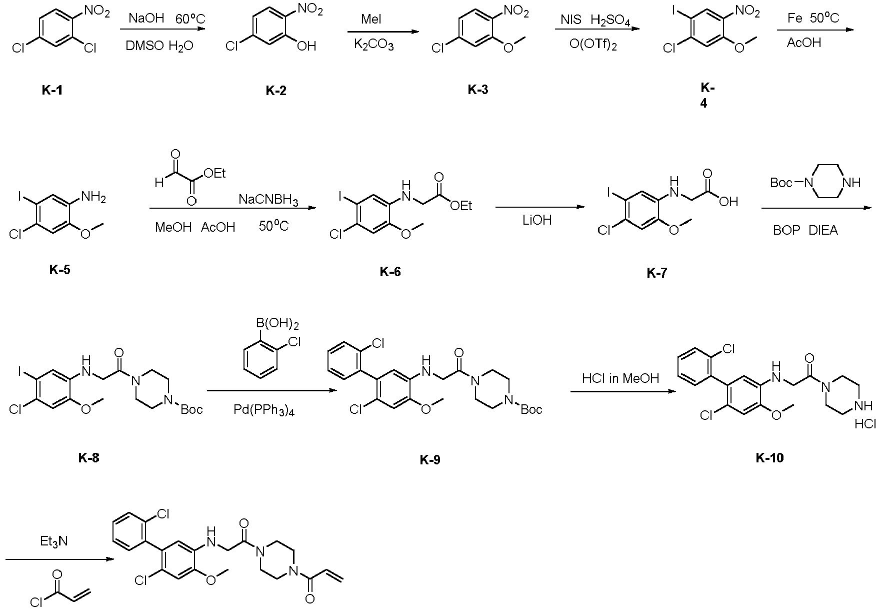

### 5-Chloro-2-nitrophenol

To a solution of 2,4-dichloro-1-nitrobenzene (100 g, 0.52 mol) in DMSO (200 mL), aqueous solution of NaOH (41.6 g, 1.04 mol) in water (42 mL) was added and the resulting mixture was stirred 60°C for 16 h. The mixture was allowed to cool to room temperature, poured to ice water, and then acidified with aqueous HCl (1 M) to adjusted the pH to 3 - 4. The mixture was extracted with ethyl acetate. The combined organic layer was washed with brine, dried over Na₂SO₄, filtered and concentrated *in vacuo.* The residue was used directly in the next step (80 g, 88% yield).

### 4-Chloro-2-methoxy-1-nitrobenzene

To a solution of 5-chloro-2-nitrophenol (40 g, 0.23 mol) in DMF (200 mL), K₂CO₃ (47.6 g, 0.345 mol) and iodomethane (49 g, 0.345 mol) were added and the resulting mixture was stirred at room temperature for 16h. The mixture was partitioned between ethyl acetate and water. The organic layer was washed with brine, dried over MgSO₄, filtered and concentrated *in vacuo.* The residue was purified by flash column chromatography on silica gel (petroleum ether) to afford the desired product (30 g, 70% yield).

### 1-Chloro-2-iodo-5-methoxy-4-nitrobenzene

To a solution of H₂SO₄ (600 mL, 90%), trifluoromethanesulfonic anhydride (11.3g, 0.04mol) and NIS (49.68 g, 0.22 mol) were added and resulting mixture was stirred at room temperature for 1 h. To this mixture, 4-chloro-2-methoxy-1-nitrobenzene (69 g, 0.368 mol) was added quickly. The mixture was stirred for 1 h, and then NIS (33.12 g, 0.148 mol) was slowly added to the mixture. The mixture was stirred at room temperature for 1 h and then was poured into ice-water. The precipitate collected by filtration, rinsed with water, aqueous NaSO₃ and NaHCO₃ solutions, and then dried *in vacuo* to afford the desired product (113 g, 98% yield).

### 4-Chloro-5-iodo-2-methoxybenzenamine

To a solution of 1-chloro-2-iodo-5-methoxy-4-nitrobenzene (113 g, 0.361 mol) in acetic acid (1 L) and water (50 mL) at 50°C, Fe (50.5 g, 0.903 mol) was added and the resulting mixture was stirred at 50°C for 2 h. The mixture was allowed to cool to room temperature and then poured into ice-water. The precipitate was collected by filtration and rinsed with water. This crude product was dissolved with ethyl acetate (1 L) and filtered. The filtrate was washed with saturated NaHCO₃ solution and brine. The organic layer was dried over MgSO₄, filtered, and concentrated *in vacuo* to afford the desired product (87 g, 85% yield).

### Ethyl 2-(4-chloro-5-iodo-2-methoxyphenylamino)acetate

To a solution of 4-chloro-5-iodo-2-methoxybenzenamine (6 g, 21.2 mmol) in MeOH (50 mL) at room temperature, AcOH (3 drops) and ethyl glyoxalate (5.6 g, 27.5 mmol, 50% in toluene) were added. The mixture was stirred at room temperature for 2 h and then sodium cyanoborohydride (5.32 g, 84.8 mmol) was added to the mixture. The resulting mixture was stirred at 50°C for 16 h. The mixture was allowed to cool to room temperature, and partitioned between ethyl acetate and water. The organic layer was dried over MgSO₄, filtered, and concentrated *in vacuo* to afford the crude product (8.6 g).

### 2-(4-Chloro-5-iodo-2-methoxyphenylamino)acetic acid

To a solution of ethyl 2-(4-chloro-5-iodo-2-methoxyphenylamino)acetate (8.6 g, 22.9 mmol) in THF (50 mL) and water (50 mL), LiOH.HzO (1.96 g, 45.9 mmol) was added and the resulting mixture was stirred at room temperature for 2 h. The mixture was washed with 20% ethyl acetate/petroleum ether. The aqueous layer was acidified with aqueous HCl (1 M) to adjust PH to 3-4 and extracted with ethyl acetate. The organic layer was dried over MgSO₄, filtered, and concentrated *in vacuo* to afford the desired product (5 g, 64% yield).

### tert-Butyl 4-(2-(4-chloro-5-iodo-2-methoxyphenylamino)acetyl)piperazine-1-carboxylate

To a solution of 2-(4-chloro-5-iodo-2-methoxyphenylamino)acetic acid (280 mg, 0.83 mmol) and tert-butyl piperazine-1-carboxylate (185 mg, 0.99 mmol) in DMF (10 mL) at room temperature, BOP (550 mg, 1.25 mmol) and DIEA (321 mg, 2.4 9 mmol) were added and the resulting mixture was stirred at room temperature for 1 h. The mixture was partitioned between ethyl acetate and water. The organic layer was washed brine, dried over MgSO₄, filtered and concentrated *in vacuo.* The residue was purified by flash column chromatography on silica gel (petroleum ether/ethyl acetate = 3:1) to afford the desired product (350 mg, 68.7% yield). **ESI-MS** *m*/*z:* 510.3 [M+1]⁺.

### tert-Butyl-4-(2-(5-(2-Chloropheny)-4-chloro-2-methoxyphenylamino)acetyl)piperazine-1-carboxylate

To a solution of tert-butyl 4-(2-(4-chloro-5-iodo-2-methoxyphenylamino)acetyl)piperazine-1-carboxylate (200 mg, 0.4 mmol) and 2-chlorophenylboronic acid (69 mg, 0.44 mmol) in 1,4-dioxane (10 mL) and water (2 mL), Pd(PPh₃)₄ (40 mg, 0.035 mmol) and Na₂CO₃ (212 mg, 2 mmol) were added. The mixture was stirred at 80°C for 16 h. The mixture was allowed to cool to room temperature and concentrated *in vacuo.* The residue was purified by flash column chromatography on silica gel (petroleum ether/ethyl acetate = 4:1) to afford the desire product (150 mg, 76% yield).

### 2-(5-(2-Chloropheny)-4-chloro-2-methoxyphenylamino)-1-(piperazin-1-yl)ethanone

To a solution of tert-butyl-4-(2-(5-(2-chloropheny)-4-chloro-2-methoxyphenylamino)acetyl)piperazine-1-carboxylate (150 mg, 0.304 mmol) in DCM (2 mL), a solution of HCl in MeOH (10 mL, 29 mmol) was added. The mixture was stirred at room temperature for 1 h and then concentrated *in vacuo* to afford the crude product which was used in the next step without further purification.

### 1-(4-(2-(4-chloro-5-(2-Chloropheny)-2-methoxyphenylamino)acetyl)piperazin-1-yl)prop-2-en-1-one

To a solution of the crude 2-(5-(2-Chloropheny)-4-chloro-2-methoxyphenylamino)-1-(piperazin-1-yl)ethanone (0.304 mmol) and Et₃N in DCM (5 mL) at 0 °C, acryloyl chloride (27.5 mg, 0.304 mmol) was slowly added and the resulting mixture was stirred at room temperature for 1 h. The mixture was quenched with saturated NaHCO₃ solution and extracted with DCM. The organic layer was washed with brine, dried over MgSO₄, filtered and concentrated *in vacuo.* The residue was purified by flash column chromatography on silica gel to afford the desired product (80 mg, 58.7% yield, 2 steps). **¹H NMR** (400 MHz, DMSO-*d6*) δ: 7.55-7.52 (m, 1H), 7.45-7.39 (m, 2H), 7.32-7.29 (m, 1H), 6.97 (s, 1H), 6.80 (dd, *J* = 10.4, 16.4 Hz, 1H), 6.54 (s, 1H), 6.12 (dd, *J* = 2.0, 16.8 Hz, 1H), 5.70 (dd, *J* = 2.0, 10.4 Hz, 1H), 5.35 (bs., 1H), 3.93 (d, *J* = 4.0 Hz, 2H), 3.89 (s, 1H), 3.55-3.49 (m, 8H). **ESI-MS** *m*/*z:* 448.2 [M+H]⁺.

### EXAMPLE 5

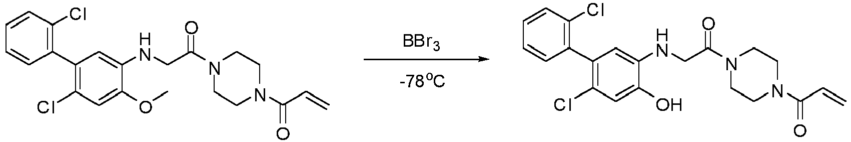

### 1-(4-(2-(4-chloro-2-hydroxy-5-(2-Chloropheny)phenylamino)acetyl)piperazin-1-yl)prop-2-en-1-one

To a solution of 1-(4-(2-(4-chloro-5-(2-Chloropheny)-2-methoxyphenylamino)acetyl)piperazin-1-yl)prop-2-en-1-one (70 mg, 0.147 mmol) in DCM (15 mL) -78°C under argon, BBr₃ (187 mg, 0.754 mmol) was added. The mixture was stirred at room temperature for 1 h. The mixture was poured into ice water and extracted ethyl acetate. The organic layer was dried over MgSO₄, filtered and concentrated *in vacuo.* The residue was purified by flash column chromatography on silica gel (dichloromethane/methanol = 40:1) to afford the desired product (15 mg, 24% yield). **¹H NMR** (400 MHz, DMSO-*d6*) δ: 10.08 (s, 1H), 7.53-7.51 (m, 1H), 7.41-7.37 (m, 2H), 7.30-7.28 (m, 1H), 6.84-6.77 (m, 2H), 6.48 (s, 1H), 6.12 (dd, *J* = 2.4, 16.4 Hz, 1H), 5.70 (dd, *J* = 2.0, 10.4 Hz, 1H), 5.24 (t, *J* = 4.0 Hz, 1H), 3.90 (d, *J* = 4.4 Hz, 2H), 3.56-3.49 (m, 8H). **ESI-MS** *m*/*z:* 434.2 [M+H]⁺.

### EXAMPLE 6

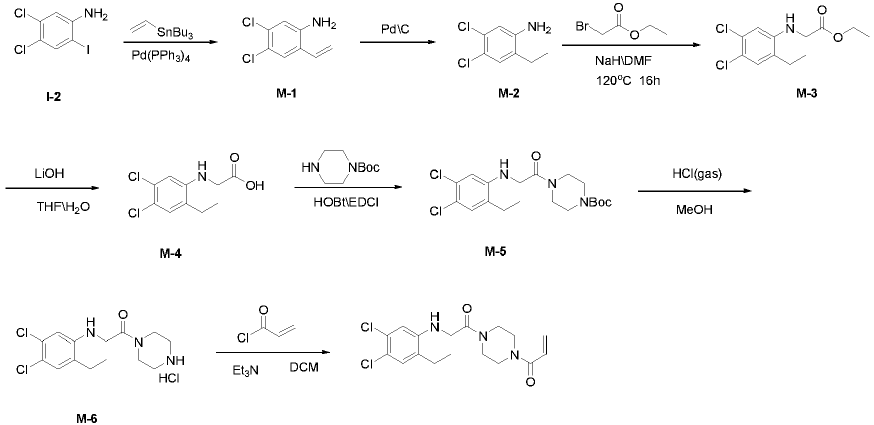

### 4,5-Dichloro-2-vinylbenzenamine

A mixture of 4,5-dichloro-2-iodobenzenamine (6 g, 20.8 mmol), tributyl(vinyl)stannane (6.6 g, 20.8 mmol), Pd(PPh₃)₄ (2.4 g, 2.1 mmol) in toluene (60 mL) was stirred at reflux under argon for 6 h. The mixture was allowed to cool to room temperature, quenched with aqueous KF solution and then extracted with ethyl acetate. The organic layer was washed with brine, dried over Na₂SO₄, filtered and concentrated *in vacuo.* The residue was purified by flash column chromatography on silica gel (petroleum ether/ethyl acetate = 4 : 1) to afford the desired product (3.0 g, 76.7% yield) as a colorless oil.

### 4,5-Dichloro-2-ethylbenzenamine.

A mixture of 4,5-dichloro-2-vinylbenzenamine (3.0 g, 15.95 mmol), Pd/C (3.3 g, 10%) in MeOH (20 mL) was stirred at room temperature under H₂ (1 atm) atmosphere for 4 h. The mixture was filtered and the filtrate was concentrated *in vacuo* to afford the desired product (2.4 g, 80% yield).

### Ethyl 2-(4,5-dichloro-2-ethylphenylamino)acetate

To a stirring solution of 4,5-dichloro-2-ethylbenzenamine (600 mg, 3.15 mmol) in DMF at 0°C,NaH (150 mg, 3.78 mmol) was added in portions. After stirring for 30 min, ethyl 2-bromoacetate (789 mg, 4.74 mmol) was added to the mixture. The resulting mixture was stirred at 120 °C for 16 h. The mixture was allowed to cool to room temperature, poured into ice-water and extracted with ethyl acetate. The organic layer was washed with brine, dried over Na₂SO₄ and concentrated. The residue was purified by flash column chromatography on silica gel (petroleum ether/ethyl acetate = 5 : 1) to afford the desired product (380 mg, 44.7% yield) as a yellow solid.

### 2-(4,5-Dichloro-2-ethylphenylamino)acetic acid

A mixture of ethyl 2-(4,5-dichloro-2-ethylphenylamino)acetate (390 mg, 1.41 mmol) and LiOH.HzO (592 mg, 14.1 mmol) in THF (8 mL) and water (2 mL) was stirred at room temperature for 2 h. The mixture was concentrated *in vacuo* and the residue was dissolved in H₂O and diluted with ethyl acetate. The mixture was acidified with aqueous HCl (10%) to adjust the pH to 3 - 4 and then extracted with ethyl acetate. The organic layer was dried over Na₂SO₄, filtered and concentrated *in vacuo* to afford the desired product (300 mg, 85.75% yield) as a yellow solid. **ESI-MS** *m*/*z:* 246.2 [M-H]⁻.

### tert-Butyl 4-(2-(4,5-dichloro-2-ethylphenylamino)acetyl)piperazine-1-carboxylate

To a stirred mixture of 2-(4,5-dichloro-2-ethylphenylamino)acetic acid (100 mg, 0.403 mmol), Et₃N (122 mg, 1.21 mmol) in DCM (3 mL) at 0°C, EDCI.HCl (123 mg, 0.604 mmol) and HOBt (8 2mg, 0.604 mmol) were added. The resulting mixture was stirred at 0°C for 30 min, and then tert-butyl piperazine-1-carboxylate (90 mg, 0.483 mmol) was added. The mixture was stirred at room temperature for 16 h and partitioned between ethyl acetate and water. The organic layer was washed with brine, dried over Na₂SO₄, filtered and concentrated *in vacuo.* The residue was purified by flash column chromatography on silica gel (petroleum ether/ethyl acetate = 5 : 1) to afford the desired product (120 mg, 71.5% yield) as an off-white solid. **ESI-MS** *m*/*z:* 414.3 [M-H]⁻.

### 2-(4,5-Dichloro-2-ethylphenylamino)-1-(piperazin-1-yl)ethanone hydrochloride

A mixture of tert-butyl 4-(2-(4,5-dichloro-2-ethylphenylamino)acetyl)piperazine-1-carboxylate (120 mg, 0.288 mmol) in HCl/MeOH (2.86 M, 10 mL) was stirred at room temperature for 1 h. The mixture was concentrated *in vacuo* to afford the crude product (82 mg) as a yellow solid which was used directly in the next step without further purification.

### 1-(4-(2-(4,5-Dichloro-2-ethylphenylamino)acetyl)piperazin-1-yl)prop-2-en-1-one

To a mixture of above crude 2-(4,5-dichloro-2-ethylphenylamino)-1-(piperazin-1-yl)ethanone hydrochloride (82 mg) and Et₃N (69 mg, 0.686 mmol) in DCM (3 mL) 0°C, a solution of acryloyl chloride (23 mg, 0.251 mmol) in DCM (1 mL) was added. The resulting mixture was stirred at room temperature for 1 h and then was quenched withed saturated NaHCO₃ solution. The mixture was diluted with ethyl acetate, washed with saturated NaHCO₃ solution and brine, dried over Na₂SO₄ and concentrated *in vacuo.* The residue was purified by flash column chromatography on silica gel (DCM/MeOH = 30:1) to afford the desired product (20 mg, 18.76% yield, 2 steps) as an off-white solid. **¹H NMR** (400 MHz, CDCl₃) δ: 7.11 (s, 1H), 6.58 (dd, *J* = 10.4, 16.4 Hz, 1H), 6.54 (s, 1H), 6.36 (dd, *J* = 1.6, 16.4 Hz, 1H), 5.79 (dd, *J* = 1.6, 10.4 Hz, 1H), 5.08 (bs., 1H), 3.89-3.52 (m, 10H), 2.52 (q, *J* = 7.2 Hz, 2H), 1.29-1.26 (t, *J* = 7.6 Hz, 3H). **ESI-MS** *m*/*z:* 370.2 [M+H]⁺.

### EXAMPLE 7

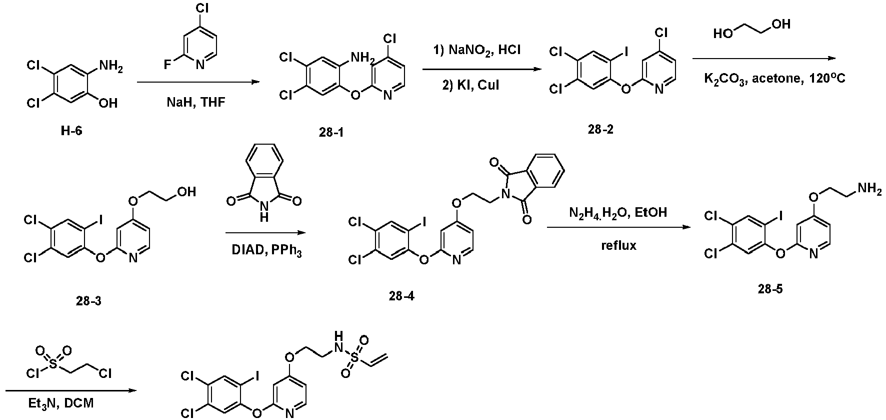

### 2-(4-chloropyridin-2-yloxy)-4,5-dichlorobenzenamine

To a solution of 2-amino-4,5-dichlorophenol (1.5 g, 8.47 mmol) in THF (20 mL) at 0°C, NaH (60% dispersed in oil, 373 mg, 9.33 mmol) was added and the resulting mixture was stirred for 20 min. To this mixture, 4-chloro-2-fluoropyridine (1.67 g, 12.7 mmol) was added and the resulting mixture was stirred at reflux under argon for 15 h. The mixture was allowed to cool to room temperature and concentrated *in vacuo.* The residue was purified by flash column chromatography on silica gel (petroleum ether/ethyl acetate = 20 : 1 to 10 : 1) to afford the desired product (1.1 g, 45% yield). **ESI-MS** *m*/*z:* 287.0 [M-H]⁻.

### 2-(4,5-Dichloro-2-iodophenoxy)-4-chloropyridine

The mixture of 2-(4-chloropyridin-2-yloxy)-4,5-dichlorobenzenamine (1.0 g, 3.47 mmol) in concentrated HCl (5 mL) and H₂O (15 mL) was cooled to -10 °C-0 °C, NaNO₂ (0.359 g, 5.21 mmol) was added and the resulting mixture was stirred at this temperature for 50 min. This mixture was added dropwise to the mixture of KI (10 g, 60.2 mmol), CuI (330 mg, 1.73 mmol) in H₂O (40 mL) and then stirred at room temperature for 15 h. The mixture was partitioned between ethyl acetate and water. The organic layer was washed withed brine, dried over Na₂SO₄ and concentrated. The residue was purified by flash column chromatography on silica gel (petroleum ether/ethyl acetate = 30 : 1) to afford the desired product (900 mg, 65% yield). **ESI-MS** *m*/*z:* 399.9 [M+H]⁺.

### 2-(2-(4,5-Dichloro-2-iodophenoxy)pyridin-4-yloxy)ethanol

A mixture of 2-(4,5-dichloro-2-iodophenoxy)-4-chloropyridine (0.45 g, 1.128 mmol), ethane-1,2-diol (4 mL, 71.89 mmol), K₂CO₃ (0.45mg, 3.26 mmol) in acetone (6 mL) was stirred at 120°C for 5 h. The mixture was poured into water and extracted with ethyl acetate. The organic layer was washed withed brine, dried over Na₂SO₄ and concentrated. The residue was purified by flash column chromatography on silica gel (petroleum ether/ethyl acetate = 20:1 to 4:1) to afford the desired product (160 mg, 33% yield). **ESI-MS** *m*/*z:* 426.0 [M+H]⁺.

### 2-(2-(2-(4,5-Dichloro-2-iodophenoxy)pyridin-4-yloxy)ethyl)isoindoline-1,3-dione

A mixture of 2-(2-(4,5-dichloro-2-iodophenoxy)pyridin-4-yloxy)ethanol (130 mg, 0.31 mmol), isoindoline-1,3-dione (54 mg, 0.37 mmol), PPh₃ (160 mg, 0.61 mmol) in THF (5 mL) at 0°C, DIAD (123 mg, 0.61 mmol) was added. The resulting mixture was allowed to warm to room temperature and stirred for 15 h. The mixture was concentrated *in vacuo* and the residue was purified by flash column chromatography on silica gel (petroleum ether/ethyl acetate = 20:1 to 5:1) to afford the desired product (120 mg, 71% yield). **ESI-MS** *m*/*z:* 555.0 [M+H]⁺.

### 2-(2-(4,5-Dichloro-2-iodophenoxy)pyridin-4-yloxy)ethanamine

The mixture of 2-(2-(2-(4,5-dichloro-2-iodophenoxy)pyridin-4-yloxy)ethyl)isoindoline-1,3-dione (120 mg, 0.22 mmol), N₂H₄.H₂O (106 mg, 1.80 mmol) and EtOH (5 mL) was stirred at reflux for 1 h. The mixture was allowed to cool to room temperature and then concentrated *in vacuo.* The residue was slurried in DCM (5 mL) and MeOH (1 mL). The precipitate was removed by filtration, and the filtrate was concentrated *in vacuo.* The residue was purified by flash column chromatography on silica gel (DCM/MeOH = 10:1) to afford the desired product (35 mg, 38% yield).

### N-(2-(2-(4,5-Dichloro-2-iodophenoxy)pyridin-4-yloxy)ethyl)ethenesulfonamide

To a mixture of 2-(2-(4,5-dichloro-2-iodophenoxy)pyridin-4-yloxy)ethanamine (30 mg, 0.07 mmol), Et₃N (35.7 mg, 0.35 mmol) in DCM (5 mL), 2-chloroethanesulfonyl chloride (11.5 mg, 0.07 mmol) was added and the resulting mixture was stirred at room temperature for 1 h. Then solvent was removed under reduced pressure. The residue was taken in THF (2 mL) and H₂O (2 mL), then K₂CO₃ (100 mg) was added and the resulting mixture was stirred at room temperature for 1 h. The mixture was partitioned between DCM and water. The organic layer was washed with brine, dried over Na₂SO₄ and concentrated. The residue was purified by flash column chromatography on silica gel (DCM/ethyl acetate = 5:1) to afford the desired product (56 mg, 56% yield). **¹H NMR** (400 MHz, CDCl₃) δ: 7.98 (d, *J* = 5.6 Hz, 1H), 7.92 (s, 1H), 7.25 (s, 1H), 6.61-6.57 (m, 2H), 6.47 (d, *J* = 2.0 Hz, 1H), 6.32 (d, *J* = 16.4 Hz, 1H), 6.00 (d, *J* = 10.0 Hz, 1H), 4.77 (t, *J* = 5.2 Hz, 1H), 4.18 (t, *J* = 5.2 Hz, 2H), 3.51-3 .47 (m, 2H). **ESI-MS** *m*/*z:* 515.0 [M+H]⁺.

### EXAMPLE 8

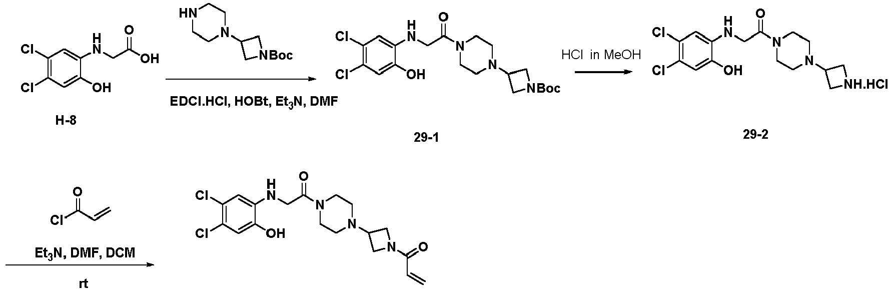

### tert-Butyl 3-(4-(2-(4,5-dichloro-2-hydroxyphenylamino)acetyl)piperazin-1-yl)azetidine-1-carboxylate

A mixture of 2-(4,5-dichloro-2-hydroxyphenylamino)acetic acid (500 mg, 2.12 mmol), *tert*-butyl 3-(piperazin-1-yl)azetidine-1-carboxylate (565 mg, 2.34mmol), EDCI.HCl (488 mg, 2.54 mmol), HOBt (343 mg, 2.54 mmol), Et₃N (428 mg, 4.24 mmol) in DMF (20 mL) was stirred at room temperature for 15 h. The mixture was poured into water and extracted with ethyl acetate. The organic layer was washed withed saturated aqueous NaHCO₃ solution and brine, dried over Na₂SO₄ and concentrated *in vacuo.* The residue was purified by flash column chromatography on silica gel (DCM/MeOH = 30:1) to afford the desired product (300 mg, 31% yield). **ESI-MS** *m*/*z:* 457.4 [M-H]⁻.

### 2-(4,5-Dichloro-2-hydroxyphenylamino)-1-(4-(azetidin-3-yl)piperazin-1-yl)ethanone hydrochloride

A mixture of *tert*-butyl 3-(4-(2-(4,5-dichloro-2-hydroxyphenylamino)acetyl)piperazin-1-yl)azetidine-1-carboxylate (150 mg, 0.33 mmol) in HCl-MeOH (20 mL, 57 mmol) was stirred at room temperature for 1 h. The mixture was concentrated *in vacuo* to afford the crude product (130 mg) which was used directly in the next step without further purification.

### 1-(3-(4-(2-(4,5-Dichloro-2-hydroxyphenylamino)acetyl)piperazin-1-yl)azetidin-1-yl)prop-2-en-1-one

2-(4,5-dichloro-2-hydroxyphenylamino)-1-(4-(azetidin-3-yl)piperazin-1-yl)ethanone hydrochloride (120 mg, 0.30 mmol) was added to the mixture of Et₃N (0.2 mL, 1.44 mmol) in DCM (10 mL) followed by addition of DMF (1 drop). The mixture was stirred for 5 min and then acryloyl chloride (27 mg, 0.30 mmol) was added. The resulting mixture was stirred at room temperature for 1 h, poured into water and then extracted with MeOH/DCM. The organic layer was washed with brine, dried over Na₂SO₄ and concentrated. The residue was purified by flash column chromatography on silica gel (DCM/MeOH/NH₃.H₂O = 50:1:0.1 to 20:1:0.2) to afford the desired product (30 mg, 24% yield). **¹H NMR** (400 MHz, DMSO-_{d6}) δ: 10.17 (s, 1H), 6.78 (s, 1H), 6.71 (s, 1H), 6.30 (dd, *J* = 10.4, 17.2 Hz, 1H), 6.09 (dd, *J* = 2.0, 17.2Hz, 1H), 5.67 (dd, *J* = 2.4, 10.4 Hz, 1H), 5.32 (t, *J* = 4.4 Hz, 1H), 4.26-4.22 (m, 1H), 4.11 - 4.04 (m, 1H), 3.93-3.91 (m, 3H), 3.79-3.75 (m, 3H), 3.52-3.51 (m, 4H), 3.19-3.16 (m, 1H), 2.36-2.30 (m, 4H). **ESI-MS** *m*/*z:* 411.2 [M-H]⁻.

### EXAMPLE 9

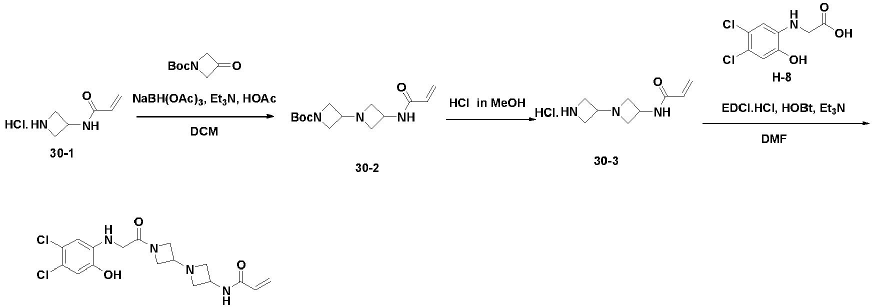

### tert-Butyl 3-(3-(acrylamido)azetidin-1-yl)azetidine-1-carboxylate

To a mixture of *N*-(azetidin-3-yl)acrylamide hydrochloride (500 mg, 3.40 mmol), *tert*-butyl 3-oxoazetidine-1-carboxylate (684 mg, 4.0 mmol), Et₃N (343 mg, 3.40 mmol) and AcOH (100 mg, 0.167 mmol) in DCM (20 mL), NaBH(OAc)₃ (2.16 g, 10.2 mmol) was added, and the resulting mixture was stirred at room temperature for 16 h. The mixture was poured into water and extracted with ethyl acetate. The organic layer was washed with saturated NaHCO₃ solution and brine, dried over Na₂SO₄ and concentrated *in vacuo.* The residue was purified by flash column chromatography on silica gel (DCM/MeOH = 100:1 to 20:1) to afford the desired product (300 mg, 31% yield).

### N-(1-(Azetidin-3-yl)azetidin-3-yl)acrylamide hydrochloride

A mixture of *tert*-butyl 3-(3-(acrylamido)azetidin-1-yl)azetidine-1-carboxylate (300 mg, 1.07 mmol) in HCl-MeOH (30 mL, 86 mmol) was stirred at room temperature for 1 h. The mixture was concentrated *in vacuo* to afford the crude product (250 mg) which was used directly in the next step without further purification.

### N-(1-(1-(2-(4,5-Dichloro-2-hydroxyphenylamino)acetyl)azetidin-3-yl)azetidin-3-yl)acrylamide

A mixture of 2-(4,5-dichloro-2-hydroxyphenylamino)acetic acid (120 mg, 0.51 mmol), EDCI.HCl (147 mg, 0.77 mmol), HOBt (83 mg, 0.61 mmol), Et₃N (154 mg, 1.53 mmol) in DMF (20 mL) was stirred at room temperature for 5 min and then *N*-(1-(azetidin-3-yl)azetidin-3-yl)acrylamide hydrochloride (150 mg, 0.69 mmol) was added. The resulting mixture was stirred at room temperature for 15 h. The mixture was poured into water and extracted with ethyl acetate. The organic layer was washed with saturated NaHCO₃ solution and brine, dried over Na₂SO₄ and concentrated *in vacuo.* The residue was purified by flash column chromatography on silica gel (DCM/MeOH/NH₃.H₂O = 100:10:1.5) to afford the desired product (6 mg, 3% yield). **¹H NMR** (400 MHz, DMSO-*d6*) δ: 10.19 (s, 1H), 8.59 (d, *J* = 8.0 Hz, 1H), 6.79 (s, 1H), 6.55 (s, 1H), 6.20 (dd, *J* = 10.0, 16.8 Hz, 1H), 6.09 (dd, *J* = 2.0, 16.8 Hz, 1H), 5.62 (dd, *J* = 2.0, 9.6 Hz, 1H), 5.20 (t, *J* = 4.0 Hz, 1H), 4.40-4.35 (m, 1H), 4.19-4.15 (m, 1H), 3.96-3.88 (m, 2H), 3.73-3.69 (m, 3H), 3.53-3.45 (m, 3H), 3.00-2.96 (m, 2H). **ESI-MS** *m*/*z:* 399.2 [M+H]⁺.

### EXAMPLE 10

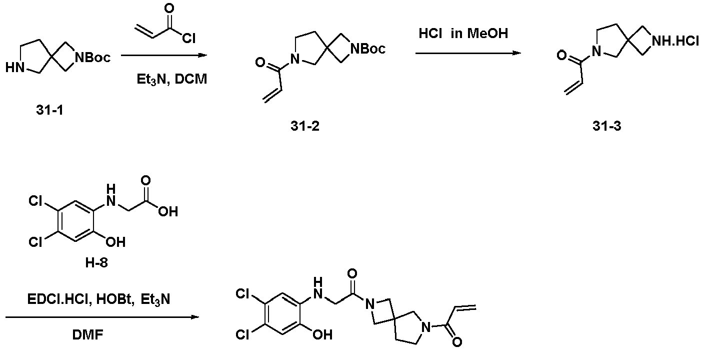

### 2,6-Diaza-spiro[3.4]octane-6-acryloyl-2-carboxylic acid tert-butyl ester

To a mixture of 2,6-diaza-spiro[3.4]octane-2-carboxylic acid tert-butyl ester (80 mg, 0.38 mmol), Et₃N (0.2 mL, 1.44 mmol) in DCM (20 mL), acryloyl chloride (34 mg, 0.38 mmol) was added and the resulting mixture was stirred at room temperature for 1 h. The mixture was poured into water and extracted with ethyl acetate. The organic layer was washed with saturated NaHCO₃ solution and brine, dried over Na₂SO₄ and concentrated *in vacuo.* The residue was purified by flash column chromatography on silica gel (DCM/MeOH = 40:1) to afford the desired product (50 mg, 50% yield). **ESI-MS** *m*/*z:* 289.2 [M+Na]⁺.

### 1-(2,6-Diazaspiro [3.4] octan-6-yl)prop-2-en-1-one

A mixture of 2,6-diaza-spiro[3.4]octane-6-acryloyl-2-carboxylic acid tert-butyl ester (50 mg, 0.19 mmol) in HCl/MeOH (10 mL, 29 mmol) was stirred at room temperature for 1 h. The mixture was concentrated *in vacuo* to afford the crude product (40 mg) which was used directly in the next step without further purification.

### 1-(2-((4,5-Dichloro-2-hydroxyphenyl)glycyl)-2,6-diazaspiro[3.4]octan-6-yl)prop-2-en-1-one

The mixture of 2-(4,5-dichloro-2-hydroxyphenylamino)acetic acid (47 mg, 0.2 mmol), 1-(2,6-diazaspiro[3.4]octan-6-yl)prop-2-en-1-one (40 mg, 0.2 mmol), EDCI.HCl (46 mg, 0.24 mmol), HOBt (32 mg, 0.24 mmol) and Et₃N (0.61 mg, 0.6 mmol) in DMF (10 mL) was stirred at room temperature for 2 h. The mixture was poured into water and extracted with ethyl acetate. The organic layer was washed withed saturated NaHCO₃ solution and brine, dried over Na₂SO₄ and concentrated *in vacuo.* The residue was purified by flash column chromatography on silica gel (DCM/MeOH = 30:1) to afford the desired product (13 mg, 17% yield). **¹H NMR** (400 MHz, DMSO-*d6*) δ: 10.17 (s, 1H), 6.78 (s, 1H), 6.60-6.50 (m, 2H), 6.13 (dt, *J* = 2.4, 16.4 Hz, 1H), 5.67 (dd, *J* = 2.4, 10.4 Hz, 1H), 5.19 (dd, *J* = 5.2, 10.0 Hz, 1H), 4.16-4.07 (m, 2H), 3.90-3.83 (m, 2H), 3.75-3.72 (m, 3H), 3.61-3.52 (m, 2H), 3.42-3.39 (m, 1H), 2.16-2.13 (m, 1H), 2.06-2.03 (m, 1H). **ESI-MS** *m*/*z:* 382.3 [M-H]⁻.

### EXAMPLE 11

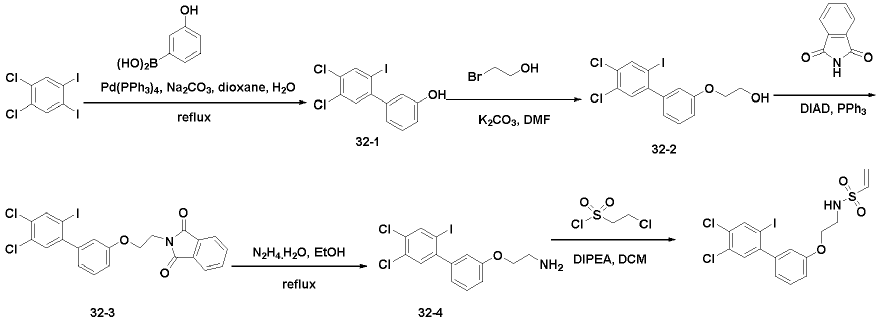

### 3-(4,5-Dichloro-2-iodophenyl)phenol

A mixture of 1,2-dichloro-4,5-diiodobenzene (1.5 g, 3.76 mmol), 3-hydroxyphenylboronic acid (0.52 g, 3.76 mmol), Na₂CO₃ (1.99 g, 18.8 mmol) and Pd(PPh₃)₄ (0.35 g, 0.30 mmol), in 1,4-dioxane (10 mL) and water (2 mL) was stirred at reflux under argon for 16 h. The mixture was allowed to cool to room temperature and then concentrated *in vacuo.* The residue was purified by flash column chromatography on silica gel (petroleum ether/ethyl acetate = 20:1) to afford the desired product (300 mg, 22% yield). **ESI-MS** *m*/*z:* 362.9 [M-H]⁻.

### 2-[3-(4,5-Dichloro-2-iodo)phenoxy] ethanol

A mixture of 3-(4,5-dichloro-2-iodophenyl)phenol (0.3 g, 0.82 mmol), 2-bromoethanol (0.20 g, 1.65 mmol), K₂CO₃ (0.57 g, 4.12 mmol) in DMF (5 mL) was stirred at 100°C under nitrogen for 16 h. The mixture was allowed to cool to room temperature and partitioned between ethyl acetate and brine. The organic layer was dried over Na2SO4, filtered and concentrated *in vacuo.* The residue was purified by flash column chromatography on silica gel (petroleum ether/ethyl acetate = 4:1) to afford the desired product (150 mg, 45% yield).

### N-(2-((4',5'-Dichloro-2'-iodo-[1,1'-biphenyl]-3-yl)oxy)ethyl)ethenesulfonamide

The title compound was prepared from 2-[3-(4,5-dichloro-2-iodo)phenoxy]ethanol in three steps followed the procedure described in Example 28. **¹H NMR** (400 MHz, CDCl₃) δ: 8.01 (s, 1H), 7.37-7.33 (m, 2H), 6.94-6.91 (m, 2H), 6.81-6.80 (m, 1H), 6.57 (dd, *J* = 10.0, 16.8 Hz, 1H), 6.30 (d, *J* = 16.4 Hz, 1H), 5.97 (d, *J* = 10.0 Hz, 1H), 4.77 (t, *J* = 6.0 Hz, 1H), 4.14 (t, *J* = 5.2 Hz, 2H), 3.49-3.45 (m, 2H). **ESI-MS** *m*/*z:* 496.0 [M-H]⁻.

### EXAMPLE 12

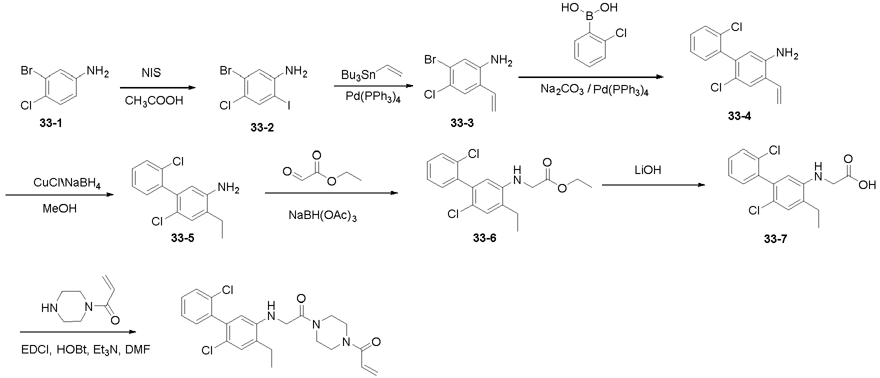

### 5-Bromo-4-chloro-2-iodobenzenamine

To a solution of 3-bromo-4-chlorobenzenamine (10.0 g, 48.5 mmol) in CH₃COOH (50 mL), NIS (10.9 g, 48.5 mmol) was added in portions and the resulting mixture was stirred at room temperature for 16 h. The mixture was concentrated *in vacuo.* The residue was dissolved in ethyl acetate, washed with saturated NaHCO₃ solution and brine, dried over Na₂SO₄ and concentrated *in vacuo.* The residue was purified by flash column chromatography on silica gel (petroleum ether/ethyl acetate = 50:1) to afford the desired product (2.5 g, 15.5 % yield). **ESI-MS** *m*/*z:* 329.9 [M-H]⁻.

### 5-Bromo-4-chloro-2-vinylbenzenamine

A mixture of 5-bromo-4-chloro-2-iodobenzenamine (2.5 g, 7.51 mmol), tributyl(vinyl)stannane (2.4 g, 7.51 mmol), Pd(PPh₃)₄ (867 mg, 0.75 mmol) in toluene (25 mL) was stirred at reflux under argon for 16 h. The reaction mixture was allowed to cool to room temperature and quenched with aqueous KF solution. The mixture was partitioned between ethyl acetate and water. The organic layer was washed with brine, dried over Na₂SO₄ and concentrated. The residue was purified by flash column chromatography on silica gel (petroleum ether/ethyl acetate = 20:1) to afford the desired product (1.0 g, 57.4% yield). **ESI-MS** *m*/*z:* 232.2 [M+H]⁺.

### 2',6-Dichloro-4-vinyl-[1,1'-biphenyl]-3-amine

A mixture of 5-bromo-4-chloro-2-vinylbenzenamine (600 mg, 2.6 mmol), 2-chlorophenylboronic acid (1.25 g, 12.9 mmol), Pd(PPh₃)₄ (300 mg, 0.2 6mmol), Na₂CO₃ (1.4 g, 13.0 mmol) in 1,4-dioxane (20 mL) and water (5 mL) was stirred at reflux under argon for 16 h. The mixture was allowed to cool to room temperature and then concentrated *in vacuo.* The residue was purified by flash column chromatography on silica gel (petroleum ether/ethyl acetate = 30: 1) to afford the desired product (500 mg, 73.1% yield). **ESI-MS** *m*/*z:* 263.1 [M+H]⁺.

### 2',6-Dichloro-4-ethyl-[1,1'-biphenyl]-3-amine

A mixture of 2',6-dichloro-4-vinyl-[1,1'-biphenyl]-3-amine (500 mg ,1.9 mmol), CuCl (225 mg, 2.28 mmol) in MeOH (10 mL) at 0°C, NaBH₄ (722 mg, 19 mmol) was added in portions and the resulting mixture was stirred at room temperature for 20 min. The reaction was quenched with water and extracted with ethyl acetate. The organic layer was washed with brine, dried over Na₂SO₄ and concentrated *in vacuo.* The residue was purified by flash column chromatography on silica gel (petroleum ether/ethyl acetate = 30: 1) to afford the desired product (300 mg, 59.4% yield). **ESI-MS** *m*/*z:* 266.0 [M+H]⁺.

### 1-(4-(2-((2',6-Dichloro-4-ethyl-[1,1'-biphenyl]-3-yl)amino)acetyl)piperazin-1-yl)prop-2-en-1-one.

The title compound was prepared from 2',6-dichloro-4-ethyl-[1,1'-biphenyl]-3-amine in three steps followed the procedure described in Example 20 and the following procedure for the amide formation.

2-((2',6-dichloro-4-ethyl-[1,1'-biphenyl]-3-yl)amino)acetic acid (150 mg, 0.466 mmol) and Et₃N (235 mg, 2.33 mmol) in DMF (5 mL) at 0°C, EDCI.HCl (178 mg, 0.932 mmol) and HOBt (126 mg, 0.932 mmol) were added and the resulting mixture was stirred at 0°C for 30 min. To this mixture, tert-butyl piperazine-1-carboxylate (123 mg, 0.698 mmol) was added and then stirred at room temperature for 16 h. The reaction mixture was partitioned between ethyl acetate and water. The organic layer was washed with brine, dried over Na₂SO₄ and concentrated *in vacuo.* The residue was purified by flash column chromatography on silica gel (DCM/MeOH = 50:1) to afford the desired product (45 mg, 21.7% yield). **¹H NMR** (400 MHz, DMSO-*d6*) δ: 7.56-7.54 (m, 1H), 7.43-7.41 (m, 2H), 7.32-7.30 (m, 1H), 7.14 (s, 1H), 6.81 (dd, *J* = 10.4, 16.4 Hz, 1H), 6.54 (s, 1H), 6.13 (dd, *J* = 2.4, 16.8 Hz, 1H), 5.71 (dd, *J* = 2.0, 10.4 Hz, 1H), 5.25 (bs., 1H), 3.96 (d, *J* = 4.4 Hz, 2H), 3.56-3.52 (m, 8H), 2.54 (q, *J* = 7.2 Hz, 2H), 1.23(t, *J* = 7.2 Hz, 3H). **ESI-MS** *m*/*z:* 444.3 [M-H]⁻.

### EXAMPLE 13

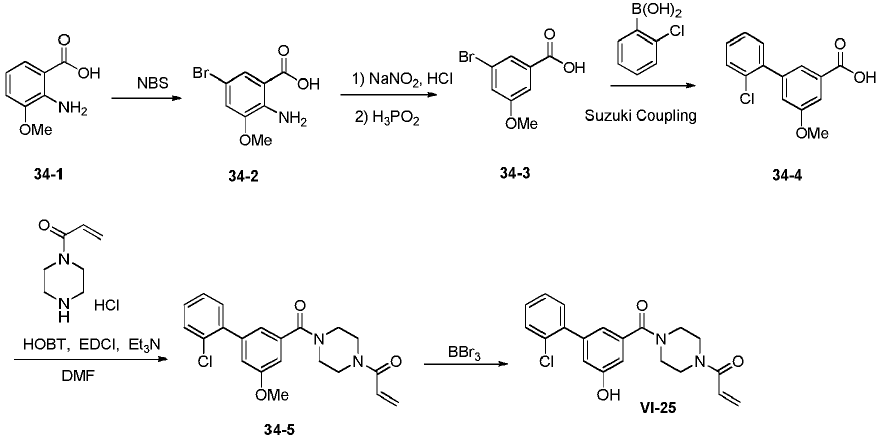

### 2-Amino-5-bromo-3-methoxybenzoic acid

To a solution of 2-amino-3-methoxybenzoic acid (5 g, 29.9 mmol) in MeOH (35 mL) at -5°C, NBS (5.59 g, 31.4 mmol) was added and the resulting mixture was stirred at 0°C for 16 h. The mixture was partitioned between ethyl acetate and water. The organic layer was washed with brine, dried over MgSO₄, filtered and concentrated *in vacuo* to afford the crude product (4 g, 54% yield). **ESI-MS** *m*/*z:* 244.2 [M-H]⁻.

### 3-Bromo-5-methoxybenzoic acid

To a solution of 2-amino-5-bromo-3-methoxybenzoic acid (4 g, 16.3 mmol) in water (20 mL) at 0°C, conc. HCl (7.5 mL, 90 mmol) and THF (20 mL) were added. The mixture was stirred for 30 min, and then NaNO₂ (3.16 g, 45.8 mmol) was added. The resulting mixture was stirred for 2 h and then hypophosphorous acid (5.1 g, 76 mmol, 50% in H₂O) was added to the reaction. The mixture was stirred at room temperature for 16 h. The precipitate was collected by filtration, washed with water and dried *in vacuo* to afford the desired product (3.2 g, 85% yield). **ESI-MS** *m*/*z*: 229.2 [M-H]⁻.

### 3-(2-Chlorophenyl)-5-methoxybenzoic acid

To a solution of 3-bromo-5-methoxybenzoic acid (1 g, 4.06 mmol) and 2-chlorophenylboronic acid (1.27 g, 8.13 mmol) in 1,4-dioxane (10 mL) and water (2 mL), Pd(PPh₃)₄ (468 mg, 0.40 mmol) and Na₂CO₃ (2.15 g, 20.3 mmol) were added and the resulting mixture was stirred at 80 °C for 16 h. The mixture was allowed to cool to room temperature and acidified with aqueous HCl (1.0 M) to adjust the pH to 3-4.The mixture was extracted with ethyl acetate. The organic layer was dried over MgSO₄, filtered and concentrated *in vacuo* to afford the desired product (800 mg, 75% yield) without further purification. **ESI-MS** *m*/*z:* 361.2 [M-H]⁻.

### 1-(4-(2'-Chloro-5-methoxy-[1,1'-biphenyl]-3-carbonyl)piperazin-1-yl)prop-2-en-1-one

To a solution of tert-butyl 4-acryloylpiperazine-1-carboxylate (260 mg, 1.07 mmol) in DCM (2 mL), a solution of HCl in MeOH (10 mL, 28.6 mmol) was added and the resulting mixture was stirred at room temperature for 1 h. The mixture was concentrated *in vacuo.* The residue was added to the solution of 3-(2-chlorophenyl)-5-methoxybenzoic acid (280 mg, 1.07 mmol), HOBt (290 mg, 2.17 mmol), EDCI.HCl (410 mg, 2.17 mmol) and Et₃N (324 mg, 3.21 mmol) in DMF (10 mL). The resulting mixture was stirred at room temperature for 16 h and partitioned between DCM and saturated NaHCO₃ solution. The organic layer was dried over MgSO₄, filtered and concentrated *in vacuo.* The residue was purified by flash column chromatography on silica gel (dichloromethane/methanol = 50:1) to afford the desired product (200 mg, 52% yield). **ESI-MS** *m*/*z:* 385.2[M+H]⁺.

### 1-(4-(2'-Chloro-5-hydroxy-[1,1'-biphenyl]-3-carbonyl)piperazin-1-yl)prop-2-en-1-one (VI-25)

To a solution of 1-(4-(2'-chloro-5-methoxy-[1,1'-biphenyl]-3-carbonyl)piperazin-1-yl)prop-2-en-1-one

(100 mg, 0.26 mmol) in DCM (15 mL) at -78°C, BBr₃ (650 mg, 2.6 mmol) was added and the resulting mixture was stirred at room temperature for 1 h. The mixture was poured into ice-water, basified with sat NaHCO₃ aqueous solution to adjust the pH to 7-8 and extracted with ethyl acetate. The organic layer was dried over MgSO₄, filtered and concentrated *in vacuo.* The residue was purified by flash column chromatography on silica gel (dichloromethane/methanol = 50: 1) to afford the desired product (25 mg, 26% yield). **¹H NMR** (400 MHz, DMSO-*d6*) δ: 9.96 (s, 1H), 7.58-7.56 (m, 1H), 7.42-7.40 (m, 3H), 6.89-6.75 (m, 4H), 6.14 (dd, *J* = 2.0, 16.8 Hz, 1H), 5.71 (dd, *J* = 2.0, 10.0 Hz, 1H), 3.68-3.44 (m, 8H). **ESI-MS** *m*/*z:* 371.2 [M+H]⁺.

### EXAMPLE 14

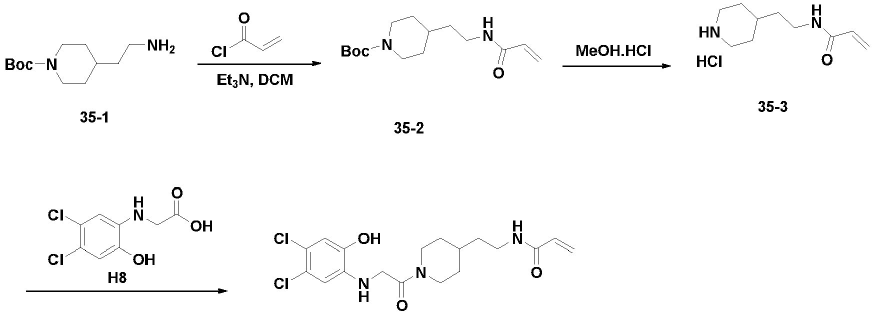

### tert-Butyl 4-(2-acrylamidoethyl)piperidine-1-carboxylate

To a stirred mixture of tert-butyl 4-(2-aminoethyl)piperidine-1-carboxylate (1.0 g, 4.38 mmol) in DCM (20 mL) at 0 °C, Et₃N (1.33 g, 13.14 mmol) was added and the resulting mixture was stirred at 0°C for 5 min. To this mixture, a solution of acryloyl chloride (0.39 g, 4.38 mmol) in DCM (5 mL) was added dropwise. The resulting mixture was stirred at room temperature for 30 min. The reaction mixture was poured into water (20 mL) and extracted with DCM (30 mL x 3). The combined organic layer was washed with brine (15 mL x 3), dried over anhydrous Na₂SO₄, filtered and concentrated *in vacuo.* The residue was purified by flash column chromatography on silica gel (10-50% ethyl acetate/dichloroethane) to afford the desired product (0.6 g, 50 % yield) as a solid.

### N-(2-(Piperidin-4-yl)ethyl)acrylamide

A mixture of tert-butyl 4-(2-acrylamidoethyl)piperidine-1-carboxylate (600 mg, 2.13 mmol) in HCl/MeOH (60 mL, 2.86 M) was stirred at room temperature for 1 h. The mixture was concentrated *in vacuo* to yield the crude product (550 mg) as a solid which was used directly in the next step without further purification.

### N-(2-(1-(2-((4,5-Dichloro-2-hydroxyphenyl)amino)acetyl)piperidin-4-yl)ethyl)acrylamide

To a stirred solution of 2-((4,5-dichloro-2-hydroxyphenyl)amino)acetic acid (200 mg, 0.85 mmol) in DMF (30 mL) at room temperature, N-(2-(piperidin-4-yl)ethyl)acrylamide (222.8 mg, 1.02 mmol) was added followed by HOBt (172.9 mg, 1.28 mmol), EDCI.HCl (244.7 mg, 1.28 mmol) and Et₃N (257.9 mg, 2.55 mmol). The reaction mixture was stirred at room temperature for 1 h and then partitioned between ethyl acetate and brine. The organic layer was dried over anhydrous Na₂SO₄, filtered and concentrated *in vacuo.* The residue was purified by flash column chromatography on silica gel (1-3% methanol/dichloroethane) to afford the desired product (120 mg, 29 % yield) as a solid. **¹H NMR** (400MHz, DMSO-*d6*) δ: 10.15 (s, 1H), 8.07 (t, *J* = 5.2 Hz, 1H), 6.78 (s, 1H), 6.72 (s, 1H), 6.18 (dd, *J* = 10.0, 17.2 Hz, 1H), 6.06 (dd, *J* = 2.4, 17.2 Hz, 1H), 5.56 (dd, *J* = 2.4, 10.4 Hz, 1H), 5.34 (t, *J* = 4.0 Hz, 1H), 4.36 (d, *J* = 12.8 Hz, 1H), 3.94-3.87 (m, 3H), 3.20-3.15 (m, 2H), 2.98-2.92 (m, 1H), 2.63-2.57 (m, 1H), 1.74-1.69 (m, 2H), 1.57-1.52 (m, 1H), 1.41-1.35 (m, 2H), 1.12-0.95 (m, 2H). **ESI-MS** *m*/*z:* 400.4[M+H]⁺.

### EXAMPLE 15

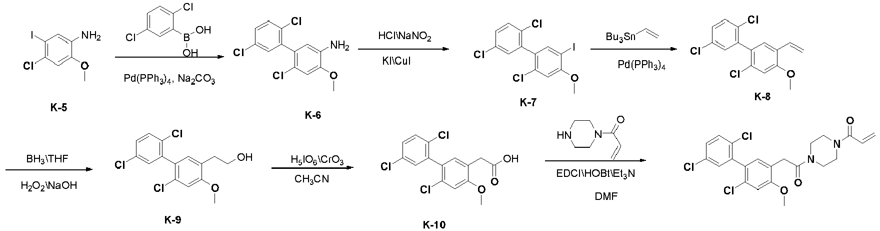

### 2',5',6-Trichloro-4-methoxy-[1,1'-biphenyl]-3-amine

A mixture of 4-chloro-5-iodo-2-methoxybenzenamine (4.1 g, 14.5 mmol), 2,5-dichlorophenylboronic acid (3.3 g, 17.4 mmol), Pd(PPh₃)₄ (500 mg, 1.45 mmol) and Na₂CO₃ (4.7 g, 43.5 mmol) in 1,4-dioxane (150 mL) and water (15 mL) was stirred at 80°C under argon for 16 h. The mixture was allowed to cool to room temperature, and then partitioned between ethyl acetate and water. The organic layer was washed with brine, dried over Na₂SO₄ and concentrated *in vacuo.* The residue was purified by flash column chromatography on silica gel (petroleum ether/ethyl acetate = 20:1) to afford the desired product (3.3 g, 75.8 % yield) as an off-white solid.

### 2,2',5'-Trichloro-5-iodo-4-methoxy-1,1'-biphenyl

A mixture of 2',5',6-trichloro-4-methoxy-[1,1'-biphenyl]-3-amine (1.0 g, 3.3 mmol) in conc. HCl (10 mL) and water (10 mL) at 0°C, NaNO₂ (350 mg, 5 mmol) was added in portions and the resulting solution was stirred at -5 - 0°C for 30 min. To this mixture, a solution of KI (2.2 g, 13.2 mmol) in H₂O (10 mL) and CuI (630 mg, 3.3 mmol) was added slowly. The resulting solution was stirred at room temperature for 45 min and then extracted with ethyl acetate. The organic layer was washed with saturated NaHCO₃ aqueous solution and brine, dried over Na₂SO₄ and concentrated *in vacuo.* The residue was purified by flash column chromatography on silica gel (petroleum ether/ethyl acetate = 20: 1) to afford the desired product (1.17 g, 87% yield) as a dark oil.

### 2,2',5'-Trichloro-4-methoxy-5-vinyl-1,1'-biphenyl

A mixture of 2,2',5'-trichloro-5-iodo-4-methoxy-1,1'-biphenyl (1.17 g, 2.9 mmol), tributyl(vinyl)stannane (1.1 g, 3.5 mmol), Pd(PPh₃)₄ (670mg, 0.6 mmol) in toluene (30 mL) was stirred at reflux under argon for 6 h. The reaction mixture was quenched with KF aqueous solution and then extracted with ethyl acetate. The organic layer was washed with brine, dried over Na₂SO₄ and concentrated *in vacuo.* The residue was purified by flash column chromatography on silica gel (petroleum ether/DCM = 100:1) to afford the desired product (310 mg, 35% yield) as a white solid.

### 2-(2',5',6-Trichloro-4-methoxy-[1,1'-biphenyl]-3-yl)ethanol

To a stirring solution of 2,2',5'-trichloro-4-methoxy-5-vinyl-1,1'-biphenyl (310 mg, 1 mmol) in THF (15 mL) under nitrogen at room temperature, BH₃ (2 mL, 1 N) was added. After stirring for 8 h, a mixture of NaOH (160 mg, 4 mmol) in water (3 mL) and H₂O₂ (30% in H₂O, 0.3 g, 4 mmol) was added and the resulting mixture was stirred for 6 h. The reaction was quenched with NaHCO₃ aqueous solution and extracted with ethyl acetate. The organic layer was washed with brine, dried over Na₂SO₄ and concentrated *in vacuo.* The residue was purified by flash column chromatography on silica gel (petroleum ether/ethyl acetate = 4:1) to afford the desired product (180 mg, 54% yield) as a colorless oil.

### 2-(2',5',6-Trichloro-4-methoxy-[1,1'-biphenyl]-3-yl)acetic acid

To a solution of 2-(2',5',6-Trichloro-4-methoxy-[1,1'-biphenyl]-3-yl)ethanol (180 mg, 0.54 mmol) in CH₃CN (20 mL) at 0°C, H₅IO₅\CrO₃ (3.7 mL, 1.63 mmol, 0.44 M in water) was added and the resulting mixture was stirred at 0°C for 1 h. The reaction was quenched with Na₂HPO₄ and diluted with ethyl acetate. The organic layer was washed with brine, dried over Na₂SO₄ and concentrated *in vacuo* to afford the product (110 mg, 59% yield) as a colorless oil. **ESI-MS** *m*/*z:* 343.1 [M-H]⁻.

### 1-(4-(2-(2',5',6-Trichloro-4-methoxy-[1,1'-biphenyl]-3-yl)acetyl)piperazin-1-yl)prop-2-en-1-one

To a solution of 2-(2',5',6Ttrichloro-4-methoxy-[1,1'-biphenyl]-3-yl)acetic acid (110 mg, 0.318 mmol) Et₃N (96 mg, 0.954 mmol) in DMF (3 mL) at 0°C, EDCI.HCl (92 mg, 0.477 mmol) and HOBt (65 mg, 0.477 mmol) were added and the resulting mixture was stirred at 0°C for 30 min. To this mixture, 1-(piperazin-1-yl)prop-2-en-1-one (67 mg, 0.382 mmol) was added. The resulting mixture was stirred at room temperature for 16 h and then partitioned between ethyl acetate and water. The organic layer was washed with brine, dried over Na₂SO₄ and concentrated *in vacuo.* The residue was purified by flash column chromatography on silica gel (DCM/MeOH = 30:1) to afford the desired product (37 mg, 24.97% yield) as an off-white solid. **¹H NMR** (400 MHz, DMSO-*d6*) δ: 7.61 (d, *J* = 8.4 Hz,1H), 7.52 (dd, *J* = 2.4, 8.8 Hz, 1H), 7.40 (d, *J* = 2.4 Hz, 1H), 7.20 (s, 1H), 7.10 (s, 1H), 6.81 (dd, *J* = 10.8, 15.2 Hz, 1H), 6.13 (dd, *J* = 2.4, 16.8 Hz, 1H), 5.71 (dd, *J* = 2.0, 10.4 Hz, 1H), 3.84 (s, 3H), 3.67 (s, 2H), 3.54-3.41 (m, 8H). **ESI-MS** *m*/*z:* 467.1 [M+H]⁺.

### EXAMPLE 16

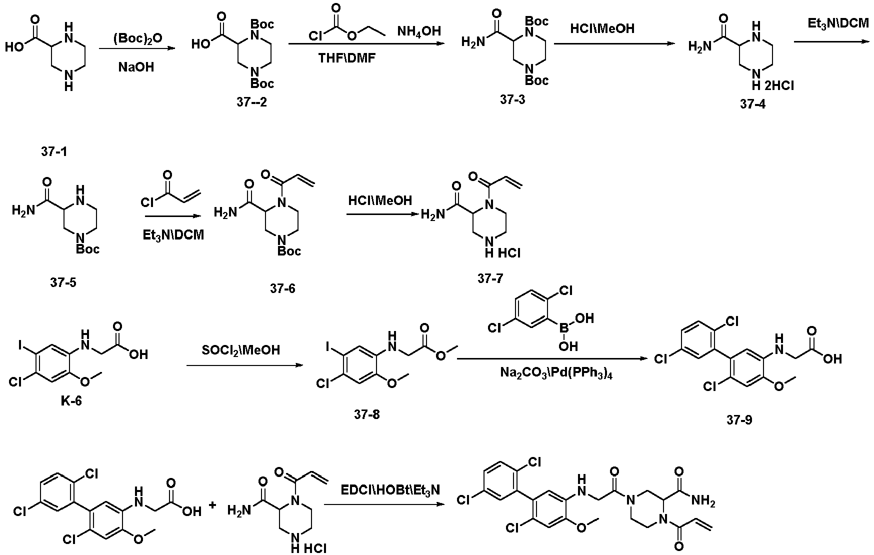

### Methyl 2-(4-chloro-5-iodo-2-methoxyphenylamino)acetate

A mixture of tert-butyl 2-(4-chloro-5-iodo-2-methoxyphenylamino)acetic acid (3.0 g, 8.7 mmol), SOCl₂ (3 mL) in MeOH (20 mL) was stirred at reflux for 2 h. The mixture was concentrated *in vacuo* to yield the crude product (3.1 g) as a yellow solid.

### 2-((2',5',6-Trichloro-4-methoxy-[1,1'-biphenyl]-3-yl)amino)acetic acid

A mixture of methyl 2-(4-chloro-5-iodo-2-methoxyphenylamino)acetate (5.0 g, 14.08 mmol), 2,5-dichlorophenylboronic acid (4.03 g, 21.12 mmol), Pd(PPh₃)₄ (1.626 g, 1.04 mmol), Na₂CO₃ (4.477 g, 42.24 mmol) in 1,4-dioxane (100 mL) and water (20 mL) was stirred at reflux under argon for 6 h. Then reaction mixture was allowed to cool to room temperature, quenched with water and acidified with HCl (10% in water) to adjust the pH to 3-4. The mixture was extracted with ethyl acetate, washed with brine, dried over Na₂SO₄ and concentrated *in vacuo.* The residue was purified by flash column chromatography on silica gel (50% petroleum ether/ethyl acetate) to afford the desired product (3 g, 59% yield) as an off-white solid. **ESI-MS** *m*/*z:* 360.2 [M-H]⁻.

### 1,4-di(tert-Butoxycarbonyl)piperazine-2-carboxylic acid

To a solution of piperazine-2-carboxylic acid (21.2 g, 0.16 mol) in 1,4-dioxane at 0°C, NaOH (80 mL, 400 mmol) was added slowly (over 15 min) followed by (Boc)₂O (71 g, 33 mol) and the resulting mixture was stirred at room temperature for 16 h. The mixture was concentrated *in vacuo.* The residue was dissolved in water (100 mL), acidified with conc. HCl at 0°C to adjust the pH to 2-3 and then extracted with ethyl acetate. The organic layer was washed with brine, dried over Na₂SO₄ and concentrated *in vacuo* to afford the product (44.2 g, 83.7% yield) as an off-white solid.

### di-tert-Butyl 2-carbamoylpiperazine-1,4-dicarboxylate

To a solution of 1,4-di(tert-butoxycarbonyl)piperazine-2-carboxylic acid (8.21 g, 24.85 mmol) was dissolved in THF (50 mL) and Et₃N (20 mL) at 0°C, ethyl chloroformate (2.8 g, 26.1 mmol) was added dropwise. After stirring at -5 - 0°C for 1 h, NH₄OH (20 mL) was added and the resulting solution was stirred at room temperature for 1 h. The mixture was poured into water and extracted with ethyl acetate. The organic layer was washed with 1N NaOH and brine, dried over Na₂SO₄ and concentrated *in vacuo* to give the product (7.5 g, 91.7% yield) as an off-white solid.

### Piperazine-2-carboxamide dihydrochloride

A mixture of di-tert-butyl 2-carbamoylpiperazine-1,4-dicarboxylate (7.5 g, 22.79 mmol), in HCl/MeOH (75 mL, 2.86 N) was stirred at room temperature for 16 h. The mixture was concentrated *in vacuo* to yield the product (4.58 g, 100% yield) as a yellow solid.

### tert-Butyl 3-carbamoylpiperazine-1-carboxylate

To a a solution of piperazine-2-carboxamide dihydrochloride (2.02 g, 10 mmol) and Et₃N (3.03 g, 30 mmol) in DCM (40 mL) at 0°C, (Boc)₂O (2.18 g, 10 mmol) was added dropwise (over 1 h). The resulting solution was stirred at room temperature for 16 h and then partitioned between ethyl acetate and water. The organic layer was washed with brine, dried over Na₂SO₄ and concentrated *in vacuo.* The residue was purified by flash column chromatography on silica gel (DCM/MeOH = 30:1) to afford the desired product (1.1 g, 48.0% yield).

### tert-Butyl 4-acryloyl-3-carbamoylpiperazine-1-carboxylate

To a solution of tert-butyl 3-carbamoylpiperazine-1-carboxylate (300 mg, 1.31 mmol) and Et₃N (396 mg, 3.93 mmol) in DCM (5 mL) at 0°C, acryloyl chloride (130 mg, 1.44 mmol) in DCM (1 mL) was added and the resulting mixture was stirred at room temperature for 1.5 h. The mixture was partitioned between DCM and saturated NaHCO₃ aqueous solution. The organic layer was washed with saturated NaHCO₃ and brine, dried over Na₂SO₄ and concentrated *in vacuo.* The residue was purified by flash column chromatography on silica gel (DCM/MeOH = 30:1) to afford the desired product (200 mg, 53.9% yield) as an off-white solid.

### 1-Acryloylpiperazine-2-carboxamide hydrochloride

A mixture of tert-butyl 4-acryloyl-3-carbamoylpiperazine-1-carboxylate (200 mg, 0.706 mmol) in HCl/MeOH (20 mL, 2.86 N) was stirred at room temperature for 1 h. The mixture was concentrated *in vacuo* to yield the crude product (160 mg) as a yellow solid which was used directly in next step without further purification.

### 1-Acryloyl-4-(2-((2',5',6-trichloro-4-methoxy-[1,1'-biphenyl]-3-yl)amino)acetyl)piperazine-2-carboxamide

2-((2',5',6-Trichloro-4-methoxy-[1,1'-biphenyl]-3-yl)amino)acetic acid (231 mg, 0.641 mmol) and Et₃N (592 mg, 2.564 mmol) in DMF (3 mL) at 0°C, EDCI.HCl(184 mg, 0.961 mmol) and HOBt (134 mg, 0.961 mmol) were added and the resulting mixture was stirred at 0°C for 30 min. To this mixture, the above 1-acryloylpiperazine-2-carboxamide hydrochloride (160 mg) was added and stirred at room temperature for 16 h. The mixture was partitioned between ethyl acetate and water. The organic layer was washed with brine, dried over Na₂SO₄ and concentrated *in vacuo.* The residue was purified by flash column chromatography on silica gel (DCM/MeOH = 30:1) to afford the desired product (10 mg, 2.97% yield) as an off-white solid. **¹H NMR** (400 MHz, DMSO-*d*6) δ: 7.60-7.38 (m, 3H), 6.70-6.99 (m, 1H), 6.86-6.78 (m, 1H), 6.54-6.49 (m, 1H), 6.16-6.10 (m, 1H), 5.76-5.69 (m, 1H), 5.32-5.31 (m, 1H), 4.93-4.84 (m, 1H), 4.93-4.84 (m, 1H), 4.70-4.62 (m, 1H), 4.35-4.32 (m, 1H), 4.04-3.96 (m, 2H), 3.89-3.63 (m, 1H), 2.90-2.86 (m, 1H). **ESI-MS** *m*/*z:* 525.2 [M+H]⁺.

### EXAMPLE 17

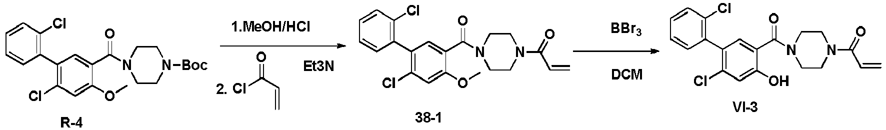

### 1-(4-(2',6-dichloro-4-methoxy-[1,1'-biphenyl]-3-carbonyl)piperazin-1-yl)prop-2-en-1-one

tert-Butyl 4-(2',6-dichloro-4-methoxy-[1,1'-biphenyl]-3-carbonyl)piperazine-1-carboxylate (200 mg, 0.43 mmol) was stirred in HCl in MeOH (2.86 M, 10 mL) for 1 h. The mixture was concentrated *in vacuo* to yield the crude product. The residue was dissolved in DCM (15 mL), triethylamine (0.5 mL), acryloyl chloride (40 mg, 0.43 mmol) was added to the mixture. The reaction mixture was stirred at room temperature for 30 min, poured into water, and extracted with DCM. The organic layer was washed with water and brine, dried over Na₂SO₄ and concentrated *in vacuo.* The residue was purified by flash column chromatography on silica gel (dichloromethane/methanol = 50:1) to afford the desired product (16 mg, 10% yield) as white solid. **ESI-MS** *m*/*z:* 419.2 [M+H]⁺.

### 1-(4-(2',6-Dichloro-4-hydroxy-[1,1'-biphenyl]-3-carbonyl)piperazin-1-yl)prop-2-en-1-one (VI-3)

To a solution of 1-(4-(2',6-Dichloro-4-methoxy-[1,1'-biphenyl]-3-carbonyl)piperazin-1-yl)prop-2-en-1-one (200 mg, 0.48 mmol) in DCM (15 mL) at - 60°C, BBr₃ (0.6 g, 2.4 mmol) was added dropwise and the resulting mixture was stirred at room temperature for 1 h. The mixture was poured into ice-water, basified with saturated NaHCO₃ solution to adjust the pH to 8 - 9, and extracted with DCM. The organic layer was dried over anhydrous sodium sulfate, filtered and concentrated *in vacuo.* The residue was purified by flash column chromatography on silica gel (dichloromethane/methanol = 20:1) to afford the desired product (10 mg, 5% yield). **¹H NMR** (400 MHz, DMSO-*d6*) δ: 10.6 (s, 1H), 7.57-7.33 (m, 5H), 7.12 (s, 1H), 7.05 (s, 1H), 6.80 (m, 1H), 6.15-6.11 (dd, *J* = 2, 16.8 Hz, 1H), 5.72-5.70 (m, 1H), 3.6 (m, 8H). **ESI-MS** *m*/*z:* 405.3 [M+H]⁺.

### EXAMPLE 18

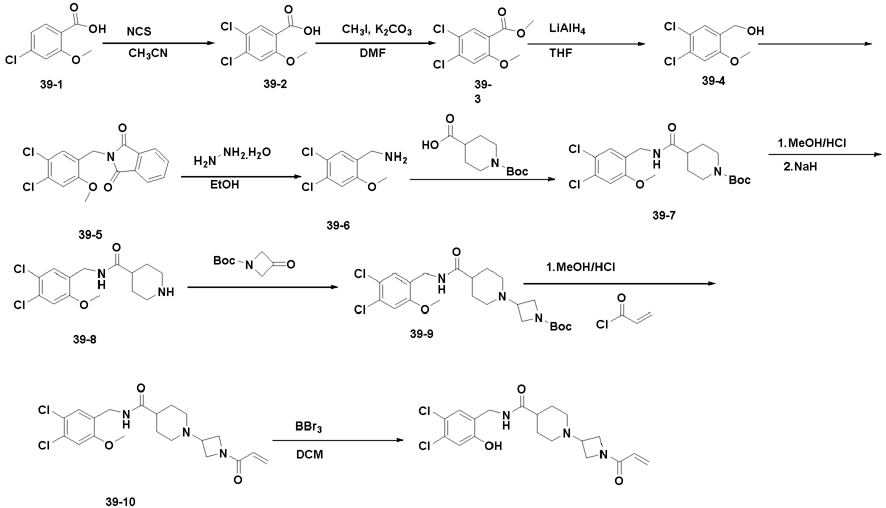

### 4,5-Dichloro-2-methoxybenzoic acid

A mixture of 4-chloro-2-methoxybenzoic acid (10 g, 53.6 mmol) and NCS (35 g, 19.2 mmol) in acetonitrile (200 mL) was stirred at room temperature for 48 h. The mixture was poured into water and extracted with ethyl acetate. The organic layer was washed with water and brine, dried over Na₂SO₄ and concentrated *in vacuo* to get the crude product (23.3 g).

### Methyl 4,5-dichloro-2-methoxybenzoate

A mixture of 4,5-dichloro-2-methoxybenzoic acid (8.2 g, 37 mmol) and K₂CO₃ (11.8 g, 111 mmol) in DMF(100 mL), CH₃I (6.3 g, 44 mmol) was added dropwise and the resulting mixture was stirred at room temperature for 16 h. The mixture was partitioned between ethyl acetate and water. The organic layer was washed with water and brine, dried over Na₂SO₄, and concentrated *in vacuo.* The residue was purified by flash column chromatography on silica gel (petroleum ether/ethyl acetate = 10:1) to afford the desired product.

### (4,5-Dichloro-2-methoxyphenyl)methanol

To a mixture of LiAlH₄ (2.42 g, 64 mmol) in THF (40 mL) at -40°C under argon, a solution of methyl 4,5-dichloro-2-methoxybenzoate (6 g, 26 mmol) in THF (50 mL) was added dropwise. The reaction mixture was stirred at -5°C to 5°C for 1 h. The mixture was cooled to -20°C and then water (2 mL) and NaOH (15%) aqueous were added. The resulting mixture was stirred for 15 min. The solid was filtered, and the cake rinsed with ethyl acetate. The combined filtrate was dried over Na₂SO₄ and concentrated *in vacuo* to afford the crude product (4.6 g).

### 2-(4,5-Dichloro-2-methoxybenzyl)isoindoline-1,3-dione

To a mixture of 4,5-dichloro-2-methoxyphenyl)methanol (4.5 g, 22 mmol), isoindoline-1,3-dione (9.6 g, 65 mmol) and PPh₃ (17 g, 65 mmol) in THF (100 mL) at room temperature, DIAD (13 g, 65 mmol) was added and the resulting mixture was stirred at room temperature for 16 h. The mixture was partitioned between ethyl acetate and water. The organic layer was washed brine, dried over Na₂SO₄, concentrated *in vacuo.* The residue was purified by flash column chromatography on silica gel (petroleum ether/ethyl acetate = 10: 1) to afford the desired product.

### (4,5-Dichloro-2-methoxyphenyl)methanamine

To a solution of 2-(4,5-dichloro-2-methoxybenzyl)isoindoline-1,3-dione (1.8 g, 5 mmol) in EtOH (5 mL), hydrazine hydrate (1.34 g, 27 mmol) was added and the resulting mixture was stirred at reflux for 1 h. The mixture was concentrated *in vacuo* and the residue was purified by flash column chromatography on silica gel (dichloromethane/methanol = 20:1) to afford the desired product (0.8 g, 78% yield).

### tert-Butyl 4-((4,5-dichloro-2-methoxybenzyl)carbamoyl)piperidine-1-carboxylate

The mixture of (4,5-dichloro-2-methoxyphenyl)methanamine (0.8 g, 3.90 mmol), 1-(tert-butoxycarbonyl)piperidine-4-carboxylic acid (0.88 g, 3.84 mmol), BOP (2 g, 1.16 mmol) and DIEA (1.6 g, 2.91 mmol) in DMF (20 mL) was stirred at room temperature for 1 h. The mixture was poured into water and extracted with ethyl acetate. The organic layer was washed with water and brine, dried over Na₂SO₄ and concentrated *in vacuo.* The residue was purified by flash column chromatography on silica gel (dichloromethane/methanol = 100:1) to afford the desired product (0.987 g, 62% yield). **ESI-MS** *m*/*z:* 415.4 [M-H]⁻.

### N-(4,5-Dichloro-2-methoxybenzyl)piperidine-4-carboxamide

The mixture of 4-((4,5-dichloro-2-methoxybenzyl)carbamoyl)piperidine-1-carboxylate (987 mg, 2.37 mmol) in HCl/MeOH (20 mL, 57.2 mmol) was stirred at room temperature for 1 h. Then the solvent was evaporated *in vacuo* and the residue was dissolved with dichloromethane (5 mL). To this mixture, NaH (85 mg, 3.55 mmol) was added. Then the resulting mixture was stirred at room temperature for 30 min. The solvent was removed under reduced pressure to yield the crude product (800 mg).

### tert-butyl 3-(4-(4,5-Dichloro-2-methoxybenzylcarbamoyl)piperidin-1-yl)azetidine-1-carboxylate

A mixture of *N*-(4,5-dichloro-2-methoxybenzyl)piperidine-4-carboxamide (750 mg, 2.37 mmol), *tert*-butyl 3-oxoazetidine-1-carboxylate (607 mg, 3.55 mmol), AcOH (1 mL) and MeOH (5 mL) was stirred at reflux for 2 h. To this mixture, NaBH₃(CN) (0.74 g, 11.85 mmol) was added and the resulting mixture was stirred at 60°C for 16 h. The mixture was allowed to cool to room temperature and partitioned between NH₄Cl aqueous solution and ethyl acetate. The organic layer was washed with water and brine, dried over Na₂SO₄ and concentrated *in vacuo.* The residue was purified by flash column chromatography on silica gel (dichloromethane/methanol = 50:1) to afford the desired product (220 mg, 18% yield). **ESI-MS** *m*/*z:* 472.3 [M+H]⁺.

### 1-(1-Acryloylazetidin-3-yl)-N-(4,5-dichloro-2-methoxybenzyl)piperidine-4-carboxamide

A mixture of *tert*-butyl 3-(4-(4,5-dichloro-2-methoxybenzylcarbamoyl)piperidin-1-yl)azetidine-1-carboxylate (210 mg, 0. 44 mmol) in HCl/MeOH (10 mL, 2.86 M) was stirred at room temperature for 1 h. The mixture was concentrated *in vacuo* to yield the crude residue. The residue was dissolved in DCM (5 mL), triethylamine (0.5 mL) and acryloyl chloride (40 mg, 0.43 mmol) were added. The reaction mixture was stirred at room temperature for 30 min and then partitioned between DCM and water. The organic layer was washed with water and brine, dried over Na₂SO₄ and concentrated *in vacuo.* The residue was purified by flash column chromatography on silica gel (dichloromethane/methanol = 50:1) to afford the desired product (150 mg, 82% yield).

### N-(4,5-Dichloro-2-hydroxybenzyl)-1-(1-acryloylazetidin-3-yl)piperidine-4-carboxamide

To a solution of 1-(1-acryloylazetidin-3-yl)-N-(4,5-dichloro-2-methoxybenzyl)piperidine-4-carboxamide (150 mg, 0.35mmol) in DCM (15 mL) at - 60°C, BBr₃ (0.6 g, 2.4 mmol) was added dropwise. The resulting mixture was allowed to warm to room temperature and stirred for 1 h. The mixture was poured into ice-water, basified with saturated NaHCO₃ solution to adjust the pH to 8 - 9, and then extracted with DCM. The organic layer was dried over anhydrous sodium sulfate and concentrated *in vacuo.* The residue was purified by flash column chromatography on silica gel (dichloromethane/methanol = 20:1) to afford the desired product (34 mg, 24% yield). **¹H NMR** (400 MHz, DMSO-*d6*) δ: 10.34 (s, 1H), 8.2-8.25 (m, 1H), 7.2 (s, 1H), 7.0 (s, 1H), 6.91 (s, 1H), 6.33-6.27 (m, 1H), 6.12-6.07 (dd, *J* = 2.4, 12.4 Hz, 1H), 5.68-5.65 (dd, *J* = 2.4, 10.4 Hz, 1H), 4.24-4.20 (m, 1H), 4.17-4.14 (m, 2H), 4.14-3.99 (m, 1H), 3.94-3.90 (m, 1H), 3.73-3.70 (m, 1H), 3.10 (s, 1H), 2.84-2.80 (m, 2H), 2.22 (m, 1H), 1.80 (s, 2H), 1.73-1.71(m, 2H), 1.63-1.57 (m, 2H). **ESI-MS** *m*/*z:* 412.2 [M+H]⁺.

### EXAMPLE 19

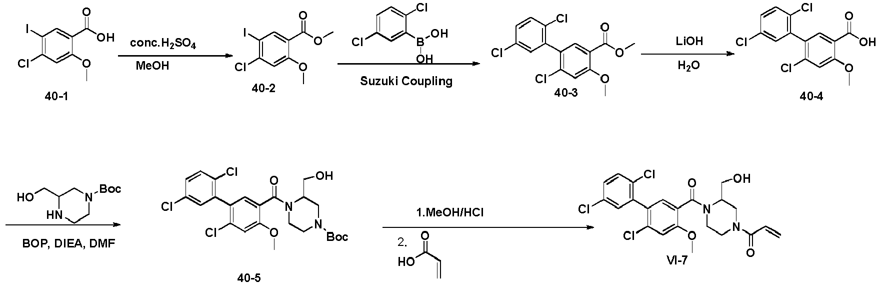

### Methyl 4-chloro-5-iodo-2-methoxybenzoate

A mixture of 4-chloro-5-iodo-2-methoxybenzoic acid (2 g, 6.41 mmol) concentrated sulfuric acid (1.5 mL) in MeOH (50 mL) was stirred at reflux for 16 h. The mixture was allowed to cool to room temperature and partitioned between water and ethyl acetate. The organic layer was washed with water and brine, dried over Na₂SO₄ and concentrated *in vacuo* to afford the desired product (1.85 g, 85% yield) as a yellow oil.

### Methyl 2',5',6-trichloro-4-methoxy-[1,1'-biphenyl]-3-carboxylate

A mixture of Methyl 4-chloro-5-iodo-2-methoxybenzoate (1.8 g, 5.51 mmol), (2,5-dichlorophenyl)boronic acid (2.1 g, 11.03 mmol), Pd(PPh₃)₄ (403 mg, 0.55 mmol), Na₂CO₃ (1.75 g, 16.54 mmol) in 1,4-dioxane (50 mL) and water (5 mL) was stirred at reflux under argon for 16 h. The mixture was filtered and filtrate was concentrated *in vacuo.* The residue was purified by flash column chromatography on silica gel (petroleum ether/ethyl acetate = 20:1) to afford the desired product (1.6 g, 85% yield).

### 1-(3-(Hydroxymethyl)-4-(2',5',6-trichloro-4-methoxy-[1,1'-biphenyl]-3-carbonyl)piperazin-1-yl)prop-2-en-1-one (VI-7)

The title compound was prepared from methyl 2',5',6-trichloro-4-methoxy-[1,1'-biphenyl]-3-carboxylate in three steps followed the procedure described in Example 20.

tert-butyl3-(hydroxymethyl)-4-(2',5',6-trichloro-4-methoxy-[1,1'-biphenyl]-3-carbonyl)piperazine-1-carboxylate (420 mg, 0.79 mmol) was stirred in HCl in MeOH (2.85 N). The solvent was removed under reduced pressure to yield the crude reside which was dissolved in DMF (20 mL), acrylic acid (57 mg, 0.79 mmol), BOP (421 mg, 0.95 mmol) and DIEA(409 mg, 3.17 mmol) were added. The reaction was stirred at room temperature for 1 h. The resulting mixture was poured into water, extracted with ethyl acetate and washed with water and brine. The combined organic layer was dried over Na₂SO₄, filtered and concentrated. The residue was purified by column chromatography on silica gel (dichloromethane/methanol = 60:1) to afford the desired product (92 mg, 24% yield). **¹H NMR** (400 MHz, DMSO-*d6*) δ: 7.64 - 7.20 (m, 5H), 6.83-6.70 (m ,1H), 6.16-6.11 (d, 1H), 5.74-5.71(d, 1H), 6.91 (s, 1H), 5.08- 4.01(m, 3H), 3.90-3.86 (d, 3H), 3.49-3.22(m, 2H), 2.93-2.74(m, 2H), 2.89-2.67 (m, 2H). **ESI-MS** *m*/*z*: 451.2 [M-H]⁻.

### EXAMPLE 20

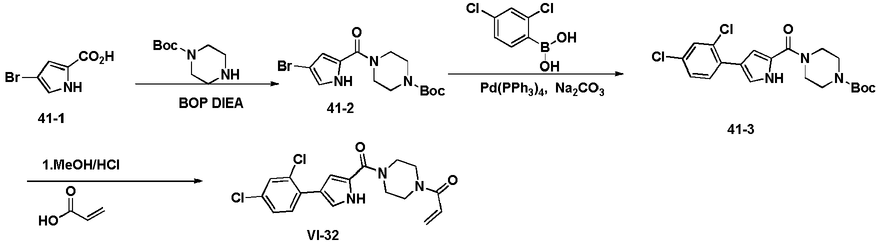

### tert-Butyl 4-(4-bromo-1H-pyrrole-2-carbonyl)piperazine-1-carboxylate

To a mixture of 4-bromo-1H-pyrrole-2-carboxylic acid (800 mg, 4.21 mmol), tert-butylpiperazine-1-carboxylate (822 mg, 4.42 mmol), BOP (2. 2g, 5.05 mmol) in DMF (5 mL), DIEA (1.63 g, 12.63 mmol) was added and the resulting mixture was stirred at room temperature for 1 h. The mixture was poured into water and extracted with ethyl acetate. The organic layer was washed with water and brine, dried over Na₂SO₄ and concentrated *in vacuo* to afford the crude product (920 mg, 61% yield) which was used directly in the next step without purification.

### tert-Butyl 4-(4-(2,4-dichlorophenyl)-1H-pyrrole-2-carbonyl)piperazine-1-carboxylate

A mixture of tert-butyl 4-(4-bromo-1H-pyrrole-2-carbonyl)piperazine-1-carboxylate (350 mg, 0.98 mmol), (2,4-dichlorophenyl) boronic acid (280 mg, 1.47 mmol), Pd(PPh₃)₄ (116 mg, 0.1 mmol), Na₂CO₃(312 mg, 2.94 mmol) in 1,4-dioxane (10 mL) and water (2 mL) was stirred at reflux under argon for 16 h. The mixture was allowed to cool to room temperature and concentrated in vacuo. The residue was purified by flash column chromatography on silica gel (petroleum ether/ethyl acetate = 30:1) to afford the desired product (273 mg, 59% yield).

### 1-(4-(4-(2,4-Dichlorophenyl)-1H-pyrrole-2-carbonyl)piperazin-1-yl)prop-2-en-1-one (VI-32)

The mixture of tert-butyl 4-(4-(2,4-dichlorophenyl)-1H-pyrrole-2-carbonyl)piperazine-1-carboxylate (270 mg, 0.64 mmol) in HCl/MeOH (20 mL, 57.2 mmol) was stirred for 1 h. The mixture was concentrated *in vacuo* and the residue was dissolved in DMF (5 mL). To this mixture, acrylic acid (50 mg, 0.7 mmol), BOP (437 mg, 0.72 mmol) and DIEA (248 mg, 1.92 mmol) were added. The reaction mixture was stirred at room temperature for 1 h. The mixture was partitioned between ethyl acetate and water. The organic layer was washed with brine, dried over Na₂SO₄ and concentrated *in vacuo.* The residue was purified by flash column chromatography on silica gel (dichloromethane/methanol = 60:1) to afford the desired product (40 mg, 27% yield) as a white solid. **¹H NMR** (400 MHz, DMSO-*d6*) δ: 11.88 (s, 1H), 7.66-7.63 (m, 2H), 7.43 (m, 1H), 7.38 (s, 1H), 6.91(s, 1H), 6.85-6.78(m, 1H), 6.18-6.13 (dd, *J* = 2.4, 12.4 Hz, 1H), 5.74-5.71 (dd, *J* = 2.4, 10.4 Hz, 1H), 3.76 (s, 4H), 3.68-3.63(m, 4H). **ESI-MS** *m*/*z:* 377.3 [M-H]⁻.

### EXAMPLE 21

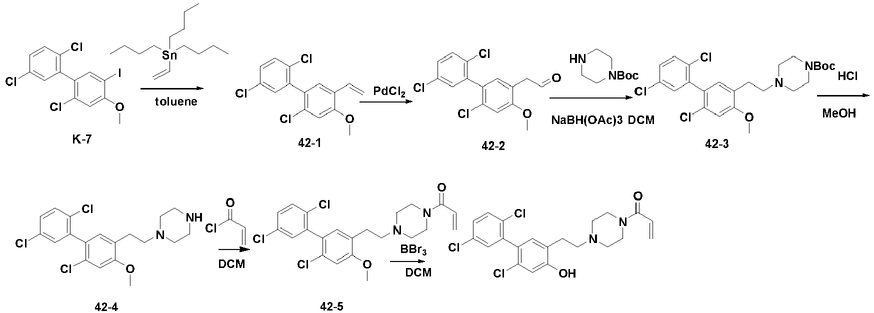

### 2,2',5'-Trichloro-4-methoxy-5-vinylbiphenyl

To a stirred solution of 2,2',5'-trichloro-5-iodo-4-methoxybiphenyl (3.4g, 8.3 mmol) in toluene (100 mL) at room temperature, tributyl(vinyl)stannane (3.1 g, 9.9 mmol) was added followed by Pd(PPh₃)₄ (2 g, 1.7 mmol). The reaction mixture was degassed and back-filled with nitrogen (several cycles) and then stirred at reflux for 16 h. The mixture was allowed to cool to room temperature, a solution of KF (1.72 g, 29.7 mmol) in H₂O (10 mL) was added and then stirred for 1 h. The mixture was partitioned between ethyl acetate and water. The organic layer was dried over anhydrous Na₂SO₄, filtered and concentrated *in vacuo.* The residue was purified by flash column chromatography on silica gel (1-2% ethyl acetate /petroleum ether) to afford the desired product (2.4 g, 92% yield) as a white solid

### 2-(2',5',6-Trichloro-4-methoxybiphenyl-3-yl)acetaldehyde

To a mixture of 2,2',5'-trichloro-4-methoxy-5-vinylbiphenyl (1.0 g, 3.2 mmol) in anhydrous DMF (10 mL), PdCl₂ (1.14 g, 6.4 mmol) was added and the resulting mixture was stirred at room temperature under nitrogen for 16 h. To this mixture, water (0.5 mL) was added and the resulting mixture was stirred at room temperature for another 6 h. The mixture was partitioned between ethyl acetate and water. The organic layer was washed with water, dried over anhydrous Na₂SO₄, filtered and concentrated *in vacuo.* The residue was purified by flash column chromatography on silica gel (1-3% ethyl acetate/petroleum ether) to afford the desired product (300 mg, 29% yield) as a white solid.

### tert-Butyl 4-(2-(2',5',6-trichloro-4-methoxybiphenyl-3-yl)ethyl)piperazine-1-carboxylate

To a stirred solution of 2-(2',5',6-trichloro-4-methoxybiphenyl-3-yl)acetaldehyde (300 mg, 0.92 mmol) in DCM (20 mL) at room temperature, tert-butyl piperazine-1-carboxylate (205 mg, 1.1 mmol) was added followed by drops of AcOH. The reaction mixture was stirred at room temperature for 1 h, and then NaBH(OAc)₃ (1.95 g, 9.2 mmol) was added. The resulting mixture was stirred at reflux for 16 h. The mixture was allowed to cool to room temperature, diluted with water and extracted with DCM. The organic layer was dried over anhydrous Na₂SO₄, filtered and concentrated *in vacuo.* The residue was purified by flash column chromatography on silica gel (1-33% ethyl acetate/petroleum ether) to afford the desired product (350 mg, 77% yield) as a white solid.

### 1-(4-(2-(2',5',6-Trichloro-4-hydroxybiphenyl-3-yl)ethyl)piperazin-1-yl)prop-2-en-1-one

The title compound was prepared from *tert*-butyl 4-(2-(2',5',6-trichloro-4-methoxybiphenyl-3-yl)ethyl)piperazine-1-carboxylate in three steps followeingthe procedure described in Example 18. **¹H NMR** (400 MHz, DMSO-*d6*) δ: 10.96 (bs., 1H), 7.54-7.59 (d, *J* = 8.6 Hz, 1H), 7.47-7.50 (m, 1H), 7.39-7.40 (d, *J* = 2.6 Hz, 1H), 7.08 (s, 1H), 6.94 (s, 1H), 6.77-6.84 (m, 1H), 6.09 (dd, *J* = 2.1, 17.0 Hz, 1H), 5.65 (dd, *J* = 2.6, 10.7 Hz, 1H), 3.50 (m, 4H), 2.72-2.76 (m, 2H), 2.54-2.57 (m, 2H), 2.47-2.50 (m, 4H). **ESI-MS** *m*/*z:* 439.1 [M + H]⁺.

### EXAMPLE 22

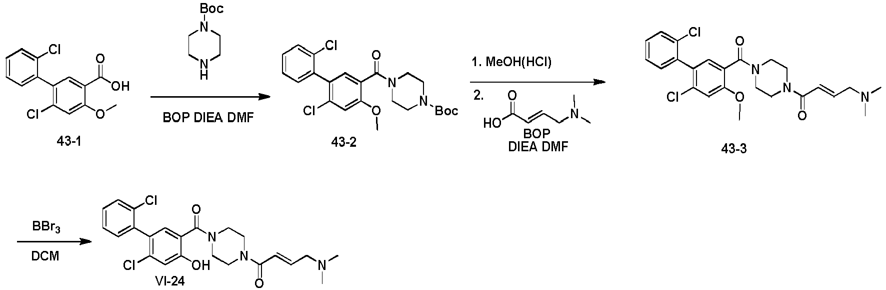

### tert-Butyl 4-(2',6-dichloro-4-methoxy-[1,1'-biphenyl]-3-carbonyl)piperazine-1-carboxylate

To a stirred solution of 2',6-dichloro-4-methoxy-[1,1'-biphenyl]-3-carboxylic acid (500 mg, 1.68 mmol) in DMF (10 mL) at room temperature, tert-butyl piperazine-1-carboxylate (345 mg, 1.85 mmol), BOP (892 mg, 2.02 mmol) and DIEA (542 mg, 4.2 mmol) were added and the resulting mixture was stirred at room temperature for 2 h. The reaction mixture was diluted with ethyl acetate and washed with brine. The organic layer was dried over anhydrous Na₂SO₄, filtered and concentrated *in vacuo.* The residue was purified by flash column chromatography on silica gel (dichloromethane/methanol = 50: 1) to afford the desired product (550 mg, 70% yield). **ESI-MS** *m*/*z:* 465.4 [M + H]⁺.

### (E)-1-(4-(2',6-dichloro-4-methoxy-[1,1'-biphenyl]-3-carbonyl)piperazin-1-yl)-4-(dimethylamino)but-2-en-1-one

A mixture of tert-Butyl 4-(2',6-dichloro-4-methoxy-[1,1'-biphenyl]-3-carbonyl)piperazine-1-carboxylate (550 mg, 1.18 mmol) in HCl/MeOH (20 mL, 57.2 mmol) was stirred at room temperature for 1 h. The solvent was removed under reduced pressure to yield the crude product. The crude residue was dissolved with DMF (10mL), 4-(dimethylamino)but-2-enoic acid (215 mg, 0.47 mmol), BOP (627 mg, 1.42 mmol) and DIEA (610 mg, 4.73 mmol) was added. The resulting mixture was stirred at room temperature for 2 h. The reaction mixture was diluted with ethyl acetate and washed with brine. The organic layer was dried over anhydrous Na₂SO₄, filtered and concentrated *in vacuo.* The residue was purified by flash column chromatography on silica gel (dichloromethane/methanol = 50: 1) to afford the desired product (450 mg, 80% yield, 2 steps). **ESI-MS** *m*/*z:* 476.4 [M + H]⁺.

### (E)-1-(4-(2',6-dichloro-4-hydroxy-[1,1'-biphenyl]-3-carbonyl)piperazin-1-yl)-4-(dimethylamino)but-2-en-1-one (VI-24)

A solution of (E)-1-(4-(2',6-dichloro-4-methoxy-[1,1'-biphenyl]-3-carbonyl)piperazin-1-yl)-4-(dimethylamino)but-2-en-1-one (270 mg, 0.57 mmol) in DCM (5 mL) at -78 °C, BBr₃ (1.43 g, 5.7 mmol) was added dropwise. The resulting mixture was stirred at room temperature for 2 h. The mixture was poured into ice-water, basified with the aqueous NaHCO₃ to adjust the pH to 7 and then extracted with DCM (3 x 20 mL). The organic layer was dried over Na₂SO₄ and concentrated *in vacuo.* The residue was purified by column chromatography on silica gel (dichloromethane/methanol = 50:1) to afford the desired product (150 mg, 57% yield). **¹HNMR** (400 MHz, DMSO-*d6*) δ: 13.12 (s, 1H), 8.02 (s, 1H), 7.81 (s, 1H), 6.81(m, 1H), 6.13 (dd, *J* = 2.8, 16.8 Hz, 1H), 5.71 (dd, *J* = 2.0, 10.4 Hz, 1H), 4.10 (s, 2H), 3.50-3.60 (m, 8H). **ESI-MS** *m*/*z:* 462.4 [M + H]⁺.

### EXAMPLE 23

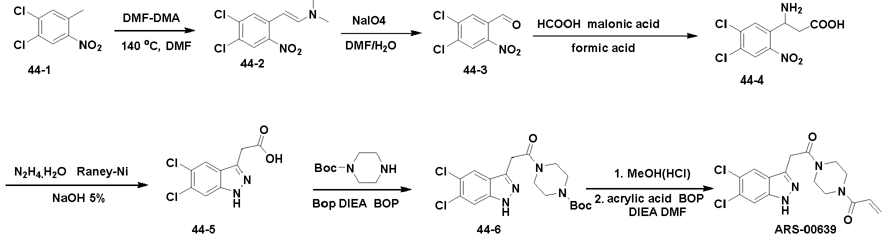

### 4,5-Dichloro-2-nitrobenzaldehyde

A mixture of 1,2-dichloro-4-methyl-5-nitrobenzene (2 g, 9.71 mmol) and DMF-DMA (3 g, 25.2 mmol) in DMF (50 mL) was stirred at 140°C for 14 h. The dark solution was cooled to 0°C, and then added to a solution of NaIO₄ (10.8 g, 50.5 mmol) in DMF/water (1:4, 25 mL) at 0°C. After stirring at room temperature for 8 h, the mixture was filtered and the cake was rinsed with ethyl acetate. The filtrate was diluted with ethyl acetate and washed with brine. The organic layer was dried over Na₂SO₄ and concentrated *in vacuo.* The residue was purified by column chromatography on silica gel (ethyl acetate/petroleum ether = 1:20) to afford the desire product (480 mg, 23% yield, 2 steps)

### 2-(5,6-Dichloro-1H-indazol-3-yl)acetic acid

To a stirred mixture of 4,5-dichloro-2-nitrobenzaldehyde (1.1 g, 5.00 mmol) and malonic acid (676 mg, 6.5 mmol) in formic acid (3 mL) at 40°C, ammonium formate (768 mg, 12.5 mmol) was added. The resulting mixture was stirred at 65°C for 1 h and then at 95°C for 4 h. To this mixture, conc. HCl (2.5 mL) was added and then stirred at 95°C for 1 h. The mixture was allowed to cool to room temperature, quenched with water (5 mL) and extracted with isobutyl ketone. The aqueous layer was adjusted the pH to 4 with KOH (50%) and filtered to afford the crude solid (500 mg). The solid was dissolved in a mixture of NaOH aqueous solution (8 mL, 5%) and N₂H₄.H₂O (90 mg, 1.79 mmol) and heated to 85°C. To this mixture, Raney-Ni was added and the resulting mixture was stirred at 85°C for 30 min. The mixture was allowed to cool to room temperature and filtered. The aqueous layer was acidified with aqueous HCl (6 M) to adjust the pH to 2. The precipitate was collected by filtration and dried *in vacuo* to afford the desired product (250 mg, yield 22%, 2 steps).

### 1-(4-(2-(5, 6-Dichloro-1H-indazol-3-yl)acetyl)piperazin-1-yl)prop-2-en-1-one

The title compound was prepared from 2-(5,6-dichloro-1H-indazol-3-yl)acetic acid in three steps followed the procedure described in Example 40. **¹H NMR** (400 MHz, DMSO-*d6*) δ: 7.56 (m, 1H), 7.33-7.45 (m, 3H), 7.12 (s, 1H), 7.07 (s, 1H), 6.62 (s, 2H), 3.60 (m, 8H), 3.10 (s, 2H), 2.20 (s, 6H). **ESI-MS** *m*/*z*: 367.2 [M + H]⁺.

### EXAMPLE 24

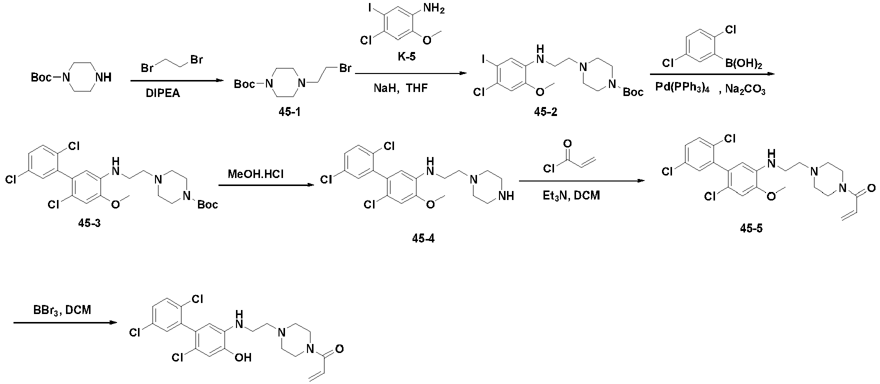

### tert-Butyl 4-(2-bromoethyl)piperazine-1-carboxylate

A mixture of tert-butyl piperazine-1-carboxylate (5.0 g, 26.9 mmol), 1,2-dibromoethane (25 mL), DIPEA (3.5 g, 26.9 mmol) was stirred at 30°C under argon for 72 h. The solvent was removed under reduced pressure and the residue was purified by column chromatography on silica gel (1-2% methanol/dichloroethane) to afford the desired product (2.8 g, 36% yield) as a solid. **ESI-MS** *m*/*z*: 293.1 [M+1]⁺*.*

### tert-Butyl 4-(2-((4-chloro-5-iodo-2-methoxyphenyl)amino)ethyl)piperazine-1-carboxylate

To a stirred solution of 4-chloro-5-iodo-2-methoxyaniline (968 mg, 3.42 mmol) in anhydrous THF (20 mL) at 0°C, NaH (60% in mineral oil, 205.2 mg, 5.13 mmol,) was added and the resulting mixture was stirred at reflux under nitrogen for 1 h. To this mixture, *tert*-butyl4-(2-bromoethyl)piperazine-1-carboxylate (500 mg, 1.71 mmol) was added and the resulting mixture was stirred at room temperature for 15 h. The mixture was concentrated *in vacuo* and the residue was partitioned between ethyl acetate and brine. The organic layer was dried over anhydrous Na₂SO₄, filtered and concentrated *in vacuo.* The residue was purified by flash column chromatography on silica gel (10-30% ethyl acetate/petroleum ether) to afford the desired product (180 mg, 21 % yield) as a solid.

### tert-Butyl4-(2-((2',5',6-trichloro-4-methoxy-[1,1'-biphenyl]-3-yl)amino)ethyl)piperazine-1-carboxylate

A mixture of tert-butyl 4-(2-((4-chloro-5-iodo-2-methoxyphenyl)amino)ethyl)piperazine-1-carboxylate (180 mg, 0.36mmol), (2,5-dichlorophenyl)boronic acid (138.9 mg, 0.72 mmol), Na₂CO₃ (114.5 mg, 1.08 mmol), Pd(PPh₃)₄ (42 mg, 0.036 mmol) in 1,4-dioxane (20 mL) and water (5mL) was stirred at 90°C under argon for 16 h. The mixture was allowed to cool to room temperature and then concentrated *in vacuo.* The residue was purified by column chromatography on silica gel (petroleum ether/ethyl acetate = 5:1) to afford the desired product (135 mg, 73% yield) as a solid. **ESI-MS** *m*/*z*: 514.3 [M+H]⁺.

### 1-(4-(2-((2',5',6-Trichloro-4-hydroxy-[1,1'-biphenyl]-3-yl)amino)ethyl)piperazin-1-yl)prop-2-en-1-one

The title compound was prepared from *tert*-butyl4-(2-((2',5',6-trichloro-4-methoxy-[1,1'-biphenyl]-3-yl)amino)ethyl)piperazine-1-carboxylate in three steps followed the procedure described in Example 18. **¹H NMR** (400 MHz, DMSO-*d6*) δ: 10.04 (s, 1H), 7.56 (d, *J* = 8.4 Hz, 1H), 7.48-7.45 (m, 1H), 7.37 (d, *J* = 2.8 Hz, 1H), 6.79 (dd, *J* = 10.4,16.8 Hz, 1H), 6.77 (s, 1H), 6.40 (s, 1H), 6.09 (dd, *J* = 2.4, 16.4 Hz, 1H), 5.67(dd, *J* = 2.4, 10.4 Hz, 1H), 4.88 (bs., 1H), 3.54-3.51 (m, 4H), 3.11(d, *J* = 4.8 Hz, 2H), 2.55 (t, *J* = 6.0 Hz, 2H), 2.39-2.38 (m, 4H). **ESI-MS** *m*/*z*: 454.1[M+H]⁺.

### EXAMPLE 25

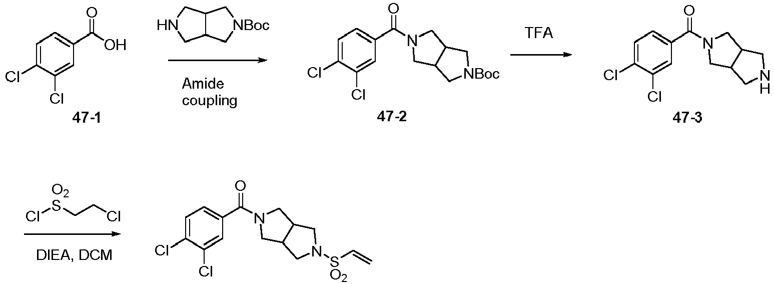

### tert-butyl 5-(3,4-dichlorobenzoyl)hexahydropyrrolo[3,4-c]pyrrole-2(1H)-carboxylate

To a solution of acid 3,4-dichlorobenzoic acid (0.45 g, 2.36 mmol), tert-butyl hexahydropyrrolo[3,4-c]pyrrole-2(1H)-carboxylate (0.5 g, 2.36 mmol) and HOBt (0.4 g, 2.83 mmol) in DMF (15 mL), EDCI.HCl (0.54 g, 2.83 mmol) and DIEA (1.5 g, 11.5 mmol) were added. The mixture was stirred at room temperature overnight and then partitioned between ethyl acetate and water. The organic layer was washed with brine, dried over Na₂SO₄, filtered and concentrated *in vacuo* to afford the crude product which was used directly in the next step.

### (3,4-dichlorophenyl)(hexahydropyrrolo[3,4-c]pyrrol-2(1H)-yl)methanone

tert-Butyl 5-(3,4-dichlorobenzoyl)hexahydropyrrolo[3,4-c]pyrrole-2(1H)-carboxylate (crude product from above step) was dissolved in 50% TFA in DCM (10 mL) and the resulting mixture was stirred at room temperature for 3 h. The mixture was concentrated *in vacuo.* The residue was dissolved in ethyl acetate and washed with saturated NaHCO₃ aqueous solution. The organic layer was dried over Na₂SO₄, filtered and concentrated *in vacuo* to afford the crude product. The crude product was used directly in the next step without further purification.

### (3,4-Dichlorophenyl)(5-(vinylsulfonyl)hexahydropyrrolo[3,4-c]pyrrol-2(1H)-yl)methanone

To a solution of (3,4-dichlorophenyl)(hexahydropyrrolo[3,4-c]pyrrol-2(1H)-yl)methanone (100 mg, 0.35 mmol) in DCM (10 mL) at 0°C, Et₃N (106 mg, 1 mmol) was added followed by sulfonyl chloride (60 mg, 0.37 mmol). The resulting mixture was stirred at room temperature overnight. The mixture was partitioned between DCM and water. The organic layer was dried over Na₂SO₄, filtered and concentrated *in vacuo.* The residue was purified by Isolera One (silica gel cartridge, 10-100% ethyl acetate/hexanes) to afford the desired product (14 mg, 11%). **¹H NMR** (300 MHz, CDCl₃) δ: 7.60 (d, *J* = 2.0 Hz, 1H), 7.51 (d, *J* = 8.2 Hz, 1H), 7.35 (dd, *J* = 2.0, 8.2 Hz, 1H), 6.50 (dd, *J* = 9.9, 16.5 Hz, 1H), 6.30 (d, *J* = 16.6 Hz, 1H), 6.09 (d, *J* = 9.9 Hz, 1H), 2.90-4.10 (m, 10H). **ESI-MS** *m*/*z:* 375.0 [M + H]⁺.

### EXAMPLE 26.

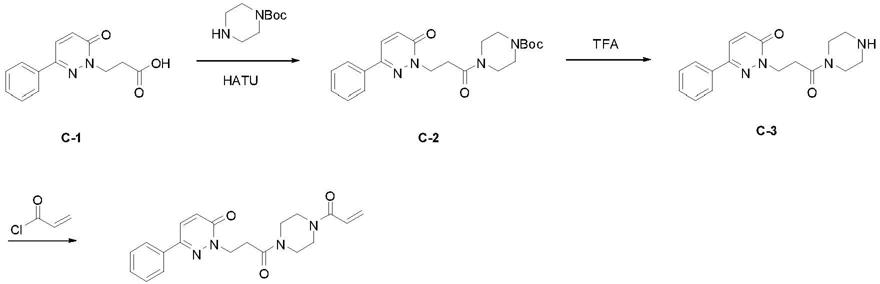

### EXAMPLE 27

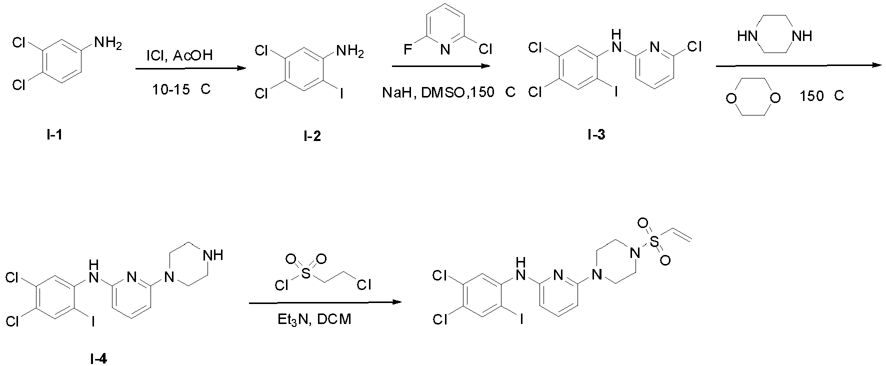

### EXAMPLE 28

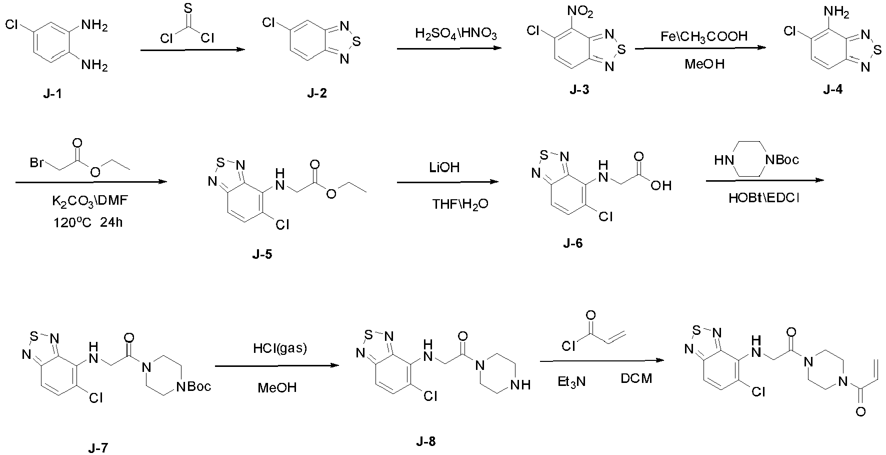

### EXAMPLE 29

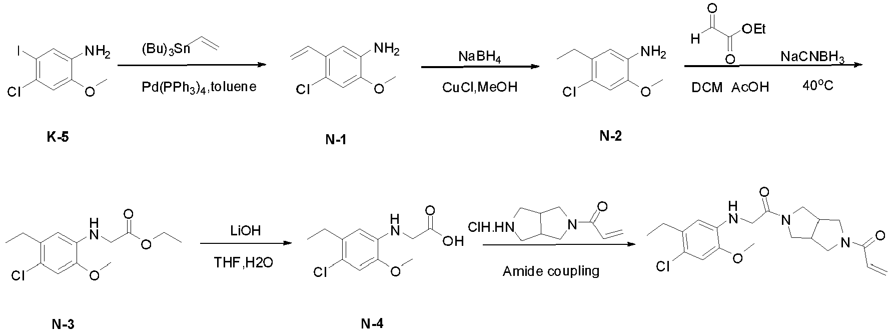

### EXAMPLE 30

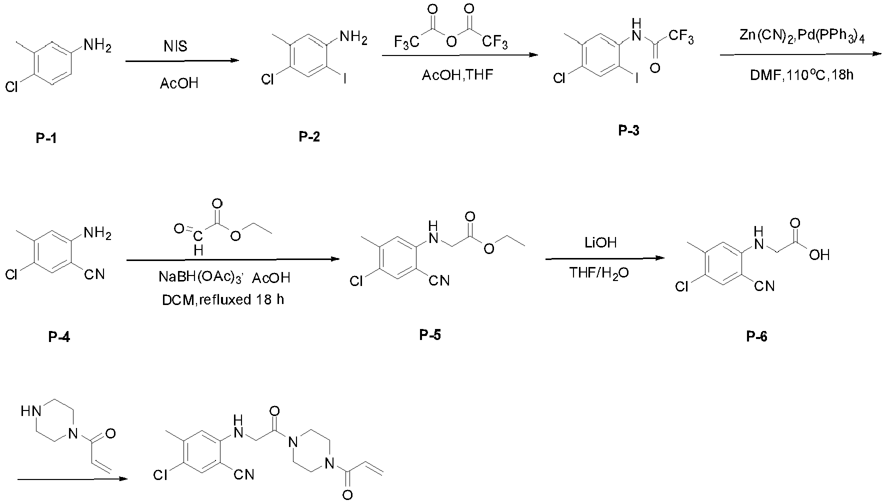

### EXAMPLE 31

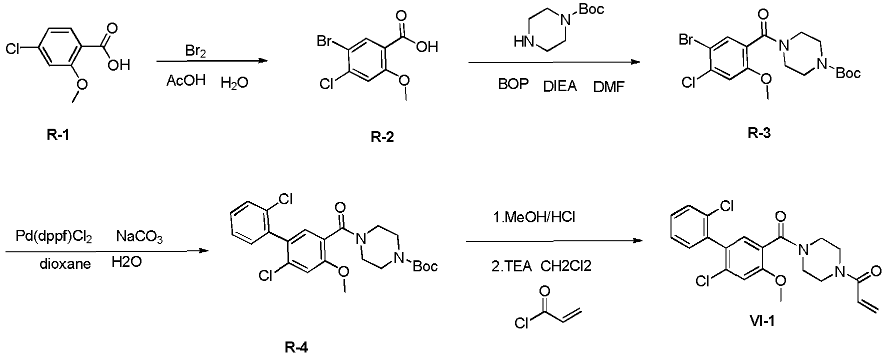

### EXAMPLE 32

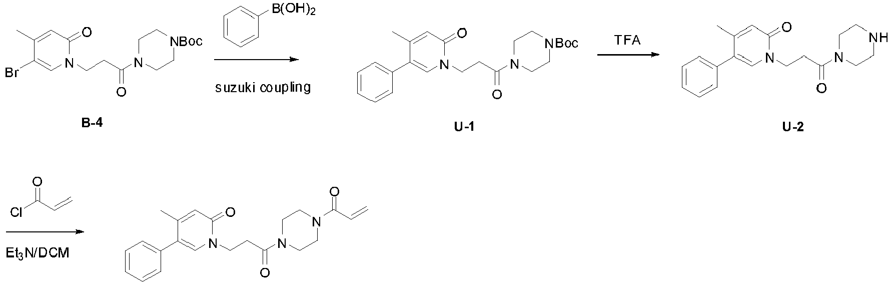

### EXAMPLE 33

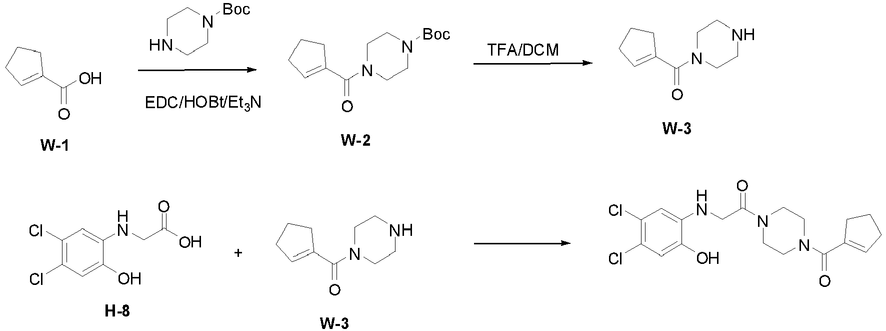

### EXAMPLE 34

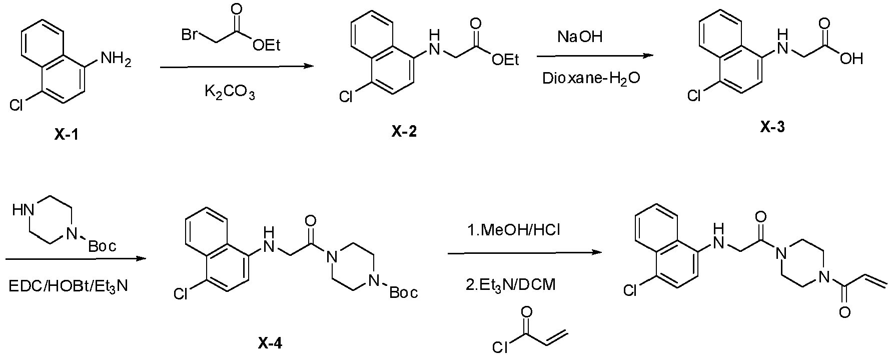

### EXAMPLE 35

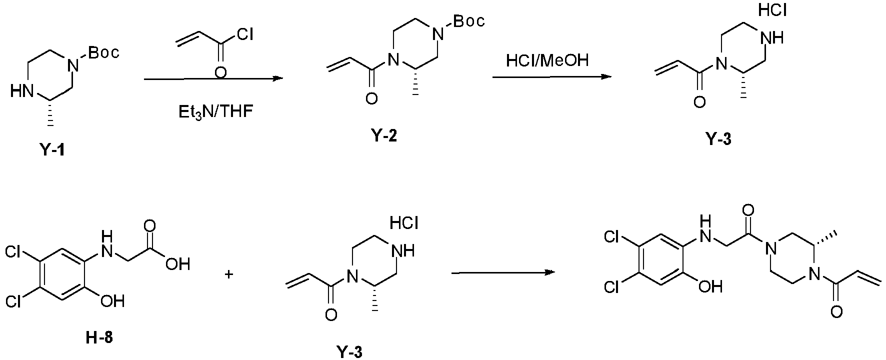

### EXAMPLE 36

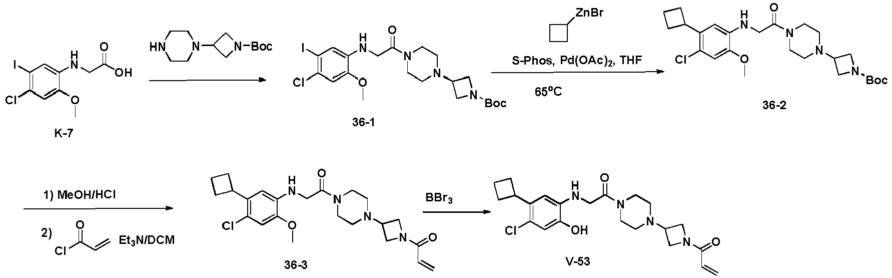

### tert-Butyl 3-(4-(2-(4-Chloro-5-iodo-2-methoxyphenylamino)acetyl)piperazin-1-yl)azetidine-1-carboxylate

To a solution of 2-(4-chloro-5-iodo-2-methoxyphenylamino)acetic acid (2.0 g, 5.88 mmol), tert-butyl 3-(piperazin-1-yl)azetidine-1-carboxylate (1.84 g, 7.64 mmol), EDCI.HCl (2.26 g, 11.76 mmol), and HOBt (1.59 g, 11.76 mmol) in DMF (3 mL) at 0°C, Et₃N (3.28 mL, 23.52 mmol) was added. The resulting mixture was stirred at RT for 16 h and then partitioned between ethyl acetate and water. The organic layer was washed with brine, dried over Na₂SO₄ and concentrated *in vacuo.* The residue was washed by a mixture of ethyl acetate / petroleum ether = 1:5 to afford the desired product (2.24 g, 67% yield) as a white solid. **ESI-MS** *m*/*z*: 565.4 [M + H]⁺.

### tert-Butyl 3-(4-(2-(4-chloro-5-cyclobutyl-2-methoxyphenylamino)acetyl)piperazin-1-yl)azetidine-1-carboxylate

A mixture of tert-butyl 3-(4-(2-(4-chloro-5-iodo-2-methoxyphenylamino)acetyl)piperazin-1-yl)azetidine-1-carboxylate (697 mg, 1.24 mmol), cyclobutylzinc bromide (4.46 mL, 2.23 mmol, 0.5 M in THF ), Pd(OAc)₂ (56 mg, 0.248 mmol), and S-Phos(102 mg, 0.248 mmol) in THF (15 mL) was stirred at 65°C under argon for 16 h. The mixture was allowed to cool to RT, quenched with aqueous NH₄Cl solution and then extracted with ethyl acetate. The organic layer was washed with brine, dried over Na₂SO₄, filtered and concentrated *in vacuo.* The residue was purified by flash column chromatography on silica gel (methanol/ dichloromethane = 1:30) to afford the desired product (596 mg, 98% yield) as a brown oil. **ESI-MS** *m*/*z:* 493.5 [M + H]⁺.

### 1-(3-(4-(2-(4-Chloro-5-cyclobutyl-2-hydroxyphenylamino)acetyl)piperazin-1-yl)azetidin-1-yl)prop-2-en-1-one (V-53) (Reference Example)

The title compound was prepared from tert-butyl 3-(4-(2-(4-chloro-5-cyclobutyl-2-methoxyphenylamino)acetyl)piperazin-1-yl)azetidine-1-carboxylate in three steps according to the procedure described in **Example 17**. **¹H NMR** (400 MHz, DMSO-*d6*) δ: 9.66 (s, 1H), 6.63 (s, 1H), 6.52 (s, 1H), 6.31 (dd, *J=* 10.2, 16.9 Hz, 1H), 6.10 (dd, *J* = 2.1, 16.8 Hz, 1H), 5.68 (dd, *J* = 2.1, 10.2 Hz, 1H), 5.16 (t, *J* = 4.4 Hz, 1H), 4.27-4.23 (m, 1H), 4.08-4.04 (m, 1H), 3.97-3.93 (m, 3H), 3.80-3.76 (m, 1H), 3.65-3.59 (m, 1H), 3.56-3.54 (m, 4H), 3.20-3.14 (m, 1H), 2.40-2.25 (m, 4H), 2.20-2.15 (m, 2H), 2.09-2.05 (m, 2H), 1.97-1.90 (m, 1H), 1.80-1.74 (m, 1H). **ESI-MS** *m*/*z*: 433.4 [M + H]⁺.

### EXAMPLE 37

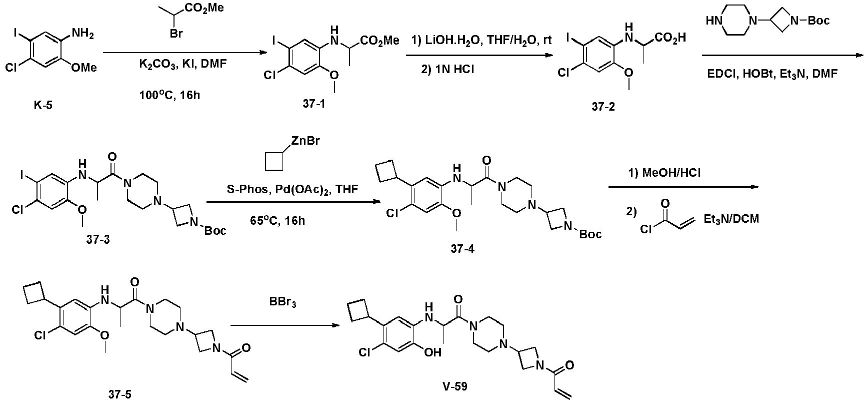

### Methyl 2-(4-chloro-5-iodo-2-methoxyphenylamino)propanoate

A mixture of tert-butyl 4-chloro-5-iodo-2-methoxybenzenamine (2 g, 7.07 mmol), methyl 2-bromopropanoate (1.17 g, 7.07 mmol), K₂CO₃ (1.94 g, 14.14 mmol) and KI (0.235 g, 1.414 mmol) in DMF (25 mL) was stirred at 100°C for 16 h. The mixture was allowed to cool to RT, quenched with aqueous NaHCO₃ solution and then extracted with ethyl acetate. The organic layer was washed with brine, dried over Na₂SO₄, filtered and concentrated *in vacuo.* The residue was purified by flash column chromatography on silica gel (ethyl acetate / petroleum ether = 1:20) to afford the desired product (1.12 g, 43% yield) as a yellow solid. ESI-MS *m*/*z*: 370.1 [M + H]⁺.

### 2-(4-Chloro-5-iodo-2-methoxyphenylamino)propanoic acid

To a solution of methyl 2-(4-chloro-5-iodo-2-methoxyphenylamino)propanoate (1.12 g, 3.04 mmol) in mixture of tetrahydrofuran (20 mL) and water (10 mL) at RT, LiOH.H₂O (0.51 g, 12.16 mmol) was added and the resulting mixture was stirred for 1 h. The aqueous phase was washed with TBME and then acidified with aqueous HCl (1 N) to adjust the pH to 5. The mixture was extracted with ethyl acetate. The organic layer was washed with brine, dried over anhydrous Na₂SO₄, filtered and concentrated *in vacuo* to afford the crude product (760 mg) which was used directly in the next step without further purification. **ESI-MS** *m*/*z*: 356.1 [M + H]+.

### Tert-Butyl 3-(4-(2-((4-Chloro-5-iodo-2-methoxyphenyl)amino)propanoyl)piperazin-1-yl)azetidine-1-carboxylate

To a solution of 2-(4-chloro-5-iodo-2-methoxyphenylamino)propanoic acid (760 mg, 2.13 mmol), tert-butyl 3-(piperazin-1-yl)azetidine-1-carboxylate (669 mg, 2.78 mmol), EDCI.HCl (818 mg, 4.26 mmol), HOBt (575 mg, 4.26 mmol) in DMF (8 mL) at 0°C, Et₃N (861 mg, 8.52 mmol) was added. The resulting mixture was stirred at RT for 16 h and then partitioned between ethyl acetate and water. The organic layer was washed with saturated NaHCO₃ solution and brine, dried over Na₂SO₄ and concentrated *in vacuo.* The residue was purified by flash column chromatography on silica gel (ethyl acetate / petroleum ether = 1 : 1) to afford the desired product (673 mg, 55% yield) as a white solid. **ESI-MS** *m*/*z*: 579.4 [M + H]⁺.

### Tert-Butyl 3-(4-(2-((4-Chloro-5-cyclobutyl-2-methoxyphenyl)amino)propanoyl)piperazin-1-yl)azetidine-1-carboxylate

A mixture of tert-butyl 3-(4-(2-((4-chloro-5-iodo-2-methoxyphenyl)amino)propanoyl)piperazin-1-yl)azetidine-1-carboxylate (673 mg, 1.162 mmol), cyclobutylzinc bromide (5.11 mL, 2.556 mmol, 0.5 M in THF ), Pd(Oac)₂ (52 mg, 0.23 mmol), S-Phos (95 mg, 0.23 mmol) in THF (10 mL) was stirred at 65°C under argon for 16 h. The mixture was allowed to cool to RT, quenched with aqueous NH₄Cl solution and then extracted with ethyl acetate. The organic layer was washed with brine, dried over Na₂SO₄, filtered and concentrated *in vacuo.* The residue was purified by flash column chromatography on silica gel (ethyl acetate / petroleum ether = 1 : 1) to afford the desired product (565 mg, 96% yield) as a light yellow solid. **ESI-MS** *m*/*z*: 507.6 [M + H]⁺.

### 1-(3-(4-(2-((4-Chloro-5-cyclobutyl-2-hydroxyphenyl)amino)propanoyl)piperazin-1-yl)azetidin-1-yl)prop-2-en-1-one (V-59) (Reference Example)

The title compound was prepared from tert-butyl 3-(4-(2-((4-chloro-5-cyclobutyl-2-methoxyphenyl)amino)propanoyl)piperazin-1-yl)azetidine-1-carboxylate in three steps according to the procedure described in **Example 17. ¹H NMR** (400 MHz, DMSO-*d6*) δ: 9.63 (s, 1H), 6.62 (s, 1H), 6.49 (s, 1H), 6.30 (dd, *J* = 10.1, 16.8 Hz, 1H), 6.10 (d, *J=* 18.7 Hz, 1H), 5.68 (d, *J* = 10.4 Hz, 1H), 4.86 (d, *J* = 9.2 Hz, 1H), 4.69-4.63 (m, 1H), 4.27-4.23 (m, 1H), 4.07-4.03 (m, 1H), 3.97-3.62 (m, 1H), 3.82-3.76 (m, 2H), 3.64-3.55 (m, 3H), 3.77-3.11 (m, 1H), 2.44-2.15 (m, 6H), 2.08-1.90 (m, 4H), 1.80-1.72 (m, 2H), 1.97-1.90 (m, 1H), 1.24 (d, *J* = 6.4 Hz, 3H). **ESI-MS** *m*/*z*: 447.4 [M + H]+.

### EXAMPLE 38

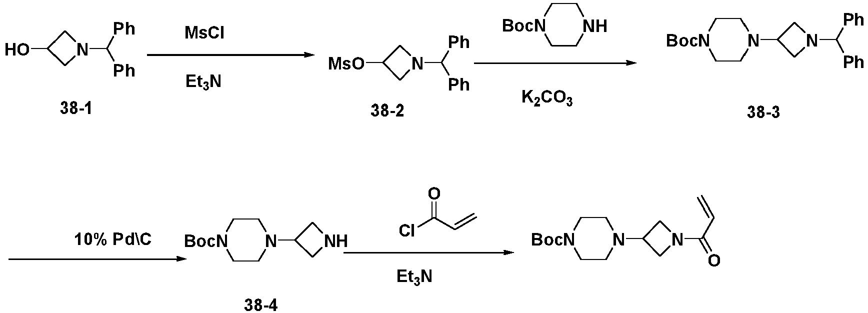

### 1-Benzhydrylazetidin-3-yl methanesulfonate

A mixture of 1-benzhydrylazetidin-3-ol (20.0 g, 83.68 mmol) and Et₃N (12.68 g, 125.52 mmol) in DCM (200 mL) at 0°C, MsCl (11.447 mg, 100.41 mmol) was added in portions and the resulting solution was stirred at RT for 1 h. The reaction mixture was diluted with ethyl acetate and washed with brine. The organic layer was dried over anhydrous Na₂SO₄, filtered and concentrated *in vacuo* to afford the desired product (26.526 g, 100% yield).

### tert-Butyl 4-(1-benzhydrylazetidin-3-yl)piperazine-1-carboxylate

A mixture of 1-benzhydrylazetidin-3-yl methanesulfonate (26.53 g, 83.68 mmol), tert-butyl piperazine-1-carboxylate( 18.68 g, 100.41 mmol) and K₂CO₃ (23.09 g, 163.36 mmol) in CH₃CN (200 mL) was stirred at 80°C for 16 h. The reaction mixture was cooled to RT and diluted with ethyl acetate and washed with brine. The organic layer was dried over anhydrous Na₂SO₄, filtered and concentrated *in vacuo.* The residue was purified by flash column chromatography on silica gel (dichloromethane/methanol = 50:1) to afford the desired product (25.5g, 80% yield).

### tert-Butyl 4-(azetidi-3-yl)piperazine-1-carboxylate

A mixture of tert-butyl 4-(1-benzhydrylazetidin-3-yl)piperazine-1-carboxylate (10.0 g, 24.57 mmol) and 10% Pd\C (2.5 g) in MeOH (100 mL) was stirred under H₂ atmosphere at 50°C for 48 h. The reaction mixture was cooled to RT and filtered. The filtrate was diluted concentrated *in vacuo* to afford a crude desire product (6.7 g) as a colorless oil.

### tert-Butyl 4-(1-acryloylazetidin-3-yl)piperazine-1-carboxylate

A mixture of tert-butyl 4-(azetidi-3-yl)piperazine-1-carboxylate (6.7 g, 27.80 mmol) and Et₃N (8.43 g, 83.40 mmol) in DCM (100 mL) at 0°C, acryloyl chloride (3.77 g, 41.7 mmol) was added in portions and the resulting solution was stirred at RT for 1 h. The reaction mixture was diluted with DCM and washed with brine. The organic layer was dried over anhydrous Na₂SO₄, filtered and concentrated *in vacuo.* The residue was purified by column chromatography on silica gel (dichloromethane/methanol = 50:1) to afford the desired product (3.6 g, 49.66% yield, 2 steps). **¹H NMR** (400 MHz, DMSO-*d6*) δ: 6.30 (dd, *J* = 10.4, 16.8 Hz, 1H), 6.09 (dd, *J* = 2.4, 17.2 Hz, 1H), 5.67 (dd, *J* = 2.4, 10.4 Hz, 1H), 4.22 (t, *J* = 8, 1H), 4.03-4.00 (m, 1H), 3.94-3.90 (m, 1H), 3.75-3.70 (m, 1H), 3.32 (t, *J* = 8.8 Hz, 4H), 3.10-3.18 (m, 1H), 2.22-2.30 (m, 1H), 1.40 (s, 9H).

### EXAMPLE 39

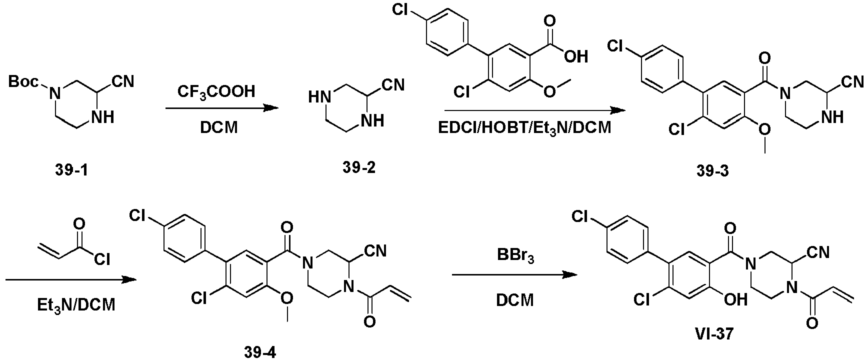

### Piperazine-2-carbonitrile

To a mixture of tert-butyl 3-cyanopiperazine-1-carboxylate (200 mg, 0.95 mmol) in dichloromethane (10 mL), CF₃COOH (2 mL) was added and the resulting was stirred at RT for 1 h. The mixture was concentrated *in vacuo* to afford the crude product.

### 4-(4',6-Dichloro-4-methoxy-[1,1'-biphenyl]-3-carbonyl)piperazine-2-carbonitrile

To a mixture of 4',6-dichloro-4-methoxy-[1,1'-biphenyl]-3-carboxylic acid (309 mg, 1.04 mmol), EDCI (272 mg, 1.43 mmol), HOBt (195 mg, 1.43 mmol), Et₃N (288 mg, 2.85 mmol) in dichloromethane (10 mL) at 0°C, piperazine-2-carbonitrile was added at 0°C and the resulting mixture was stirred at RT for 8 h. The mixture was partitioned between dichloromethane and water. The organic layer was washed brine, dried over MgSO₄, filtered and concentrated *in vacuo.* The residue was purified by flash column chromatography on silica gel (dichloromethane/methanol = 50: 1) to afford the desired product (225 mg, 61% yield). **ESI-MS** m/z: 444.3 [M+H]⁺.

### 1-Acryloyl-4-(4',6-dichloro-4-hydroxy-[1,1'-biphenyl]-3-carbonyl)piperazine-2-carbonitrile (VI-37)

The title compound was prepared from 4-(4',6-dichloro-4-methoxy-[1,1'-biphenyl]-3-carbonyl)piperazine-2-carbonitrile in two steps according to the procedure described in **Example 17**. **¹H NMR** (400 MHz, CDCl₃) δ: 9.24 (s, 1H), 7.44-7.33 (m, 5H), 7.20 (s, 1H), 6.57-6.45 (m, 2H), 6.79 (s, 1H), 5.94-5.91 (m, 1H), 5.75 (s, 1H), 4.62-4.61 (m, 1H), 4.50-4.46(m, 1H), 4.06 (s, 1H), 3.61 (s, 1H), 3.36-3.33 (m, 1H), 3.16-3.10(m, 1H). **ESI-MS** m/z: 428.4 [M+H]⁺.

### EXAMPLE 40

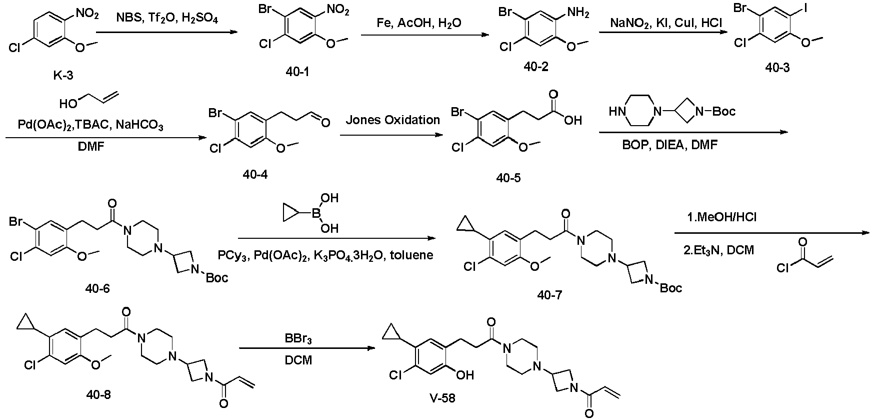

### 5-Bromo-4-chloro-2-methoxybenzenamine

The title compound was prepared from 4-chloro-2-methoxy-1-nitrobenzene in two steps according to the procedure described in **Example 4.**

### 1-Bromo-2-chloro-5-iodo-4-methoxybenzene

To a mixture of 5-bromo-4-chloro-2-methoxyaniline (3 g, 12.7 mmol) in 6N HCl (60 mL, 360 mmol) at 0°C, a solution of NaNO₂ (963 mg, 13.9 mmol) in water (20 mL) was added dropwise while keeping the internal temperature around 0°C. KI (10.5 g, 63.4 mmol) and CuI (4.8 g, 25.4 mmol) were dissolved in water (20 mL) and added to the stirred reaction mixture. The reaction was kept at 5°C for 2 h. The reaction mixture was extracted with ethyl acetate. The combined organic layer was washed with water, Na₂SO₃ (aq, 10%) and brine, dried over anhydrous Na₂SO₄, an concentrated. The residue was purified by flash column chromatography on silica gel (ethyl acetate/petroleum ether = 1:100) to afford the desired product (3.2 g, 73% yield).

### 3-(5-Bromo-4-chloro-2-methoxyphenyl)propanal

A mixture of 1-bromo-2-chloro-5-iodo-4-methoxybenzene (3.2 g, 9.2 mmol), prop-2-en-1-ol (1.3 g, 23.0 mmol), Pd(OAc)₂ (206 mg, 0.9 mmol), TBAC (2.56 g, 9.2 mmol), NaHCO₃ (2.3 g, 27.6mmol) in DMF (50 mL) was stirred under Argon at 60°C for 16 h. The mixture was allowed to cool to RT, and then partitioned between ethyl acetate and water. The combined organic layer was washed with brine, dried over anhydrous Na₂SO₄ and concentrated. The residue was purified by flash column chromatography on silica gel (ethyl acetate / petroleum ether = 1:20) to afford the desired product (860 mg, 34% yield).

### 3-(5-Bromo-4-chloro-2-methoxyphenyl)propanoic acid

To a stirred solution of Jones reagent (3 mL, 5.4 mmol, 2.8 M) in acetone (20 mL), 3-(5-bromo-4-chloro-2-methoxyphenyl)propanal (860 mg, 3.1 mmol) was added. The reaction was stirred at RT for 12 h, quenched with iso-propylalcohol and then stirred for 10 min. The resulting mixture was diluted with water, extracted with ethyl acetate. The combined organic layer was washed with water and brine, dried over Na₂SO₄ and concentrated. The residue was purified by flash column chromatography on silica gel (ethyl acetate/petroleum ether = 1:1) to afford the desired product (358 mg, 38% yield). **ESI-MS** m/z: 291.1 [M+H]⁻.

### tert-Butyl-3-(4-(3-(5-bromo-4-chloro-2-methoxyphenyl)propanoyl)piperazin-1-yl)azetidine-1-carboxylate

To a stirred solution of 3-(5-bromo-4-chloro-2-methoxyphenyl)propanoic acid (350 mg, 1.2 mmol) in DMF (30 mL) at RT, tert-butyl 3-(piperazin-1-yl)azetidine-1-carboxylate (317 mg, 1.3 mmol), BOP (731 mg, 1.4 mmol) and DIEA (461 mg, 3.6 mmol) were added and the mixture was stirred at RT for 1 h. The reaction mixture was partitioned between ethyl acetate and water. The organic layer was washed with brine and dried over anhydrous Na₂SO₄, filtered and concentrated *in vacuo.* The residue was purified by flash column chromatography on silica gel (dichloromethane/methanol = 50:1) to afford the desired product (285 mg, 46% yield).

### tert-Butyl 3-(4-(3-(4-chloro-5-cyclopropyl-2-hydroxyphenyl)propanoyl)piperazin-1-yl)azetidine-1-carboxylate

A mixture of tert-butyl 3-(4-(3-(5-bromo-4-chloro-2-methoxyphenyl)propanoyl)piperazin-1-yl)azetidine-1-carboxylate (280 mg, 0.54 mmol), cyclopropylboronic acid (185 mg, 2.2 mmol), K₃PO₄.3H₂O (444 mg, 1.9 mmol), tricyclohexylphosphine (30 mg, 0.1 mmol), Pd(OAc)₂ (24 mg, 0.11 mmol) in toluene (10 mL) and water (1 mL) was stirred at reflux under argon for 16 h. The mixture was allowed to cool to RT and concentrated *in vacuo.* The residue was purified by flash column chromatography on silica gel (dichloromethane/methanol = 60:1) to afford the desired product (194 mg, 75% yield). **ESI-MS** m/z: 477.3 [M+H]⁺.

### 1-(3-(4-(3-(4-Chloro-5-cyclopropyl-2-hydroxyphenyl)propanoyl)piperazin-1-yl)azetidin-1-yl)prop-2-en-1-one (V-58) (Reference Example)

The title compound was prepared from tert-butyl 3-(4-(3-(4-chloro-5-cyclopropyl-2-hydroxyphenyl)propanoyl)piperazin-1-yl)azetidine-1-carboxylate in three steps according to the procedure described in **Example 17**. **¹H NMR** (400 MHz, DMSO-*d6*) δ: 9.68 (s, 1H), 6.79 (s, 1H), 6.74 (s, 1H), 6.33-6.26 (m, 1H), 6.12-6.07 (dd, *J* = 1.9, 17.2 Hz, 1H), 5.68-5.65 (dd, *J* = 2.0, 10.2 Hz, 1H), 4.24-4.20 (m, 1H), 4.05-4.01 (m, 1H), 3.94-3.90 (m, 1H), 3.76-3.72 (m, 1H), 3.45-3.42 (m, 4H), 3.13-3.11(m, 1H), 2.69-2.65 (m, 2H), 2.53-2.51 (m, 2H), 2.25-2.23 (bs, 4H), 1.97-1.93 (m, 1H), 0.90-0.86 (m, 1H), 0.60-0.55 (m, 1H). **ESI-MS** m/z: 418.4 [M+H]⁺.

### EXAMPLE 41

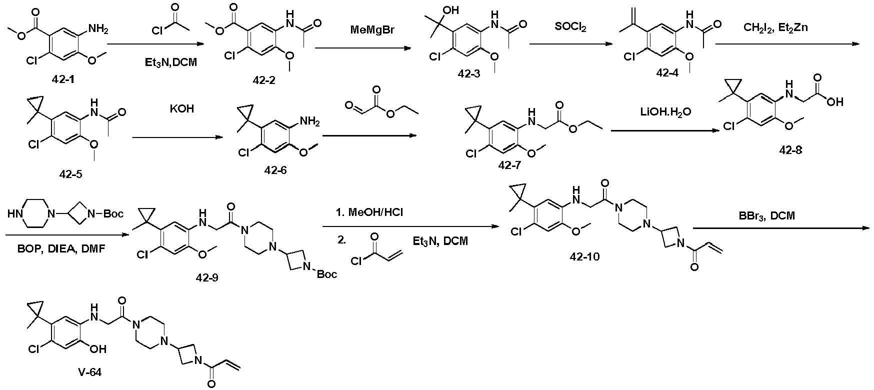

### Methyl 5-acetamido-2-chloro-4-methoxybenzoate

To a mixture of methyl 5-amino-2-chloro-4-methoxybenzoate (3.6 g, 16.7 mmol), Et₃N (6.7 g, 66.8 mmol) and DCM (100 mL) at RT, acetyl chloride (1.57 g, 20.1 mmol) was added dropwise and the resulting mixture was stirred for 12h. The reaction mixture was partitioned between dichloromethane and water. The organic layer was washed with saturated NaHCO₃ solution and brine, dried over anhydrous Na₂SO₄ and concentrated *in vacuo.* The residue was purified by flash column chromatography on silica gel (ethyl acetate / petroleum ether = 1:1) to afford the desired product (2.7 g, 63% yield).

### N-(4-Chloro-5-(2-hydroxypropan-2-yl)-2-methoxyphenyl)acetamide

To a solution of methyl 5-acetamido-2-chloro-4-methoxybenzoate (2.7 g, 11.1 mmol), in THF (40 mL) at - 40°C under Argon, methylmagnesium bromide (21mL, 21 mmol, 1M in ether) was added dropwise while keeping the internal temperature at - 40°C. Then the mixture was allowed to warm to RT, and stirred for 2 h. The reaction mixture was poured into ice-cooled NH₄Cl (10%) solution, and extracted with ethyl acetate. The combined organic layer was washed with water and brine, dried over anhydrous Na₂SO₄ and concentrated *in vacuo* to afford the desire product (2.3 g, 80% yield).

### N-(4-Chloro-2-methoxy-5-(prop-1-en-2-yl)phenyl)acetamide

To a solution of N-(4-chloro-5-(2-hydroxypropan-2-yl)-2-methoxyphenyl)acetamide (3.2 g, 12.4 mmol) in DCM (20 mL) at - 5°C, SOCl₂ (3.7g, 37.25 mmol) was added dropwise. The mixture was warmed to RT, and then stirred at reflux for 2 h. The reaction mixture was concentrated and the residue was purified by flash column chromatography on silica gel (ethyl acetate/petroleum ether = 3:1) to afford the desired product (1.9 g, 64% yield).

### N-(4-Chloro-2-methoxy-5-(1-methylcyclopropyl)phenyl)acetamide

To a solution of N-(4-chloro-2-methoxy-5-(prop-1-en-2-yl)phenyl)acetamide (1.0 g, 4.17 mmol) in toluene (20 mL) at 0°C, CH₂I₂ (5.6 g, 20.86 mmol) and Et₂Zn (41.7 mL, 41.7 mmol, 1.0 M in hexane) was added. The mixture was kept at 0°C for 30 min, and then stirred at RT for 16 h. The reaction mixture was quenched with saturated NH₄Cl solution and stirred for 15 min. The mixture was concentrated *in vacuo* to remove toluene and the resulting mixture was extracted with dichloromethane. The combined organic layer was washed with water and brine, dried over anhydrous Na₂SO₄, concentrated to afford the desired product (820 mg, 77% yield).

### 4-Chloro-2-methoxy-5-(1-methylcyclopropyl)aniline

A mixture of N-(4-chloro-2-methoxy-5-(1-methylcyclopropyl)phenyl)acetamide (820 mg, 3.23 mmol), KOH (1.8 g, 32.3 mmol), ethanol (40 mL) and water (20 mL) was stirred at reflux for 12 h. The reaction mixture was extracted with ethyl acetate. The combined organic layer was washed with water and brine, dried over anhydrous Na₂SO₄ and filtered. The filtrate was concentrated *in vacuo* and the residue was purified by flash column chromatography on silica gel (ethyl acetate / petroleum ether = 20:1) to afford the desired product (460 mg, 67% yield). **ESI-MS** m/z: 212.4 [M+H]⁺.

### Ethyl 2-((4-chloro-2-methoxy-5-(1-methylcyclopropyl)phenyl)amino)acetate

To a solution of 4-chloro-2-methoxy-5-(1-methylcyclopropyl)aniline (450 mg, 2.13 mmol) in MeOH (20 mL) at RT, AcOH (3 drops) and ethyl glyoxalate (326 mg, 3.19 mmol, 50% in toluene) were added. The mixture was stirred at RT for 2 h and then sodium cyanoborohydride (403 mg, 6.39 mmol) was added to the mixture. The resulting mixture was stirred at 50°C for 16 h. The mixture was allowed to cool to RT, and partitioned between ethyl acetate and water. The organic layer was dried over MgSO₄, filtered, and concentrated *in vacuo* to afford the crude product (636 mg). **ESI-MS** m/z: 298.2 [M+H]⁺.

### 2-((4-Chloro-2-methoxy-5-(1-methylcyclopropyl)phenyl)amino)acetic acid

To a solution of ethyl 2-((4-chloro-2-methoxy-5-(1-methylcyclopropyl)phenyl)amino)acetate (630 mg, 2.12 mmol) in THF (15 mL) and water (5 mL), LiOH.H2O (889mg, 21.2 mmol) was added and the resulting mixture was stirred at RT for 2 h. The mixture was washed with 20% ethyl acetate/petroleum ether. The aqueous layer was acidified with aqueous HCl (1 N) to adjust pH to 3-4 and extracted with ethyl acetate. The organic layer was dried over MgSO₄, filtered and concentrated *in vacuo* to afford the desired product (200 mg, 33% yield).

### tert-Butyl 3-(4-(2-((4-chloro-2-methoxy-5-(1-methylcyclopropyl)phenyl)amino)acetyl)piperazin-1-yl)azetidine-1-carboxylate

To a solution of 2-((4-chloro-2-methoxy-5-(1-methylcyclopropyl)phenyl)amino)acetic acid (110 mg, 0.41 mmol) and tert-butyl 3-(piperazin-1-yl)azetidine-1-carboxylate (118 mg, 0.49 mmol) in DMF (15 mL) at RT, BOP (217 mg, 0.49 mmol) and DIEA (159 mg, 1.23 mmol) were added and the resulting mixture was stirred at RT for 1 h. The mixture was partitioned between ethyl acetate and water. The organic layer was washed with brine, dried over MgSO₄, filtered and concentrated *in vacuo* to afford the desired product (192 mg, 95% yield).

### 1-(3-(4-(2-((4-Chloro-2-hydroxy-5-(1-methylcyclopropyl)phenyl)amino)acetyl)piperazin-1-yl)azetidin-1-yl)prop-2-en-1-one (V-64) (Reference Example)

The title compound was prepared from tert-butyl 3-(4-(2-((4-chloro-2-methoxy-5-1-methylcyclopropyl)phenyl)amino)acetyl)piperazin-1-yl)azetidine-1 - carboxylate in three steps according to the procedure described in **Example 17. ¹H NMR** (400 MHz, DMSO-*d6*) δ: 9.70 (s, 1H), 6.64 (s, 1H), 6.51 (s, 1H), 6.35-6.28 (m ,1H), 6.13-6.08 (dd, *J* =1.9, 17.9 Hz, 1H), 5.69-5.66 (dd, *J* = 2.1, 10.1 Hz, 1H), 5.13-5.11 (m, 1H), 4.25-4.23 (m, 1H), 4.08-4.05 (m, 1H), 3.95-3.91 (m, 3H), 3.80-3.76 (m, 1H), 3.53 (bs, 4H), 3.18-3.16 (m, 1H), 2.38-2.31 (m, 4H), 1.26 (s, 3H), 0.72-0.64 (m, 4H). **ESI-MS** m/z: 434.4 [M+H]⁺.

### EXAMPLE 42

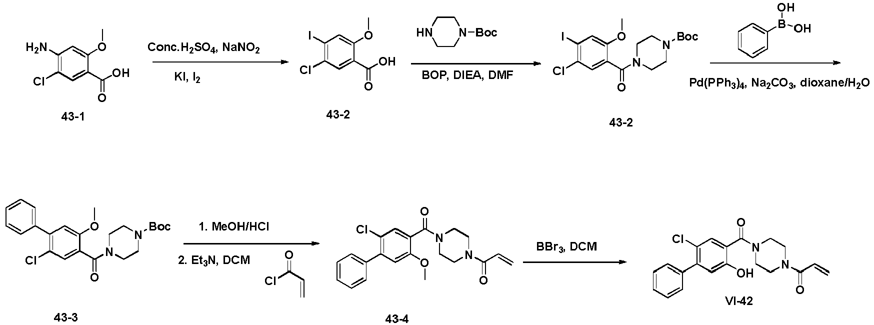

### 5-Chloro-4-iodo-2-methoxybenzoic acid

To a stirred solution of 4-amino-5-chloro-2-methoxybenzoic acid (5 g, 24.8 mmol) in water (10 mL) at 0°C, concentrated sulfuric acid (50 mL) was added. Then a solution of NaNO₂ (1.9 g, 27.3 mmol) in water (10 mL) was added dropwise while keeping the internal temperature around 0°C. KI (4.5 g, 27.3 mmol) and I₂ (3.5 g, 13.64 mmol) were dissolved in water and added dropwise to the stirred reaction mixture. The reaction was stirred at 5°C for 2 h and then extracted with ethyl acetate. The organic layer was washed with water, Na₂SO₃ (aq, 10%) and brine, dried over anhydrous Na₂SO₄ and concentrated *in vacuo* to afford desired product (1.55 g, 19% yield). **ESI-MS** m/z: 311.1 [M+H]⁺.

### tert-Butyl 4-(5-chloro-4-iodo-2-methoxybenzoyl)piperazine-1-carboxylate

To a stirred solution of 5-chloro-4-iodo-2-methoxybenzoic acid (1.55 g, 4.9 mmol) in DMF (30 mL) at RT, tert-butyl piperazine-1-carboxylate (1.02 g, 5.5 mmol), BOP (2.63 g, 25.9 mmol) and DIEA (1.92 g, 14.9 mmol) were added and the resulting mixture was stirred at RT for 1 h. The reaction mixture was extracted with ethyl acetate and washed with brine. The organic layer was dried over anhydrous Na₂SO₄, filtered and concentrated *in vacuo.* The residue was purified by flash column chromatography on silica gel (dichloromethane/methanol = 50:1) to afford the desired product (1.96 g, 76% yield).

### tert-Butyl-4-(2-chloro-5-methoxy-[1,1'-biphenyl]-4-carbonyl)piperazine-1-carboxylate

A mixture of tert-butyl 4-(5-chloro-4-iodo-2-methoxybenzoyl)piperazine-1-carboxylate (300 mg, 0.56 mmol), phenylboronic acid (82 mg, 0.67 mmol), Pd(PPh₃)₄ (129 mg, 0.1 mmol), Na₂CO₃(180 mg, 1.68 mmol) in 1,4-dioxane (10 mL) and water (2 mL) was stirred at reflux under argon for 16 h. The mixture was allowed to cool to room temperature and concentrated *in vacuo.* The residue was purified by flash column chromatography on silica gel (petroleum ether/ethyl acetate = 30:1) to afford the desired product (219 mg, 80% yield).

### 1-(4-(2-Chloro-5-hydroxy-[1,1'-biphenyl]-4-carbonyl)piperazin-1-yl)prop-2-en-1-one (VI-42)

The title compound was prepared from tert-butyl-4-(2-chloro-5-methoxy-[1,1'-biphenyl]-4-carbonyl)piperazine-1-carboxylatein three steps according to the procedure described in **Example 17**. **¹H NMR** (400 MHz, DMSO-*d6*) δ: 10.40 (s, 1H), 7.50-7.41 (m, 5H), 7.35 (s, 1H), 6.89 (s, 1H), 6.84 (m, 1H), 6.17-6.13 (d, 1H), 5.73-5.71 (m, 1H), 3.63 (s, 6H), 3.30 (s, 2H). **ESI-MS** m/z: 371.2 [M+H]⁺.

### EXAMPLE 43

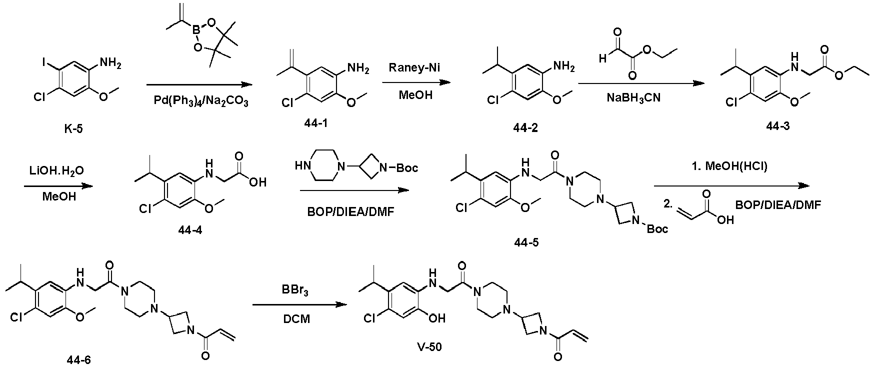

### 4-Chloro-2-methoxy-5-(prop-1-en-2-yl)benzenamine

A mixture of 4-chloro-5-iodo-2-methoxybenzenamine (1.0 g, 3.53 mmol), 4,4,5,5-tetramethyl-2-(prop-1-en-2-yl)-1,3,2-dioxaborolane (889 mg, 5.29 mmol), Pd(PPh₃)₄ (363 mg, 0.353 mmol), Na₂CO₃ (1.12 g, 10.6 mmol) in DME (10 mL) and water (3 mL) was stirred at reflux under argon for 6 h. The reaction mixture was allowed to cool to RT and extracted with ethyl acetate. The organic layer was washed with brine, dried over Na₂SO₄ and concentrated *in vacuo.* The residue was purified by flash column chromatography on silica gel (5% petroleum ether/ethyl acetate) to afford the desired product (173 mg, 25% yield) as an off-white solid. **ESI-MS** *m*/*z*: 198.5[M+H]⁺.

### 4-Chloro-5-isopropyl-2-methoxybenzenamine

A mixture of 4-chloro-2-methoxy-5-(prop-1-en-2-yl)benzenamine (160 mg, 0.81 mmol), Raney-Ni (20 mg) in MeOH (5 mL) was stirred at RT under H₂ (1 atm) atmosphere for 8 h. The mixture was filtered and the filtrate was concentrated *in vacuo* to afford the desired product (150 mg, 93% yield).

### tert-Butyl3-(4-(2-(4-chloro-5-isopropyl-2-methoxyphenylamino)acetyl)piperazin-1-yl)azetidine-1-carboxylate

The title compound was prepared from 4-chloro-5-isopropyl-2-methoxybenzenamine in three steps according to the procedure described in **Example 42**.

### 1-(3-(4-(2-(4-Chloro-5-isopropyl-2-methoxyphenylamino)acetyl)piperazin-1-yl)azetidin-1-yl)prop-2-en-1-one

A mixture of tert-butyl 3-(4-(2-(4-chloro-5-isopropyl-2-methoxyphenylamino)acetyl)piperazin-1-yl)azetidine-1-carboxylate (102 mg, 0.212 mmol) in HCl/MeOH (2.86 M, 5 mL) was stirred at RT for 1 h. The mixture was concentrated *in vacuo* to afford the crude product, the crude was dissolved in DMF (5 mL) at RT, acrylic acid (17 mg, 0.233 mmol), BOP (113 mg, 0.254 mmol) and DIEA (82 mg, 0.636 mmol) were added and the resulting mixture was stirred at RT for 1 h. The mixture was partitioned between ethyl acetate and water. The organic layer was washed brine, dried over MgSO₄, filtered and concentrated *in vacuo.* The residue was purified by flash column chromatography on silica gel (dichloromethane/methanol = 50:1) to afford the desired product (77 mg, 85% yield, 2 steps). **ESI-MS** *m*/*z*: 435.4 [M+H]⁺.

### 1-(3-(4-(2-(4-Chloro-2-hydroxy-5-isopropylphenylamino)acetyl)piperazin-1-yl)azetidin-1-yl)prop-2-en-1-one (V-50) (Reference Example)

To a solution of 1-(3-(4-(2-(4-chloro-5-isopropyl-2-methoxyphenylamino)acetyl)piperazin-1-yl)azetidin-1-yl)prop-2-en-1-one (77 mg, 0.18 mmol) in DCM (15 mL) at - 60°C, BBr₃ (443 mg, 1.8 mmol) was added dropwise and the resulting mixture was stirred at RT for 1 h. The mixture was cooled to -60°C, MeOH was added dropwise and then basified with Et₃N to adjust the pH to 8 - 9. The mixture was poured into water and extracted with dichloromethane. The organic layer was dried over anhydrous Na₂SO₄, filtered and concentrated *in vacuo* The residue was purified by flash column chromatography on silica gel (dichloromethane/methanol = 50:1) to afford the desired product (25 mg, 33% yield). **¹H NMR** (400 MHz, DMSO-*d6)* δ: 9.50 (bs, 1H), 6.62 (s, 1H), 6.470 (s, 1H), 6.30 (m, 1H), 6.10 (dd, *J* = 2.4, 17.2 Hz, 1H), 5.68 (dd, *J* = 2.0, 10.4 Hz, 1H), 5.14 (m, 1H), 4.25 (m, 1H), 4.06 (m, 1H), 3.96 (m, 1H), 3.91 (m, 2H), 3.78 (m, 1H), 3.54 (m, 4H), 3.17 (m, 2H), 2.35 (m, 4H), 1.21 (m, 6H). **ESI-MS** *m*/*z*: 421.4 [M+H]⁺.

### EXAMPLE 44

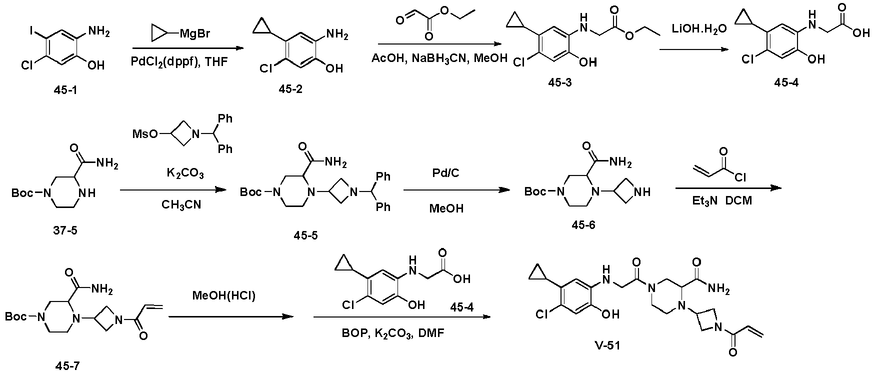

### 2-Amino-5-chloro-4-cyclopropylphenol

To a mixture of 2-amino-5-chloro-4-iodophenol (500 mg, 1.9 mmol), PdCl₂(dppf) (136 mg, 0.19 mmol) in THF (10 mL) under argon at RT, cyclopropylmagnesium bromide (16 mL, 11.4 mmol, 0.7 M in THF) was added and the mixture was stirred at reflux for 15 h. The mixture was allowed to cool to RT, and partitioned between ethyl acetate and water. The organic layer was washed with brine, dried over Na₂SO₄ and concentrated. The residue was purified by flash column chromatography on silica gel (10-20% ethyl acetate / hexanes) to afford the desired product (220 mg, 63% yield) as a brown solid. **¹H NMR** (400 MHz, DMSO-*d6*) δ: 9.27 (s, 1H), 6.62 (s, 1H), 6.22 (s, 1H), 4.53(s, 2H), 1.89-1.93 (m, 1H), 0.83-0.87 (m, 2H), 0.46-0.49 (m, 2H).

### Ethyl 2-(4-chloro-5-cyclopropyl-2-hydroxyphenylamino)acetate

To a solution of 2-amino-5-chloro-4-cyclopropylphenol (200 mg, 1.01 mmol) in MeOH (20 mL) at RT, AcOH (3 drops) and ethyl glyoxalate (416 mg, 2.02 mmol, 50% in toluene) were added. The mixture was stirred at RT for 2 h and then sodium cyanoborohydride (190 mg, 3.03 mmol) was added to the mixture. The resulting mixture was stirred at 40°C for 15 h. The mixture was allowed to cool to RT and partitioned between ethyl acetate and water. The organic layer was washed with brine, dried over Na₂SO₄ and concentrated. The residue was purified by flash column chromatography on silica gel (10-20% methanol/dichloromethane) to afford the desired product (290 mg, 100% yield) as a solid. **¹H NMR** (400 MHz, DMSO-*d6*) δ: 9.63 (s, 1H), 6.66 (s, 1H), 5.93 (s, 1H), 5.07 (t, *J* = 6.4 Hz, 1H), 4.12 (q, *J* = 7.2 Hz, 2H), 3.91 (d, *J=* 6.4 Hz, 2H), 1.92-1.97 (m, 1H), 1.20 (t, *J* = 6.8 Hz, 2H), 0.84-0.87 (m, 2H), 0.51-0.55 (m, 2H).

### 2-(4-Chloro-5-cyclopropyl-2-hydroxyphenylamino)acetic acid

To a solution of ethyl 2-(4-chloro-5-cyclopropyl-2-hydroxyphenylamino)acetate (290mg, 0.89mmol) in of 4:1 mixture of tetrahydrofuran and water (30 mL) at RT, LiOH.H₂O (226mg, 5.34mmol) was added and the resulting mixture was stirred for 2 h at 60°C. The mixture was acidified with aqueous HCl (1 N) to adjust the pH to 3 - 5 and then extracted with ethyl acetate. The organic layer was washed with brine, dried over anhydrous Na₂SO₄, filtered and concentrated *in vacuo* to afford the product (100 mg, 47% yield). **¹H NMR** (400 MHz, DMSO-*d6*) δ: 9.64 (s, 1H), 6.66 (s, 1H), 5.96 (s, 1H), 3.81 (s, 2H), 1.89-1.96 (m, 1H), 0.84-0.87 (m, 2H), 0.54-0.56 (m, 2H).

### tert-Butyl 4-(1-benzhydrylazetidin-3-yl)-3-carbamoylpiperazine-1-carboxylate

A mixture of 1-benzhydrylazetidin-3-yl methanesulfonate (2.69 g, 8.5 mmol), K₂CO₃ (1.76 g, 12.8 mmol), tert-butyl 3-carbamoylpiperazine-1-carboxylate (1.95 g, 8.5 mmol) in CH₃CN (40 mL) was stirred at reflux for 16 h. The mixture was partitioned between ethyl acetate and water. The organic layer was washed with water and brine, dried over Na₂SO₄, and concentrated *in vacuo.* The residue was purified by flash column chromatography on silica gel (dichloromethane/methanol = 50:1) to afford the desired product. (2.08 g, 54% yield).

### tert-Butyl 4-(azetidin-3-yl)-3-carbamoylpiperazine-1-carboxylate

A mixture of 4-chloro-2-methoxy-5-(prop-1-en-2-yl)benzenamine (1 g, 2.22 mmol), Pd/C (300 mg) in MeOH (25 mL) was stirred at 50°C under H₂ (1 atm) atmosphere for 12 h. The mixture was cooled and filtered. The filtrate was concentrated *in vacuo* to afford the desired product (640 mg, 100% yield).

### tert-Butyl 4-(1-acryloylazetidin-3-yl)-3-carbamoylpiperazine-1-carboxylate

To a solution of tert-butyl 4-(azetidin-3-yl)-3-carbamoylpiperazine-1-carboxylate (640 mg, 2.22 mmol) and Et₃N (463 mg, 4.58mmol) in DCM (10 mL) at 0°C, acryloyl chloride (248 mg, 2.74 mmol) was added dropwise and the resulting mixture was stirred at RT for 1.5 h. The mixture was partitioned between dichloromethane and saturated NaHCO₃ solution. The organic layer was washed with saturated brine, dried over Na₂SO₄ and concentrated *in vacuo.* The residue was purified by flash column chromatography on silica gel (dichloromethane/methanol = 50:1) to afford the desired product (350 mg, 47% yield).

### 1-(1-acryloylazetidin-3-yl)-4-(2-(4-chloro-5-cyclopropyl-2-hydroxyphenylamino)acetyl)piperazine-2-carboxamide (V-51) (Reference Example)

A mixture of tert-butyl 4-(1-acryloylazetidin-3-yl)-3-carbamoylpiperazine-1-carboxylate (120 mg, 0.35 mmol) in HCl/MeOH (2.86 M, 10 mL) was stirred at RT for 1 h. The mixture was concentrated *in vacuo* to afford the crude residue. It was dissolved in DMF (5 mL) at 0°C, 2-(4-chloro-5-cyclopropyl-2-hydroxyphenylamino)acetic acid (31 mg, 0.427 mmol), BOP (206 mg, 0.466 mmol) and K₂CO₃ (150 mg, 1.164 mmol) were added and the resulting mixture was stirred at RT for 1 h. The mixture was partitioned between ethyl acetate and water. The organic layer was washed brine, dried over MgSO₄, filtered and concentrated *in vacuo.* The residue was purified by flash column chromatography on silica gel (dichloromethane/methanol = 20:1) to afford the desired product (126 mg, 75% yield, 2 steps). **¹H NMR** (400 MHz, DMSO-*d6*) δ: 9.63 (bs, 1H), 7.53 (d, 1H), 7.26-6.94 (m, 2H), 6.67 (s, 1H), 6.34-6.27 (m, 1H), 6.10 (m, 2H), 5.68 (d, *J* = 10.4, 1H), 5.08 (m, 1H), 4.30 (m, 2H), 3.93 (m, 6H), 3.52 (m, 2H), 3.29 (m, 1H), 3.15 (m, 1H), 3.06 (m, 1H), 2.47 (m, 1H), 1.98 (m, 1H), 0.87 (m, 2H), 0.64 (m, 2H). **ESI-MS** *m*/*z*: 462.5 [M+H]⁺.

### EXAMPLE 45

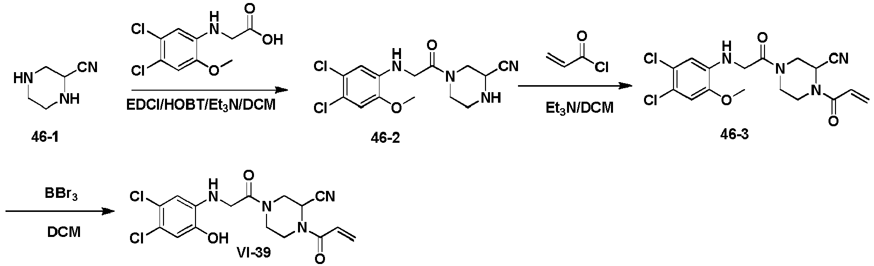

### 4-(2-(4,5-Dichloro-2-methoxyphenylamino)acetyl)piperazine-2-carbonitrile

To a mixture of 2-(4,5-dichloro-2-methoxyphenylamino)acetic acid (260 mg, 1.04 mmol), EDCI (273 mg, 1.43 mmol), HOBt (194 mg, 1.43 mmol) and Et₃N (288 mg 2.85 mmol) in DCM (10 mL) at 0°C, piperazine-2-carbonitrile was added. The resulting mixture was stirred at RT for 8 h. The mixture was partitioned between dichloromethane and water. The organic layer was washed with brine, dried over MgSO₄, filtered and concentrated *in vacuo.* The residue was purified by flash column chromatography on silica gel (dichloromethane/methanol = 50: 1) to afford the desired product (108 mg, 30% yield).

### 1-acryloyl-4-(2-(4,5-dichloro-2-hydroxyphenylamino)acetyl)piperazine-2-carbonitrile (VI-39)

The title compound was prepared from 4-(2-(4,5-dichloro-2-methoxyphenylamino)acetyl)piperazine-2-carbonitrile in two steps according to the procedure described in **Example 17**. **¹H NMR** (400 MHz, DMSO-*d6*) δ: 10.3 (s, 1H), 6.90 (m, 1H), 6.87(s, 1H), 6.73(s, 1H), 6.28 (d, *J* = 16.4 Hz, 1H), 5.88 (d ,*J* = 10.4 Hz, 1H), 5.68 (m, 1H), 5.33 (m, 1H), 4.66 (m, 1H), 4.40 (m, 1H), 4.02 (m, 2H), 3.22 (m, 1H), 3.04 (m, 1H), 2.91 (m, 1H). **ESI-MS** *m*/*z:* 383.2 [M+H]⁺.

### EXAMPLE 46

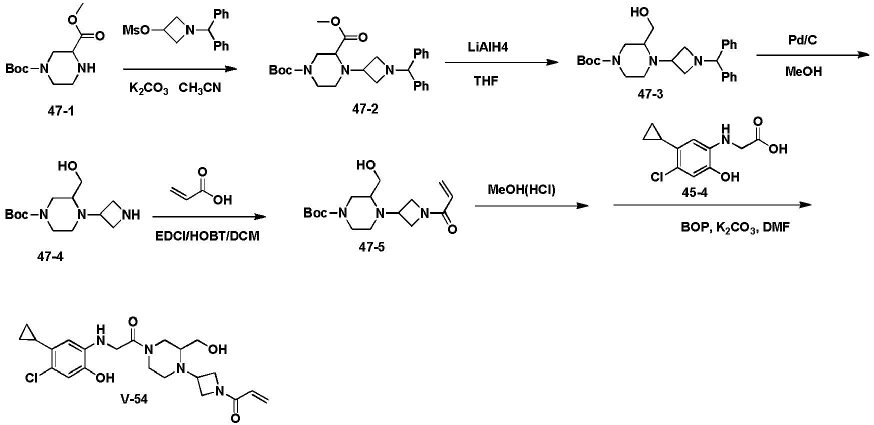

### 1-tert-Butyl 3-methyl 4-(1-benzhydrylazetidin-3-yl)piperazine-1,3-dicarboxylate

A mixture of 1-benzhydrylazetidin-3-yl methanesulfonate (2.4 g, 7.56 mmol), tert-butyl methyl piperazine-1,3-dicarboxylate (1.85 g, 7.56 mmol), K₂CO₃ (1.6 g, 11.34 mmol) in CH₃CN (40 mL) was stirred at reflux for 16 h. The mixture was partitioned between ethyl acetate and water. The organic layer was washed with water and brine, dried over Na₂SO₄, and concentrated *in vacuo.* The residue was purified by flash column chromatography on silica gel (10% petroleum ether/ethyl acetate) to afford the desired product (1.85 g, 51% yield).

### tert-Butyl 4-(1-benzhydrylazetidin-3-yl)-3-(hydroxymethyl)piperazine-1-carboxylate

To a mixture of LiAlH₄ (500 mg, 13.5 mmol) in THF (40 mL) at - 40°C under argon, a solution of 1-tert-butyl 3-methyl 4-(1-benzhydrylazetidin-3-yl) piperazine-1,3-dicarboxylate (1.8 g, 3.87 mmol) in THF (10 mL) was added dropwise. The reaction mixture was stirred at -5°C to 5°C for 1 h and cooled to -20°C. Then water (2 mL) and NaOH (15%) aqueous were added. The resulting mixture was stirred for 15 min. The solid was filtered, and the cake rinsed with ethyl acetate. The combined filtrate was dried over Na₂SO₄ and concentrated *in vacuo* to afford the product (1.6 g, 94% yield).

### 1-(3-(4-(2-(4-Chloro-5-cyclopropyl-2-hydroxyphenylamino)acetyl)-2-(hydroxymethyl)piperazin-1-yl)azetidin-1-yl)prop-2-en-1-one (V-54) (Reference Example)

The title compound was prepared from 4-(2-(4,5-dichloro-2-methoxyphenylamino)acetyl)piperazine-2-carbonitrile in four steps according to the procedure described in **Example 44**. **¹H NMR** (400 MHz, DMSO-*d6*) δ: 9.68 (bs, 1H), 6.67 (s, 1H), 6.35-6.27 (m, 1H), 6.12-6.05 (m, 2H), 5.67 (dd, *J* = 1.6, 10.4 Hz, 1H), 5.11 (m, 1H), 4.82-4.63 (m, 1H), 4.24 (m, 1H), 4.13 (m, 1H), 3.95 (m, 1H), 3.88 (m, 2H), 3.85 (m, 1H), 3.77-3.67 (m, 2H), 3.20 (m, 1H), 3.15 (m, 1H), 2.76-2.60 (m, 2H), 2.40 (m, 1H), 1.95 (m, 1H), 0.87 (m, 2H), 0.62 (m, 2H). **ESI-MS** *m*/*z*: 449.4 [M+H]⁺.

### EXAMPLE 47

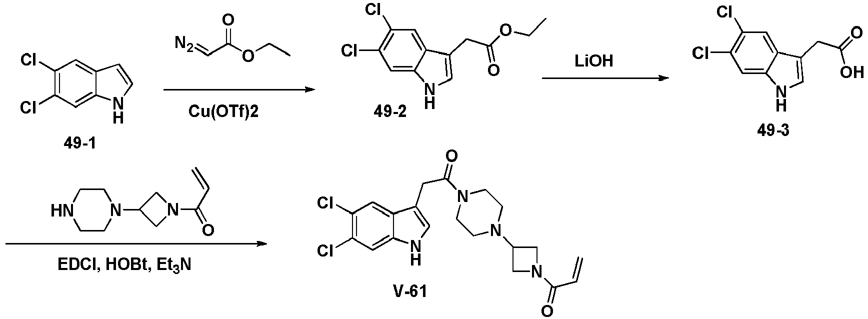

### Ethyl 2-(5,6-dichloro-1H-indol-3-yl)acetate

To a mixture of 5,6-dichloro-1H-indole (1.0 g, 5.37 mmol), Cu(OTf)₂ (194 mg, 0.537 mmol) in DCM (15 mL) at RT, ethyl 2-diazoacetate (918 mg, 8.05 mmol) was added dropwise. The resulting mixture was stirred at RT for 16 h, quenched with water, and then extracted dichloromethane. The organic layer was dried over Na₂SO₄, filtered and concentrated *in vacuo.* The residue was purified by Prep-HPLC to afford the desired product (120 mg, 8.2% yield) as light yellow solid. **ESI-MS** *m*/*z*: 272.1 [M+H]⁺.

### 2-(5,6-Dichloro-1H-indol-3-yl)acetic acid

A mixture of ethyl 2-(5,6-dichloro-1H-indol-3-yl)acetate (120 mg, 0.44 mmol), LiOH (90 mg, 2.20 mmol) in THF (3 mL) and H₂O (1 mL) was stirred at RT for 16 h. The solution was poured into water, adjusted pH to 3-4 with 1N HCl and extracted with ethyl acetate. The organic layer was dried over Na₂SO₄ and concentrated *in vacuo* to afford the desired product (90 mg, 84.5% yield) as a yellow solid.

### 1-(3-(4-(2-(5,6-Dichloro-1H-indol-3-yl)acetyl)piperazin-1-yl)azetidin-1-yl)prop-2-en-1-one (V-61) (Reference Example)

A mixture of 2-(5,6-dichloro-1H-indol-3-yl)acetic acid (90 mg, 0.372 mmol), 1-(3-(piperazin-1-yl)azetidin-1-yl)prop-2-en-1-one (87 mg, 0.446 mmol), EDCI.HCl (107 mg, 0.558 mmol), HOBt (75 mg, 0.558 mmol) in DMF (3 mL) at 0°C, Et₃N (112 mg, 1.11 mmol) was added. The resulting mixture was stirred at RT for 16 h. The mixture was partitioned between ethyl acetate and water. The organic layer was washed with brine, dried over Na₂SO₄ and concentrated *in vacuo.* The residue was purified by flash column chromatography on silica gel (dichloromethane/methanol = 30:1) to afford the desired product (12 mg, 7.66% yield) as an off-white solid. **¹H NMR** (400 MHz, DMSO-*d6*) δ:11.19 (bs, 1H), 7.79 (s, 1H), 7.59 (s, 1H), 7.35 (d, 1H), 6.28 (dd, *J* = 9.6, 16.8 Hz, 1H), 6.09 (dd, *J* = 2.4, 17.2 Hz, 1H), 5.66 (dd, *J* = 2.4, 10.4 Hz, 1H), 4.21-4.18 (m, 1H), 4.02-3.98 (m, 1H), 3.93-3.88 (m, 1H), 3.78 (s, 2H), 3.74-3.70 (m, 1H), 3.53-3.47 (m, 4H), 3.10-3.07 (m, 1H), 2.25-2.19 (m, 4H). **ESI-MS** *m*/*z*: 423.3 [M+1]⁺.

### EXAMPLE 48

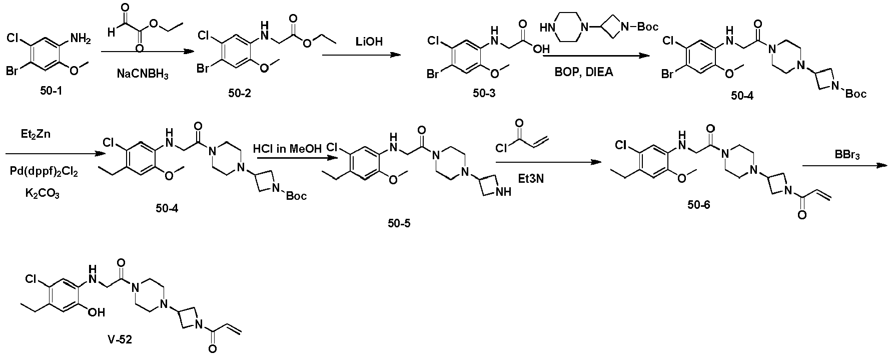

### tert-Butyl 3-(4-(2-(4-bromo-5-chloro-2-methoxyphenylamino)acetyl)piperazin-1-yl)azetidine-1-carboxylate

The title compound was prepared from 4-bromo-5-chloro-2-methoxybenzenamine in three steps according to the procedure described in **Example** 4.

### tert-Butyl 3-(4-(2-(5-chloro-4-ethyl-2-methoxyphenylamino)acetyl)piperazin-1-yl)azetidine-1-carboxylate

To a mixture of tert-butyl 3-(4-(2-(4-bromo-5-chloro-2-methoxyphenylamino)acetyl)piperazin-1-yl)azetidine-1-carboxylate (100 mg, 0.193 mmol), Pd(dppf)₂Cl₂ (29 mg, 0.04 mmol) and K₂CO₃ (55 mg, 0.386 mmol) in DMF (10 mL) at RT, Et₂Zn (0.8 mL, 0.8 mmol, 1.0 M in hexane) was added. The resulting mixture was stirred at 80°C for 16 h. The mixture was partitioned between ethyl acetate and water. The organic layer was washed with brine, dried over Na₂SO₄ and concentrated *in vacuo.* The residue was purified by flash column chromatography on silica gel (dichloromethane/methanol = 50:1) to afford the crude product (100 mg). **ESI-MS** *m*/*z*: 467.5 [M+1]⁺.

### 1-(3-(4-(2-(5-Chloro-4-ethyl-2-hydroxyphenylamino)acetyl)piperazin-1-yl)azetidin-1-yl)prop-2-en-1-one (V-52) (Reference Example)

The title compound was prepared from tert-butyl 3-(4-(2-(5-chloro-4-ethyl-2-methoxyphenylamino)acetyl)piperazin-1-yl)azetidine-1-carboxylate in 3 steps according to the procedure described in **Example 17**. **¹H NMR** (400 MHz, DMSO-*d6*) δ: 9.6 (s, 1H), 6.6 (s, 1H), 6.5 (s, 1H), 6.3 (dd, *J* = 10.4, 17.2 Hz, 1H), 6.1 (dd, *J* = 2.4, 17.2 Hz, 1H), 5.7 (dd, *J* = 2.4, 10.4 Hz, 1H), 5.1 (t, *J* = 4.4 Hz, 1H), 4.2 (t, *J* = 8 Hz, 1H), 4.1 (dd, *J* = 4.8, 8.8 Hz, 1H), 3.95 (dd, *J* = 7.2, 10.0 Hz, 1H), 3.9 (d, *J* = 4.4 Hz, 2H), 3.8 (dd, *J* = 4.8, 10.4 Hz, 1H), 3.6-3.5 (m, 4H), 3.2-3.1 (m, 1H), 3.1-3.0 (m, 1H), 2.5-2.3 (m, 4H), 1.1 (t, *J* = 7.2 Hz, 3H); **ESI-MS** *m*/*z*: 407.4 [M + H]⁺.

### EXAMPLE 49

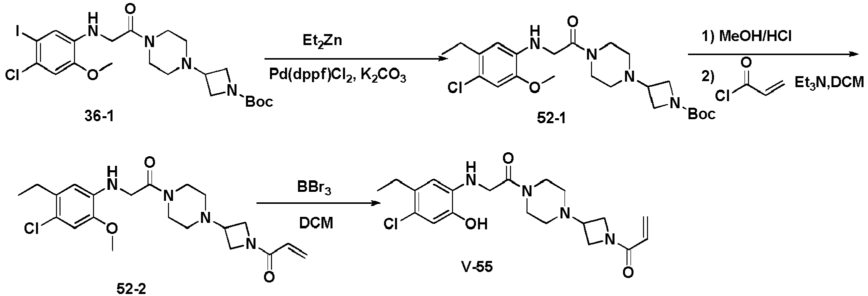

### tert-Butyl3-(4-(2-((4-chloro-5-ethyl-2-methoxyphenyl)amino)acetyl)piperazin-1-yl) azetidine-1-carboxylate

The title compound was prepared from tert-butyl 3-(4-(2-(4-chloro-5-iodo-2-methoxyphenylamino)acetyl)piperazin-1-yl)azetidine-1-carboxylate in one step according to the procedure described in **Example 50**.

### 1-(3-(4-(2-(4-chloro-5-ethyl-2-hydroxyphenylamino)acetyl)piperazin-1-yl)azetidin-1-yl)prop-2-en-1-one (V-55) (Reference Example)

The title compound was prepared from tert-butyl3-(4-(2-((4-chloro-5-ethyl-2-methoxyphenyl)amino)acetyl)piperazin-1-yl) azetidine-1-carboxylate in three steps according to the procedure described in **Example** 17. **¹H NMR** (400 MHz, DMSO-*d6*) δ:9.67 (s, 1H), 6.66 (s, 1H), 6.47 (s, 1H), 6.30 (dd, *J* = 10.5, 16.9 Hz, 1H), 6.12 (dd, *J* = 1.7, 16.7 Hz, 1H), 5.69 (dd, *J* = 1.7, 16.7 Hz, 1H), 5.1 (m, 1H), 4.26 (m, 1H), 4.07 (m, 1H), 3.96 (m, 1H), 3.88 (d, *J* = 4.4, 2H), 3.78 (m, 1H), 3.53 (m, 4H), 3.17 (m, 1H), 2.54 (m, 2H), 2.37 (m, 4H), 1.14 (m, 3H). **ESI-MS** *m*/*z*: 407.3[M+H]⁺.

### EXAMPLE 50

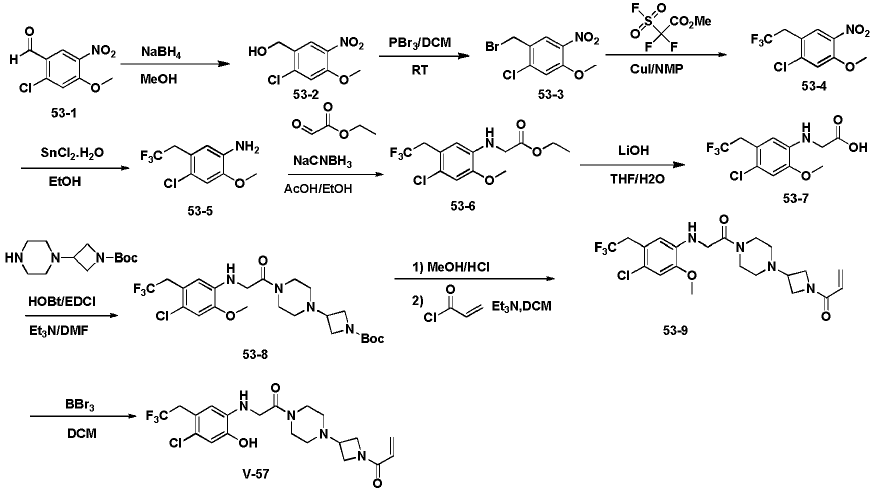

### (2-Chloro-4-methoxy-5-nitrophenyl)methanol

To a solution of 2-chloro-4-methoxy-5-nitrobenzaldehyde (6.0 g, 29 mmol) in MeOH at 0°C (50 mL), sodium borohydride (4.45 g, 117 mmol) was added in portions and the resulting mixture was stirred at RT for 30 min. The mixture was concentrated *in vacuo.* The residue was dissolved in ethyl acetate, washed with brine, dried over Na₂SO₄ and concentrated *in vacuo.* The residue was purified by flash column chromatography on silica gel (petroleum ether/ethyl acetate = 10:1) to afford the desired product (5.0 g, 78.4% yield).

### 1-(Bromomethyl)-2-chloro-4-methoxy-5-nitrobenzene

To a solution of (2-chloro-4-methoxy-5-nitrophenyl)methanol (5.0 g, 23 mmol) in dichloromethane (50 mL) at 0 °C, tribromophosphine (3.08 g, 11.5 mmol) was added in portions and the resulting mixture was stirred at RT for 2 h. The mixture was poured into ice-water and extracted with dichloromethane. The organic layer was washed with brine, dried over Na₂SO₄ and concentrated *in vacuo.* The residue was purified by flash column chromatography on silica gel (petroleum ether/ethyl acetate = 10:1) to afford the desired product (3.5 g, 54.2% yield).

### 1-Chloro-5-methoxy-4-nitro-2-(2,2,2-trifluoroethyl)benzene

A mixture of (2-chloro-4-methoxy-5-nitrophenyl)methanol (3.5 g, 12.5 mmol), methyl 2,2-difluoro-2-(fluorosulfonyl)acetate (4.8 g, 25 mmol), copper iodide (617 mg, 3.25 mmol) in NMP (20 mL) was stirred at 80°C for 24 h under Argon. After cooled to RT, the reaction mixture was dissolved in ethyl acetate, washed with brine, dried over Na₂SO₄ and concentrated *in vacuo.* The residue was purified by flash column chromatography on silica gel (petroleum ether/ethyl acetate = 100:1) to afford the desired product (1.2 g, 36.4% yield). **¹H NMR** (400 MHz, DMSO-*d6*) δ: 8.15 (s, 1H), 7.60 (s, 1H), 3.98 (s, 3H), 3.89 (dd, *J* = 1.7, 11.2 Hz, 2H).

### 4-Chloro-2-methoxy-5-(2,2,2-trifluoroethyl)aniline

A mixture of 1-chloro-5-methoxy-4-nitro-2-(2,2,2-trifluoroethyl)benzene (1.2 g, 4.51 mmol), tin(II) chloride dehydrate (5.0 g, 22.5 mmol) in EtOH (20 mL) was stirred at reflux for 2 h. After cooled to RT, the reaction mixture was added saturated NaHCO₃ solution to adjusted pH to 7-8 and then extracted with ethyl acetate. The organic layer washed with brine, dried over Na₂SO₄ and concentrated *in vacuo* to afford the desired product (900 mg, 85% yield). **¹H NMR** (400 MHz, DMSO-*d6*) δ: 7.29 (s, 1H), 7.11 (s, 1H), 5.41 (s, 2H), 4.21 (s, 3H), 3.99(dd, *J* = 1.7, 11.2 Hz, 2H).

### 1-(3-(4-(2-((4-Chloro-2-hydroxy-5-(2,2,2-trifluoroethyl)phenyl)amino)acetyl)piperazin-1-yl)azetidin-1-yl)prop-2-en-1-one (V-57) (Reference Example)

The title compound was prepared from 4-chloro-2-methoxy-5-(2,2,2-trifluoroethyl)aniline in six steps according to the procedure described in **Example 41**. **¹H NMR** (400 MHz, DMSO-*d6*) δ: 10.08 (s, 1H), 6.74 (s, 1H), 6.59 (s, 1H), 6.34 (dd, *J* = 10.5, 16.9 Hz, 1H), 6.12 (dd, *J* = 1.7, 16.7 Hz, 1H), 5.69 (dd, *J* = 1.7, 16.7 Hz, 1H), 5.22 (m, 1H), 4.24 (m, 1H), 4.04 (m, 1H), 3.94 (m, 1H), 3.88 (d, *J* = 4.4 Hz, 2H), 3.78 (m, 1H), 3.57 (m, 2H), 3.54 (m, 4H), 3.18 (m, 1H), 2.37 (m, 4H). **ESI-MS** *m*/*z*: 461.2[M+H]⁺.

### EXAMPLE 51

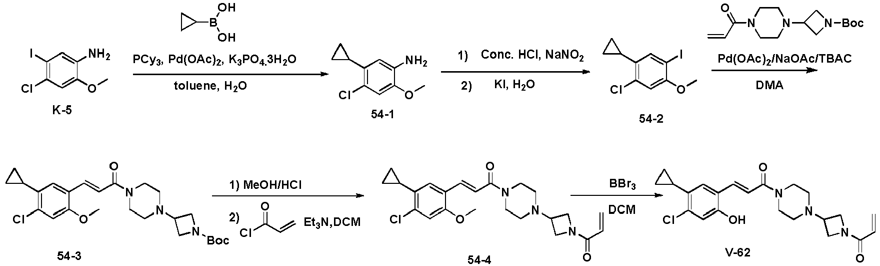

### 4-Chloro-5-cyclopropyl-2-methoxybenzenamine

A mixture of 4-chloro-5-iodo-2-methoxyaniline (5.0 g, 17.6 mmol), cyclopropylboronic acid (1.8 g, 21.1 mmol), Pd(OAc)₂(314 mg, 1.4 mmol), tricyclohexylphosphine (500 mg, 17.6mmol), K₃PO₄.3H₂O (16.4g, 61.6mmol) in toluene (62.5 mL) and H₂O (3 mL) was stirred at 80°C under argon for 16 h. The mixture was allowed to cool to RT, and then partitioned between ethyl acetate and water. The organic layer was washed with brine, dried over Na₂SO₄ and concentrated *in vacuo.* The residue was purified by flash column chromatography on silica gel (petroleum ether/ethyl acetate = 10:1) to afford the desired product (3.1 g, 88.5% yield). **ESI-MS** *m*/*z*: 198.2[M+H]⁺.

### 1-Chloro-2-cyclopropyl-4-iodo-5-methoxybenzene

To a mixture of 4-chloro-5-cyclopropyl-2-methoxyaniline (2.2 g, 11.05 mmol), conc. HCl (12 mL) and water (12 mL) at 0°C, the solution of sodium nitrate (762.8 mg, 11.05 mmol) in water (2.5 mL) was added dropwise. After stirring at 0°C for 15 min, a solution of KI (1.83 g, 11.05 mmol) in water (5 mL) was added dropwise. The resulting mixture was stirred at RT for 4 h, poured into water (20 mL) and then extracted with dichloromethane. The organic layer was washed with brine, dried over anhydrous Na₂SO₄, filtered and concentrated *in vacuo.* The residue was purified by flash column chromatography on silica gel (0-10% ethyl acetate/petroleum ether) to afford the desired product (680 mg, 20% yield) as a solid. **¹H NMR** (400 MHz, DMSO-*d6)* δ: 7.37 (s, 1H), 7.08 (s, 1H), 3.84 (s, 3H), 2.00 (m, 1H), 0.89 (m, 2H), 0.65 (m, 1H).

### (E)-1-(4-(1-Acryloylazetidin-3-yl)piperazin-1-yl)-3-(4-chloro-5-cyclopropyl-2-methoxyphenyl)prop-2-en-1-one

A mixture of 1-chloro-2-cyclopropyl-4-iodo-5-methoxybenzene (300 mg, 0.974 mmol), tert-butyl 3-(4-acryloylpiperazin-1-yl)azetidine-1-carboxylate (431 mg, 1.46 mmol), Pd(OAc)₂ (54.6 mg, 0.243 mmol), sodium acetate (239 mg, 2.92 mmol), tetrabutylammonium chloride (539 mg, 1.95 mmol) in DMF (7 mL) was stirred at 100°C for 24 h. The mixture was partitioned between ethyl acetate and water. The organic layer was washed with brine, dried over Na₂SO₄, filtered and concentrated *in vacuo* to purified by silica gel (dichloromethane/methanol = 40:1) to afford the desired product (350 mg, 84% yield). ESI-MS *m*/*z*: 476.2 [M+H]⁺.

### (E)-1-(4-(1-Acryloylazetidin-3-yl)piperazin-1-yl)-3-(4-chloro-5-cyclopropyl-2-hydroxyphenyl)prop-2-en-1-one (V-62) (Reference Example)

The title compound was prepared from (E)-1-(4-(1-acryloylazetidin-3-yl)piperazin-1-yl)-3-(4-chloro-5-cyclopropyl-2-methoxyphenyl)prop-2-en-1-one in three steps according to the procedure described in **Example 17**. **¹H NMR** (400 MHz, DMSO-*d6*) δ: 10.3 (s, 1H), 7.71 (m, 1H), 7.30 (s, 1H), 7.22 (m, 1H), 6.93 (s, 1H), 6.34 (dd, *J* = 10.5, 16.9 Hz, 1H), 6.12 (dd, *J* = 1.7, 16.7 Hz, 1H), 5.66 (dd, *J* = 1.7, 16.7 Hz, 1H), 4.26 (m, 1H), 4.08 (m, 1H), 3.94 (m, 1H), 3.79 (m, 1H), 3.69 (m, 2H), 3.58 (m, 2H), 3.18 (m, 1H), 2.33 (m, 4H), 1.99 (m, 1H), 0.92 (m, 2H), 0.73 (m, 2H). **ESI-MS** *m*/*z*: 416 [M+H]⁺.

### EXAMPLE 52

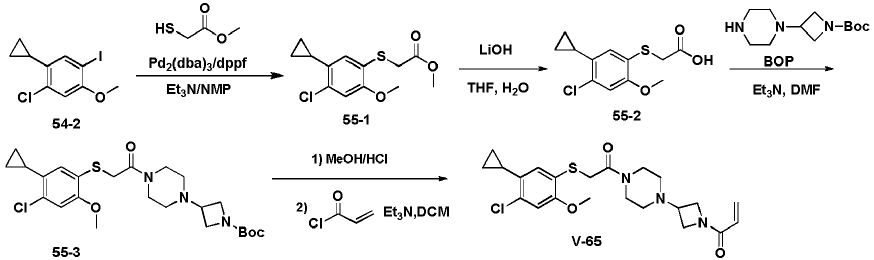

### Methyl 2-((4-chloro-5-cyclopropyl-2-methoxyphenyl)thio)acetate

A mixture of 1-chloro-2-cyclopropyl-4-iodo-5-methoxybenzene (380 mg, 1.23 mmol), Pd₂(dba)₃ (56 mg, 0.061 mmol), methyl 2-mercaptoacetate (196 mg, 1.85 mmol), 1,1'-Bis(diphenylphosphino)ferrocene (136 mg, 0.246 mmol), Et₃N (372 mg, 3.69 mmol) in NMP (8 mL) was stirred under argon at 80°C for 24 h. After cooled to RT, the reaction mixture was extracted with ethyl acetate. The organic layer was washed with brine, dried over Na₂SO₄ and concentrated *in vacuo.* The residue was purified by flash column chromatography on silica gel (petroleum ether/ethyl acetate = 20:1) to afford the desired product (340 mg, 92% yield). **¹H NMR** (400 MHz, DMSO-*d6*) δ: 7.05 (s, 1H), 6.81 (s, 1H), 3.84 (s, 2H), 3.82 (s, 3H), 3.62 (s, 3H), 2.00 (m, 1H), 0.94 (m, 2H), 0.64 (m, 2H).

### 1-(3-(4-(2-(4-Chloro-5-cyclopropyl-2-methoxyphenylthio)acetyl)piperazin-1-yl)azetidin-1-yl)prop-2-en-1-one (V-65) (Reference Example)

The title compound was prepared from methyl 2-((4-chloro-5-cyclopropyl-2-methoxyphenyl)thio)acetate in four steps according to the procedure described in **Example 40.¹H NMR** (400 MHz, DMSO-*d6*) δ: 7.03 (s, 1H), 6.93 (s, 1H), 6.31 (dd, *J* = 10.5, 16.9 Hz, 1H), 6.11 (dd, *J* = 1.7, 16.7 Hz, 1H), 5.68 (dd, *J* = 1.7, 16.7 Hz, 1H), 4.25 (m, 1H), 4.04 (m, 1H), 3.88 (s, 2H) 3.81 (s, 3H), 3.75 (m, 1H), 3.52 (m, 4H), 3.16 (m, 1H), 2.36-2.25 (m, 4H), 2.02 (m, 1H), 0.93 (m, 2H), 0.66 (m, 2H). **ESI-MS** *m*/*z*: 450 [M+H]⁺.

### EXAMPLE 53

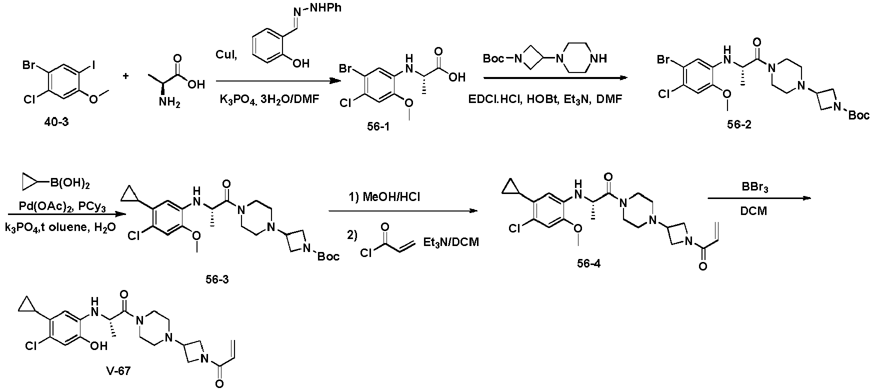

### (S)-2-(5-Bromo-4-chloro-2-methoxyphenylamino)propanoic acid

A mixture of 1-bromo-2-chloro-5-lodo-4-methoxybenzene (3 g, 8.64 mmol), (S)-2-aminopropanoic acid (769 mg, 8.64 mmol), CuI (164 mg, 0.864 mmol), 2-hydroxybenzaldehyde phenylhydrazone (366 mg, 1.73 mmol), K₃PO₄.3H₂O (4.6 g, 17.28 mmol) in DMF (10 mL) was stirred under argon at 80°C for 16 h. The mixture was allowed to cool to RT, H₂O and Et₂O were added to the solution. The resulting solution was partitioned into two phases, the aqueous phase was separated, and the organic layer was extracted with 5% NaOH. The combined aqueous phase was acidified to pH 4 with 20% HCl, and then extracted with Et₂O. The resulting organic layer was dried over MgSO₄ and concentrated *in vacuo* to afford the desired product (1.7 g, 64% yield). **ESI-MS** *m*/*z*: 306.1 [M+H]⁻

### (S)-tert-Butyl 3-(4-(2-((5-bromo-4-chloro-2-methoxyphenyl)amino)propanoyl)piperazin-1-yl)azetidine-1-carboxylate

To a solution of (S)-2-(5-bromo-4-chloro-2-methoxyphenylamino)propanoic acid (1.6 g, 5.21 mmol), tert-butyl 3-(piperazin-1-yl)azetidine-1-carboxylate (1.88 g, 7.82 mmol), EDCI.HCl (2.0 g, 10.42 mmol), HOBt (1.41 g, 10.42 mmol) in DMF (20 mL) at 0°C, Et₃N (1.58 g, 15.63 mmol) was added. The resulting mixture was stirred at RT for 16 h and then partitioned between ethyl acetate and water. The organic layer was washed with brine, dried over Na₂SO₄ and concentrated *in vacuo.* The residue was purified by flash column chromatography on silica gel (methanol/ dichloroethane = 1:50) to afford the desired product (2.1 g, 76% yield). **ESI-MS** *m*/*z*: 531.3 [M+H]⁺.

### ((S)-tert-Butyl 3-(4-(2-((4-chloro-5-cyclopropyl-2-methoxyphenyl)amino)propanoyl)piperazin-1-yl)azetidine-1-carboxylate

A mixture of (S)-tert-butyl 3-(4-(2-((5-bromo-4-chloro-2-methoxyphenyl)amino)propanoyl)piperazin-1-yl)azetidine-1-carboxylate (700 mg, 1.32 mmol), cyclopropylboronic acid (114 mg, 1.32 mmol), Pd(OAc)₂ (15 mg, 0.066 mmol), tricyclohexylphosphine (37 mg, 0.132 mmol), K₃PO₄.3H₂O (974 mg, 4.62 mmol) in DMF (10 mL) and H₂O (0.5 mL) was stirred under argon at 80°C for 16 h. The mixture was allowed to cool to RT, and then partitioned between ethyl acetate and water. The organic layer was washed with brine, dried over Na₂SO₄ and concentrated *in vacuo.* The residue was purified by flash column chromatography on silica gel (methanol/ dichloroethane = 1:100) to afford the desired product (400 mg, 62%). **ESI-MS** *m*/*z*: 493.2[M+H]⁺.

### (S)-1-(3-(4-(2-((4-Chloro-5-cyclopropyl-2-hydroxyphenyl)amino)propanoyl)piperazin-1-yl)azetidin-1-yl)prop-2-en-1-one (V-67) (Reference Example)

The title compound was prepared from (S)-tert-butyl 3-(4-(2-((4-chloro-5-cyclopropyl-2-methoxyphenyl)amino)propanoyl)piperazin-1-yl)azetidine-1-carboxylate in three steps according to the procedure described in **Example 17**. **¹H NMR** (400 MHz, DMSO-*d6*) δ: 9.69 (s, 1H), 6.66 (s, 1H), 6.29 (dd, *J* = 10.5, 16.9 Hz, 1H), 6.12 (dd, *J* = 1.7, 16.7 Hz, 1H), 6.05 (s, 1H), 5.68 (dd, *J* = 1.7, 16.7 Hz, 1H), 4.84 (m, 1H), 4.61 (m, 1H), 4.24 (m, 1H), 4.06 (m, 1H), 3.94 (m, 1H), 3.78 (m, 4H), 3.55 (m, 1H), 2.43-2.17 (m, 4H), 1.97 (m, 1H), 0.88 (m, 2H), 0.63 (m, 2H). **ESI-MS** *m*/*z*: 433.3 [M+H]⁺.

### EXAMPLE 54

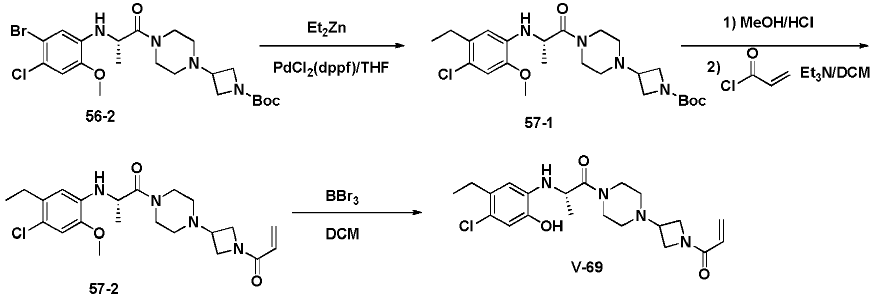

### (S)-tert-Butyl 3-(4-(2-((4-chloro-5-ethyl-2-methoxyphenyl)amino)propanoyl)piperazin-1-yl)azetidine-1-carboxylate

To a solution of (S)-tert-butyl 3-(4-(2-((5-bromo-4-chloro-2-methoxyphenyl)amino)propanoyl)piperazin-1-yl)azetidine-1-carboxylate (400 mg, 0.75 mmol), PdCl₂(dppf) (95 mg, 0.13 mmol) in THF (20 mL) at RT, Et₂Zn (2.86 mL, 2.86 mmol, 1.0 M in hexane) was added. The resulting mixture was stirred under argon at 80°C for 4 h and then partitioned between ethyl acetate and water. The organic layer was washed with brine, dried over Na₂SO₄ and concentrated *in vacuo.* The residue was purified by flash column chromatography on silica gel (methanol/ dichloroethane = 1:80) to afford the desired product (250 mg, 69% yield). **ESI-MS** *m*/*z:* 481.2 [M+H]⁺.

### (S)-1-(3-(4-(2-((4-Chloro-5-ethyl-2-hydroxyphenyl)amino)propanoyl)piperazin-1-yl)azetidin-1-yl)prop-2-en-1-one (V-69) (Reference Example)

The title compound was prepared from (S)-tert-butyl 3-(4-(2-((4-chloro-5-ethyl-2-methoxyphenyl)amino)propanoyl)piperazin-1-yl)azetidine-1-carboxylate in three steps according to the procedure described in **Example 17. ¹H NMR** (400 MHz, DMSO-*d6*) δ: 9.61 (s, 1H), 6.64 (s, 1H), 6.48 (s, 1H), 6.29 (dd, *J* = 10.5, 16.9 Hz, 1H), 6.12 (dd, *J* = 1.7, 16.7 Hz, 1H), 5.68 (dd, *J* = 1.7, 16.7 Hz, 1H), 4.89 (m, 1H), 4.61 (m, 1H), 4.25 (m, 1H), 4.06 (m, 1H), 3.94 (m, 1H), 3.72-3.53 (m, 4H), 3.16 (m, 1H), 2.5 (m, 2H), 2.43-2.17 (m, 4H), 1.21 (dd, 3H), 1.15 (m, 3H). **ESI-MS** *m*/*z*: 406.2 [M+H]⁺.

### EXAMPLE 55

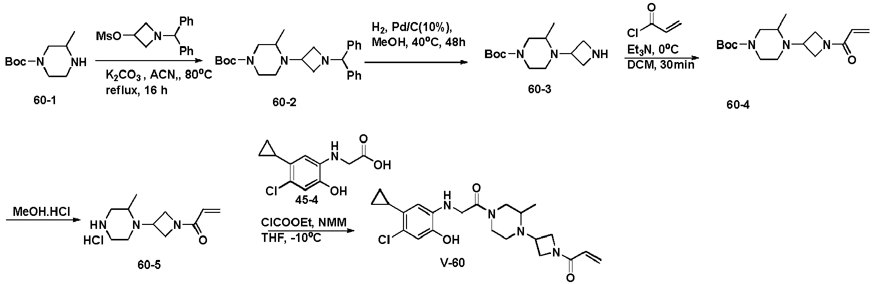

### tert-Butyl 4-(1-acryloylazetidin-3-yl)-3-methylpiperazine-1-carboxylate

The title compound was prepared from tert-butyl 3-methylpiperazine-1-carboxylate in three steps according to the procedure described in **Example 38**.

### 1-(3-(2-Methylpiperazin-1-yl)azetidin-1-yl)prop-2-en-1-one hydrochloride

The mixture of tert-butyl 4-(1-acryloylazetidin-3-yl)-3-methylpiperazine-1-carboxylate (62 mg, 0.199 mmol) in MeOH/HCl (20 mL, 2.9 M) was stirred at RT for 1 h. The mixture was concentrated *in vacuo* to afford the crude product (59 mg). The crude product was used directly in the next step without further purification.

### 1-(3-(4-(2-((4-Chloro-5-cyclopropyl-2-hydroxyphenyl)amino)acetyl)-2-methylpiperazin-1-yl)azetidin-1-yl)prop-2-en-1-one (V-60) (Reference Example)

To the mixture of 2-((4-chloro-5-cyclopropyl-2-hydroxyphenyl)amino)acetic acid (30 mg, 0.124 mmol) and NMM (50 mg, 0.496 mmol) in dry THF (30 mL) at -10°C, ethyl chloroformate (15 mg, 0.136 mmol) was added and the resulting mixture was stirred at -10°C for 45 min. Then it was added a mixture of 1-(3-(2-methylpiperazin-1-yl)azetidin-1-yl)prop-2-en-1-one hydrochloride (37 mg, 0.149 mmol), Et₃N (50 mg, 0.496 mmol) and dichloromethane (3 mL). The resulting mixture was stirred at RT for 30 min. The mixture was partitioned between ethyl acetate and water. The organic layer was washed with saturated NaHCO₃ solution and brine, dried over Na₂SO₄, filtered and concentrated *in vacuo.* The residue was purified with column chromatography on silica gel (dichloromethane/methanol = 40: 1) to afford the desired product (10 mg, 18.6% yield) as a white solid. **¹H** NMR (400 MHz, DMSO-*d6*) δ: 9.65 (s, 1H), 6.66 (s, 1H), 6.34-6.27 (m, 1H), 6.10-6.07 (m, 2H), 5.68-5.65 (d, *J* = 10.4 Hz, 1H), 5.12 (m, 1H), 4.29-4.19 (m, 1H), 4.12-4.10 (m, 1H), 4.08-3.81 (m, 4H) , 3.78 (s, 4H), 2.63 (m, 2H), 2.25 (m, 1H), 1.96 (m, 1H), 1.24 (s, 1H), 0.96-0.87 (m, 5H), 0.63 (m, 2H). **ESI-MS** *m*/*z*: 433.5 [M+H]⁺.

### EXAMPLE 56

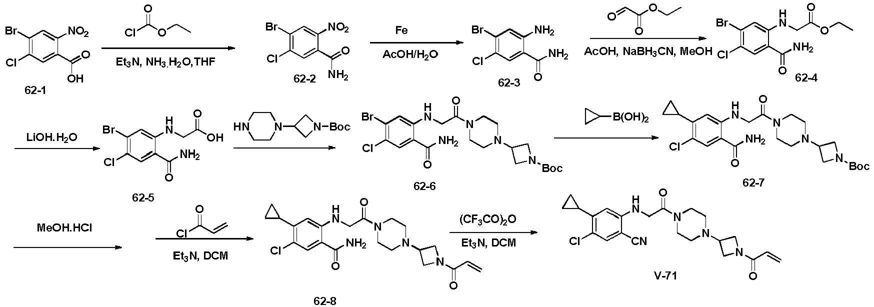

### 4-Bromo-5-chloro-2-nitrobenzamide

A mixture of 4-bromo-5-chloro-2-nitrobenzoic acid (1.3 g, 4.63 mmol), Et₃N (1.4 g, 13.9 mmol) in THF (20 mL) at 0°C, ethyl chloroformate (1.5 g, 13.9 mmol) was added. The resulting mixture was stirred at 0°C for 1 h. Then NH₃.H₂O (4 mL) was added and stirred for 0.5 h. The mixture was extracted with ethyl acetate. The organic layer was washed with brine, dried over anhydrous Na₂SO₄, filtered and concentrated *in vacuo* to afford the crude product (900 mg).

### 2-Amino-4-bromo-5-chlorobenzamide

To a solution of 4-bromo-5-chloro-2-nitrobenzamide (900 mg, 3.2 mmol) in AcOH (20 mL) and water (5 mL) at 70°C, Fe powder (900 mg, 16.1 mmol) was added and the resulting mixture was stirred at 70°C for 1 h. The mixture was allowed to cool to RT and poured into ice-water. The precipitate was collected by filtration and rinsed with water. This crude product was dissolved with ethyl acetate and filtered. The filtrate was washed with saturated NaHCO₃ solution and brine. The organic layer was dried over MgSO₄, filtered, and concentrated *in vacuo* to afford the desired product (770 mg, 97% yield). **ESI-MS** *m*/*z:* 250.1 [M+H]⁺.

### tert-Butyl 3-(4-(2-((5-bromo-2-carbamoyl-4-chlorophenyl)amino)acetyl)piperazin-1-yl)azetidine-1-carboxylate

The title compound was prepared from 2-amino-4-bromo-5-chlorobenzamide in three steps according to the procedure described in **Example 41. ESI-MS** *m*/*z:* 532.5 [M+H]⁺.

### tert-Butyl 3-(4-(2-((2-carbamoyl-4-chloro-5-cyclopropylphenyl)amino)acetyl)piperazin-1-yl)azetidine-1-carboxylate

To a solution of tert-butyl 3-(4-(2-((5-bromo-2-carbamoyl-4-chlorophenyl)amino)acetyl)piperazin-1-yl)azetidine-1-carboxylate (350 mg, 0.66 mmol) and cyclopropylboronic acid (226 mg, 2.64 mmol) in toluene (10 mL) and water (2 mL), Pd(OAc) ₂(15 mg, 0.07 mmol), PCy₃ (37 mg, 0.132 mmol) and K₃PO₄ (487 mg, 2.31 mmol) were added. The mixture was stirred at 80°C for 16 h. The mixture was allowed to cool to RT and concentrated *in vacuo.* The residue was purified by flash column chromatography on silica gel (1-5% methanol/dichloroethane) to afford the desired product (150 mg, 46 % yield) as a solid.

### 2-((2-(4-(1-Acryloylazetidin-3-yl)piperazin-1-yl)-2-oxoethyl)amino)-5-chloro-4-cyclopropylbenzamide

The title compound was prepared from tert-butyl 3-(4-(2-((2-carbamoyl-4-chloro-5-cyclopropylphenyl)amino)acetyl)piperazin-1-yl)azetidine-1-carboxylate in two steps according to the procedure described in **Example 17**. **ESI-MS** *m*/*z:* 446.4 [M+H]⁺.

### 2-((2-(4-(1-Acryloylazetidin-3-yl)piperazin-1-yl)-2-oxoethyl)amino)-5-chloro-4-cyclopropylbenzonitrile (V-71) (Reference Example)

A mixture of 2-((2-(4-(1-acryloylazetidin-3-yl)piperazin-1-yl)-2-oxoethyl)amino)-5-chloro-4-cyclopropylbenzamide (3 0mg, 0.067 mmol) and Et₃N (41mg, 0.404 mmol) in DCM (10 mL) at RT, trifluoroacetic anhydride (56 mg, 0.268 mmol) was added. The resulting mixture was stirred at RT for 0.5 h, poured into water and then extracted with dichloromethane. The organic layer was washed with brine, dried over Na₂SO₄ and concentrated. The residue was purified by flash column chromatography on silica gel (1-4% methanol/dichloroethane) to afford the desired product (20 mg, 72% yield). **¹H NMR** (400 MHz, DMSO-*d6*) δ: 7.60 (s, 1H), 6.34-6.30 (m,1H), 6.27 (s, 1H), 6.12-6.07 (m, 1H), 6.01-5.99 (t, *J* = 4 Hz, 1H), 5.69-5.65 (m, 1H), 4.26-4.22 (m, 1H), 4.07-4.04 (m, 3H), 3.96-3.92 (m, 1H), 3.80-3.76 (m, 1H), 3.53-3.51 (m, 4H), 3.19-3.13 (m, 1H), 2.45-2.30 (m, 4H), 2.16-2.09 (m, 1H), 1.08-1.03 (m, 2H), 0.87-0.80 (m, 2H). **ESI-MS** *m*/*z*: 428.4 [M+H]⁺.

### EXAMPLE 57

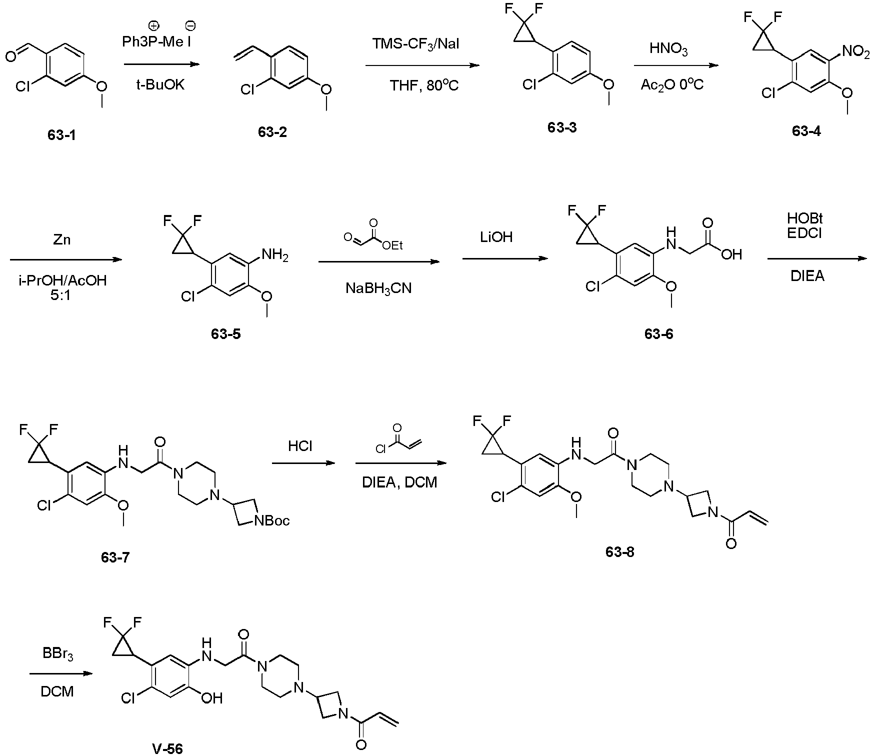

### 2-Chloro-4-methoxy-1-vinylbenzene

To a suspension of phosphonium salt (2.05 g, 5 mmol) in THF (50 mL), was added t-BuOK (0.84g, 7.5 mmol). The mixture turned to yellow and was kept stirring at RT for 1h. 2-Chloro-4-methoxybenzaldehyde (0.85g, 5 mmol) was added to the mixture. The mixture was stirred for 24h, diluted with sat. NaHCO3 and then extracted with hexane. Organic layer was dried over Na2SO4, filtered and concentrated in vacuo. The residue was purified by Isolera One (100% hexanes to afford the desired product (0.45g, 53% yield). 1H NMR (CDCl3, δ): 7.49 (d, J = 6.8Hz, 1H), 7.03 (dd, J = 8.8, 14.0 Hz, 1H), 6.90 (d, J = 2.0, 1H), 6.79 (dd, J = 2.0, 6.8 Hz, 1H), 5.62 (d, J = 14.0 Hz, 1H), 5.26 (d, J = 8.8 Hz, 1H), 3.80 (s, 3H).

### 2-Chloro-1-(2,2-difluorocyclopropyl)-4-methoxybenzene

The solution of 2-chloro-4-methoxy-1-vinylbenzene (290 mg, 1.72 mmol) in dry THF (4 mL) was degassed, and then TMS-CF3 and NaI were added. The mixture was stirred at 80oC overnight. TLC (100% Hexane) showed the reaction as complete. The mixture was diluted with hexane (20 mL). The inorganic salt was removed by filtration. The filtrate was concentrate in vacuo. The residue was purified via Isolera One (Hexane = 100%).

### 1-Chloro-2-(2,2-difluorocyclopropyl)-5-methoxy-4-nitrobenzene

To a solution of 2-chloro-1-(2,2-difluorocyclopropyl)-4-methoxybenzene (328 mg, 1.5 mmol) in Ac2O (2 mL), was added HNO3 (10 drops) at 0oC. The mixture was stirred from 0oC to rt. Ac2O was removed in vacuo. The residue was diluted with DCM and washed with water. The organic layer was dried over Na2SO4. The solvent was removed in vacuo. The residue was purified via Isolera One (EtOAc/Hexane = 0 -15%) to afford the desired product. 1H NMR (CDCl3, δ): 7.77 (s, 1H), 7.16 (s, 1H), 3.98 (s, 3H), 2.78-2.90 (m, 1H), 1.90-1.98 (m, 1H), 1.60-1.68 (m, 1H). ESI-MS m/z: 264.1 [M+H]+.

### 4-Chloro-5-(2,2-difluorocyclopropyl)-2-methoxyaniline

The above obtained 1-chloro-2-(2,2-difluorocyclopropyl)-5-methoxy-4-nitrobenzene was dissolved in 10 mL of co-solvent of AcOH/i-PrOH (1:5). Zn dust was added to the mixture. The mixture was stirred at 60oC for 30 min. The solvent was removed in vacuo. The residue was diluted was DCM and the inorganic salt was removed by filtration. The filtrate was concentrated to give crude product which was used in next step without further purification.

### 1-(3-(4-((4-chloro-5-(2,2-difluorocyclopropyl)-2-hydroxyphenyl)glycyl)piperazin-1-yl)azetidin-1-yl)prop-2-en-1-one (V-56) (Reference Example)

The title compound was prepared from 4-chloro-5-(2,2-difluorocyclopropyl)-2-methoxyaniline in 6 steps according to the procedure described in Example 41.

1H NMR (CDCl3, δ): 9.90 (s, 1H), 6.73 (s, 1H), 6.40 (s, 1H), 6.30 (dd, J = 8.4, 13.6 Hz, 1H), 6.10 (dd, J = 1.6, 12.0 Hz, 1H), 5.66 (dd, J = 1.6, 8.4 Hz, 1H), 5.18 (t, J = 3.2, 3.6 Hz, 1H), 4.24 (t, J = 6.0, 6.8 Hz, 1H), 4.03-4.08 (m, 1H), 3.86-3.97 (m, 3H), 3.74-3.80 (m, 1H), 3.52 (br. s, 4H), 3.13-3.20 (m, 1H), 2.77-2.87 (m, 1H), 2.25-2.43 (m, 4H), 1.87-1.97 (m, 2H). ESI-MS m/z: 455.2 [M+H]+.

### EXAMPLE 58

### BIOCHEMICAL ASSAY OF THE COMPOUNDS

Test compounds were prepared as 10 mM stock solutions in DMSO (Fisher cat# BP-231-100). KRAS G12C 1-169, his-tagged protein, GDP-loaded was diluted to 2 µm in buffer (20mM Hepes, 150mM NaCl, 1mM MgCl2). Compounds were tested for activity as follows:
Compounds were diluted to 50X final test concentration in DMSO in 96-well storage plates. Compound stock solutions were vortexed before use and observed carefully for any sign of precipitation. Dilutions were as follow:
- For 100µM final compound concentration, compounds were diluted to 5000µM (5µl 10mM compound stock + 5µl DMSO and mixed well by pipetting.
- For 30µM final compound concentration, compounds were diluted to 1500µM (3µl 10mM compound stock + 17µl DMSO) and mixed well by pipetting.
- For 10µM final compound concentration, compounds were diluted to 500µM (2µl 10mM compound stock + 38µl DMSO) and mixed well by pipetting.
49µl of the stock protein solution was added to each well of a 96-well PCR plate (Fisher cat# 1423027). 1µl of the diluted 50X compounds were added to appropriate wells in the PCR plate using 12-channel pipettor. Reactions were mixed carefully and thoroughly by pipetting up/down with a 200µl multi-channel pipettor. The plate was sealed well with aluminum plate seal, and stored in a drawer at room temperature for 2hrs or 24hrs. 5µl of 2% formic acid (Fisher cat# A117) in DI H2O was then added to each well followed by mixing with a pipette. The plate was then resealed with aluminum seal and stored on dry ice until analyzed as described below.

The above described assays were analyzed by mass spectrometry according to the following procedure:
The MS instrument is set to positive polarity, 2 GHz resolution, and low mass (1700) mode and allowed to equilibrate for 30 minutes. The instrument is then calibrated, switched to acquisition mode and the appropriate method loaded.

After another 30 minute equilibration time, a blank batch (i.e., buffer) is run to ensure equipment is operating properly. The samples are thawed at 37°C for 10 minutes, briefly centrifuged, and transfer to the bench top. Wells A1 and H12 are spiked with 1 uL 500 uM internal standard peptide, and the plates centrifuged at 2000 x g for 5 minutes. The method is then run and masses of each individual well recorded.

The masses (for which integration data is desired) for each well are pasted into the platemap and exported from the analysis. Masses for the internal standards are exported as well. The data at 50 ppm is extracted for the +19 charge state, and identity of well A1 is assigned using the internal standard spike and integrated. Peak data is exported as a TOF list and the above steps are repeated individually, for the +20, 21, 22, 23, 24, and 25 charge states.

Other in vitro analyses are as follows:

### Inhibition of Cell Growth:

The ability of the subject compounds to inhibit Ras-mediated cell growth is assessed and demonstrated as follows. Cells expressing a wildtype or a mutant Ras are plated in white, clear bottom 96 well plates at a density of 5,000 cells per well. Cells are allowed to attach for about 2 hours after plating before a compound disclosed herein is added. After certain hours (e.g., 24 hours, 48 hours, or 72 hours of cell growth), cell proliferation is determined by measuring total ATP content using the Cell Titer Glo reagent (Promega) according to manufacturer's instructions. Proliferation EC50s is determined by analyzing 8 point compound dose responses at half-log intervals decreasing from 100 µM.

### Inhibition of Ras-mediated signaling transduction:

The ability of the compounds disclosed herein in inhibiting Ras-mediated signaling is assessed and demonstrated as follows. Cells expressing wild type or a mutant Ras (such as G12C, G12V, or G12A) are treated with or without (control cells) a subject compound. Inhibition of Ras signaling by one or more subject compounds is demonstrated by a decrease in the steady-state level of phosphorylated MEK, and/or Raf binding in cells treated with the one or more of the subject compounds as compared to the control cells.

### Inhibition of Ras-mediated signaling transduction:

The ability of the compounds disclosed herein in inhibiting Ras-mediated signaling is assessed and demonstrated as follows. Cells expressing wild type or a mutant Ras (such as G12C, G12V, or G12A) are treated with or without (control cells) a subject compound. Inhibition of Ras signaling by one or more subject compounds is demonstrated by percentage binding of compound to the G12C mutated Ras protein in cells treated with the one or more of the subject compounds as compared to the control cells.

### Inhibition of Ras-mediated signaling transduction:

The ability of the compounds disclosed herein in inhibiting Ras-mediated signaling is assessed and demonstrated as follows. Cells expressing wild type or a mutant Ras (such as G12C, G12V, or G12A) are treated with or without (control cells) a subject compound. Inhibition of Ras signaling by one or more subject compounds is demonstrated by a decrease in binding of Ras complex to downstream signaling molecules (for example Raf) in cells treated with the one or more of the subject compounds as compared to the control cells.

The compounds in Tables 5-6 were tested according to the above procedures. Results for the compounds of Tables 5 and 6 are presented below. Each of the compounds in Tables 5 and 6 were found to covalently bind to KRAS G12C to the extent of at least about 5% (*i.e.*, at least about 5% of the protein present in the well was found to be covalently bound to test compound).

**Table 7a**

| Activity of Reference Compounds* | | | | | | | |
|---|---|---|---|---|---|---|---|
| No. | Binding % | No. | Binding % | No. | Binding % | No. | Binding % |
| V-1 | ++++ | V-2 | +++ | V-3 | ++++ | V-4 | +++ |
| V-5 | + | V-6 | + | V-7 | ++ | V-8 | ++ |
| V-9 | + | V-10 | + | V-11 | ++++ | V-12 | +++ |
| V-13 | ++ | V-14 | +++ | V-15 | + | V-16 | + |
| V-17 | ++ | V-18 | ++++ | V-19 | +++ | V-20 | ++ |
| V-21 | + | V-22 | + | V-23 | + | V-24 | + |
| V-25 | + | V-26 | + | V-27 | ++ | V-28 | +++ |
| V-29 | + | V-30 | + | V-31 | ++++ | V-32 | ++++ |
| V-33 | + | V-34 | + | V-35 | ++++ | V-36 | ++++ |
| V-37 | ++ | V-3 8 | + | V-39 | +++ | V-40 | ++++ |
| V-41 | ++++ | V-42 | + | V-43 | ++++ | V-44 | + |
| V-45 | ++++ | V-46 | + | V-47 | + | V-48 | ++ |
| V-49 | ++++ | N/A | N/A | N/A | N/A | N/A | N/A |

| | | | | | | | |
|---|---|---|---|---|---|---|---|
| * Binding activity determined at 24hrs. + indicates binding activity from 5% to 15% ++ indicates binding activity greater than 15% and up to 25% +++ indicates binding activity greater than 25% and up to 50% ++++ indicates binding activity greater than 50% | | | | | | | |

**Table 7b**

| Activity of Reference Compounds* | | | | | | | |
|---|---|---|---|---|---|---|---|
| No. | Binding % | No. | Binding % | No. | Binding % | No. | Binding % |
| V-50 | ++ | V-51 | ++ | V-52 | + | V-53 | ++ |
| V-54 | ++ | V-55 | ++ | V-56 | ++ | V-57 | ++ |
| V-58 | + | V-59 | ++ | V-60 | ++ | V-61 | + |
| V-62 | + | V-63 | ++ | V-64 | ++ | V-65 | + |
| V-66 | + | V-67 | ++ | V-68 | ++ | V-69 | ++ |
| V-70 | ++ | V-71 | + | V-72 | + | V-73 | + |
| V-74 | + | V-75 | + | V-76 | N/A | N/A | N/A |

| | | | | | | | |
|---|---|---|---|---|---|---|---|
| * Binding activity determined at 2hrs. + indicates binding activity from 5% to 20% ++ indicates binding activity greater than 20% | | | | | | | |

**Table 8a**

| Activity of Representative and Reference Compounds* | | | | | | | |
|---|---|---|---|---|---|---|---|
| No. | Binding % | No. | Binding % | No. | Binding % | No. | Binding % |
| VI-1 | + | VI-2 | ++ | VI-3 | ++++ | VI-4 | ++ |
| VI-5 | ++++ | VI-6 | ++ | VI-7 | +++ | VI-8 | + |
| VI-9 | + | VI-10 | + | VI-11 | + | VI-12 | +++ |
| VI-13 | + | VI-14 | + | VI-15 | + | VI-16 | + |
| VI-17 | ++++ | VI-18 | + | VI-19 | + | VI-20 | + |
| VI-21 | ++ | VI-22 | + | VI-23 | +++ | VI-24 | ++++ |
| VI-25 | +++ | VI-26 | ++ | VI-27 | +++ | VI-28 | ++ |
| VI-29 | +++ | VI-30 | ++ | VI-31 | + | VI-32 | ++++ |
| VI-33 | + | VI-34 | +++ | VI-35 | ++ | VI-36 | + |

| | | | | | | | |
|---|---|---|---|---|---|---|---|
| * Binding activity determined at 24hrs. + indicates binding activity from 5% to 10% ++ indicates binding activity greater than 10% and up to 20% +++ indicates binding activity greater than 20% and up to 30% ++++ indicates binding activity greater than 30% | | | | | | | |

**Table 8b**

| Activity of Representative Compounds* | | | | | | | |
|---|---|---|---|---|---|---|---|
| No. | Binding % | No. | Binding % | No. | Binding % | No. | Binding % |
| VI-37 | ++ | VI-38 | ++ | VI-39 | ++ | VI-40 | + |
| VI-41 | + | VI-42 | + | VI-43 | + | N/A | N/A |

| | | | | | | | |
|---|---|---|---|---|---|---|---|
| * Binding activity determined at 2hrs. + indicates binding activity from 5% to 20% ++ indicates binding activity greater than 20% | | | | | | | |

## Claims

1. A compound having one of the following structures (VIa'), (VIb'), (VIc'), (VId'), (VIe'), (VIf') or (VIg'): 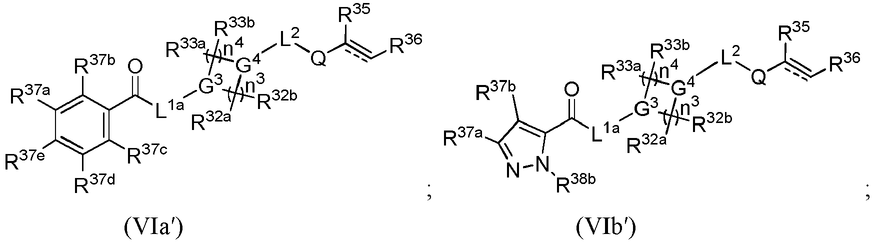 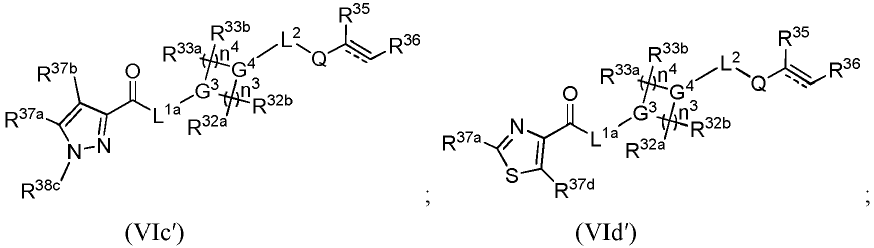 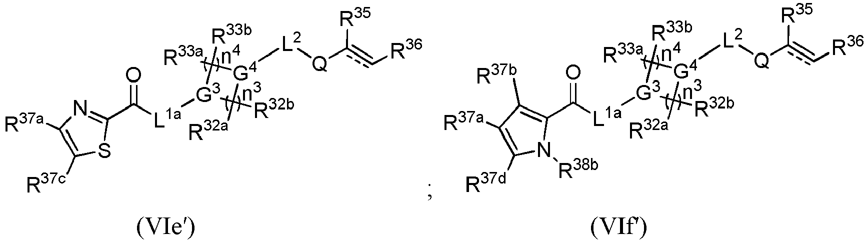 or 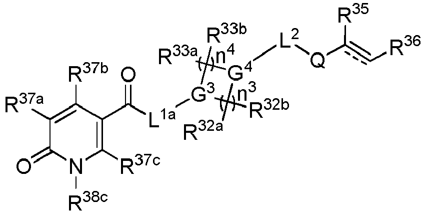 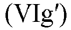 or a pharmaceutically acceptable salt, tautomer, stereoisomer or prodrug thereof, wherein the prodrug is selected from the group consisting of acetate, formate and benzoate derivatives of a hydroxy functional group, or acetamide, formamide and benzamide derivatives of an amine functional group in the compound; wherein:
i) G³ is N and G⁴ is CR; or
ii) G³ is CR and G⁴ is N; or
iii) G³ is N and G⁴ is N,
wherein R is H, cyano, halo or C₁-C₆alkyl;
L^{1a} is a bond, -NH-, alkylene or heteroalkylene;
L² is a bond or alkylene;
R^{32a} and R^{32b} are, at each occurrence, independently H, -OH, -NH₂, -CO₂H, cyano, cyanoalkyl, C₁-C₆alkyl, C₃-C₈cycloalkyl, hydroxylalkyl, aminoalkyl, carboxylalkyl or aminocarbonyl; or R^{32a} and R^{32b} join to form a carbocyclic or heterocyclic ring; or R^{32a} is H, -OH, -NH₂, -CO₂H, cyano, C₁-C₆alkyl, C₃-C₈cycloalkyl, hydroxylalkyl, aminoalkyl, carboxylalkyl or aminocarbonyl and R^{32b} joins with R^{33b} to form a carbocyclic or heterocyclic ring;
R^{33a} and R^{33b} are, at each occurrence, independently H, -OH, -NH₂, -CO₂H, cyano, cyanoalkyl, C₁-C₆alkyl, C₃-C₈cycloalkyl, hydroxylalkyl, aminoalkyl, carboxylalkyl or aminocarbonyl; or R^{33a} and R^{33b} join to form a carbocyclic or heterocyclic ring; or R^{33a} is H, -OH, -NH₂, -CO₂H, cyano, C₁-C₆alkyl, C₃-C₈cycloalkyl, hydroxylalkyl, aminoalkyl, carboxylalkyl or aminocarbonyl and R^{33b} joins with R^{32b} to form a carbocyclic or heterocyclic ring;
R^{37a}, R^{37b}, R^{37c}, R^{37d} and R^{37e} are each independently H, halo, oxo, hydroxyl, cyano, aminocarbonyl, formyl, C₁-C₆alkyl, C₁-C₆alkylsulfonyl, C₁-C₆haloalkyl, C₃-C₈cycloalkyl, C₁-C₆alkoxy, C₁-C₆hydroxylalkyl, C₁-C₆alkoxyalkyl, C₁-C₆aminoalkyl, heterocyclyl or aryl, wherein: i) R^{37a} or R^{37e} is aryl, or ii) R^{37a} or R^{37e}, or both, are chloro, bromo or iodo;
R^{38a}, R^{38b} and R^{38c} are each independently H, C₁-C₆alkyl or aryl;
n³ and n⁴ are each independently 1, 2 or 3;
Q is -C(=O)-, -NR³⁴C(=O)-, -S(=O)₂- or - NR³⁴S(=O)₂-;
R³⁴ is H, C₁-C₆alkyl or hydroxylalkyl;
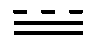 is a carbon-carbon double bond or a carbon-carbon triple bond; and
R³⁵ and R³⁶ are each independently H, cyano, C₁-C₆alkyl, aminoalkyl, alkylaminoalkyl, or hydroxylalkyl, wherein at least one of R³⁵ or R³⁶ is H,
wherein each occurrence of alkyl, alkylene, heteroalkylene, cyanoalkyl, cycloalkyl, hydroxylalkyl, aminoalkyl, carboxylalkyl, aminocarbonyl, alkylsulfonyl, carbocyclic ring, heterocyclic ring, haloalkyl, alkoxy, alkoxyalkyl, heterocyclyl, aryl or alkylaminoalkyl is optionally substituted unless otherwise specified.

2. The compound of claim 1 or a pharmaceutically acceptable salt, tautomer, stereoisomer or prodrug thereof, wherein the prodrug is selected from the group consisting of acetate, formate and benzoate derivatives of a hydroxy functional group, or acetamide, formamide and benzamide derivatives of an amine functional group in the compound, wherein:
i) n³ is 2 and n⁴ is 2; or
ii) n³ is 1 and n⁴ is 1; or
ii) n³ is 2 and n⁴ is 1.

3. The compound of claim 1 or 2, or a pharmaceutically acceptable salt, tautomer, stereoisomer or prodrug thereof, wherein the prodrug is selected from the group consisting of acetate, formate and benzoate derivatives of a hydroxy functional group, or acetamide, formamide and benzamide derivatives of an amine functional group in the compound, wherein each occurrence of alkyl, alkylene, heteroalkylene, cyanoalkyl, cycloalkyl, hydroxylalkyl, aminoalkyl, carboxylalkyl, aminocarbonyl, alkylsulfonyl, carbocyclic ring, heterocyclic ring, haloalkyl, alkoxy, alkoxyalkyl, heterocyclyl, aryl or alkylaminoalkyl is optionally substituted unless otherwise specified, wherein R^{37a}, R^{37b}, R^{37c}, R^{37d} and R^{37e} are each independently H, -OH, halo, oxo, C₁-C₆alkyl, C₁-C₆alkoxy, heterocyclyl or aryl, and wherein: i) R^{37a} or R^{37e} is aryl, or ii) R^{37a} or R^{37e}, or both, are chloro, bromo or iodo.

4. The compound of any one of claims 1-3, or a pharmaceutically acceptable salt, tautomer, stereoisomer or prodrug thereof, wherein the prodrug is selected from the group consisting of acetate, formate and benzoate derivatives of a hydroxy functional group, or acetamide, formamide and benzamide derivatives of an amine functional group in the compound, wherein each occurrence of alkyl, alkylene, heteroalkylene, cyanoalkyl, cycloalkyl, hydroxylalkyl, aminoalkyl, carboxylalkyl, aminocarbonyl, alkylsulfonyl, carbocyclic ring, heterocyclic ring, haloalkyl, alkoxy, alkoxyalkyl, heterocyclyl, aryl or alkylaminoalkyl is optionally substituted unless otherwise specified, wherein R^{37a} or R^{37e} is aryl, preferably phenyl, more preferably R^{37a} or R^{37e} is aryl or phenyl substituted with one or more halo substituents, even more preferably R^{37a} or R^{37e} is aryl or phenyl substituted with halo substituents selected from fluoro and chloro.

5. The compound of any one of claims 1-3, or a pharmaceutically acceptable salt, tautomer, stereoisomer or prodrug thereof, wherein the prodrug is selected from the group consisting of acetate, formate and benzoate derivatives of a hydroxy functional group, or acetamide, formamide and benzamide derivatives of an amine functional group in the compound, wherein R^{37a} or R^{37e}, or both, are chloro, bromo or iodo.

6. The compound of any one of claims 1-4, or a pharmaceutically acceptable salt, tautomer, stereoisomer or prodrug thereof, wherein the prodrug is selected from the group consisting of acetate, formate and benzoate derivatives of a hydroxy functional group, or acetamide, formamide and benzamide derivatives of an amine functional group in the compound, wherein R^{37a} or R^{37e} has one of the following structures:

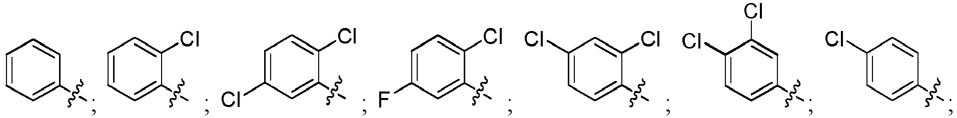

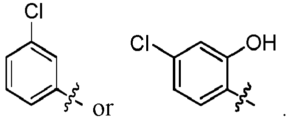

7. The compound of any one of claims 1-6, or a pharmaceutically acceptable salt, tautomer, stereoisomer or prodrug thereof, wherein the prodrug is selected from the group consisting of acetate, formate and benzoate derivatives of a hydroxy functional group, or acetamide, formamide and benzamide derivatives of an amine functional group in the compound, wherein Q is -C(=O)-.

8. The compound of any one of claims 1-7, or a pharmaceutically acceptable salt, tautomer, stereoisomer or prodrug thereof, wherein the prodrug is selected from the group consisting of acetate, formate and benzoate derivatives of a hydroxy functional group, or acetamide, formamide and benzamide derivatives of an amine functional group in the compound, wherein at least one of R³⁵ or R³⁶ is H, preferably wherein each of R³⁵ and R³⁶ are H.

9. The compound of any one of claims 1-8, or a pharmaceutically acceptable salt, tautomer, stereoisomer or prodrug thereof, wherein the prodrug is selected from the group consisting of acetate, formate and benzoate derivatives of a hydroxy functional group, or acetamide, formamide and benzamide derivatives of an amine functional group in the compound, wherein each occurrence of alkyl, alkylene, heteroalkylene, cyanoalkyl, cycloalkyl, hydroxylalkyl, aminoalkyl, carboxylalkyl, aminocarbonyl, alkylsulfonyl, carbocyclic ring, heterocyclic ring, haloalkyl, alkoxy, alkoxyalkyl, heterocyclyl, aryl or alkylaminoalkyl is optionally substituted unless otherwise specified, wherein:
i) L^{1a} is a bond; or
ii) L^{1a} is -NH-, alkylene or heteroalkylene; or
iii) L^{1a} is alkylene, preferably wherein L^{1a} is 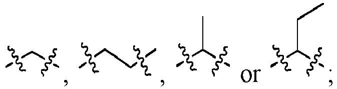 or
iv) L^{1a} is heteroalkylene, preferably wherein L^{1a} is aminoalkylene or thioalkylene, more preferably, wherein L^{1a} has one of the following structures:

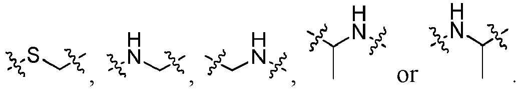

10. The compound of any one of claims 1-9, or a pharmaceutically acceptable salt, tautomer, stereoisomer or prodrug thereof, wherein the prodrug is selected from the group consisting of acetate, formate and benzoate derivatives of a hydroxy functional group, or acetamide, formamide and benzamide derivatives of an amine functional group in the compound, wherein each occurrence of alkyl, alkylene, heteroalkylene, cyanoalkyl, cycloalkyl, hydroxylalkyl, aminoalkyl, carboxylalkyl, aminocarbonyl, alkylsulfonyl, carbocyclic ring, heterocyclic ring, haloalkyl, alkoxy, alkoxyalkyl, heterocyclyl, aryl or alkylaminoalkyl is optionally substituted unless otherwise specified, wherein:
i) at least one of R^{32a}, R^{32b}, R^{33a} or R^{33b} is H, preferably wherein each of R^{32a}, R^{32b}, R^{33a} or R^{33b} is H; or
ii) at least one of R^{32a}, R^{32b}, R^{33a} or R^{33b} is hydroxylalkyl; or
iii) at least one of R^{32a}, R^{32b}, R^{33a} or R^{33b} is cyano; or
iv) at least one of R^{32a}, R^{32b}, R^{33a} or R^{33b} is aminocarbonyl; or
v) at least one of R^{32a}, R^{32b}, R^{33a} or R^{33b} is C₁-C₆ alkyl.

11. The compound of claim 1, or a pharmaceutically acceptable salt, tautomer, stereoisomer or prodrug thereof, wherein the prodrug is selected from the group consisting of acetate, formate and benzoate derivatives of a hydroxy functional group, or acetamide, formamide and benzamide derivatives of an amine functional group in the compound, wherein the compound has one of the following structures:

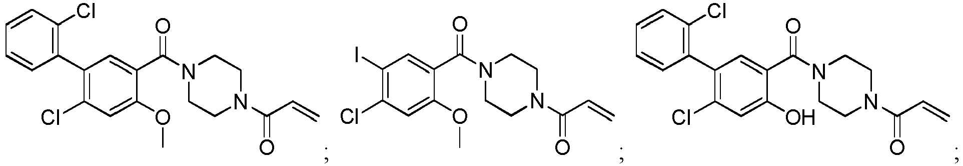

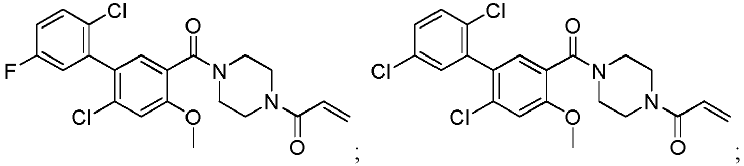

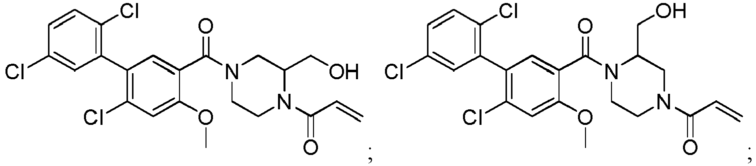

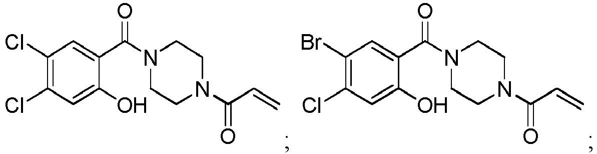

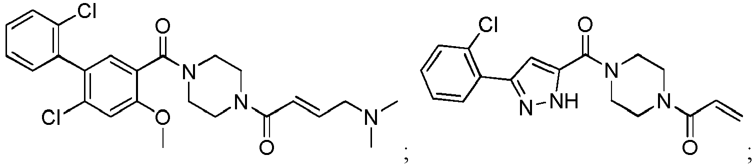

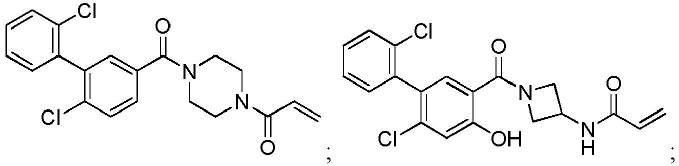

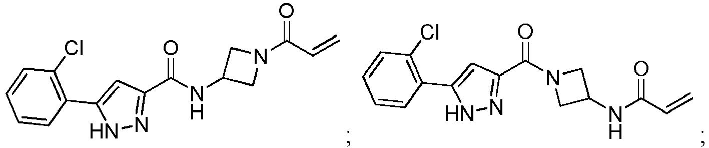

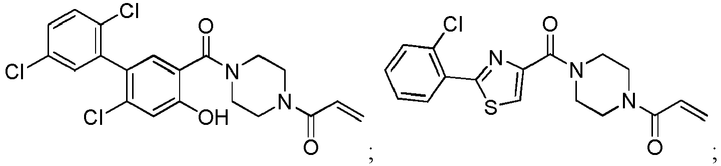

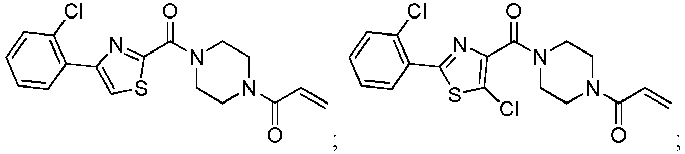

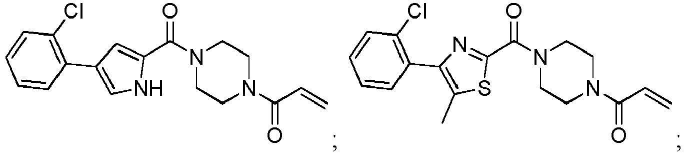

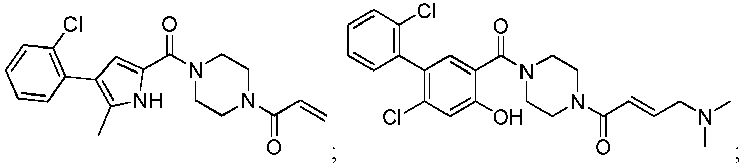

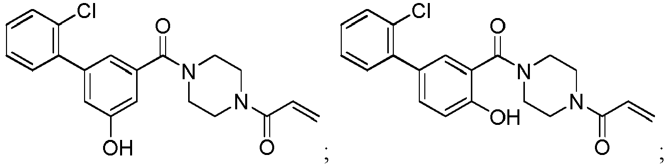

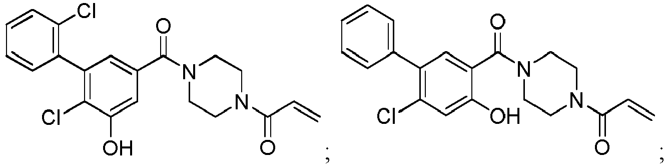

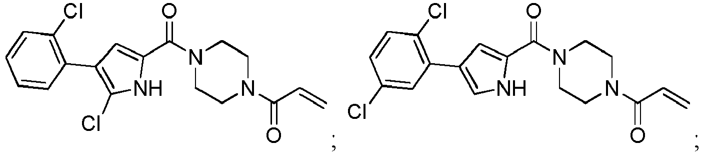

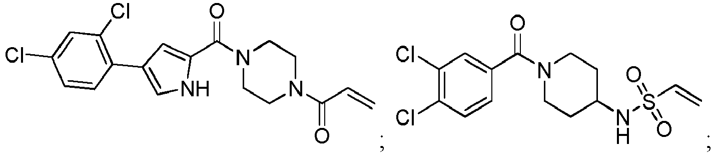

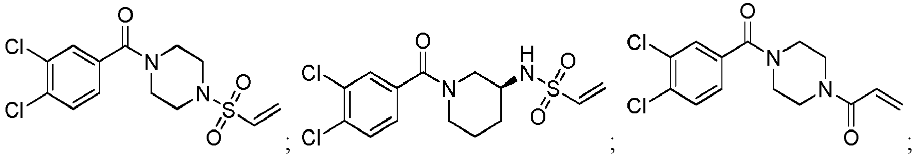

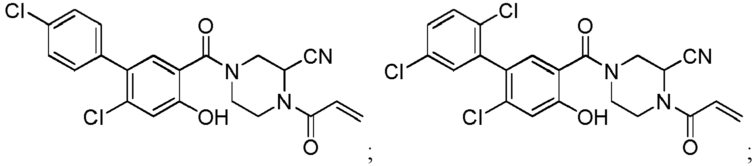

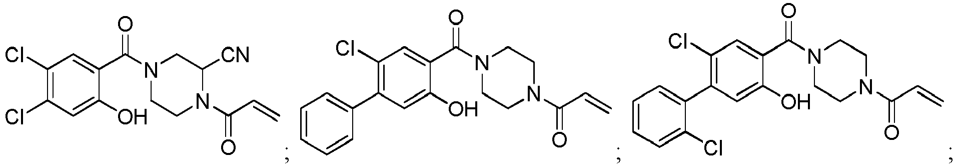

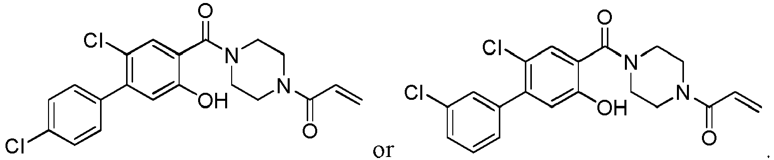

12. A pharmaceutical composition comprising a compound of any one of claims 1-11 and a pharmaceutically acceptable carrier.

13. The pharmaceutical composition of claim 12 for use in a method for treatment of cancer.

14. The pharmaceutical composition for use of claim 13, wherein the cancer is mediated by a K-Ras G12C, H-Ras G12C or N-Ras G12C mutation, preferably wherein the cancer is a hematological cancer, pancreatic cancer, MYH associated polyposis, colorectal cancer or lung cancer.

## Patentansprüche

1. Verbindung mit einer der folgenden Strukturen (Vla'), (Vlb'), (Vlc'), (Vld'), (Vle'), (Vlf') oder (Vlg'): 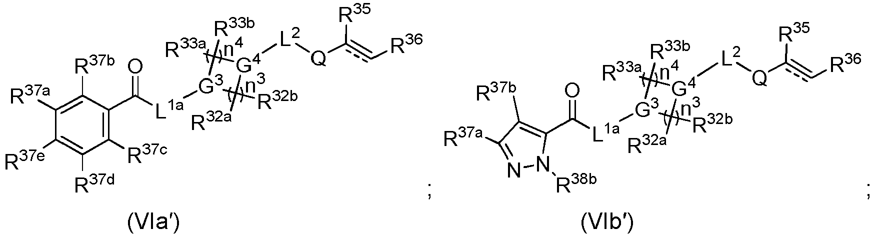 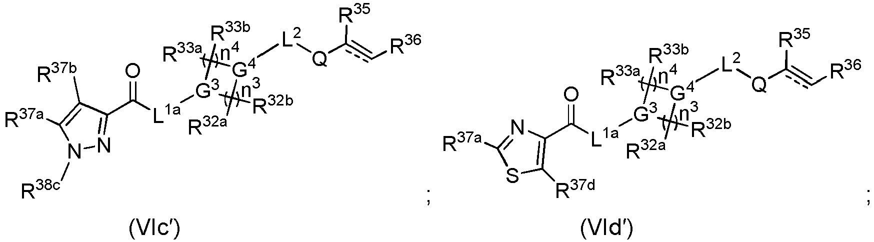 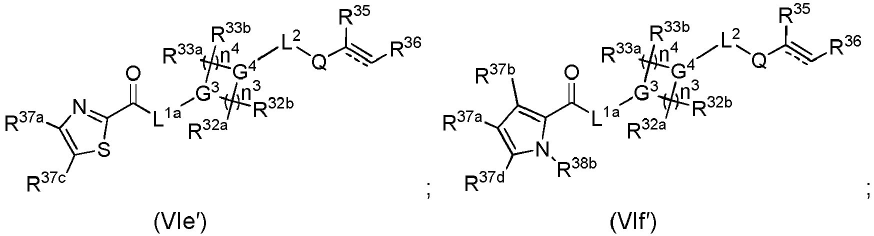 oder 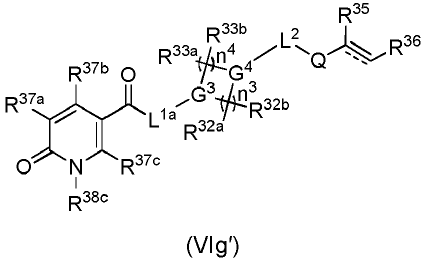 oder ein pharmazeutisch verträgliches Salz, Tautomer, Stereoisomer oder Prodrug davon, wobei das Prodrug ausgewählt ist aus der Gruppe bestehend aus Acetat-, Formiat- und Benzoatderivaten einer funktionellen Hydroxygruppe oder Acetamid-, Formamid- und Benzamidderivaten einer funktionellen Amingruppe in der Verbindung; wobei
i) G³ ist N und G⁴ ist CR; oder
ii) G³ ist CR und G⁴ ist N; oder
iii) G³ ist N und G⁴ ist N,
wobei R H, Cyano, Halogen oder C₁-C₆-Alkyl ist;
L^{1a} eine Bindung, -NH-, Alkylen oder Heteroalkylen ist;
L² eine Bindung oder Alkylen ist;
R^{32a} und R^{32b} sind bei jedem Auftreten unabhängig voneinander H, OH, NH₂, CO₂H, Cyano, Cyanoalkyl, C₁-C₆-Alkyl, C₃-C₈-Cycloalkyl, Hydroxylalkyl, Aminoalkyl, Carboxylalkyl oder Aminocarbonyl; oder R^{32a} und R^{32b} verbinden sich, um einen carbocyclischen oder heterocyclischen Ring zu bilden; oder R^{32a} ist H, OH, NH₂, CO₂H, Cyano, C₁-C₆-Alkyl, C₃-C₈-Cycloalkyl, Hydroxylalkyl, Aminoalkyl, Carboxylalkyl oder Aminocarbonyl und R^{32b} verbindet sich mit R^{33b}, um einen carbocyclischen oder heterocyclischen Ring zu bilden;
R^{33a} und R^{33b} sind bei jedem Auftreten unabhängig voneinander H, OH, NH₂, CO₂H, Cyano, Cyanoalkyl, C₁-C₆-Alkyl, C₃-C₈-Cycloalkyl, Hydroxylalkyl, Aminoalkyl, Carboxylalkyl oder Aminocarbonyl; oder R^{33a} und R^{33b} verbinden sich, um einen carbocyclischen oder heterocyclischen Ring zu bilden; oder R^{33a} ist H, OH, NH₂, CO₂H, Cyano, C₁-C₆-Alkyl, C₃-C₈-Cycloalkyl, Hydroxylalkyl, Aminoalkyl, Carboxylalkyl oder Aminocarbonyl und R^{33b} verbindet sich mit R^{32b}, um einen carbocyclischen oder heterocyclischen Ring zu bilden;
R^{37a}, R^{37b} R^{37c}, R^{37d} und R^{37e} sind jeweils unabhängig voneinander H, Halogen, Oxo, Hydroxyl, Cyano, Aminocarbonyl, Formyl, C₁-C₆-Alkyl, C₁-C₆-Alkylsulfonyl, C₁-C₆-Halogenalkyl, C₃-C₈-Cycloalkyl, C₁-C₆-Alkoxy, C₁-C₆-Hydroxylalkyl, C₁-C₆-Alkoxyalkyl, C₁-C₆-Aminoalkyl, Heterocyclyl oder Aryl, wobei: i) R^{37a} oder R^{37e} Aryl ist, oder ii) R^{37a} oder R^{37e}, oder beide, Chlor, Brom oder lod sind;
R^{38a}, R^{38b} und R^{38c} sind jeweils unabhängig voneinander H, C₁-C₆-Alkyl oder Aryl;
n³ und n⁴ sind jeweils unabhängig voneinander 1, 2 oder 3;
Q ist -C(=O)-, -NR³⁴C(=O)-, -S(=O)₂- oder-NR³⁴S(=O)₂-;
R³⁴ ist H, C₁-C₆-Alkyl oder Hydroxylalkyl;
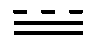 ist eine Kohlenstoff-Kohlenstoff-Doppelbindung oder eine Kohlenstoff-Kohlenstoff-Dreifachbindung; und
R³⁵ und R³⁶ sind jeweils unabhängig voneinander H, Cyano, C₁-C₆-Alkyl, Aminoalkyl, Alkylaminoalkyl oder Hydroxylalkyl, wobei mindestens eines von R³⁵ oder R³⁶ H ist,
wobei jedes Auftreten von Alkyl, Alkylen, Heteroalkylen, Cyanoalkyl, Cycloalkyl, Hydroxylalkyl, Aminoalkyl, Carboxylalkyl, Aminocarbonyl, Alkylsulfonyl, carbocyclischem Ring, heterocyclischem Ring, Halogenalkyl, Alkoxy, Alkoxyalkyl, Heterocyclyl, Aryl oder Alkylaminoalkyl gegebenenfalls substituiert ist, sofern nicht anders angegeben.

2. Verbindung nach Anspruch 1 oder ein pharmazeutisch verträgliches Salz, Tautomer, Stereoisomer oder Prodrug davon, wobei das Prodrug ausgewählt ist aus der Gruppe bestehend aus Acetat-, Formiat- und Benzoatderivaten einer funktionellen Hydroxygruppe oder Acetamid-, Formamid- und Benzamidderivaten einer funktionellen Amingruppe in der Verbindung, wobei:
i) n³ ist 2 und n⁴ ist 2; oder
ii) n³ ist 1 und n⁴ ist 1; oder
ii) n³ ist 2 und n⁴ ist 1.

3. Verbindung nach Anspruch 1 oder 2 oder ein pharmazeutisch verträgliches Salz, Tautomer, Stereoisomer oder Prodrug davon, wobei das Prodrug ausgewählt ist aus der Gruppe bestehend aus Acetat-, Formiat- und Benzoatderivaten einer funktionellen Hydroxygruppe oder Acetamid-, Formamid- und Benzamidderivaten einer funktionellen Amingruppe in der Verbindung, wobei jedes Auftreten von Alkyl, Alkylen, Heteroalkylen, Cyanoalkyl, Cycloalkyl, Hydroxylalkyl, Aminoalkyl, Carboxylalkyl, Aminocarbonyl, Alkylsulfonyl, carbocyclischem Ring, heterocyclischem Ring, Halogenalkyl, Alkoxy, Alkoxyalkyl, Heterocyclyl, Aryl oder Alkylaminoalkyl gegebenenfalls substituiert ist, sofern nicht anders angegeben, wobei R^{37a}, R^{37b}, R^{37c}, R^{37d} und R^{37e} jeweils unabhängig voneinander H, OH, Halogen, Oxo, C₁-C₆-Alkyl, C₁-C₆-Alkoxy, Heterocyclyl oder Aryl sind, und wobei: i) R^{37a}, oder R^{37e} Aryl ist, oder ii) R^{37a} oder R^{37e}, oder beide, Chlor, Brom oder lod sind.

4. Verbindung nach einem der Ansprüche 1-3 oder ein pharmazeutisch verträgliches Salz, Tautomer, Stereoisomer oder Prodrug davon, wobei das Prodrug ausgewählt ist aus der Gruppe bestehend aus Acetat-, Formiat- und Benzoatderivaten einer funktionellen Hydroxygruppe oder Acetamid-, Formamid- und Benzamidderivaten einer funktionellen Amingruppe in der Verbindung, wobei jedes Auftreten von Alkyl, Alkylen, Heteroalkylen, Cyanoalkyl, Cycloalkyl, Hydroxylalkyl, Aminoalkyl, Carboxylalkyl, Aminocarbonyl, Alkylsulfonyl, carbocyclischem Ring, heterocyclischem Ring, Halogenalkyl, Alkoxy, Alkoxyalkyl, Heterocyclyl, Aryl oder Alkylaminoalkyl gegebenenfalls substituiert ist, sofern nicht anders angegeben, wobei R^{37a} oder R^{37e} Aryl, vorzugsweise Phenyl ist, bevorzugter R^{37a} oder R^{37e} Aryl oder Phenyl ist, das mit einem oder mehreren Halogensubstituenten substituiert ist, besonders bevorzugt R^{37a} oder R^{37e} Aryl oder Phenyl ist, das mit Halogensubstituenten, ausgewählt aus Fluor und Chlor, substituiert ist.

5. Verbindung nach einem der Ansprüche 1-3 oder ein pharmazeutisch verträgliches Salz, Tautomer, Stereoisomer oder Prodrug davon, wobei das Prodrug ausgewählt ist aus der Gruppe bestehend aus Acetat-, Formiat- und Benzoatderivaten einer funktionellen Hydroxygruppe oder Acetamid-, Formamid- und Benzamidderivaten einer funktionellen Amingruppe in der Verbindung, wobei R^{37a} oder R^{37e}, oder beide, Chlor, Brom oder lod sind.

6. Verbindung nach einem der Ansprüche 1-4 oder ein pharmazeutisch verträgliches Salz, Tautomer, Stereoisomer oder Prodrug davon, wobei das Prodrug ausgewählt ist aus der Gruppe bestehend aus Acetat-, Formiat- und Benzoatderivaten einer funktionellen Hydroxygruppe oder Acetamid-, Formamid- und Benzamidderivaten einer funktionellen Amingruppe in der Verbindung, wobei R^{37a} oder R^{37e} eine der folgenden Strukturen aufweist:

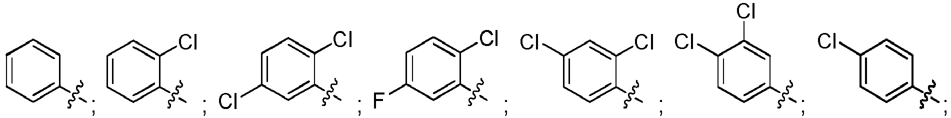

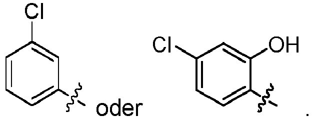

7. Verbindung nach einem der Ansprüche 1-6 oder ein pharmazeutisch verträgliches Salz, Tautomer, Stereoisomer oder Prodrug davon, wobei das Prodrug ausgewählt ist aus der Gruppe bestehend aus Acetat-, Formiat- und Benzoatderivaten einer funktionellen Hydroxygruppe oder Acetamid-, Formamid- und Benzamidderivaten einer funktionellen Amingruppe in der Verbindung, wobei Q -C(=O)- ist.

8. Verbindung nach einem der Ansprüche 1-7 oder ein pharmazeutisch verträgliches Salz, Tautomer, Stereoisomer oder Prodrug davon, wobei das Prodrug ausgewählt ist aus der Gruppe bestehend aus Acetat-, Formiat- und Benzoatderivaten einer funktionellen Hydroxygruppe oder Acetamid-, Formamid- und Benzamidderivaten einer funktionellen Amingruppe in der Verbindung, wobei mindestens eines von R³⁵ oder R³⁶ H ist, bevorzugt wobei R³⁵ und R³⁶ jeweils H sind.

9. Verbindung nach einem der Ansprüche 1-8 oder ein pharmazeutisch verträgliches Salz, Tautomer, Stereoisomer oder Prodrug davon, wobei das Prodrug ausgewählt ist aus der Gruppe bestehend aus Acetat-, Formiat- und Benzoatderivaten einer funktionellen Hydroxygruppe oder Acetamid-, Formamid- und Benzamidderivaten einer funktionellen Amingruppe in der Verbindung, wobei jedes Auftreten von Alkyl, Alkylen, Heteroalkylen, Cyanoalkyl, Cycloalkyl, Hydroxylalkyl, Aminoalkyl, Carboxylalkyl, Aminocarbonyl, Alkylsulfonyl, carbocyclischem Ring, heterocyclischem Ring, Halogenalkyl, Alkoxy, Alkoxyalkyl, Heterocyclyl, Aryl oder Alkylaminoalkyl gegebenenfalls substituiert ist, sofern nicht anders angegeben, wobei:
i) L^{1a} eine Bindung ist; oder
ii) L^{1a} -NH-, Alkylen oder Heteroalkylen ist; oder
iii) L^{1a} Alkylen ist, bevorzugt wobei L^{1a} 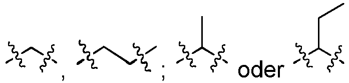 ist; oder
iv) L^{1a} Heteroalkylen ist, bevorzugt wobei L^{1a} Aminoalkylen oder Thioalkylen ist, besonders bevorzugt wobei L^{1a} eine der folgenden Strukturen aufweist:

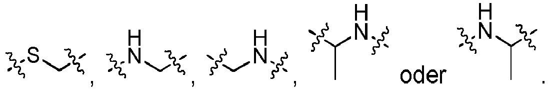

10. Verbindung nach einem der Ansprüche 1-9 oder ein pharmazeutisch verträgliches Salz, Tautomer, Stereoisomer oder Prodrug davon, wobei das Prodrug ausgewählt ist aus der Gruppe bestehend aus Acetat-, Formiat- und Benzoatderivaten einer funktionellen Hydroxygruppe oder Acetamid-, Formamid- und Benzamidderivaten einer funktionellen Amingruppe in der Verbindung, wobei:
i) mindestens eines von R^{32a}, R^{32b}, R^{33a} oder R^{33b} H ist, bevorzugt wobei jedes von R^{32a}, R^{32b}, R^{33a} oder R^{33b} H ist; oder
ii) mindestens eines von R^{32a}, R^{32b}, R^{33a} oder R^{33b} Hydroxylalkyl ist; oder
iii) mindestens eines von R^{32a}, R^{32b}, R^{33a} oder R^{33b} Cyano ist; oder
iv) mindestens eines von R^{32a}, R^{32b}, R^{33a} oder R^{33b} Aminocarbonyl ist; oder
v) mindestens eines von R^{32a}, R^{32b}, R^{33a} oder R^{33b} C₁-C₆-Alkyl ist.

11. Verbindung nach Anspruch 1 oder ein pharmazeutisch verträgliches Salz, Tautomer, Stereoisomer oder Prodrug davon, wobei das Prodrug ausgewählt ist aus der Gruppe bestehend aus Acetat-, Formiat- und Benzoatderivaten einer funktionellen Hydroxygruppe oder Acetamid-, Formamid- und Benzamidderivaten einer funktionellen Amingruppe in der Verbindung, wobei die Verbindung eine der folgenden Strukturen aufweist:

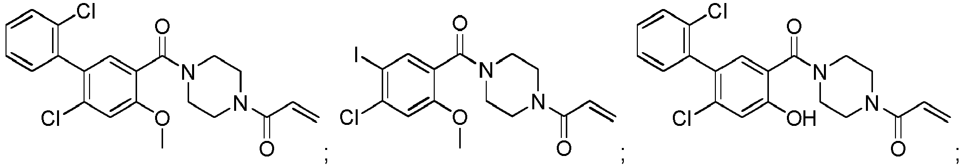

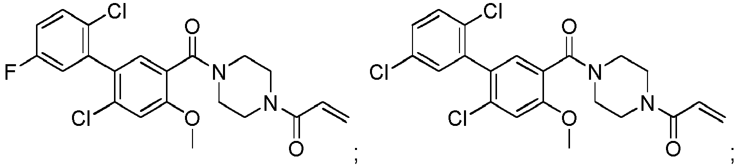

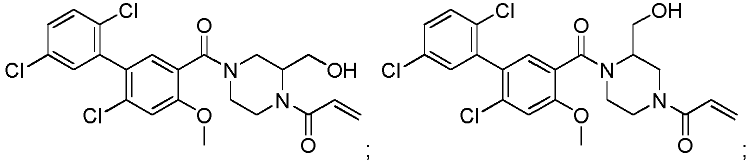

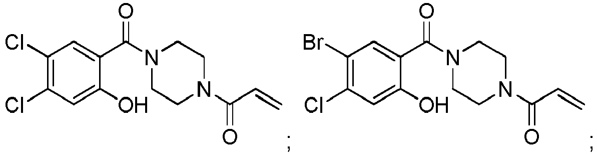

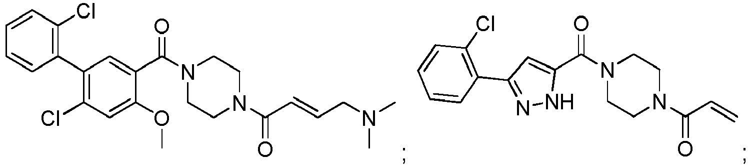

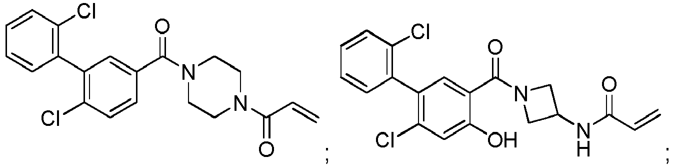

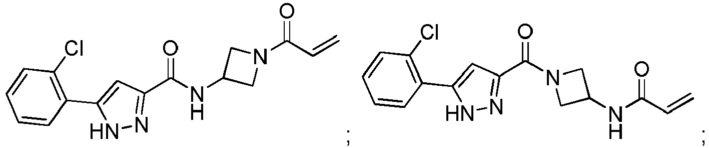

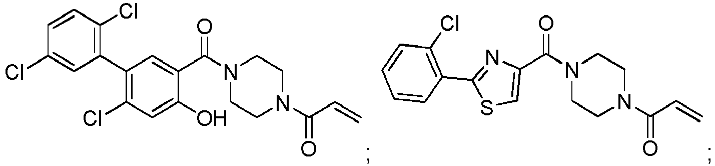

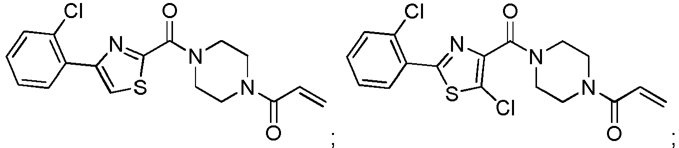

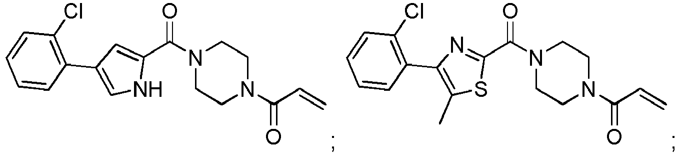

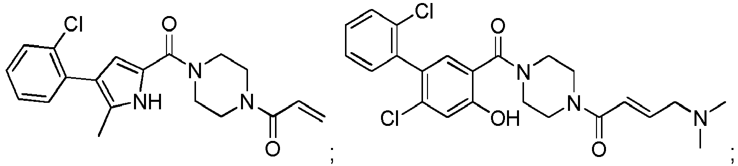

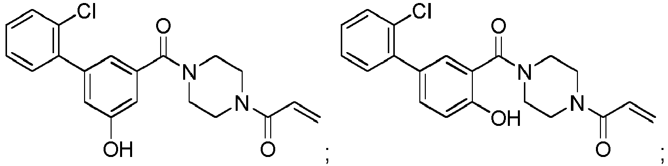

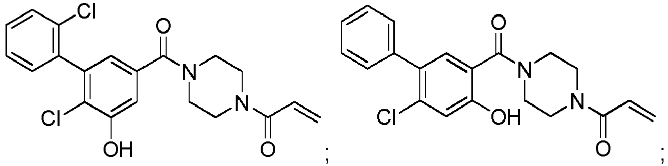

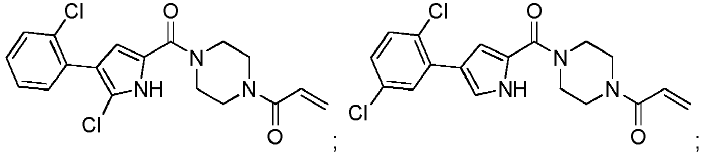

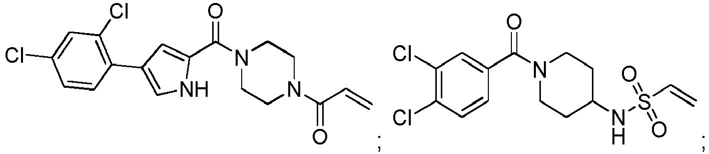

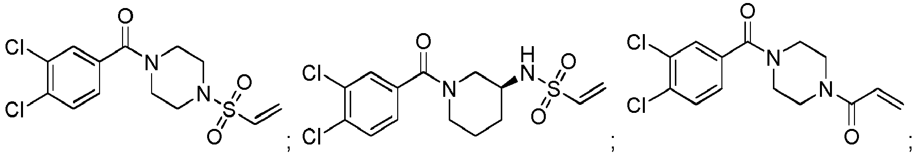

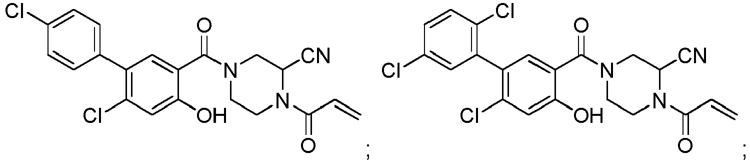

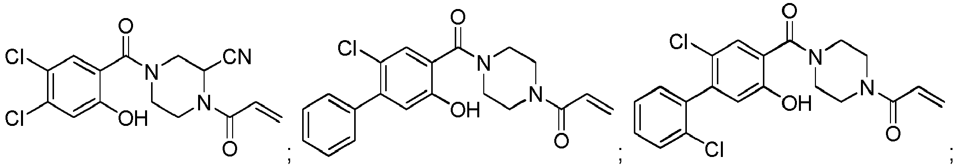

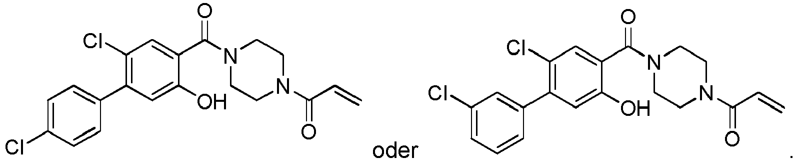

12. Pharmazeutische Zusammensetzung, umfassend eine Verbindung nach einem der Ansprüche 1-11 und einen pharmazeutisch verträglichen Träger.

13. Pharmazeutische Zusammensetzung nach Anspruch 12 zur Verwendung in einem Verfahren zur Behandlung von Krebs.

14. Pharmazeutische Zusammensetzung zur Verwendung nach Anspruch 13, wobei der Krebs durch eine K-Ras G12C-, H-Ras G12C- oder N-Ras G12C-Mutation vermittelt wird, bevorzugt wobei der Krebs ein hämatologischer Krebs, Bauchspeicheldrüsenkrebs, MYHassoziierte Polyposis, Kolorektalkrebs oder Lungenkrebs ist.

## Revendications

1. Composé ayant l'une des structures (Vla'), (Vlb'), (Vlc'), (Vld'), (Vle'), (Vlf') ou (Vlg') suivantes : 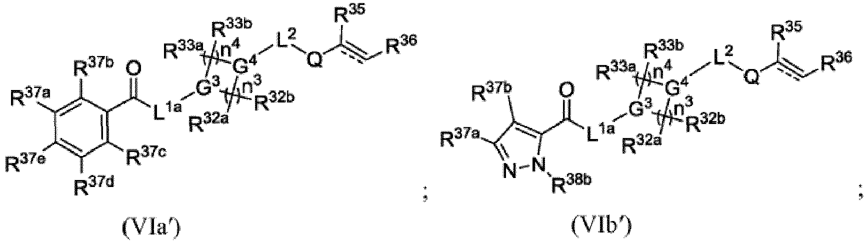 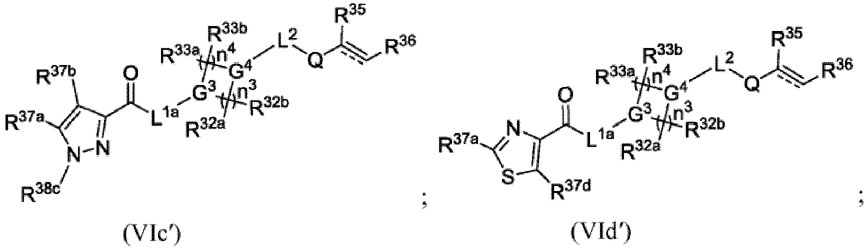 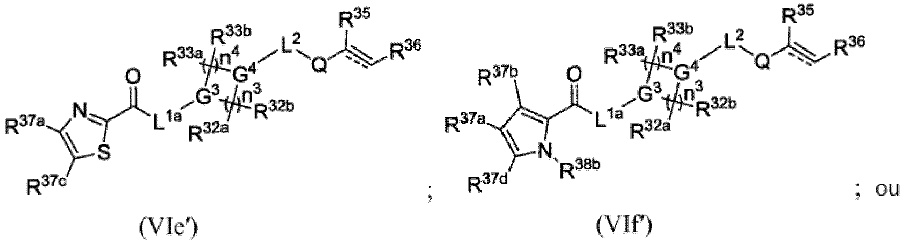 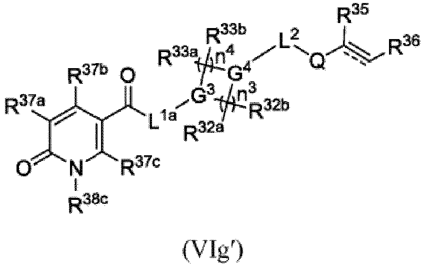 ou sel, tautomère, stéréoisomère ou promédicament pharmaceutiquement acceptable de celui-ci, le promédicament étant choisi dans le groupe constitué par des dérivés acétate, formiate et benzoate d'un groupe fonctionnel hydroxy, ou des dérivés acétamide, formamide et benzamide d'un groupe fonctionnel amine dans le composé ; où :
i) G³ est N et G⁴ est CR ; ou
ii) G³ est CR et G⁴ est N ; ou
iii) G³ est N et G⁴ est N,
où R est H, un cyano, un halogéno ou un alkyle en C₁ à C₆ ;
L^{1a} est une liaison, -NH-, un alkylène ou un hétéroalkylène ;
L₂ est une liaison ou un alkylène ;
R^{32a} et R^{32b} sont indépendamment, pour chaque occurrence, H, -OH,-NH₂, -CO₂H, cyano, cyanoalkyle, alkyle en C₁ à C₆, cycloalkyle en C₃ à C₈, hydroxyalkyle, aminoalkyle, carboxyalkyle ou aminocarbonyle ; ou R^{32a} et R^{32b} se lient pour former un noyau carbocyclique ou hétérocyclique ; ou R^{32a} est H, -OH,-NH₂, -CO₂H, cyano, alkyle en C₁ à C₆, cycloalkyle en C₃ à C₈, hydroxyalkyle, aminoalkyle, carboxyalkyle ou aminocarbonyle et R^{32b} se lie à R^{33b} pour former un noyau carbocyclique ou hétérocyclique ;
R^{33a} et R^{33b} sont indépendamment, pour chaque occurrence, H, -OH,-NH₂, -CO₂H, cyano, cyanoalkyle, alkyle en C₁ à C₆, cycloalkyle en C₃ à C₈, hydroxyalkyle, aminoalkyle, carboxyalkyle ou aminocarbonyle ; ou R^{33a} et R^{33b} se lient pour former un noyau carbocyclique ou hétérocyclique ; ou R^{33a} est H, -OH,-NH₂, -CO₂H, cyano, alkyle en C₁ à C₆, cycloalkyle en C₃ à C₈, hydroxyalkyle, aminoalkyle, carboxyalkyle ou aminocarbonyle et R^{33b} se lie à R^{32b} pour former un noyau carbocyclique ou hétérocyclique ;
R^{37a} R^{37b} R^{37c}, R^{37d} et R^{37e} sont chacun indépendamment H, halogéno, oxo, hydroxyle, cyano, aminocarbonyle, formyle, alkyle en C₁ à C₆, alkylsulfonyle en C₁ à C₆, haloalkyle en C₁ à C₆, cycloalkyle en C₃ à C₈, alcoxy en C₁ à C₆, hydroxyalkyle en C₁ à C₆, alcoxyalkyle en C₁ à C₆, aminoalkyle en C₁ à C₆, hétérocyclyle ou aryle , dans lequel : i) R^{37a} ou R^{37e} est un aryle, ou ii) R^{37a} ou R^{37e}, ou les deux, sont un chloro, bromo ou iodo ;
R^{38a}, R^{38b} et R^{38c} sont chacun indépendamment H, un alkyle en C₁ à C₆ ou un aryle ;
n³ et n⁴ valent chacun indépendamment 1, 2 ou 3 ;
Q est -C(=O)-, -NR³⁴C(=O)-, -S(=O)₂- ou -NR³⁴S(=O)₂- ;
R³⁴ est H, un alkyle en Ci à C₆ ou un hydroxyalkyle ;
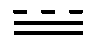 est une double liaison carbone-carbone ou une triple liaison carbone-carbone ; et
R³⁵ et R³⁶ sont chacun indépendamment H, un cyano, un alkyle en C₁ à C₆, un aminoalkyle, un alkylaminoalkyle ou un hydroxyalkyle, dans lequel au moin l'un de R³⁵ ou R³⁶ est H,
où chaque occurrence d'alkyle, d'alkylène, d'hétéroalkylène, de cyanoalkyle, de cycloalkyle, d'hydroxylalkyle, d'aminoalkyle, de carboxylalkyle, d'aminocarbonyle, d'alkylsulfonyle, d'anneau carbocyclique, d'anneau hétérocyclique, d'haloalkyle, d'alcoxy, d'alcoxyalkyle, d'hétérocyclyle, d'aryle ou d'alkylaminoalkyle est éventuellement substituée, sauf indication contraire.

2. Composé selon la revendication 1 ou sel, tautomère, stéréoisomère ou promédicament pharmaceutiquement acceptable de celui-ci, le promédicament étant choisi dans le groupe constitué par des dérivés acétate, formiate et benzoate d'un groupe fonctionnel hydroxy, ou des dérivés acétamide, formamide et benzamide d'un groupe fonctionnel amine dans le composé, où :
i) n³ vaut 2 et n⁴ vaut 2 ; ou
ii) n³ vaut 1 et n⁴ vaut 1 ; ou
iii) n³ vaut 2 et n⁴ vaut 1.

3. Composé selon la revendication 1 ou 2, ou sel, tautomère, stéréoisomère ou promédicament pharmaceutiquement acceptable de celui-ci, le promédicament étant choisi dans le groupe constitué par des dérivés acétate, formiate et benzoate d'un groupe fonctionnel hydroxy, ou des dérivés acétamide, formamide et benzamide d'un groupe fonctionnel amine dans le composé, où chaque occurrence d'alkyle, d'alkylène, d'hétéroalkylène, de cyanoalkyle, de cycloalkyle, d'hydroxylalkyle, d'aminoalkyle, de carboxylalkyle, d'aminocarbonyle, d'alkylsulfonyle, d'anneau carbocyclique, d'anneau hétérocyclique, d'haloalkyle, d'alcoxy, d'alcoxyalkyle, d'hétérocyclyle, d'aryle ou d'alkylaminoalkyle est éventuellement substituée, sauf indication contraire, où R^{37a}, R^{37b}, R^{37c}, R^{37d} et R^{37e} sont chacun indépendamment H, -OH, halogéno, oxo, alkyle en C₁ à C₆, alcoxy en C₁ à C₆, hétérocyclyle ou aryle, et dans lequel : i) R^{37a} ou R^{37e} et un aryle, ou ii) R^{37a} ou R^{37e}, ou les deux, sont un chloro, bromo ou iodo.

4. Composé selon l'une quelconque des revendications 1 à 3, ou sel, tautomère, stéréoisomère ou promédicament pharmaceutiquement acceptable de celui-ci, le promédicament étant choisi dans le groupe constitué par des dérivés acétate, formiate et benzoate d'un groupe fonctionnel hydroxy, ou des dérivés acétamide, formamide et benzamide d'un groupe fonctionnel amine dans le composé, où chaque occurrence d'alkyle, d'alkylène, d'hétéroalkylène, de cyanoalkyle, de cycloalkyle, d'hydroxylalkyle, d'aminoalkyle, de carboxylalkyle, d'aminocarbonyle, d'alkylsulfonyle, d'anneau carbocyclique, d'anneau hétérocyclique, d'haloalkyle, d'alcoxy, d'alcoxyalkyle, d'hétérocyclyle, d'aryle ou d'alkylaminoalkyle est éventuellement substituée, sauf indication contraire, où R^{37a} ou R^{37e} est un aryle, de préférence un phényle, R^{37a} ou R^{37e} étant plus préférablement un aryle ou un phényle substitué par un ou plusieurs substituants halogéno, R^{37a} ou R^{37e} étant encore plus préférablement un aryle ou un phényle substitué par des substituants halogéno choisis parmi fluoro et chloro.

5. Composé selon l'une quelconque des revendications 1 à 3, ou sel, tautomère, stéréoisomère ou promédicament pharmaceutiquement acceptable de celui-ci, le promédicament étant choisi dans le groupe constitué par des dérivés acétate, formiate et benzoate d'un groupe fonctionnel hydroxy, ou des dérivés acétamide, formamide et benzamide d'un groupe fonctionnel amine dans le composé, où R^{37a} ou R^{37e}, ou les deux, sont un chloro, bromo ou iodo.

6. Composé selon l'une quelconque des revendications 1 à 4, ou sel, tautomère, stéréoisomère ou promédicament pharmaceutiquement acceptable de celui-ci, le promédicament étant choisi dans le groupe constitué par des dérivés acétate, formiate et benzoate d'un groupe fonctionnel hydroxy, ou des dérivés acétamide, formamide et benzamide d'un groupe fonctionnel amine dans le composé, où R^{37a} ou R^{37e} a l'une des structures suivantes :

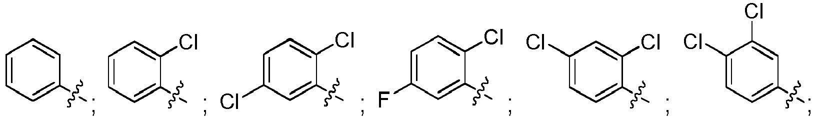

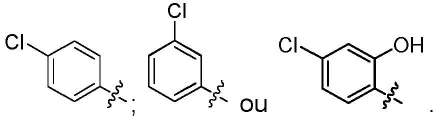

7. Composé selon l'une quelconque des revendications 1 à 6, ou sel, tautomère, stéréoisomère ou promédicament pharmaceutiquement acceptable de celui-ci, le promédicament étant choisi dans le groupe constitué par des dérivés acétate, formiate et benzoate d'un groupe fonctionnel hydroxy, ou des dérivés acétamide, formamide et benzamide d'un groupe fonctionnel amine dans le composé, où Q est -C(=O)-.

8. Composé selon l'une quelconque des revendications 1 à 7, ou sel, tautomère, stéréoisomère ou promédicament pharmaceutiquement acceptable de celui-ci, le promédicament étant choisi dans le groupe constitué par des dérivés acétate, formiate et benzoate d'un groupe fonctionnel hydroxy, ou des dérivés acétamide, formamide et benzamide d'un groupe fonctionnel amine dans le composé, où l'un au moins de R³⁵ ou R³⁶ est H, chacun de R³⁵ et R³⁶ étant de préférence H.

9. Composé selon l'une quelconque des revendications 1 à 8, ou sel, tautomère, stéréoisomère ou promédicament pharmaceutiquement acceptable de celui-ci, le promédicament étant choisi dans le groupe constitué par des dérivés acétate, formiate et benzoate d'un groupe fonctionnel hydroxy, ou des dérivés acétamide, formamide et benzamide d'un groupe fonctionnel amine dans le composé, où chaque occurrence d'alkyle, d'alkylène, d'hétéroalkylène, de cyanoalkyle, de cycloalkyle, d'hydroxylalkyle, d'aminoalkyle, de carboxylalkyle, d'aminocarbonyle, d'alkylsulfonyle, d'anneau carbocyclique, d'anneau hétérocyclique, d'haloalkyle, d'alcoxy, d'alcoxyalkyle, d'hétérocyclyle, d'aryle ou d'alkylaminoalkyle est éventuellement substituée, sauf indication contraire, où :
i) L^{1a} est une liaison ; ou
ii) L^{1a} est -NH-, un alkylène ou un hétéroalkylène ; ou
iii) L^{1a} est un alkylène, L^{1a} étant de préférence 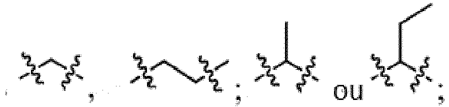 ou
iv) L^{1a} est un hétéroalkylène, L^{1a} étant de préférence un aminoalkylène ou un thioalkylène, L^{1a} ayant l'une des structures suivantes :

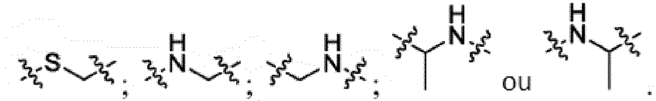

10. Composé selon l'une quelconque des revendications 1 à 9, ou sel, tautomère, stéréoisomère ou promédicament pharmaceutiquement acceptable de celui-ci, le promédicament étant choisi dans le groupe constitué par des dérivés acétate, formiate et benzoate d'un groupe fonctionnel hydroxy, ou des dérivés acétamide, formamide et benzamide d'un groupe fonctionnel amine dans le composé, où chaque occurrence d'alkyle, d'alkylène, d'hétéroalkylène, de cyanoalkyle, de cycloalkyle, d'hydroxylalkyle, d'aminoalkyle, de carboxylalkyle, d'aminocarbonyle, d'alkylsulfonyle, d'anneau carbocyclique, d'anneau hétérocyclique, d'haloalkyle, d'alcoxy, d'alcoxyalkyle, d'hétérocyclyle, d'aryle ou d'alkylaminoalkyle est éventuellement substituée, sauf indication contraire, où :
i) l'un au moins parmi R^{32a}, R^{32b}, R^{33a} ou R^{33b} est H, chacun parmi R^{32a}, R^{32b}, R^{33a} ou R^{33b} étant de préférence H ; ou
ii) l'un au moins parmi R^{32a}, R^{32b}, R^{33a} ou R^{33b} est un hydroxyalkyle ; ou
iii) l'un au moins parmi R^{32a}, R^{32b}, R^{33a} ou R^{33b} est un cyano ; ou
iv) l'un au moins parmi R^{32a}, R^{32b}, R^{33a} ou R^{33b} est un aminocarbonyle ; ou
v) l'un au moins parmi R^{32a}, R^{32b}, R^{33a} ou R^{33b} est un alkyle en C₁ à C₆.

11. Composé selon la revendication 1, ou sel, tautomère, stéréoisomère ou promédicament pharmaceutiquement acceptable de celui-ci, le promédicament étant choisi dans le groupe constitué par des dérivés acétate, formiate et benzoate d'un groupe fonctionnel hydroxy, ou des dérivés acétamide, formamide et benzamide d'un groupe fonctionnel amine dans le composé, le composé ayant l'une des structures suivantes : 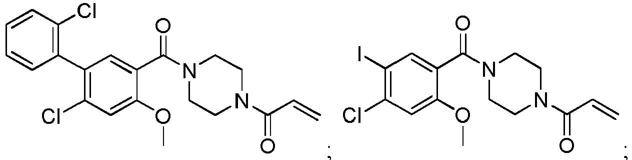 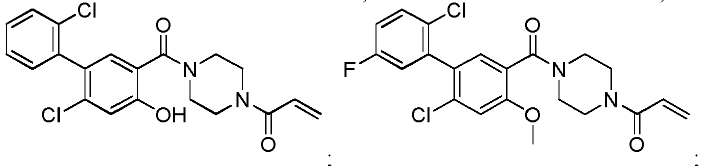 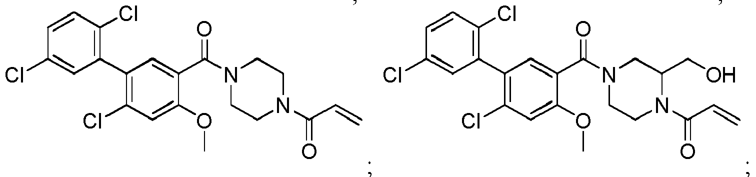 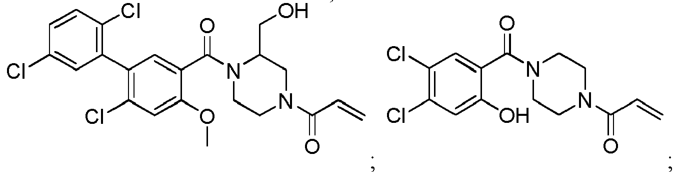 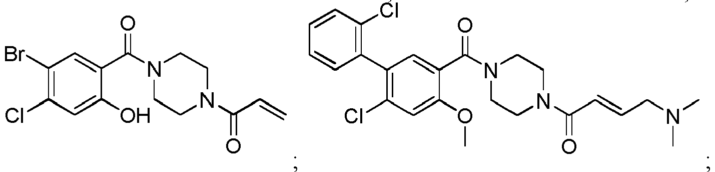 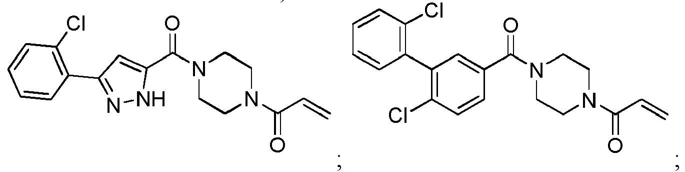 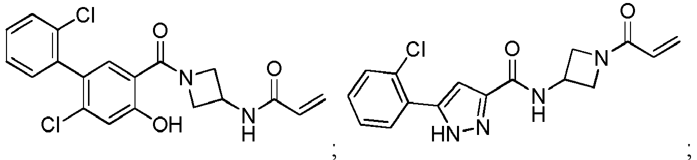 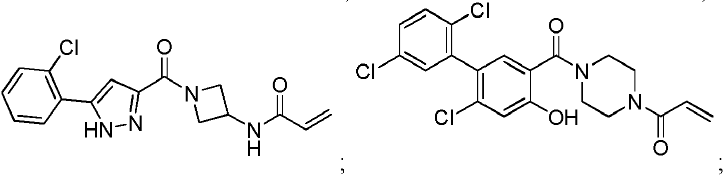 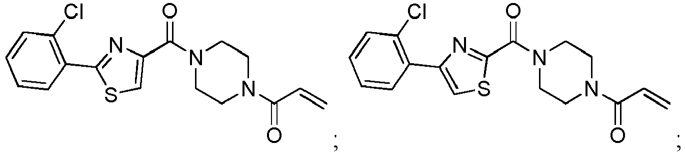 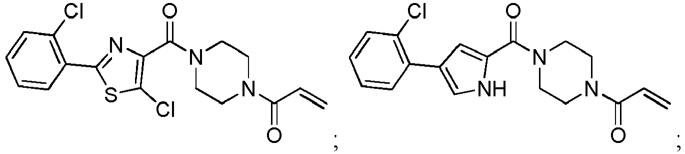 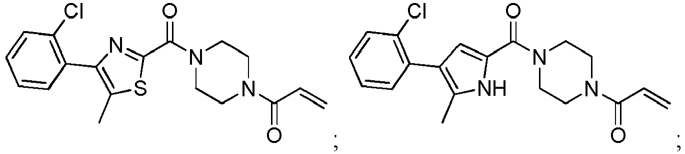 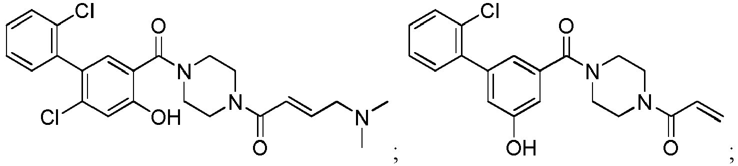 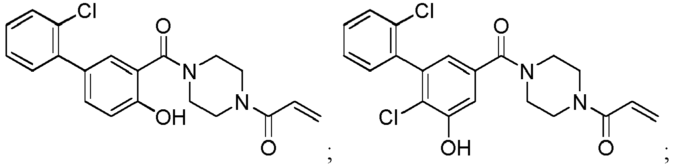 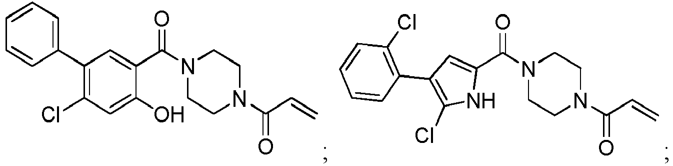 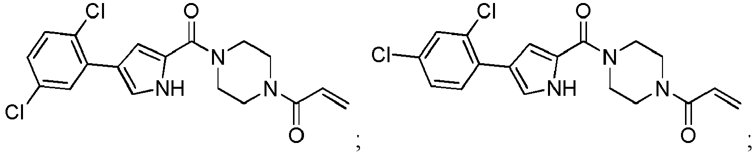 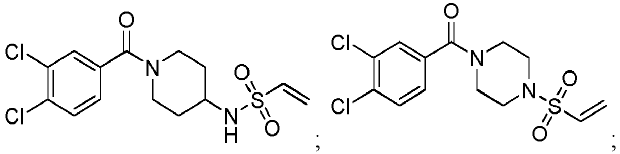 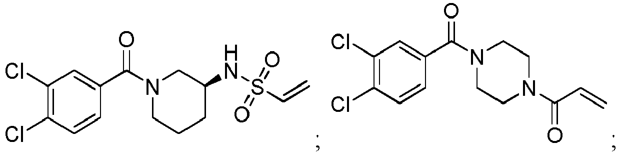 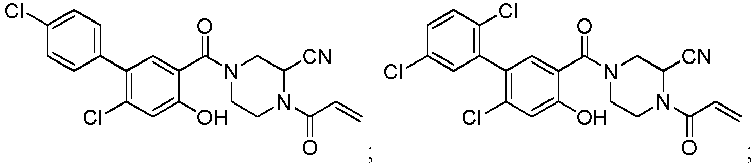 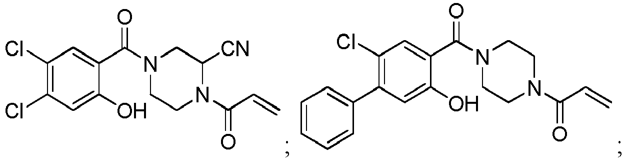 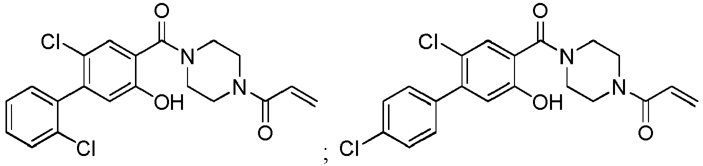 ou

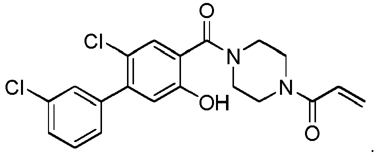

12. Composition pharmaceutique comprenant un composé selon l'une quelconque des revendications 1 à 11 et un véhicule pharmaceutiquement acceptable.

13. Composition pharmaceutique selon la revendication 12 destinée à être utilisée dans un procédé pour le traitement d'un cancer.

14. Composition pharmaceutique destinée à être utilisée selon la revendication 13, le cancer étant médié par une mutation K-Ras G12C, H-Ras G12C ou N-Ras G12C, le cancer étant de préférence un cancer hématologique, un cancer pancréatique, une polypose associée à MYH, un cancer colorectal ou un cancer du poumon.
